# EUROPEAN PATENT APPLICATION

(11) **EP 4 119 570 A1**
(43) Date of publication of application: **18.01.2023**
(21) Application number: 21768058.6
(22) Date of filing: 11.03.2021
(51) Int. Cl.: C07K 5/10, A61K 38/07, A61K 38/08, A61P 3/10, A61P 3/04, A61K 8/64, A61Q 19/06, A23L 33/18

(54) **COMPOSITION FOR ANTI-DIABETES AND ANTI-OBESITY COMPRISING NOVEL COMPOUND**

(30) Priority: 11.03.2020 KR 20200030303
(71) Applicant: Anygen Co., Ltd., Gwangju 61008 (KR)
(72) Inventor: KIM, Jae Il, Gwangju 61008 (KR); RYU, Jae Ha, Gwangju 61008 (KR); PARK, Bong Gyu, Gwangju 61008 (KR)
(74) Representative: HGF
(86) International application number: PCT/KR2021/003045
(87) International publication number: WO 2021/182898

(57) **Abstract**

The present invention relates to a novel compound and a composition for an antidiabetes and anti-obesity comprising the same as an active ingredient. The compound or pharmaceutically acceptable salt thereof according to the present invention selectively and specifically acts as an agonist of GPR39, activates the mechanism of glucose-dependent insulin secretion in pancreatic beta cells, and significantly increases glucose tolerance and an anti-diabetic effect, and therefore can be utilized as a composition for improving, preventing, or treating diabetes mellitus. In addition, the compound or pharmaceutically acceptable salt thereof according to the present invention acts on GPR39 so as to have lipolytic ability in adipocytes and adipose tissue, and therefore can be utilized as a composition for improving, preventing, or treating obesity.

## Description

### Technical Field

The present invention relates to a novel compound and a composition for an antidiabetes and anti-obesity comprising the same as an active ingredient.

### Background Art

About 880 G protein-coupled receptors (GPCRs), which are the most abundant membrane proteins in all mammals, are currently known. Of these, about 60% are related to the senses of sight, smell, taste, and the like, and about 400 GPCRs, including the remaining orphan GPCRs, are regulated by various ligands (e.g., proteins, peptide hormones, amino acids, amines and lipids) *in vivo* and are involved in various life phenomena. In particular, GPCRs are associated with diseases such as metabolic diseases, cardiovascular diseases, degenerative diseases and carcinogenesis. Therefore, it is an important drug target for new drug development, and more than 50% of the drugs developed to date are directly or indirectly related to the activity of GPCR.

On the other hand, GPR39 (G protein-coupled receptor 39) is one of the orphan GPCRs, and an intrinsic ligand has not been discovered to date. GPR39 belongs to the Ghrelin receptor (GHSR, growth hormone secretagogue receptor) family, and is known to be distributed in the stomach, intestine, pancreas, liver, kidney, reproductive and adipose tissue (Cellular and Molecular Life Sciences, January 2011, Volume 68, Issue 1, pp 85-95). GPR39 was found to be associated with body weight gain and body fat accumulation through *in vitro* GPR39 silencing and metabolic function studies through high-fat or low-fat diet control in GPR39 knockout (KO) mice (Gastroenterology 2006, 131:1131-1141). In addition, through the study of GPR39 KO mice, it was confirmed that the blood glucose level of GPR39 KO mice was increased in high-fat diet control, whereas insulin secretion was impaired and glucose-dependent insulin secretion (glucose stimulated insulin secretion) was decreased (Endocrinology 2009, 150:2577-2585).

The above results suggest that GPR39 plays an important role in the physiological function of the body, and a modulator for GPR39 is expected to be a new drug candidate for the treatment of metabolic diseases such as diabetes mellitus and obesity.

### Prior Art Document

### Non-Patent Document

(Non-Patent Document 1) Cellular and Molecular Life Sciences, January 2011, Volume 68, Issue 1, pp 85-95
(Non-Patent Document 2) Gastroenterology 2006, 131:1131-1141
(Non-Patent Document 3) Endocrinology 2009, 150:2577-2585

### Detailed Description of Invention

### Technical Problem

The present inventors have completed the present invention by confirming that a peptide consisting of a specific amino acid sequence or a variant thereof effectively regulates the activity of GPR39 to exhibit anti-diabetic and anti-obesity effects.

Therefore, an object of the present invention is to provide a compound represented by Formula 1 below or a pharmaceutically acceptable salt thereof.

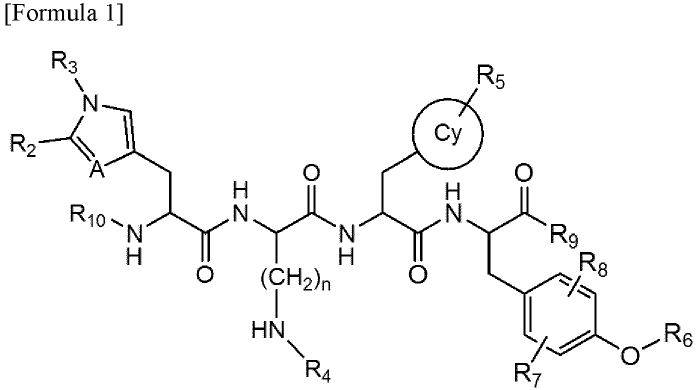

### Solution to Problem

In order to achieve the above object, in one aspect of the present invention, there is provided a compound represented by Formula 1 below or a pharmaceutically acceptable salt thereof: 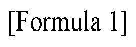 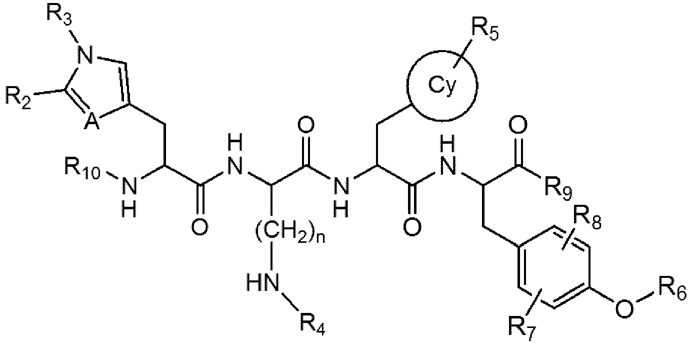 in Formula 1
A is -C(-Ro)-, -N= or -N(-R₁)-,
Cy is C₆₋₁₄ aryl or 5 to 6-membered heteroaryl,
R₁ and R₃ are each independently hydrogen, Ra, an amine protecting group, C₁₋₆ alkyl substituted with 1 to 3 Ra, C₃₋₁₀ cycloalkyl, or 5 to 6-membered heterocyclyl, wherein said heterocyclyl has or does not have 1 to 3 substituents selected from phenylethyl and cyclohexylethyl,
R₂ is hydrogen, Ra, or 5 to 6-membered heterocyclyl,
Ro is hydrogen, or R₀ and R₂ are linked to each other to form a benzene ring together with the two carbon atoms to which they are attached,
Ra is each independently C₃₋₁₀ cycloalkyl or C₆₋₁₄ aryl, wherein said aryl has or does not have one or more substituents selected from the group consisting of halogen, -OH, C₆₋₁₄ aryl substituted with 5 to 6-membered heteroaryl, C₁₋₆ alkyl and C₁₋₆ alkoxy,
n is 1 to 10,
R₄ is hydrogen, C₁₋₁₀ alkyl or C₁-C₂₀ alkylcarbonyl,
R₅ is hydrogen, halogen, CF₃ or C₁₋₆ alkyl,
R₆ is hydrogen, C₁₋₁₀ alkyl or -S(=O)₂OH,
R₇ and R₅ are each independently hydrogen, halogen, nitro, amine or C₁₋₆ alkyl,
R₉ is -OH or -NH₂, and
R₁₀ is hydrogen, an amine protecting group or biotin, wherein said heterocyclyl and heteroaryl each independently contain at least one heteroatom selected from the group consisting of N, S and O.

In another aspect of the present invention, there is provided a pharmaceutical composition for preventing or treating diabetes mellitus, comprising the compound or pharmaceutically acceptable salt thereof as an active ingredient.

In another aspect of the present invention, there is provided a method for preventing or treating diabetes mellitus, comprising administering the compound or pharmaceutically acceptable salt thereof to a mammal.

In another aspect of the present invention, there is provided the use of the compound or pharmaceutically acceptable salt thereof, for the manufacture of a medicament for preventing or treating diabetes mellitus.

In another aspect of the present invention, there is provided a health functional food composition for preventing or improving diabetes mellitus, comprising the compound or nutritionally acceptable salt thereof as an active ingredient.

In another aspect of the present invention, there is provided a pharmaceutical composition for preventing or treating obesity, comprising the compound or pharmaceutically acceptable salt thereof as an active ingredient.

In another aspect of the present invention, there is provided a method for preventing or treating obesity, comprising administering the compound or pharmaceutically acceptable salt thereof to a mammal.

In another aspect of the present invention, there is provided the use of the compound or pharmaceutically acceptable salt thereof, for the manufacture of a medicament for preventing or treating obesity.

In another aspect of the present invention, there is provided a health functional food composition for preventing or improving obesity, comprising the compound or nutritionally acceptable salt thereof as an active ingredient.

In another aspect of the present invention, there is provided a cosmetic composition for preventing or improving obesity, comprising the compound or pharmaceutically acceptable salt thereof as an active ingredient.

### Effects of Invention

The compound or pharmaceutically acceptable salt thereof according to the present invention selectively and specifically acts as an agonist of GPR39, activates the mechanism of glucose-dependent insulin secretion in pancreatic beta cells, and significantly increases glucose tolerance and an anti-diabetic effect, and therefore can be utilized as a composition for improving, preventing, or treating diabetes mellitus. In addition, the compound or pharmaceutically acceptable salt thereof according to the present invention acts on GPR39 so as to have lipolytic ability in adipocytes and adipose tissue, and therefore can be utilized as a composition for improving, preventing, or treating obesity.

### Brief Description of Drawings

FIG. 1 illustrates the results obtained by confirming the purity of HKFY-1 prepared according to one embodiment of the present invention through HPLC.
FIG. 2 illustrates the results obtained by confirming the purity of HKFY-2 prepared according to one embodiment of the present invention through HPLC.
FIG. 3 illustrates the results obtained by confirming the purity of HKFY-3 prepared according to one embodiment of the present invention through HPLC.
FIG. 4 illustrates the results obtained by confirming the purity of HKFY-4 prepared according to one embodiment of the present invention through HPLC.
FIG. 5 illustrates the results obtained by confirming the purity of HKFY-5 prepared according to one embodiment of the present invention through HPLC.
FIG. 6 illustrates the results obtained by confirming the purity of HKFY-6 prepared according to one embodiment of the present invention through HPLC.
FIG. 7 illustrates the results obtained by confirming the purity of HKFY-7 prepared according to one embodiment of the present invention through HPLC.
FIG. 8 illustrates the results obtained by confirming the purity of HKFY-8 prepared according to one embodiment of the present invention through HPLC.
FIG. 9 illustrates the results obtained by confirming the purity of HKFY-9 prepared according to one embodiment of the present invention through HPLC.
FIG. 10 illustrates the results obtained by confirming the purity of HKFY-10 prepared according to one embodiment of the present invention through HPLC.
FIG. 11 illustrates the results obtained by confirming the purity of HKFY-11 prepared according to one embodiment of the present invention through HPLC.
FIG. 12 illustrates the results obtained by confirming the purity of HKFY-12 prepared according to one embodiment of the present invention through HPLC.
FIG. 13 illustrates the results obtained by confirming the purity of HKFY-13 prepared according to one embodiment of the present invention through HPLC.
FIG. 14 illustrates the results obtained by confirming the purity of HKFY-14 prepared according to one embodiment of the present invention through HPLC.
FIG. 15 illustrates the results obtained by confirming the purity of HKFY-15 prepared according to one embodiment of the present invention through HPLC.
FIG. 16 illustrates the results obtained by confirming the purity of HKFY-16 prepared according to one embodiment of the present invention through HPLC.
FIG. 17 illustrates the results obtained by confirming the purity of HKFY-17 prepared according to one embodiment of the present invention through HPLC.
FIG. 18 illustrates the results obtained by confirming the purity of HKFY-18 prepared according to one embodiment of the present invention through HPLC.
FIG. 19 illustrates the results obtained by confirming the purity of HKFY-19 prepared according to one embodiment of the present invention through HPLC.
FIG. 20 illustrates the results obtained by confirming the purity of HKFY-20 prepared according to one embodiment of the present invention through HPLC.
FIG. 21 illustrates the results obtained by confirming the purity of HKFY-21 prepared according to one embodiment of the present invention through HPLC.
FIG. 22 illustrates the results obtained by confirming the purity of HKFY-22 prepared according to one embodiment of the present invention through HPLC.
FIG. 23 illustrates the results obtained by confirming the purity of HKFY-23 prepared according to one embodiment of the present invention through HPLC.
FIG. 24 illustrates the results obtained by confirming the purity of HKFY-24 prepared according to one embodiment of the present invention through HPLC.
FIG. 25 illustrates the results obtained by confirming the purity of HKFY-25 prepared according to one embodiment of the present invention through HPLC.
FIG. 26 illustrates the results obtained by confirming the purity of HKFY-26 prepared according to one embodiment of the present invention through HPLC.
FIG. 27 illustrates the results obtained by confirming the purity of HKFY-27 prepared according to one embodiment of the present invention through HPLC.
FIG. 28 illustrates the results obtained by confirming the purity of HKFY-28 prepared according to one embodiment of the present invention through HPLC.
FIG. 29 illustrates the results obtained by confirming the purity of HKFY-29 prepared according to one embodiment of the present invention through HPLC.
FIG. 30 illustrates the results obtained by confirming the purity of HKFY-30 prepared according to one embodiment of the present invention through HPLC.
FIG. 31 illustrates the results obtained by confirming the purity of HKFY-31 prepared according to one embodiment of the present invention through HPLC.
FIG. 32 illustrates the results obtained by confirming the purity of HKFY-32 prepared according to one embodiment of the present invention through HPLC.
FIG. 33 illustrates the results obtained by confirming the purity of HKFY-33 prepared according to one embodiment of the present invention through HPLC.
FIG. 34 illustrates the results obtained by confirming the purity of HKFY-34 prepared according to one embodiment of the present invention through HPLC.
FIG. 35 illustrates the results obtained by confirming the purity of HKFY-3 5 prepared according to one embodiment of the present invention through HPLC.
FIG. 36 illustrates the results obtained by confirming the purity of HKFY-36 prepared according to one embodiment of the present invention through HPLC.
FIG. 37 illustrates the results obtained by confirming the molecular weight of HKFY-1 prepared according to one embodiment of the present invention through Ion-Mass.
FIG. 38 illustrates the results obtained by confirming the molecular weight of HKFY-2 prepared according to one embodiment of the present invention through Ion-Mass.
FIG. 39 illustrates the results obtained by confirming the molecular weight of HKFY-3 prepared according to one embodiment of the present invention through Ion-Mass.
FIG. 40 illustrates the results obtained by confirming the molecular weight of HKFY-4 prepared according to one embodiment of the present invention through Ion-Mass.
FIG. 41 illustrates the results obtained by confirming the molecular weight of HKFY-5 prepared according to one embodiment of the present invention through Ion-Mass.
FIG. 42 illustrates the results obtained by confirming the molecular weight of HKFY-6 prepared according to one embodiment of the present invention through Ion-Mass.
FIG. 43 illustrates the results obtained by confirming the molecular weight of HKFY-7 prepared according to one embodiment of the present invention through Ion-Mass.
FIG. 44 illustrates the results obtained by confirming the molecular weight of HKFY-8 prepared according to one embodiment of the present invention through Ion-Mass.
FIG. 45 illustrates the results obtained by confirming the molecular weight of HKFY-9 prepared according to one embodiment of the present invention through Ion-Mass.
FIG. 46 illustrates the results obtained by confirming the molecular weight of HKFY-10 prepared according to one embodiment of the present invention through Ion-Mass.
FIG. 47 illustrates the results obtained by confirming the molecular weight of HKFY-11 prepared according to one embodiment of the present invention through Ion-Mass.
FIG. 48 illustrates the results obtained by confirming the molecular weight of HKFY-12 prepared according to one embodiment of the present invention through Ion-Mass.
FIG. 49 illustrates the results obtained by confirming the molecular weight of HKFY-13 prepared according to one embodiment of the present invention through Ion-Mass.
FIG. 50 illustrates the results obtained by confirming the molecular weight of HKFY-14 prepared according to one embodiment of the present invention through Ion-Mass.
FIG. 51 illustrates the results obtained by confirming the molecular weight of HKFY-15 prepared according to one embodiment of the present invention through Ion-Mass.
FIG. 52 illustrates the results obtained by confirming the molecular weight of HKFY-16 prepared according to one embodiment of the present invention through Ion-Mass.
FIG. 53 illustrates the results obtained by confirming the molecular weight of HKFY-17 prepared according to one embodiment of the present invention through Ion-Mass.
FIG. 54 illustrates the results obtained by confirming the molecular weight of HKFY-18 prepared according to one embodiment of the present invention through Ion-Mass.
FIG. 55 illustrates the results obtained by confirming the molecular weight of HKFY-19 prepared according to one embodiment of the present invention through Ion-Mass.
FIG. 56 illustrates the results obtained by confirming the molecular weight of HKFY-20 prepared according to one embodiment of the present invention through Ion-Mass.
FIG. 57 illustrates the results obtained by confirming the molecular weight of HKFY-21 prepared according to one embodiment of the present invention through Ion-Mass.
FIG. 58 illustrates the results obtained by confirming the molecular weight of HKFY-22 prepared according to one embodiment of the present invention through Ion-Mass.
FIG. 59 illustrates the results obtained by confirming the molecular weight of HKFY-23 prepared according to one embodiment of the present invention through Ion-Mass.
FIG. 60 illustrates the results obtained by confirming the molecular weight of HKFY-24 prepared according to one embodiment of the present invention through Ion-Mass.
FIG. 61 illustrates the results obtained by confirming the molecular weight of HKFY-25 prepared according to one embodiment of the present invention through Ion-Mass.
FIG. 62 illustrates the results obtained by confirming the molecular weight of HKFY-26 prepared according to one embodiment of the present invention through Ion-Mass.
FIG. 63 illustrates the results obtained by confirming the molecular weight of HKFY-27 prepared according to one embodiment of the present invention through Ion-Mass.
FIG. 64 illustrates the results obtained by confirming the molecular weight of HKFY-28 prepared according to one embodiment of the present invention through Ion-Mass.
FIG. 65 illustrates the results obtained by confirming the molecular weight of HKFY-29 prepared according to one embodiment of the present invention through Ion-Mass.
FIG. 66 illustrates the results obtained by confirming the molecular weight of HKFY-30 prepared according to one embodiment of the present invention through Ion-Mass.
FIG. 67 illustrates the results obtained by confirming the molecular weight of HKFY-31 prepared according to one embodiment of the present invention through Ion-Mass.
FIG. 68 illustrates the results obtained by confirming the molecular weight of HKFY-32 prepared according to one embodiment of the present invention through Ion-Mass.
FIG. 69 illustrates the results obtained by confirming the molecular weight of HKFY-33 prepared according to one embodiment of the present invention through Ion-Mass.
FIG. 70 illustrates the results obtained by confirming the molecular weight of HKFY-34 prepared according to one embodiment of the present invention through Ion-Mass.
FIG. 71 illustrates the results obtained by confirming the molecular weight of HKFY-35 prepared according to one embodiment of the present invention through Ion-Mass.
FIG. 72 illustrates the results obtained by confirming the molecular weight of HKFY-36 prepared according to one embodiment of the present invention through Ion-Mass.
FIG. 73 illustrates the results obtained by analyzing the specific binding of HKFY-34 to the GPR39 receptor using a luciferase assay system (top) and a calcium influx assay system (bottom).
FIG. 74 illustrates the results obtained by analyzing the binding capacity of each intrinsic ligand for NTSR1, NTSR2, GHSR, MTLR, NMUR1, NMUR2 and TRHR belonging to the GHSR family and HKFY-34, a HKFY derivative, using a luciferase assay system.
FIG. 75 illustrates the results obtained by analyzing the effect of HKFY-34 on insulin secretion ability through rat insulin ELISA.
FIG. 76 illustrates the results obtained by analyzing the effect of HKFY-34 on glucose-dependent insulin secretion ability through rat insulin ELISA.
FIG. 77 illustrates the results obtained by analyzing the mRNA expression levels of Irs2 and Ins2 according to HKFY-34 treatment through RT-PCR.
FIG. 78 illustrates the results obtained by analyzing the expression level of the GPR39 receptor in adipocytes and adipose tissue through western blot.
FIG. 79 illustrates the results obtained by analyzing the mRNA expression levels of HSL, ATGL and Perilipin A when 3T3-L1 cells are treated with HKFY-34.
FIG. 80 illustrates the results obtained by analyzing the mRNA expression levels of HSL, ATGL and Perilipin A when WAT tissues are treated with HKFY-34.
FIG. 81 illustrates the results obtained by analyzing the mRNA expression level of HSL when 3T3-L1 cells are treated with HKFY-34.
FIG. 82 illustrates the results obtained by analyzing the mRNA expression level of ATGL when 3T3-L1 cells are treated with HKFY-34.
FIG. 83 illustrates the results obtained by analyzing the mRNA expression level of Perilipin A when 3T3-L1 cells are treated with HKFY-34.
FIG. 84 illustrates the results obtained by analyzing the lipolytic ability by concentration according to HKFY-34 treatment in 3T3-L1 cells and WAT tissues.
FIG. 85 illustrates the results obtained by analyzing the expression levels of pERK and pPKA according to HKFY-34 treatment in 3T3-L1 cells through western blot.
FIG. 86 illustrates the results obtained by analyzing the expression levels of pERK and pPKA according to HKFY-34 treatment in WAT tissues through western blot.
FIG. 87 illustrates the results obtained by analyzing the glucose tolerance level in a mouse model of diabetes mellitus disease when treated with HKFY-34 using a blood glucose meter.
FIG. 88 illustrates the results obtained by analyzing the insulin level in a mouse model of diabetes mellitus disease when treated with HKFY-34 using mouse ELISA.
FIG. 89 illustrates the results obtained by analyzing the glucose tolerance level in a mouse model of obesity disease when treated with HKFY-34 using a blood glucose meter.
FIG. 90 illustrates the results obtained by analyzing the insulin level in a mouse model of obesity disease when treated with HKFY-34 using mouse ELISA.
FIG. 91 illustrates the results obtained by analyzing the glucose tolerance level in the GPR39 KO mouse model induced by a high-fat diet according to HKFY-34 treatment using a blood glucose meter.
FIG. 92 illustrates the results obtained by analyzing the glucose tolerance level in a mouse model of diabetes mellitus disease according to HKFY-34 treatment using a blood glucose meter.
FIG. 93 illustrates the results obtained by confirming ¹H NMR of HKFY-1 prepared according to one embodiment of the present invention.
FIG. 94 illustrates the results obtained by confirming ¹H NMR of HKFY-2 prepared according to one embodiment of the present invention.
FIG. 95 illustrates the results obtained by confirming ¹H NMR of HKFY-34 prepared according to one embodiment of the present invention.
FIG. 96 illustrates the results obtained by confirming the purity of HKFY-37 prepared according to one embodiment of the present invention through HPLC.
FIG. 97 illustrates the results obtained by confirming the molecular weight of HKFY-37 prepared according to one embodiment of the present invention through Ion-Mass.
FIG. 98 illustrates the results obtained by confirming the purity of HKFY-38 prepared according to one embodiment of the present invention through HPLC.
FIG. 99 illustrates the results obtained by confirming the molecular weight of HKFY-38 prepared according to one embodiment of the present invention through Ion-Mass.
FIG. 100 illustrates the results obtained by confirming the purity of HKFY-39 prepared according to one embodiment of the present invention through HPLC.
FIG. 101 illustrates the results obtained by confirming the molecular weight of HKFY-39 prepared according to one embodiment of the present invention through Ion-Mass.
FIG. 102 illustrates the results obtained by confirming the purity of HKFY-40 prepared according to one embodiment of the present invention through HPLC.
FIG. 103 illustrates the results obtained by confirming the molecular weight of HKFY-40 prepared according to one embodiment of the present invention through Ion-Mass.
FIG. 104 illustrates the results obtained by confirming the purity of HKFY-41 prepared according to one embodiment of the present invention through HPLC.
FIG. 105 illustrates the results obtained by confirming the molecular weight of HKFY-41 prepared according to one embodiment of the present invention through Ion-Mass.
FIG. 106 illustrates the results obtained by confirming the purity of HKFY-42 prepared according to one embodiment of the present invention through HPLC.
FIG. 107 illustrates the results obtained by confirming the molecular weight of HKFY-42 prepared according to one embodiment of the present invention through Ion-Mass.
FIG. 108 illustrates the results obtained by confirming the purity of HKFY-43 prepared according to one embodiment of the present invention through HPLC.
FIG. 109 illustrates the results obtained by confirming the molecular weight of HKFY-43 prepared according to one embodiment of the present invention through Ion-Mass.
FIG. 110 illustrates the results obtained by confirming the purity of HKFY-44 prepared according to one embodiment of the present invention through HPLC.
FIG. 111 illustrates the results obtained by confirming the molecular weight of HKFY-44 prepared according to one embodiment of the present invention through Ion-Mass.
FIG. 112 illustrates the results obtained by confirming the purity of HKFY-45 prepared according to one embodiment of the present invention through HPLC.
FIG. 113 illustrates the results obtained by confirming the molecular weight of HKFY-45 prepared according to one embodiment of the present invention through Ion-Mass.
FIG. 114 illustrates the results obtained by confirming the purity of HKFY-46 prepared according to one embodiment of the present invention through HPLC.
FIG. 115 illustrates the results obtained by confirming the molecular weight of HKFY-46 prepared according to one embodiment of the present invention through Ion-Mass.
FIG. 116 illustrates the results obtained by confirming the purity of HKFY-47 prepared according to one embodiment of the present invention through HPLC.
FIG. 117 illustrates the results obtained by confirming the molecular weight of HKFY-47 prepared according to one embodiment of the present invention through Ion-Mass.
FIG. 118 illustrates the results obtained by confirming the purity of HKFY-48 prepared according to one embodiment of the present invention through HPLC.
FIG. 119 illustrates the results obtained by confirming the molecular weight of HKFY-48 prepared according to one embodiment of the present invention through Ion-Mass.
FIG. 120 illustrates the results obtained by confirming the purity of HKFY-49 prepared according to one embodiment of the present invention through HPLC.
FIG. 121 illustrates the results obtained by confirming the molecular weight of HKFY-49 prepared according to one embodiment of the present invention through Ion-Mass.
FIG. 122 illustrates the results obtained by confirming the purity of HKFY-50 prepared according to one embodiment of the present invention through HPLC.
FIG. 123 illustrates the results obtained by confirming the molecular weight of HKFY-50 prepared according to one embodiment of the present invention through Ion-Mass.
FIG. 124 illustrates the results obtained by confirming the purity of HKFY-51 prepared according to one embodiment of the present invention through HPLC.
FIG. 125 illustrates the results obtained by confirming the molecular weight of HKFY-51 prepared according to one embodiment of the present invention through Ion-Mass.
FIG. 126 illustrates the results obtained by confirming the purity of HKFY-52 prepared according to one embodiment of the present invention through HPLC.
FIG. 127 illustrates the results obtained by confirming the molecular weight of HKFY-52 prepared according to one embodiment of the present invention through Ion-Mass.
FIG. 128 illustrates the results obtained by confirming the purity of HKFY-53 prepared according to one embodiment of the present invention through HPLC.
FIG. 129 illustrates the results obtained by confirming the molecular weight of HKFY-53 prepared according to one embodiment of the present invention through Ion-Mass.
FIG. 130 illustrates the results obtained by confirming the purity of HKFY-54 prepared according to one embodiment of the present invention through HPLC.
FIG. 131 illustrates the results obtained by confirming the molecular weight of HKFY-54 prepared according to one embodiment of the present invention through Ion-Mass.
FIG. 132 illustrates the results obtained by confirming the purity of HKFY-55 prepared according to one embodiment of the present invention through HPLC.
FIG. 133 illustrates the results obtained by confirming the molecular weight of HKFY-55 prepared according to one embodiment of the present invention through Ion-Mass.
FIG. 134 illustrates the results obtained by confirming the purity of HKFY-56 prepared according to one embodiment of the present invention through HPLC.
FIG. 135 illustrates the results obtained by confirming the molecular weight of HKFY-56 prepared according to one embodiment of the present invention through Ion-Mass.
FIG. 136 illustrates the results obtained by confirming the purity of HKFY-57 prepared according to one embodiment of the present invention through HPLC.
FIG. 137 illustrates the results obtained by confirming the molecular weight of HKFY-57 prepared according to one embodiment of the present invention through Ion-Mass.
FIG. 138 illustrates the results obtained by confirming the purity of HKFY-58 prepared according to one embodiment of the present invention through HPLC.
FIG. 139 illustrates the results obtained by confirming the molecular weight of HKFY-58 prepared according to one embodiment of the present invention through Ion-Mass.
FIG. 140 illustrates the results obtained by confirming the purity of HKFY-59 prepared according to one embodiment of the present invention through HPLC.
FIG. 141 illustrates the results obtained by confirming the molecular weight of HKFY-59 prepared according to one embodiment of the present invention through Ion-Mass.
FIG. 142 illustrates the results obtained by confirming the purity of HKFY-60 prepared according to one embodiment of the present invention through HPLC.
FIG. 143 illustrates the results obtained by confirming the molecular weight of HKFY-60 prepared according to one embodiment of the present invention through Ion-Mass.
FIG. 144 illustrates the results obtained by confirming the purity of HKFY-61 prepared according to one embodiment of the present invention through HPLC.
FIG. 145 illustrates the results obtained by confirming the molecular weight of HKFY-61 prepared according to one embodiment of the present invention through Ion-Mass.
FIG. 146 illustrates the results obtained by confirming the purity of HKFY-62 prepared according to one embodiment of the present invention through HPLC.
FIG. 147 illustrates the results obtained by confirming the molecular weight of HKFY-62 prepared according to one embodiment of the present invention through Ion-Mass.
FIG. 148 illustrates the results obtained by confirming the purity of HKFY-63 prepared according to one embodiment of the present invention through HPLC.
FIG. 149 illustrates the results obtained by confirming the molecular weight of HKFY-63 prepared according to one embodiment of the present invention through Ion-Mass.
FIG. 150 illustrates the results obtained by confirming the purity of HKFY-64 prepared according to one embodiment of the present invention through HPLC.
FIG. 151 illustrates the results obtained by confirming the molecular weight of HKFY-64 prepared according to one embodiment of the present invention through Ion-Mass.
FIG. 152 illustrates the results obtained by confirming the purity of HKFY-65 prepared according to one embodiment of the present invention through HPLC.
FIG. 153 illustrates the results obtained by confirming the molecular weight of HKFY-65 prepared according to one embodiment of the present invention through Ion-Mass.
FIG. 154 illustrates the results obtained by confirming the purity of HKFY-66 prepared according to one embodiment of the present invention through HPLC.
FIG. 155 illustrates the results obtained by confirming the molecular weight of HKFY-66 prepared according to one embodiment of the present invention through Ion-Mass.
FIG. 156 illustrates the results obtained by confirming the purity of HKFY-67 prepared according to one embodiment of the present invention through HPLC.
FIG. 157 illustrates the results obtained by confirming the molecular weight of HKFY-67 prepared according to one embodiment of the present invention through Ion-Mass.
FIG. 158 illustrates the results obtained by confirming the purity of HKFY-68 prepared according to one embodiment of the present invention through HPLC.
FIG. 159 illustrates the results obtained by confirming the molecular weight of HKFY-68 prepared according to one embodiment of the present invention through Ion-Mass.
FIG. 160 illustrates the results obtained by confirming the purity of HKFY-69 prepared according to one embodiment of the present invention through HPLC.
FIG. 161 illustrates the results obtained by confirming the molecular weight of HKFY-69 prepared according to one embodiment of the present invention through Ion-Mass.
FIG. 162 illustrates the results obtained by confirming the purity of HKFY-70 prepared according to one embodiment of the present invention through HPLC.
FIG. 163 illustrates the results obtained by confirming the molecular weight of HKFY-70 prepared according to one embodiment of the present invention through Ion-Mass.
FIG. 164 illustrates the results obtained by confirming the purity of HKFY-71 prepared according to one embodiment of the present invention through HPLC.
FIG. 165 illustrates the results obtained by confirming the molecular weight of HKFY-71 prepared according to one embodiment of the present invention through Ion-Mass.
FIG. 166 illustrates the results obtained by confirming the purity of HKFY-72 prepared according to one embodiment of the present invention through HPLC.
FIG. 167 illustrates the results obtained by confirming the molecular weight of HKFY-72 prepared according to one embodiment of the present invention through Ion-Mass.
FIG. 168 illustrates the results obtained by confirming the purity of HKFY-73 prepared according to one embodiment of the present invention through HPLC.
FIG. 169 illustrates the results obtained by confirming the molecular weight of HKFY-73 prepared according to one embodiment of the present invention through Ion-Mass.
FIG. 170 illustrates the results obtained by confirming the purity of HKFY-74 prepared according to one embodiment of the present invention through HPLC.
FIG. 171 illustrates the results obtained by confirming the molecular weight of HKFY-74 prepared according to one embodiment of the present invention through Ion-Mass.
FIG. 172 illustrates the results obtained by confirming the purity of HKFY-75 prepared according to one embodiment of the present invention through HPLC.
FIG. 173 illustrates the results obtained by confirming the molecular weight of HKFY-75 prepared according to one embodiment of the present invention through Ion-Mass.
FIG. 174 illustrates the results obtained by confirming the purity of HKFY-76 prepared according to one embodiment of the present invention through HPLC.
FIG. 175 illustrates the results obtained by confirming the molecular weight of HKFY-76 prepared according to one embodiment of the present invention through Ion-Mass.
FIG. 176 illustrates the results obtained by confirming the purity of HKFY-77 prepared according to one embodiment of the present invention through HPLC.
FIG. 177 illustrates the results obtained by confirming the molecular weight of HKFY-77 prepared according to one embodiment of the present invention through Ion-Mass.
FIG. 178 illustrates the results obtained by confirming the purity of HKFY-78 prepared according to one embodiment of the present invention through HPLC.
FIG. 179 illustrates the results obtained by confirming the molecular weight of HKFY-78 prepared according to one embodiment of the present invention through Ion-Mass.
FIG. 180 illustrates the results obtained by confirming the purity of HKFY-79 prepared according to one embodiment of the present invention through HPLC.
FIG. 181 illustrates the results obtained by confirming the molecular weight of HKFY-79 prepared according to one embodiment of the present invention through Ion-Mass.

### Best Mode for Carrying out the Invention

Hereinafter, the present invention will be described in more detail.

In one aspect of the present invention, there is provided a compound represented by Formula 1 below or a pharmaceutically acceptable salt thereof. 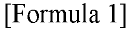 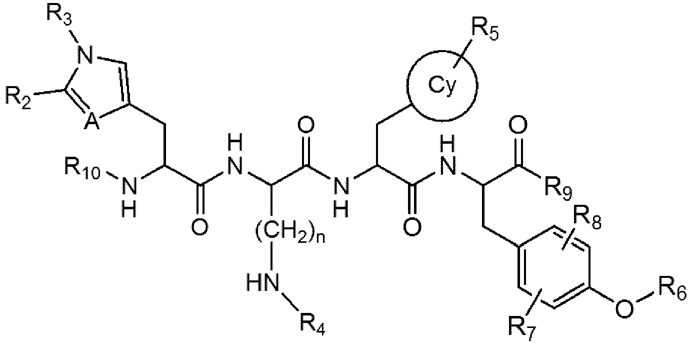 in Formula 1
A is -C(-Ro)-, -N= or -N(-R₁)-,
Cy is C₆₋₁₄ aryl or 5 to 6-membered heteroaryl,
R₁ and R₃ are each independently hydrogen, Ra, an amine protecting group, C₁₋₆ alkyl substituted with 1 to 3 Ra, C₃₋₁₀ cycloalkyl, or 5 to 6-membered heterocyclyl, wherein said heterocyclyl has or does not have 1 to 3 substituents selected from phenylethyl and cyclohexylethyl,
R₂ is hydrogen, Ra, or 5 to 6-membered heterocyclyl, and
Ro is hydrogen, or R₀ and R₂ are linked to each other to form a benzene ring together with the two carbon atoms to which they are attached,
Ra is each independently C₃₋₁₀ cycloalkyl or C₆₋₁₄ aryl, wherein said aryl has or does not have one or more substituents selected from the group consisting of halogen, -OH, C₆₋₁₄ aryl substituted with 5 to 6-membered heteroaryl, C₁₋₆ alkyl and C₁₋₆ alkoxy,
n is 1 to 10,
R₄ is hydrogen, C₁₋₁₀ alkyl or C₁-C₂₀ alkylcarbonyl,
R₅ is hydrogen, halogen, CF₃ or C₁₋₆ alkyl,
R₆ is hydrogen, C₁₋₁₀ alkyl or -S(=O)₂OH,
R₇ and R₈ are each independently hydrogen, halogen, nitro, amine or C₁₋₆ alkyl,
R₉ is -OH or -NH₂, and
R₁₀ is hydrogen, an amine protecting group or biotin,
wherein said heterocyclyl and heteroaryl each independently contain at least one heteroatom selected from the group consisting of N, S and O.

In one embodiment of the present invention, the compound may be a peptide, and the peptide may be a derivative of a peptide having a four amino acid sequence, and the four amino acid sequence may be His-Lys-Phe-Tyr (SEQ ID NO: 1).

In accordance with convention used in the art, in the Formula herein, 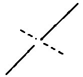 is used to denote a bond that is the point of attachment of a moiety or substituent to a nucleus or framework structure.

As used herein, the term "alkyl" is a hydrocarbon having normal (primary), secondary, tertiary or cyclic carbon atoms. For example, an alkyl group may have 1 to 20 carbon atoms (i.e., C₁-C₂₀ alkyl), 1 to 10 carbon atoms (i.e., C₁-C₁₀ alkyl), or 1 to 6 carbon atoms (i.e., C₁-C₆ alkyl). Examples of suitable alkyl groups include methyl (Me, -CH₃), ethyl (Et, -CH₂CH₃), 1-propyl (n-Pr, n-propyl, -CH₂CH₂CH₃), 2-propyl (i-Pr, i-propyl, -CH(CH₃)₂), 1-butyl (n-Bu, n-butyl, -CH₂CH₂CH₂CH₃), 2-methyl-1-propyl (i-Bu, i-butyl, -CH₂CH(CH₃)₂), 2-butyl (s-Bu, s-butyl, -CH(CH₃)CH₂CH₃), 2-methyl-2-propyl (t-Bu, t-butyl, -C(CH₃)₃), 1-pentyl (n-pentyl, - CH₂CH₂CH₂CH₂CH₃), 2-pentyl (-CH(CH₃)CH₂CH₂CH₃), 3-pentyl (-CH(CH₂CH₃)₂), 2-methyl-2-butyl (-C(CH₃)₂CH₂CH₃), 3-methyl-2-butyl (-CH(CH₃)CH(CH₃)₂), 3-methyl-1-butyl (-CH₂CH₂CH(CH₃)₂), 2-methyl-1-butyl (-CH₂CH(CH₃)CH₂CH₃), 1-hexyl (-CH₂CH₂CH₂CH₂CH₂CH₃), 2-hexyl (-CH(CH₃)CH₂CH₂CH₂CH₃), 3-hexyl (-CH(CH₂CH₃)(CH₂CH₂CH₃)), 2-methyl-2-pentyl (-C(CH₃)₂CH₂CH₂CH₃), 3-methyl-2-pentyl (-CH(CH₃)CH(CH₃)CH₂CH₃), 4-methyl-2-pentyl (-CH(CH₃)CH₂CH(CH₃)₂), 3-methyl-3-pentyl (-C(CH₃)(CH₂CH₃)₂), 2-methyl-3-pentyl (-CH(CH₂CH₃)CH(CH₃)₂), 2,3-dimethyl-2-butyl (-C(CH₃)₂CH(CH₃)₂), 3,3-dimethyl-2-butyl (-CH(CH₃)C(CH₃)₃, and octyl (-(CH₂)₇CH₃), but are not limited thereto.

As used herein, the term "cycloalkyl" refers to a saturated monocycle or poly-cycle containing only carbon atoms in the ring. A cycloalkyl may have 3 to 7 carbon atoms as a monocycle, 7 to 12 carbon atoms as a bicyclic cycloalkyl, and up to about 20 carbon atoms as a poly-cycle. A monocyclic cycloalkyl has 3 to 6 ring atoms, and more typically 5 or 6 ring atoms. A bicyclic cycloalkyl has 7 to 12 ring atoms arranged in a bicyclo [4,5], [5,5], [5,6] or [6,6] system, or 9 to 10 ring atoms arranged in a bicyclo [5,6] or [6,6] system or a spiro-fused ring. Non-limiting examples of monocyclic cycloalkyls may include cyclopropyl, cyclobutyl, cyclopentyl, and cyclohexyl (each of which may be substituted or unsubstituted).

As used herein, the term "heterocyclyl" includes the radicals of heterocycles described in the literature [Paquette, Leo A.; Principles of Modern Heterocyclic Chemistry (W.A. Benjamin, New York, 1968), specifically Chapters 1, 3, 4, 6, 7 and 9; The Chemistry of Heterocyclic Compounds, A Series of Monographs" (John Wiley & Sons, New York, from 1950 to present), specifically Volumes 13, 14, 16, 19 and 28; and J. Am. Chem. Soc. (1960) 82:5566], but is not limited thereto. The term "heterocyclyl" refers to an aromatic or nonaromatic ring radical that is formed as saturated or unsaturated single or multiple rings and has one or more heteroatoms. For example, "5 to 6-membered heterocyclyl" may refer to a heterocyclyl having a total of 5 to 6 heteroatoms and/or carbon atoms. As used herein, when "heterocyclyl" is described as a substituent, it can be used as a term encompassing "heterocycloalkyl" and "heteroaryl." Herein, "heterocycloalkyl" refers to a saturated ring radical that is formed as single or multiple rings and has one or more heteroatoms, and "heteroaryl" refers to an aromatic ring radical that is formed as single or multiple rings and has one or more heteroatoms, and "heteroatom" may be selected from N, O and S. Non-limiting examples of heteroaryl rings include all those listed in the definition of "heterocyclyl," including pyridinyl, pyrrolyl, oxazolyl, indolyl, isoindolyl, purinyl, furanyl, thienyl, benzofuranyl, benzothiophenyl, carbazolyl, imidazolyl, thiazolyl, isoxazolyl, pyrazolyl, isothiazolyl, quinolyl, isoquinolyl, pyridazyl, pyrimidyl, pyrazyl (which may be substituted or unsubstituted), and the like.

As used herein, the term "heteroaryl" refers to an aromatic heterocyclyl having one or more heteroatoms in the ring. Non-limiting examples of suitable heteroatoms that may be included in the aromatic ring include oxygen, sulfur and nitrogen. Non-limiting examples of heteroaryl rings include all those listed in the definition of "heterocyclyl," including pyridinyl, pyrrolyl, oxazolyl, indolyl, isoindolyl, purinyl, furanyl, thienyl, benzofuranyl, benzothiophenyl, carbazolyl, imidazolyl, thiazolyl, isoxazolyl, pyrazolyl, isothiazolyl, quinolyl, isoquinolyl, pyridazyl, pyrimidyl, pyrazyl (which may be substituted or unsubstituted), and the like.

As used herein, the term "alkoxy" refers to a group having the Formula -O-alkyl, in which an alkyl group as defined above is attached to a parent compound through an oxygen atom. The alkyl portion of the alkoxy group may have 1 to 20 carbon atoms (i.e., C₁-C₂₀ alkoxy), 1 to 12 carbon atoms (i.e., C₁-C₁₂ alkoxy), or 1 to 6 carbon atoms (i.e., C₁-C₆ alkoxy). Examples of suitable alkoxy groups include methoxy (-O-CH₃ or -OMe), ethoxy (-OCH₂CH₃ or -OEt), t-butoxy (-O-C(CH₃)₃ or -O-tBu), and the like, but are not limited thereto.

As used herein, the term "alkylcarbonyl" refers to a group having the Formula -C(=O)-alkyl, in which an alkyl group as defined above is attached to a parent compound through a carbon atom. The alkyl portion of the alkylcarbonyl group may have 1 to 20 carbon atoms (i.e., C₁-C₂₀ alkylcarbonyl) or 5 to 17 carbon atoms (i.e., C₅-C₁₇ alkylcarbonyl). Examples of suitable alkylcarbonyl groups include caprylic (-C(=O)(CH₂)₆CH₃), capric (-C(=O)(CH₂)₈CH₃), lauric (-C(=O)(CH₂)₁₀CH₃), myristic (-C(=O)(CH₂)₁₂CH₃), palmitic (-C(=O)(CH₂)₁₄CH₃), and the like, but are not limited thereto.

The term "substituted" with respect to alkyl, aryl, heteroaryl, heterocyclyl, carbocyclyl (for example, cycloalkyl), and the like, for example, "substituted alkyl", "substituted aryl", "substituted heteroaryl", "substituted heterocyclyl" and "substituted carbocyclyl (for example, substituted cycloalkyl)" refer to alkyl, aryl, heteroaryl, heterocyclyl, carbocyclyl (for example, cycloalkyl) in which one or more hydrogen atoms are each independently substituted with a non-hydrogen substituent. Typical substituents include -X, -R, -O-, =O, -OR, -SR, -S-, -NR₂, - N+R₃, =NR, -C(X)₃, -CN, -OCN, -SCN, -N=C=O, -NCS, -NO, -NO₂, =N-OH, =N₂, -N₃, - NHC(=O)R, -C(=O)R, -C(=O)NRR -S(=O)₂O-, -S(=O)₂OH, -S(=O)₂R, -OS(=O)₂OR, - S(=O)₂NR, -S(=O)R, -OP (=O)(OR)₂, -C(=O)R, alkylene-C(=O)R, -C(S)R, -C(=O)OR, alkylene-C(=O)OR, -C(=O)O-, alkylene-C(=O)O-, -C(=S)OR, -C(=O)SR, -C(=S)SR, - C(=O)NRR, alkylene-C(=O)NRR, -C(=S)NRR, -C(-NR)NRR (wherein, each X is independently halogen: F, Cl, Br, or I, and R is independently H, alkyl, aryl, arylalkyl or heterocycle), but are not limited thereto. Alkylene, alkenylene, and alkynylene groups may also be similarly substituted.

In addition, the present invention includes pharmaceutically acceptable salts and addition salts of the compounds of the present invention, such as hydrochloride, hydrobromide or trifluoroacetate addition salts and sodium, potassium and magnesium salts, but is not limited thereto.

It will be appreciated by one of ordinary skill in the art that when a moiety such as "alkyl", "aryl", "heterocyclyl" is substituted by one or more substituents, it may optionally be referred to as a moiety such as "alkylene", "arylene", "heterocyclylene" (i.e., meaning that one or more hydrogen atoms of the parent "alkyl", "aryl", "heterocyclyl" moiety are substituted with the substituents as stated above). When a moiety such as "alkyl", "aryl", "heterocyclyl," etc. is referred to herein as "substituted" or shown as substituted in the drawings (or optionally substituted, for example, when the number of substituents is from 0 to a positive number), the terms "alkyl", "aryl", "heterocyclyl" and the like should be understood as interchangeable with "alkylene", "arylene", "heterocyclylene" and the like.

It will be appreciated by one of ordinary skill in the art that the substituents and other moieties of the compound of Formula 1 should be selected to provide a compound that is sufficiently stable to provide a pharmaceutically useful compound that can be formulated into an acceptable stable pharmaceutical composition. The compound of Formula 1 having such stability is considered to be included in the scope of the present invention.

As used herein, the term "optionally substituted" refers to a particular moiety of a compound of Formula 1 having one, two, or more substituents.

In the present invention, the compound represented by Formula 1 may be an optical isomer of an L-form or a D-form.

In the present invention, in the compound represented by Formula 1, A may be -C(-R₀)- or -N(-R₁)-, and R₁ may be hydrogen, 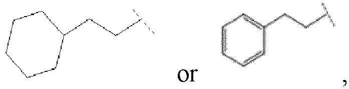 but is not limited thereto. In addition, R₀ may be hydrogen, and R₂ may be hydrogen, 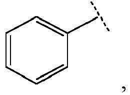 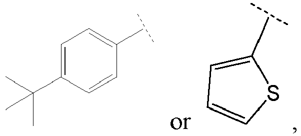 or R₀ and R₂ may be linked to each other to form a benzene ring together with the two carbon atoms to which they are attached, but is not limited thereto. In addition, R₃ may be one selected from the group consisting of 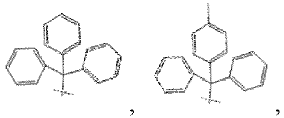 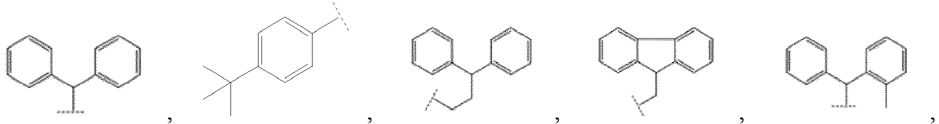 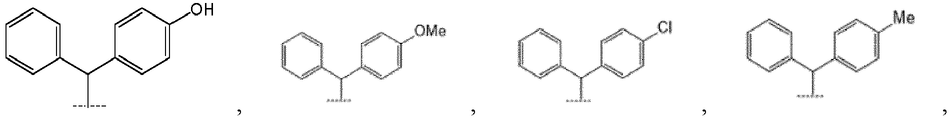 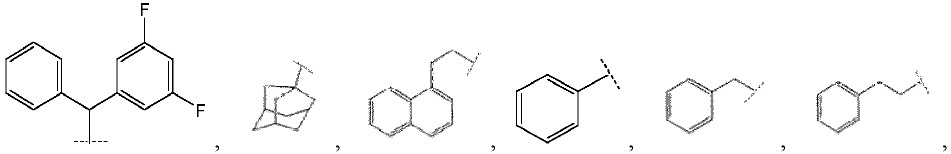 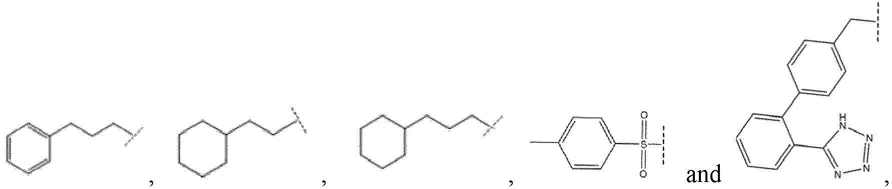 and preferably, it may be 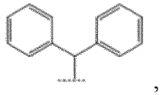 but is not limited thereto.

In addition, n may be 3 or 4, R₄ may be heptylcarbonyl, R₅ may be methyl or CF₃, R₆ may be hydrogen or S(=O)₂OH, and R₇ and R₈ may be each independently hydrogen or amine. In addition, R₇ and R₈ may be each independently hydrogen, R₉ may be -OH, and R₁₀ may be hydrogen, p-toluenesulfonyl (p-Ts), t-butoxycarbonyl (t-Boc) or biotin, but is not limited thereto. In addition, R₅, R₇ and R₈ are not particularly limited at positions to be substituted on the benzene ring. For example, R₅ may be substituted at an ortho (o-), meta (m-) or para (p-) position.

It may be any one selected from the group consisting of:
1) His(trt)-Lys(caprylic)-Phe-Tyr,
2) Biotin-His(trt)-Lys(caprylic)-Phe-Tyr,
3) His(benzyl)-Lys(caprylic)-Phe-Tyr,
4) d-His(trt)-Lys(caprylic)-Phe-Tyr,
5) His(4-methyltrityl)-Lys(caprylic)-Phe-Tyr,
6) His(DAMP-3)-Lys(caprylic)-Phe-Tyr,
7) His(DAMP-5)-Lys(caprylic)-Phe-Tyr,
8) His(DAMP-2)-Lys(caprylic)-Phe-Tyr,
9) His(2-phenylethyl)-Lys(caprylic)-Phe-Tyr,
10) His(2-naphthalen-1-methyl)-Lys(caprylic)-Phe-Tyr,
11) His(2-cyclohexylethyl)-Lys(caprylic)-Phe-Tyr,
12) His(trt)-Lys(caprylic)-d-Phe-Tyr,
13) His(trt)-Lys(caprylic)-d-Phe(4-Cl)-Tyr,
14) d-His(trt)-Lys(caprylic)-d-Phe(4-Cl)-d-Tyr,
15) His(trt)-Lys(caprylic)-d-Phe(4-Cl)-d-Tyr,
16) His(trt)-d-Lys(caprylic)-d-Phe(4-Cl)-Tyr,
17) His(3,3-diphenylethyl)-Lys(caprylic)-Phe-Tyr,
18) His(Fm)-Lys(caprylic)-Phe-Tyr,
19) His(phenylethyl)-Lys(caprylic)-Phe-Tyr,
20) His(4-methoxylbenzhydryl)-Lys(caprylic)-Phe-Tyr,
21) His(4-chlorobenzhydryl)-Lys(caprylic)-Phe-Tyr,
22) His(4-methylbenzhydryl)-Lys(caprylic)-Phe-Tyr,
23) His(adamantan-1-yl)-Lys(caprylic)-Phe-Tyr,
24) His(trt)-Lys(capric)-Phe-Tyr,
25) His(trt)-Lys(lauric)-Phe-Tyr,
26) His(trt)-Lys(myristic)-Phe-Tyr,
27) His(trt)-Lys(palmitic)-Phe-Tyr,
28) His(trt)-Lys(caprylic)-d-Phe(F)-Tyr,
29) His(trt)-Lys(caprylic)-d-Phe(Br)-Tyr,
30) His(trt)-Lys(caprylic)-d-Phe(4-methyl)-Tyr,
31) d-His(trt)-Lys(caprylic)-d-Phe(4-methyl)-Tyr,
32) His(1,3-difluorobenzhydryl)-Lys(caprylic)-d-Phe(4-methyl)-Tyr,
33) His(benzhydryl)-Lys(caprylic)-Phe-Tyr,
34) His(benzhydryl)-Lys(caprylic)-d-Phe(4-methyl)-Tyr,
35) His(benzhydryl)-Lys(caprylic)-d-Phe(4-methyl)-Tyr(SO₃H),
36) His(benzhydryl)-Lys(caprylic)-d-Phe(4-methyl)-Tyr(2,6-di-methyl),
57) His(2-phenyl)-Lys(caprylic)-d-Phe(4-methyl)-Tyr,
58) His(1-phenyl)-Lys(caprylic)-d-Phe(4-methyl)-Tyr,
59) His(1,2-diphenyl)-Lys(caprylic)-d-Phe(4-methyl)-Tyr,
60) His(2-(4-tert-butyl)phenyl)-Lys(caprylic)-d-Phe(4-methyl)-Tyr,
61) His(1-(4-tert-butyl)phenyl)-Lys(caprylic)-d-Phe(4-methyl)-Tyr,
62) d-His(benzhydryl)-Lys(caprylic)-d-Phe(4-methyl)-Tyr,
63) His(thiophene)-Lys(caprylic)-d-Phe(4-methyl)-Tyr,
64) His(4-methoxybenzhydryl)-Lys(caprylic)-d-Phe(4-methyl)-Tyr,
65) His(4-hydroxybenzhydryl)-Lys(caprylic)-d-Phe(4-methyl)-Tyr,
66) His(benzhydryl)-Lys(caprylic)-d-Phe(4-methyl)-Tyr-NH₂,
67) His(benzhydryl)-Lys(caprylic)-d-Phe(4-methyl)-Tyr(3-chloro),
68) His(benzhydryl)-Lys(caprylic)-d-Phe(4-methyl)-Tyr(3-nitro),
69) His(benzhydryl)-Lys(caprylic)-d-Phe(4-methyl)-Tyr(3,5-nitro),
70) His(benzhydryl)-Lys(caprylic)-d-Phe(4-methyl)-Tyr(3-amino),
71) His(benzhydryl)-Orn(caprylic)-d-Phe(4-methyl)-Tyr,
72) Biotin-His(benzhydryl)-Lys(caprylic)-d-Phe(4-methyl)-Tyr,
73) His(trt)-Lys(caprylic)-d-3Pal-Tyr,
74) His(trt)-Lys(caprylic)-d-2Nal-Tyr,
75) His(tosyl)-Lys(caprylic)-d-Phe(4-methyl)-Tyr,
76) His(trt)-Lys(caprylic)-d-Phe(4-CF3)-Tyr,
77) His(tbm)-Lys(caprylic)-d-Phe(4-CF3)-Tyr,
78) Boc-Trp-Lys(caprylic)-d-Phe(4-methyl)-Tyr, and
79) His(benzhydryl)Lys(caprylic).

In the present invention, the compound may be (X₁)-(X₂)-[His(trt)-Lys(caprylic)-Phe-Tyr], wherein X₁ and X₂ may be each independently any one amino acid selected from 20 amino acids or derivatives thereof, and specifically, it may be 37) Gly-Ser-His(trt)-Lys(caprylic)-Phe-Tyr (SEQ ID NO: 2).

In the present invention, in the compound represented by Formula 1, 1 to 10 amino acids or derivatives thereof may be further bound to the C-terminus of the compound. Specifically, the compound may be [His(trt)-Lys(caprylic)-Phe-Tyr]-(X₃)ₐ-(X₄)_{b}-(X₅)_{c}-(X₆)_{d}-(X₇)ₑ-(X₈)_{f}-(X₉)_{g}, wherein X₃ to X₉ may be each independently any one amino acid selected from 20 amino acids or derivatives thereof, and a to g may be 0 or 1, and at least one of them may be 1. More specifically, the compound may be any one selected from the group consisting of 38) His(trt)-Lys(caprylic)-Phe-Tyr-Ser-Asp-Gln-Gln-Ala-Arg-Phe (SEQ ID NO: 3), 39) His(trt)-Lys(caprylic)-Phe-Tyr-Ser-Gln-Asn-Gly-Ala-Arg-Phe (SEQ ID NO: 4), 40) His(trt)-Lys(caprylic)-Phe-Tyr-Ser-His-Gln-Gln-Ala-Arg-Phe (SEQ ID NO: 5), 41) His(trt)-Lys(caprylic)-Phe-Tyr-Gln-Asn-Gly-Ala-Arg-Phe (SEQ ID NO: 6), 42) His(trt)-Lys(caprylic)-Phe-Tyr-His-Gln-Gln-Ala-Arg-Phe (SEQ ID NO: 7), 43) His(trt)-Lys(caprylic)-Phe-Tyr-Gln-Asn-Gln-Ala-Arg-Phe (SEQ ID NO: 8), and 44) His(trt)-Lys(caprylic)-Phe-Tyr-Trp (SEQ ID NO: 9).

In another aspect of the present invention, there is provided a compound or a pharmaceutically acceptable salt thereof, wherein the compound consists of [His(trt)-Lys(caprylic)]-(X₁₀)ₕ-(Xₗ₁)i, wherein X₁₀ to X₁₁ may be each independently any one amino acid selected from 20 amino acids or derivatives thereof, and h and i may be 0 or 1, and at least one of them may be 1. Specifically, the compound may be any one selected from the group consisting of 45) Biotin-His(trt)-Lys(caprylic)-Ala(biphenyl)-Tyr (SEQ ID NO: 10), 46) His(trt)-Lys(caprylic)-Phe-Trp (SEQ ID NO: 11), 47) His(trt)-Lys(caprylic)-Tyr-Phe (SEQ ID NO: 12), 48) His(trt)-Lys(caprylic)-Tyr-Tyr (SEQ ID NO: 13), 49) His(trt)-Lys(caprylic)-Phe, 50) His(trt)-Lys(caprylic)-Phe-Ser (SEQ ID NO: 14), and 51) His(trt)-Lys(caprylic)-Leu-Tyr (SEQ ID NO: 15).

In another aspect of the present invention, there is provided any one selected from the group consisting of 52) His(trt)-Phe-Tyr-Asp-Gln-Gln-Ala-Arg-Phe (SEQ ID NO: 16), 53) His(trt)-Gly-Ser-Lys(caprylic)-Phe-Tyr (SEQ ID NO: 17), 54) Lys(caprylic)-Phe-Tyr, 55) Lys(caprylic)-His(trt)-Phe-Tyr (SEQ ID NO: 18), 56) His(trt)-Lys(caproic)-Phe-Tyr-Asp-Gln-Gln-Ala-Arg-Phe (SEQ ID NO: 19) and 79) His(benzhydryl)Lys(caprylic).

In the present invention, the compound may be present in a mixture of L-form and D-form amino acids (for example, which may be any one amino acid selected from the group consisting of His, Lys, Phe, Tyr, Ser, Asp, Gln, Ala, Arg and Asn, but is not limited thereto), where "d" means a D-form amino acid.

In the present invention, "trt" is triphenylmethyl, "Boc" is t-butyloxycarbonyl, "tBu" is t-butyl, "Fmoc" is 9-fluorenylmethoxyoxycarbonyl, "dde" is 1-(4,4-dimethyl-2,6-dioxocyclohexylidene)ethyl, and "Bip" is biphenylalanine.

In the present invention, the "biotin" is a kind of vitamin B complex required for the growth of animals and plants. The natural one is a D-form isomer, in which the bond of the two pentagonal rings is in the cis configuration. The other seven isomers, such as L-biotin in cis form or allobiotin in trans form, do not exhibit coenzyme activity.

In the present invention, the "amine protecting group" may be a protecting group selected from the group consisting of t-butyloxycarbonyl (Boc), p-toluenesulfonyl (Ts), fluorenylmethyloxycarbonyl, benzyl, triphenylmethyl and carboxybenzyl, and it may be preferably p-toluenesulfonyl or t-butyloxycarbonyl.

In the present invention, when R₁₀ in the compound represented by Formula 1 is biotin, the biotin may be a compound represented by Formula 2 below.

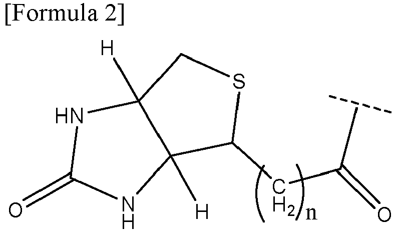

In Formula 2, n may be 1 to 10, and it may be preferably 4, but is not limited thereto.

In addition, in the present invention, the compound represented by Formula 1 may be represented by Formula 3 below, when A is -N=, R₂ is hydrogen, R₃ is 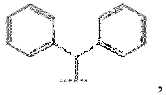 n is 4, R₄ is heptylcarbonyl, Cy is phenyl, R₅ is methyl, R₆ is S(=O)₂OH, R₇, R₈ and R₁₀ are each independently hydrogen, and R₉ is -OH, and the amino acid of SEQ ID NO: 1 may be a substituted form, such as His(benzhydryl)-Lys(caprylic)-d-Phe(4-methyl)-Tyr. The IUPAC name of the compound represented by Formula 3 below is ((R)-2-((S)-2-((S)-2-amino-3-(1-benzhydryl-1H-imidazol-4-yl)propanamido)-6-octanamidohexanamido)-3-(p-tolyl)propanoyl)-L-tyrosine, and in one embodiment of the present invention, it was named HKFY-34.

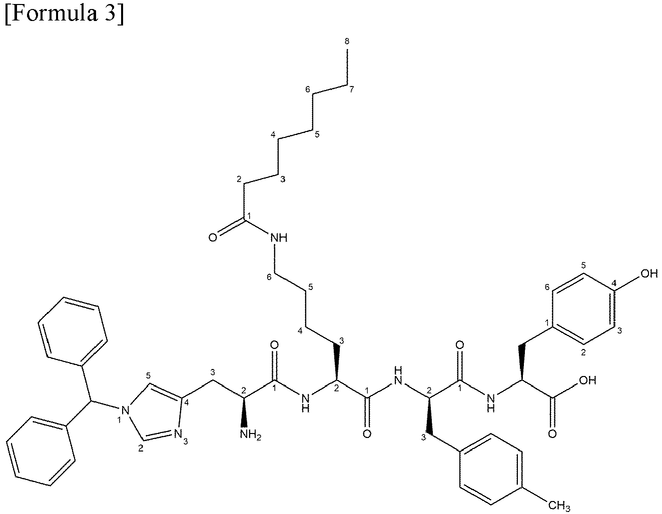

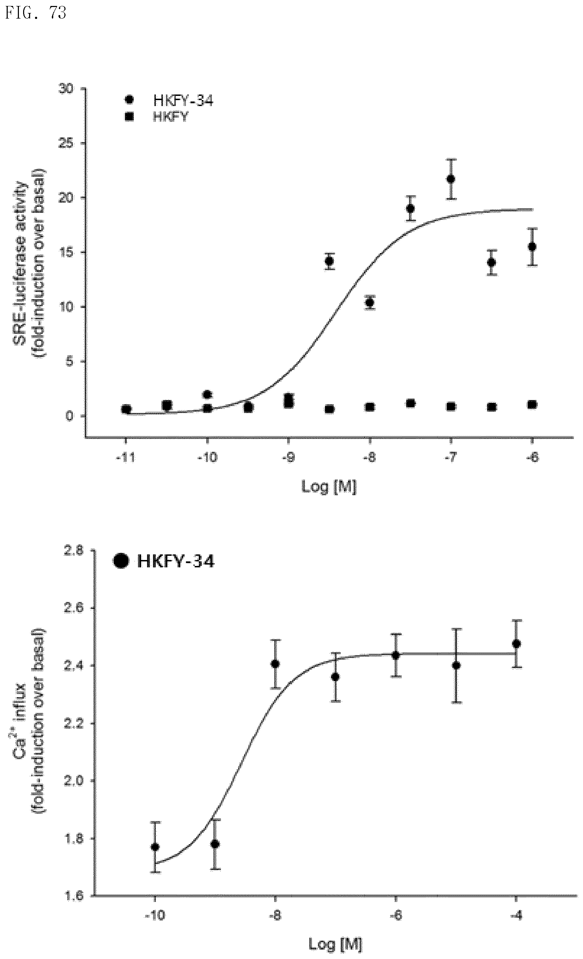

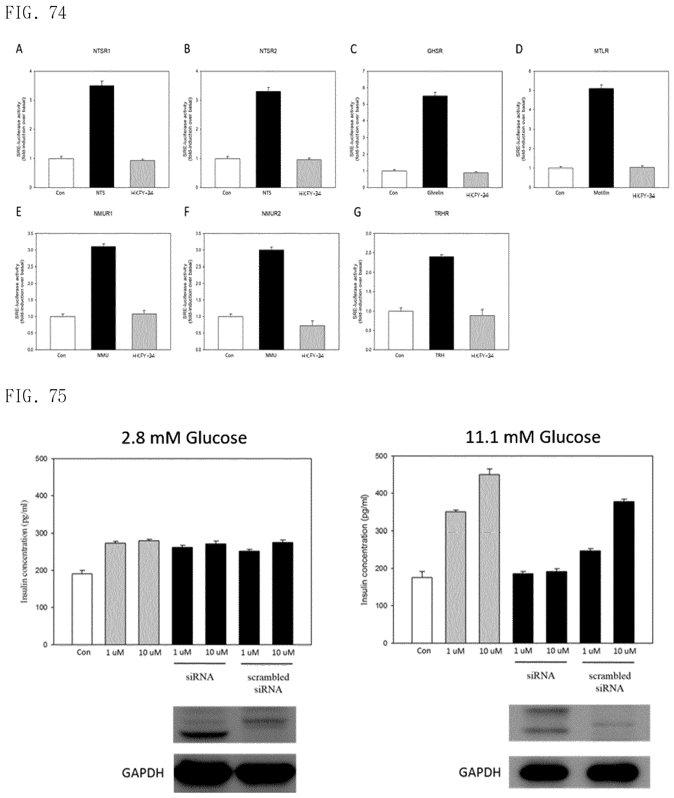

In one embodiment of the present invention, it was confirmed that the peptide derivative, which is a compound having the structure of Formula 1 according to the present invention, selectively and specifically binds to GPR39 (G protein-coupled receptor 39), one of the orphan G protein-coupled receptor (orphan GPCR), which is a membrane protein (Tables 3 to 5 and FIGs. 73 and 74).

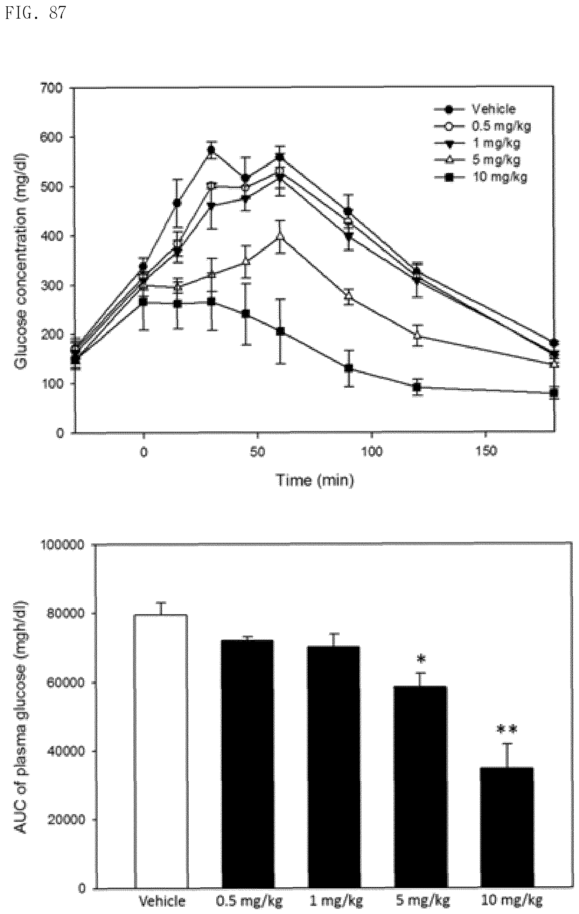

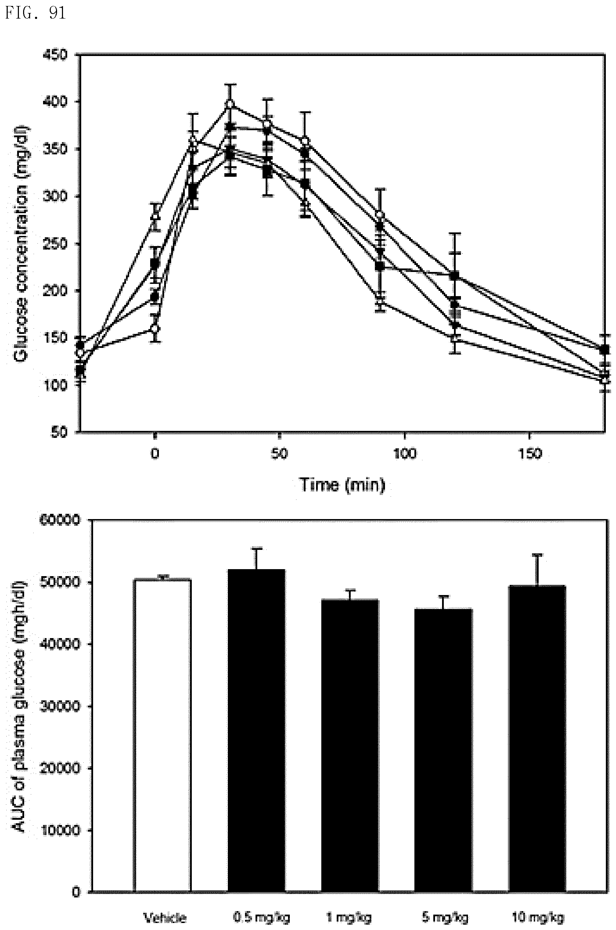

In addition, in one embodiment of the present invention, when rat insulinoma cells or rat islet cells were treated with the peptide derivative according to the present invention, glucose-dependent insulin secretion ability was enhanced through the GPR39 receptor (FIGs. 75 to 77). In addition, it was confirmed that when a mouse model of diabetes mellitus disease and a mouse model of obesity disease were treated with the peptide derivative according to the present invention, the glucose tolerance was lowered, the insulin level was increased, and the anti-diabetic effect was excellent (FIGs. 87 to 90 and 92). In addition, when GPR39 KO mice were treated with the peptide derivative according to the present invention, no significant glucose tolerance was observed (FIG. 91). Therefore, it was confirmed that the peptide derivative according to the present invention specifically acts on GPR39 in relation to glucose tolerance.

Therefore, in another aspect of the present invention, there is provided a pharmaceutical composition for preventing or treating diabetes mellitus, comprising the compound or pharmaceutically acceptable salt thereof as an active ingredient.

As used herein, the term "diabetes mellitus" is a type of metabolic diseases such as insufficient insulin secretion or failure to function normally. Diabetes mellitus is characterized by high blood glucose in which the concentration of glucose in the blood is elevated, and high blood glucose causes various symptoms and signs and causes the excretion of glucose in the urine. Diabetes mellitus is divided into type 1 and type 2. Type 1 diabetes mellitus, previously called 'juvenile diabetes mellitus', is a disease caused by the inability to produce insulin at all. Type 2 diabetes mellitus, which is relatively deficient in insulin, is characterized by insulin resistance. Type 2 diabetes mellitus seems to be largely caused by environmental factors such as high calorie, high fat, high protein diet due to westernization of diet, lack of exercise, and stress, but in addition to the above, diabetes mellitus can be caused by a defect in a specific gene, and can also be caused by pancreatic surgery, infection, or drugs. In the present invention, the "diabetes mellitus" may include type 1 or type 2, and may refer to a chronic disease caused by a lack of insulin causing glucose tolerance.

In addition, the present invention provides a method for preventing or treating diabetes mellitus, comprising administering the compound or pharmaceutically acceptable salt thereof to a mammal.

In addition, the present invention provides the use of the compound or pharmaceutically acceptable salt thereof, for the manufacture of a medicament for preventing or treating diabetes mellitus.

The "pharmaceutical composition" may be administered orally, intramuscularly or subcutaneously. Formulations for oral administration may take various forms such as syrups, tablets, capsules, creams and lozenges. Syrup formulations generally comprise a suspension or solution of the compound or a salt thereof in a liquid carrier such as ethanol, peanut oil, olive oil, glycerin or water, and optionally comprise a sweetening agent or a coloring agent. When the composition is in the form of a tablet, any pharmaceutical carrier generally used for the preparation of solid formulations may be used. Examples of such carriers include magnesium stearate, white clay, talc, gelatin, acacia, stearic acid, starch, lactose and sucrose. When the composition is in the form of a capsule, any conventional encapsulation process may be used, for example, the carrier may be included in a hard gelatin capsule shell. When the composition is formulated in the form of a soft gelatin capsule shell, any pharmaceutical carrier generally used for the preparation of dispersions or suspensions may be used, and such carriers are aqueous gum, cellulose, silicate or oil. Formulations for intramuscular or subcutaneous administration may be prepared in a liquid form, such as solutions, suspensions and emulsions including aqueous solvents such as water, physiological saline and Ringer's solution; or lipophilic solvents such as fatty oil, sesame oil, corn oil and synthetic fatty acid esters.

The composition is preferably formulated in a dosage form specific to a particular patient.

For each unit dose for oral administration, it is preferable to contain the compound of Formula 1 or a pharmaceutically acceptable salt thereof in an amount of 0.1 mg/kg to 500 mg/kg, 1 mg/kg to 100 mg/kg, or 10 mg/kg to 50 mg/kg.

A daily administration dose suitable for oral administration is about 0.01 to 40 mg/kg of the compound of Formula 1 or a pharmaceutically acceptable salt thereof, and may be administered 1 to 6 times a day depending on the condition of the patient.

In another aspect of the present invention, there is provided a health functional food composition for preventing or improving diabetes mellitus, comprising the compound or nutritionally acceptable salt thereof as an active ingredient.

The health functional food according to the present invention may be in the form of powder, granule, tablet, capsule or beverage, and may be candy, chocolate, gum, tea, vitamin complex, health supplement food, and the like.

In this case, the content of the compound or pharmaceutically acceptable salt thereof according to the present invention included in the health functional food may be typically 0.01 to 50 % by weight, or 0.1 to 20 % by weight of the total food weight. In addition, in the case of the health beverage composition, it may be included in an amount of 0.02 to 10 g, or 0.3 to 1 g, based on 100 mL of the health beverage composition.

The food may further include a nutritionally acceptable food supplementary additive together with the compound or pharmaceutically acceptable salt thereof of the present invention.

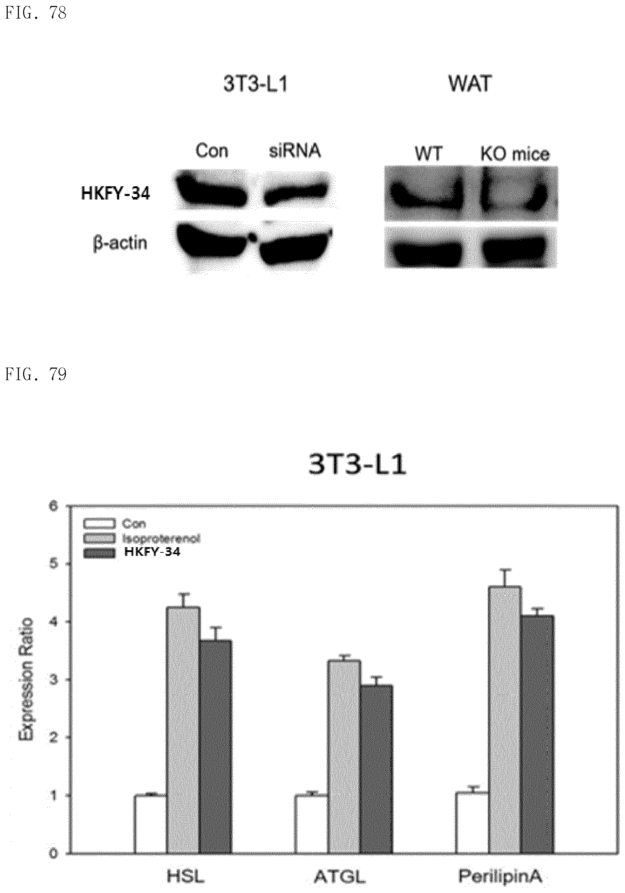

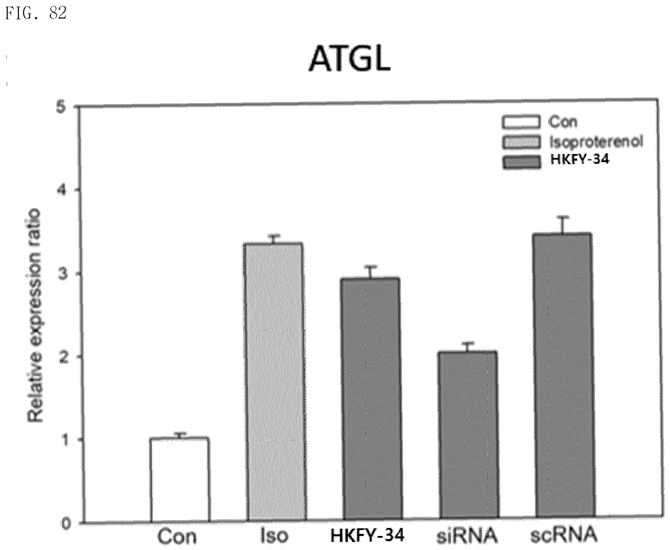

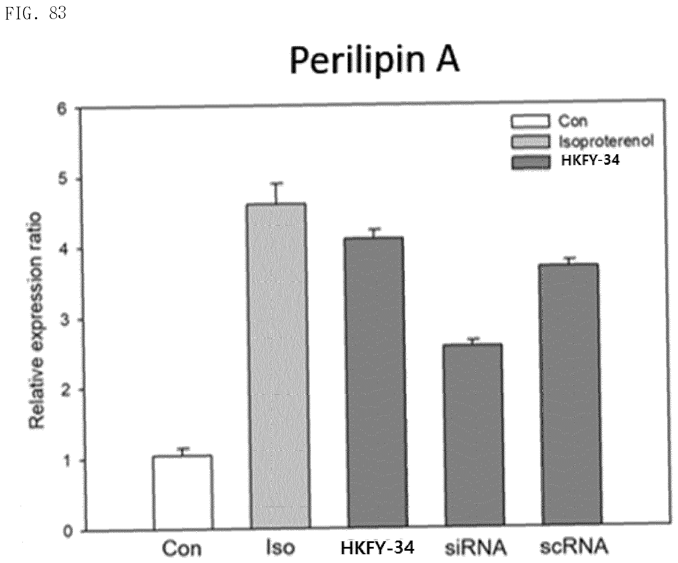

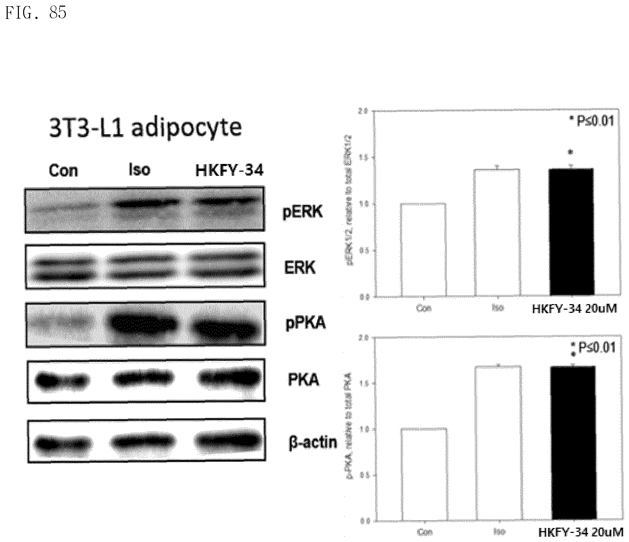

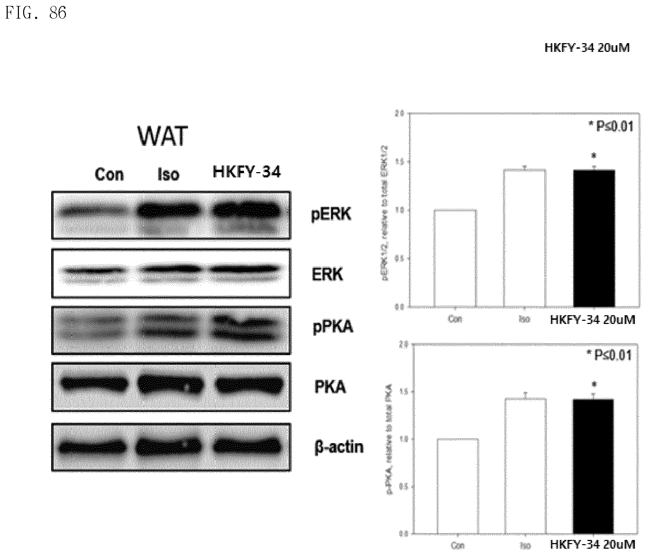

In one embodiment of the present invention, it was confirmed that the lipolytic ability was excellent when 3T3-L1 cells and WAT cells were treated with the peptide derivative according to the present invention (FIGs. 78 to 84), and by analyzing the mechanism of lipolysis, it was confirmed that it has the same signaling and lipolysis regulatory mechanism as the β-adrenergic receptor that regulates the major lipolysis of adipocytes (FIGs. 85 and 86).

Therefore, in another aspect of the present invention, there is provided a pharmaceutical composition for preventing or treating obesity, comprising the compound or pharmaceutically acceptable salt thereof as an active ingredient.

As used herein, the term "obesity" refers to a state in which adipose tissue is excessive in the body, and when an excessive amount of nutrients is consumed compared to energy consumption over a long period of time, obesity is induced by energy imbalance. There are cases where it is caused by a problem with the function of the appestat due to genetic mutations in specific genes, or it is caused by endocrine diseases such as Cushing's syndrome and various drugs that increase appetite, but in general, it occurs because energy intake is greater than energy consumption. In general, obesity is caused by a combination of genetic and environmental influences. In particular, the modern living environment, which is rich in highcalorie foods and does not cause inconvenience even with less physical activity, is causing an explosive increase in obesity.

In addition, the present invention provides a method for preventing or treating obesity, comprising administering the compound or pharmaceutically acceptable salt thereof to a mammal.

In addition, the present invention provides the use of the compound or pharmaceutically acceptable salt thereof, for the manufacture of a medicament for preventing or treating obesity.

In another aspect of the present invention, there is provided a health functional food composition for preventing or improving obesity, comprising the compound or nutritionally acceptable salt thereof as an active ingredient.

In another aspect of the present invention, there is provided a cosmetic composition for preventing or improving obesity, comprising the compound or pharmaceutically acceptable salt thereof as an active ingredient.

### Mode for Carrying out the Invention

Hereinafter, the present invention will be described in detail by way of Examples. However, the following examples are only for illustrating the present invention, and the present invention is not limited to the following examples.

### Example 1. Preparation of HKFY derivative

### Example 1.1. Preparation of HKFY-1

In order to prepare His(trt)-Lys(caprylic)-Phe-Tyr (N6-octanoyl-N2-(Nt-trityl-L-histidyl)-L-lysyl-L-phenylalanyl-L-tyrosine, HKFY-1), Fmoc-Tyr(tBu) and DMF (dimethylformamide) were loaded onto the trityl resin to prepare Fmoc-Tyr(tBu)-trityl resin. DMF containing 20% piperidine and Fmoc-Phe-OH and HOBt (hydroxyl-benzo triazole) were added to Fmoc-Tyr(tBu)-trityl resin to prepare Fmoc-Phe-Tyr(tBu)-trityl resin.

DMF containing 2% NH₂NH₂·H₂O was added to Fmoc-Lys(dde)-OH, and dde was removed to prepare Fmoc-Lys-OH. DMF containing caprylic acid and HOBt and DIC was added to the Fmoc-Lys-OH to prepare Fmoc-Lys(caprylic acid)-OH.

DMF containing 20% piperidine was added to the Fmoc-Phe-Tyr(tBu)-trityl resin, and HOBt (hydroxyl-benzo triazole) was added to the Fmoc-Lys(caprylic acid)-OH to prepare Fmoc-Lys(caprylic acid)-Phe-Tyr(tBu)-trityl resin.

DMF containing 20% piperidine was added to the Fmoc-Lys(caprylic acid)-Phe-Tyr(tBu)-trityl resin, and Boc-His(trt)-OH and HOBt (hydroxyl-benzo triazole) were added to prepare Boc-His(trt)-Lys(caprylic acid)-Phe-Tyr(tBu)-trityl resin.

The protecting group was cleaved from the Boc-His(trt)-Lys(caprylic acid)-Phe-Tyr(tBu)-trityl resin and then purified to prepare His(trt)-Lys(caprylic acid)-Phe-Tyr.

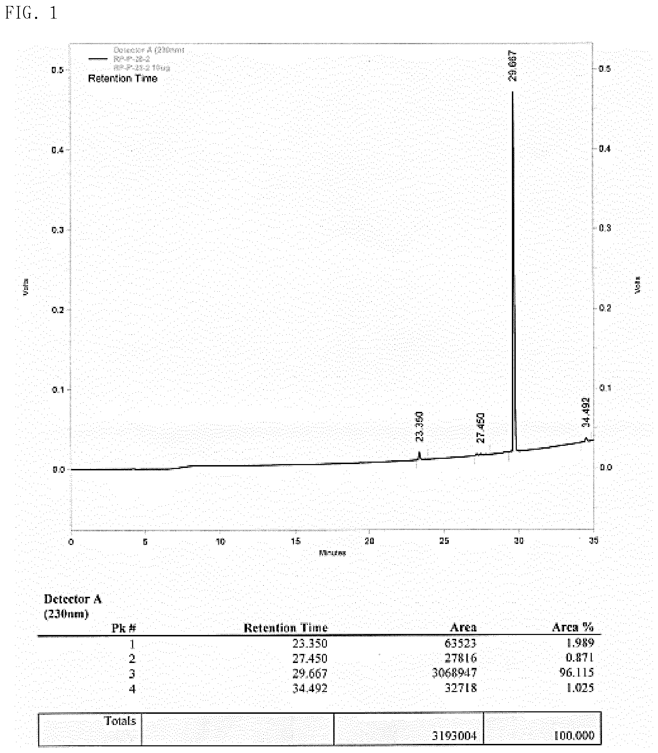

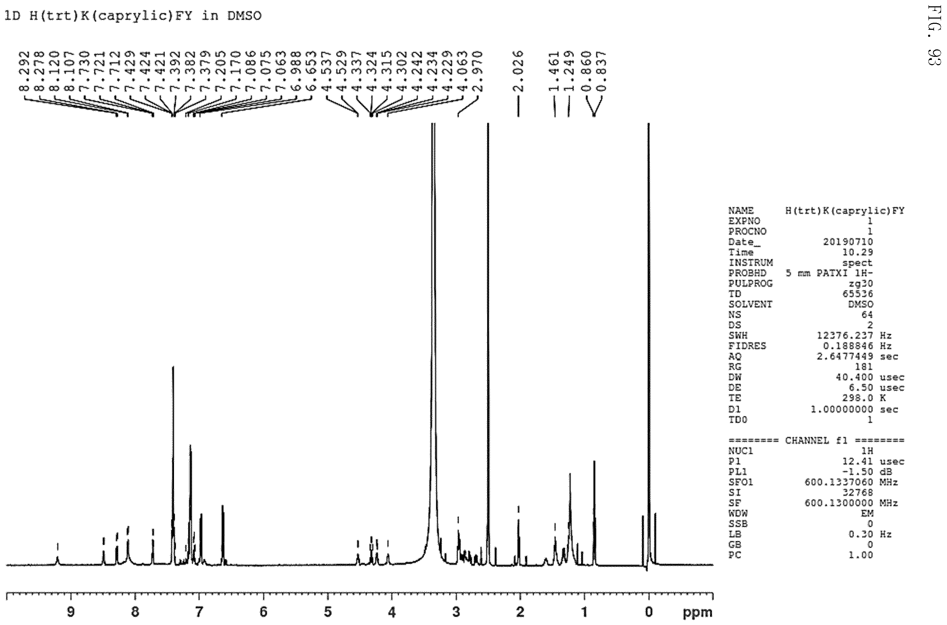

The purity, molecular weight and ¹H NMR of the HKFY-1 are shown in FIGs. 1, 37 and 93, respectively.

### Example 1.2. Preparation of HKFY-2

In order to prepare biotin ((S)-3-([1,1'-biphenyl]-4-yl)-2-((S)-6-octanamido-2-((S)-2-(5-((3aS,4S,6aR)-2-oxohexahydro-1H-thieno[3,4-d]imidazol-4-yl)pentanamido)-3-(1-trityl-1H-imidazol-4-yl)propanamido)hexanamido)propanoyl)-L-tyrosine, DMF containing 20% piperidine was added to the Fmoc-Lys(caprylic acid)-Phe-Tyr(tBu)-trityl resin obtained from Example 1.1, and Fmoc-His(trt)-OH was added to prepare Fmoc-His(trt)-Lys(caprylic acid)-Phe-Tyr(tBu)-trityl resin. D-biotin was added to the resin in the same manner to prepare D-biotin-His(trt)-Lys(caprylic acid)-Phe-Tyr(tBu)-trityl resin.

The protecting group of the resin was cleaved and then purified to prepare biotin-His(trt)-Lys(caprylic acid)-Phe-Tyr (N6-octanoyl-N2-(Na-(5-((3aS,4S,6aR)-2-oxohexahydro-1H-thieno[3,4-d]imidazol-4-yl)pentanoyl)-Nt-trityl-L-histidyl)-L-lysyl-L-phenylalanyl-L-tyrosine).

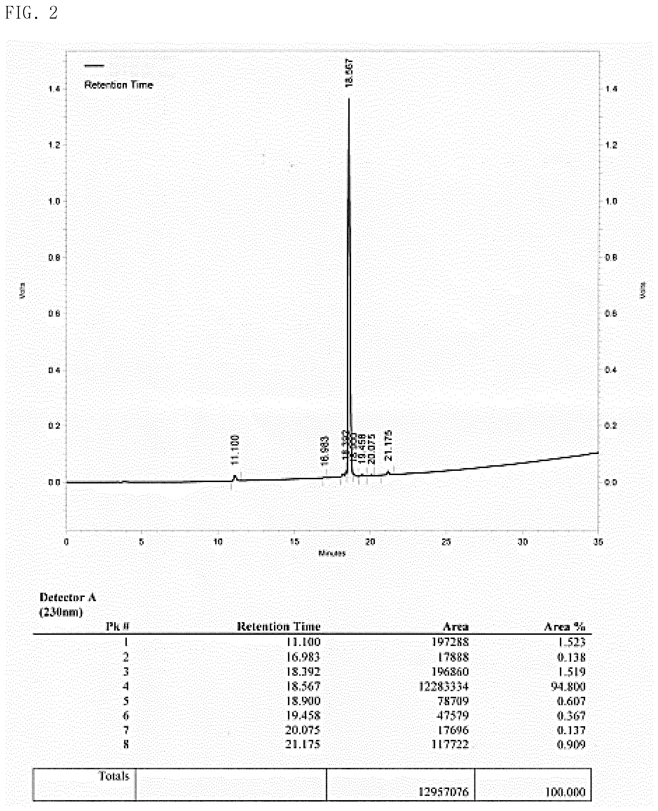

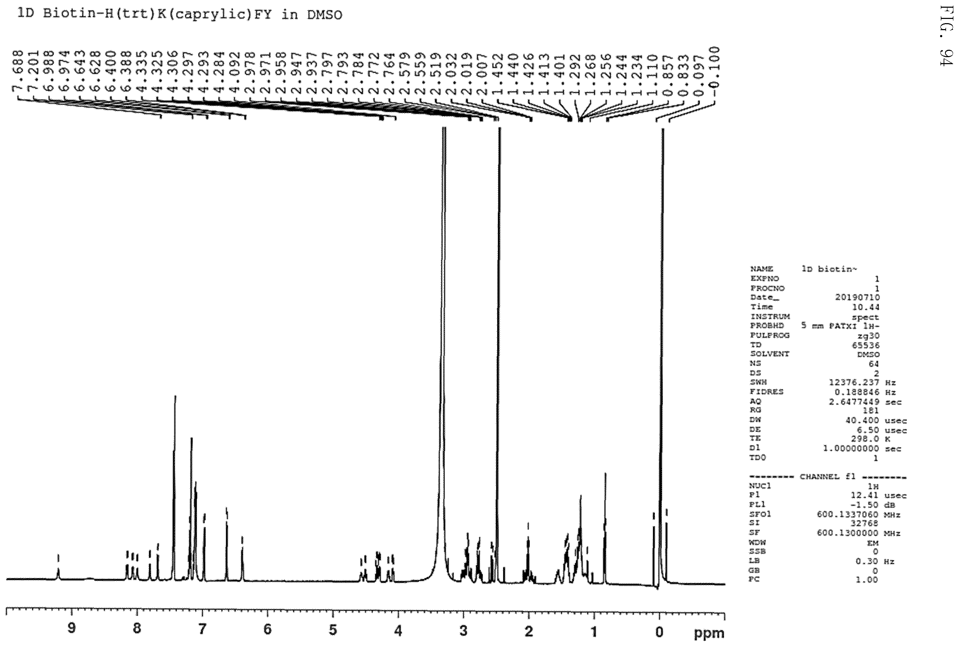

The purity, molecular weight and ¹H NMR of the HKFY-2 are shown in FIGs. 2, 38 and 94, respectively.

### Example 1.3. Preparation of HKFY-3

His(benzyl)-Lys(caprylic)-Phe-Tyr (N2-(Nt-benzyl-L-histidyl)-N6-octanoyl-L-lysyl-L-phenylalanyl-L-tyrosine) was prepared in the same manner except that Boc-His(benzyl)-OH was used instead of Boc-His(trt)-OH obtained during the preparation process of Example 1.1.

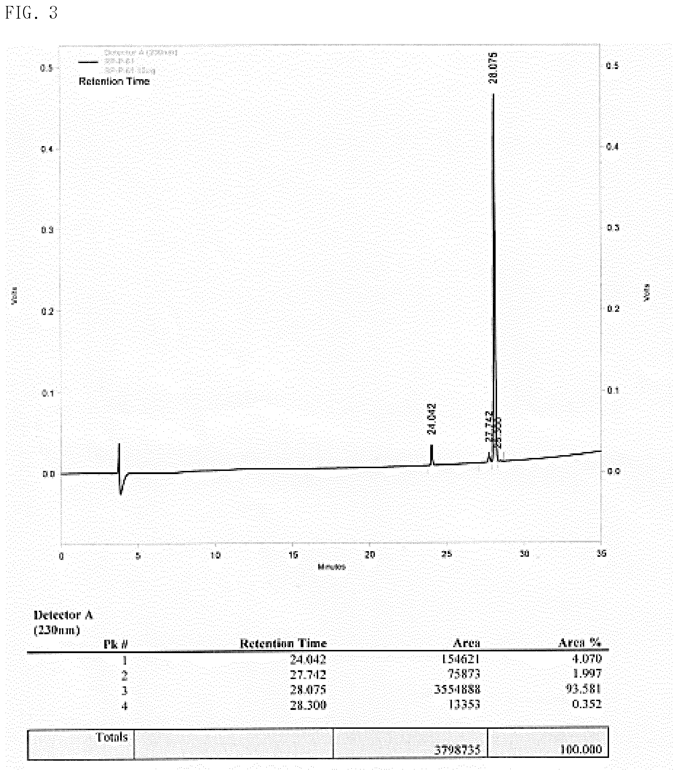

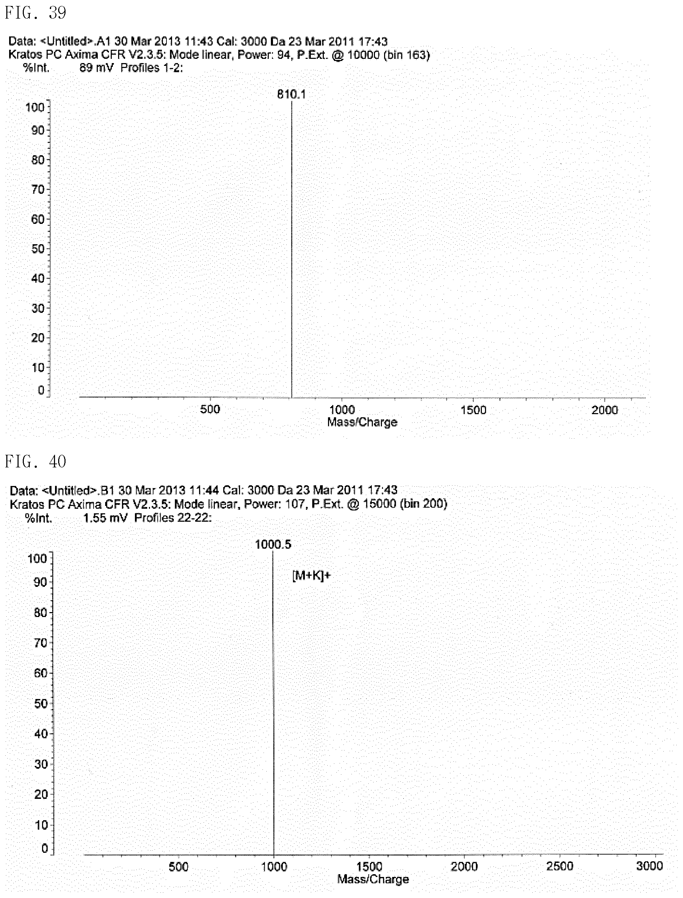

The purity and molecular weight of the HKFY-3 are shown in FIGs. 3 and 39, respectively.

### Example 1.4. Preparation of HKFY-4

d-His(trt)-Lys(caprylic)-Phe-Tyr (N6-octanoyl-N2-(tNLt-trityl-D-histidyl)-L-lysyl-L-phenylalanyl-L-tyrosine) was prepared in the same manner except that Boc-d-His(trt) was used instead of Boc-His(trt)-OH obtained during the preparation process of Example 1.1.

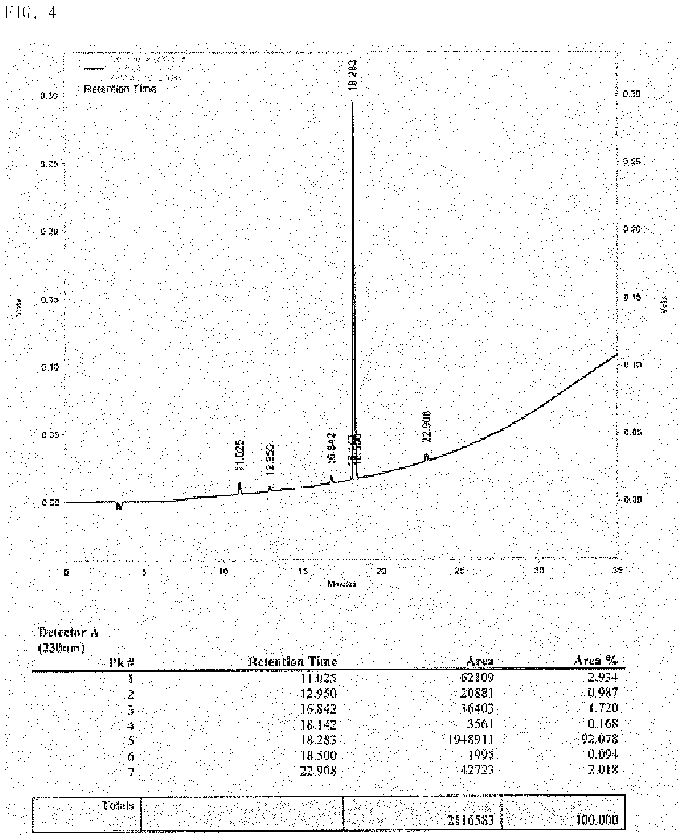

The purity and molecular weight of the HKFY-4 are shown in FIGs. 4 and 40, respectively.

### Example 1.5. Preparation of HKFY-5

His(4-methyltrityl)-Lys(caprylic)-Phe-Tyr (N2-(Nt-(diphenyl (p-tolyl)methyl)-L-histidyl)-N6-octanoyl-L-lysyl-L-phenylalanyl-L-tyrosine) was prepared in the same manner except that Boc-His(4-methyltrityl)-OH was used instead of Boc-His(trt)-OH obtained during the preparation process of Example 1.1.

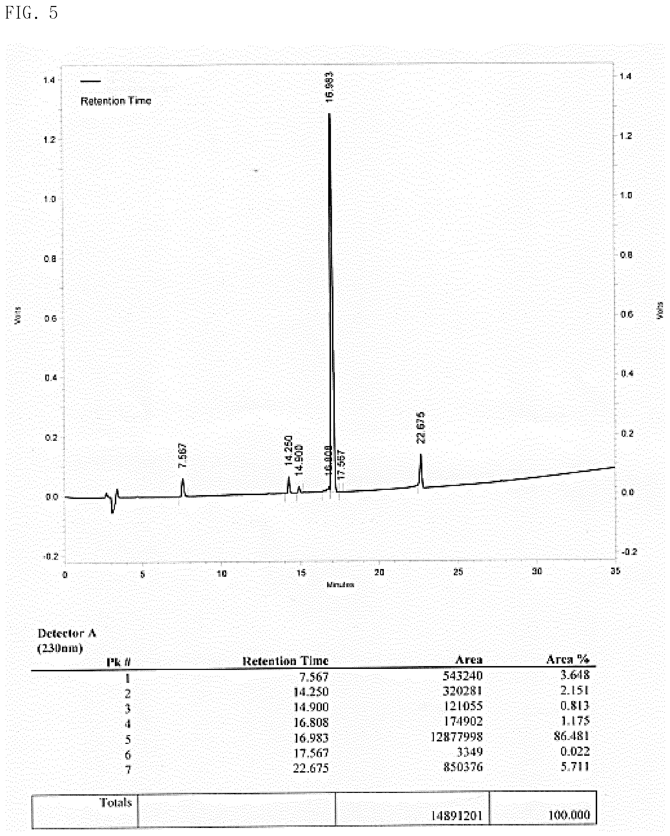

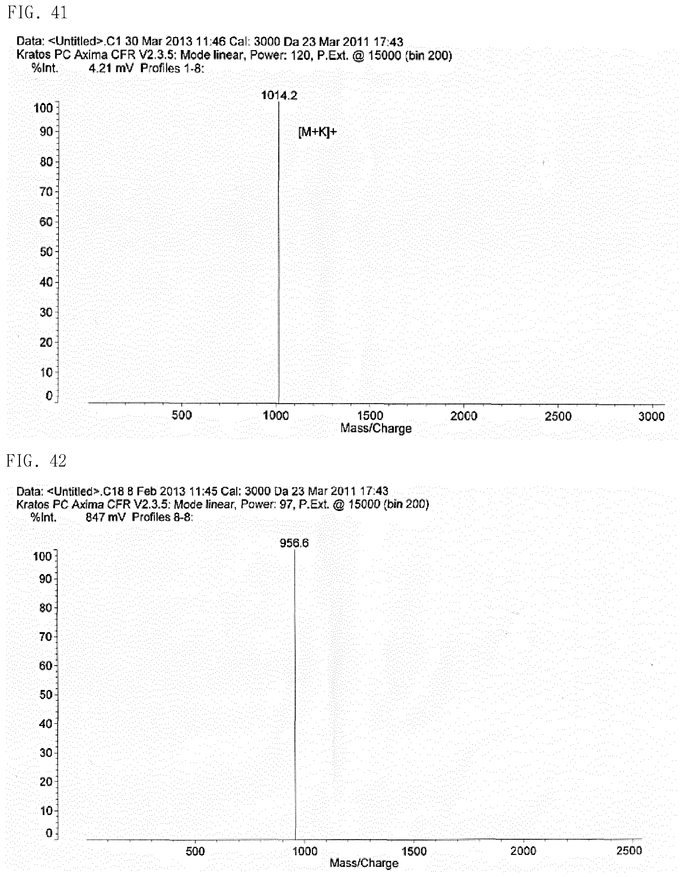

The purity and molecular weight of the HKFY-5 are shown in FIGs. 5 and 41, respectively.

### Example 1.6. Preparation of HKFY-6

His(DAMP-3)-Lys(caprylic)-Phe-Tyr (N2-((S)-2-amino-3-(1,3-diphenethyl-2,3-dihydro-1H-imidazol-4-yl)propanoyl)-N6-octanoyl-L-lysyl-L-phenylalanyl-L-tyrosine) was prepared in the same manner except that Boc-His(DAMP-3)-OH was used instead of Boc-His(trt)-OH obtained during the preparation process of Example 1.1.

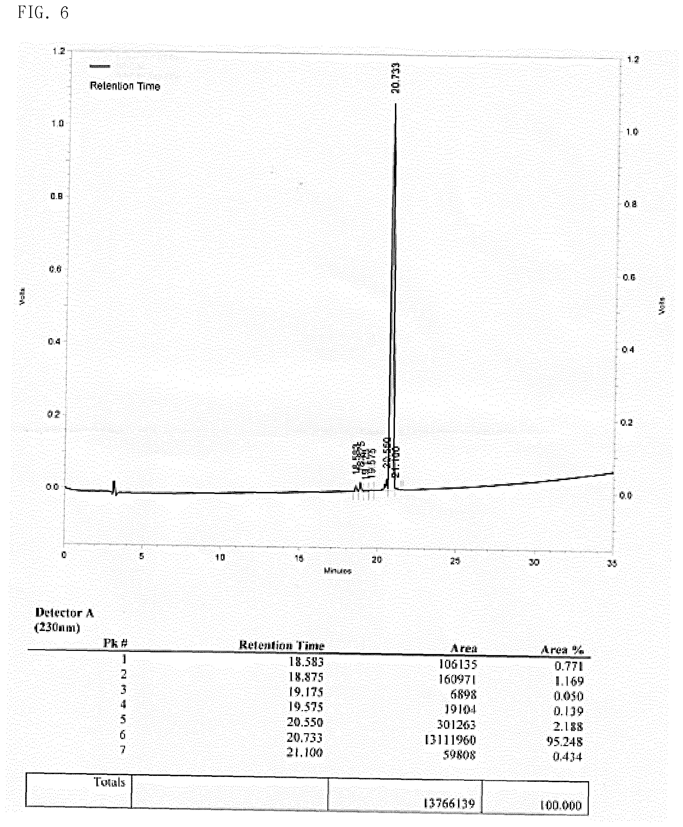

The purity and molecular weight of the HKFY-6 are shown in FIGs. 6 and 42, respectively.

### Example 1.7. Preparation of HKFY-7

His(DAMP-5)-Lys(caprylic)-Phe-Tyr (N2-((S)-2-amino-3-(3-(2-cyclohexylethyl)-2,3-dihydro-1H-imidazol-4-yl)propanoyl)-N6-octanoyl-L-lysyl-L-phenylalanyl-L-tyrosine) was prepared in the same manner except that Boc-His(DAMP-5)-OH was used instead of Boc-His(trt)-OH obtained during the preparation process of Example 1.1.

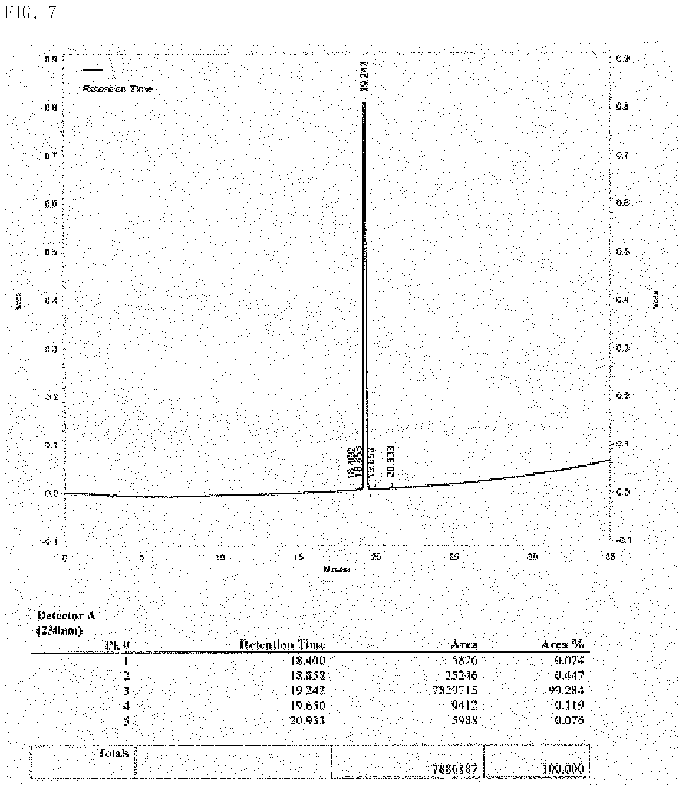

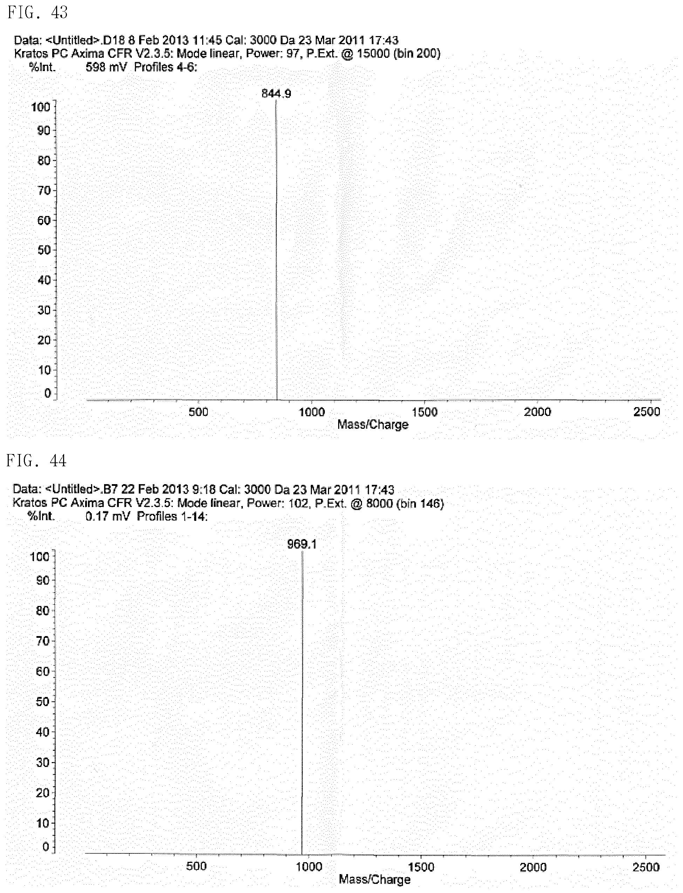

The purity and molecular weight of the HKFY-7 are shown in FIGs. 7 and 43, respectively.

### Example 1.8. Preparation of HKFY-8

His(DAMP-2)-Lys(caprylic)-Phe-Tyr (N2-((S)-2-amino-3-(1,3-bis(2-cyclohexylethyl)-2,3-dihydro-1H-imidazol-4-yl)propanoyl)-N6-octanoyl-L-lysyl-L-phenylalanyl-L-tyrosine) was prepared in the same manner except that Boc-His(DAMP-2)-OH was used instead of Boc-His(trt)-OH obtained during the preparation process of Example 1.1.

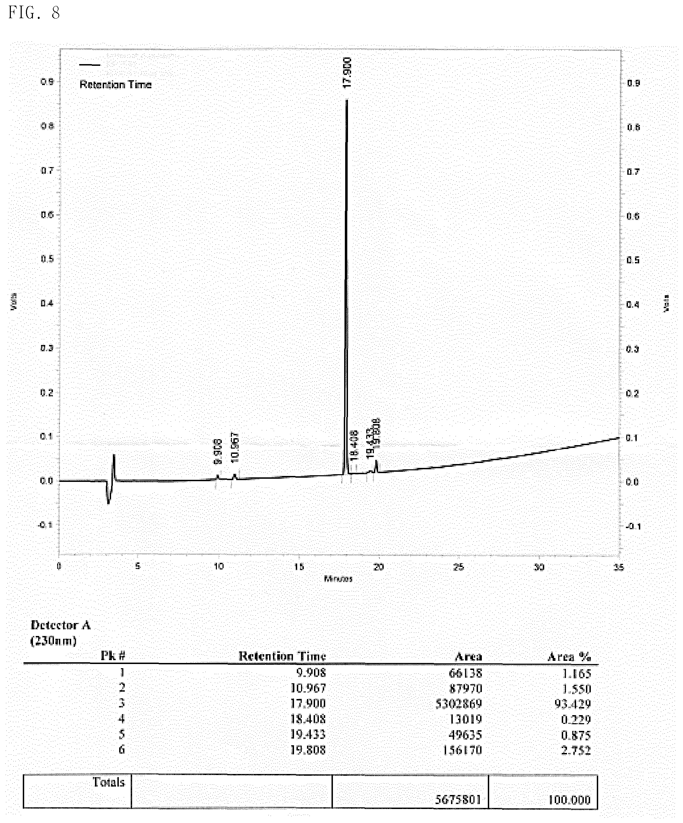

The purity and molecular weight of the HKFY-8 are shown in FIGs. 8 and 44, respectively.

### Example 1.9. Preparation of HKFY-9

His(DAMP-2)-Lys(caprylic)-Phe-Tyr (N6-octanoyl-N2-(Nt-phenethyl-L-histidyl)-L-lysyl-L-phenylalanyl-L-tyrosine) was prepared in the same manner except that Boc-His(2-phenylethyl)-OH was used instead of Boc-His(trt)-OH obtained during the preparation process of Example 1.1.

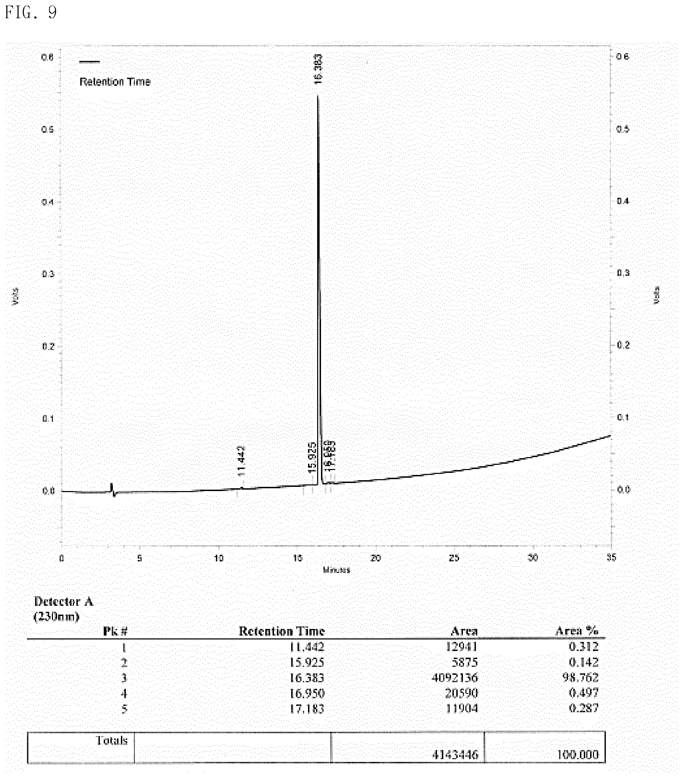

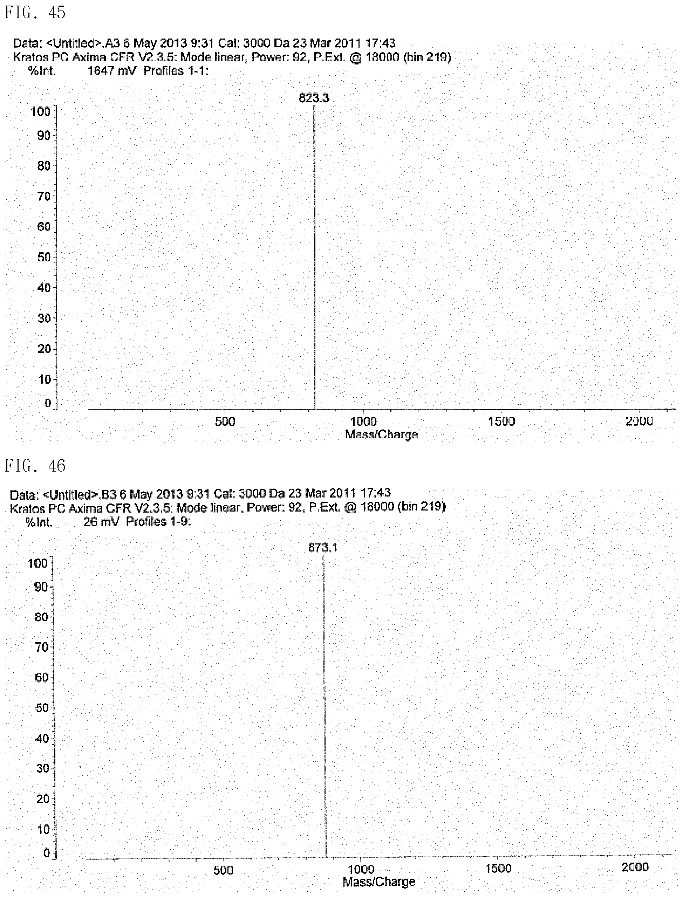

The purity and molecular weight of the HKFY-9 are shown in FIGs. 9 and 45, respectively.

### Example 1.10. Preparation of HKFY-10

His(2-naphthalen-1-methyl)-Lys(caprylic)-Phe-Tyr (N2-(Nt-(naphthalen-1-ylmethyl)-L-histidyl)-N6-octanoyl-L-lysyl-L-phenylalanyl-L-tyrosine) was prepared in the same manner except that Boc-His(2-naphthalen-1-methyl)-OH was used instead of Boc-His(trt)-OH obtained during the preparation process of Example 1.1.

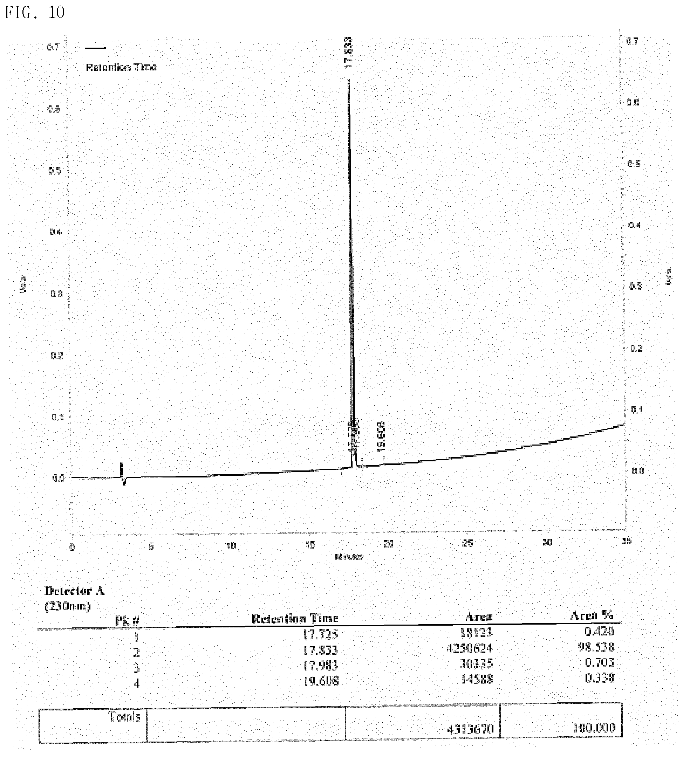

The purity and molecular weight of the HKFY-10 are shown in FIGs. 10 and 46, respectively.

### Example 1.11. Preparation of HKFY-11

His(2-cyclohexylethyl)-Lys(caprylic acid)-Phe-Tyr (N2-(Nt-(2-cyclohexylethyl)-L-histidyl)-N6-octanoyl-L-lysyl-L-phenylalanyl-L-tyrosine) was prepared in the same manner except that Boc-His(2-cyclohexylethyl)-OH was used instead of Boc-His(trt)-OH obtained during the preparation process of Example 1.1.

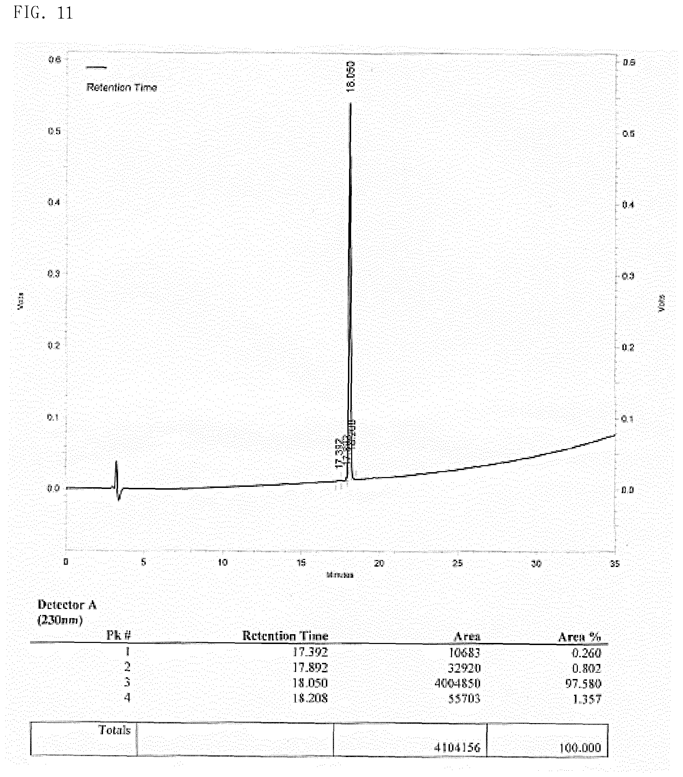

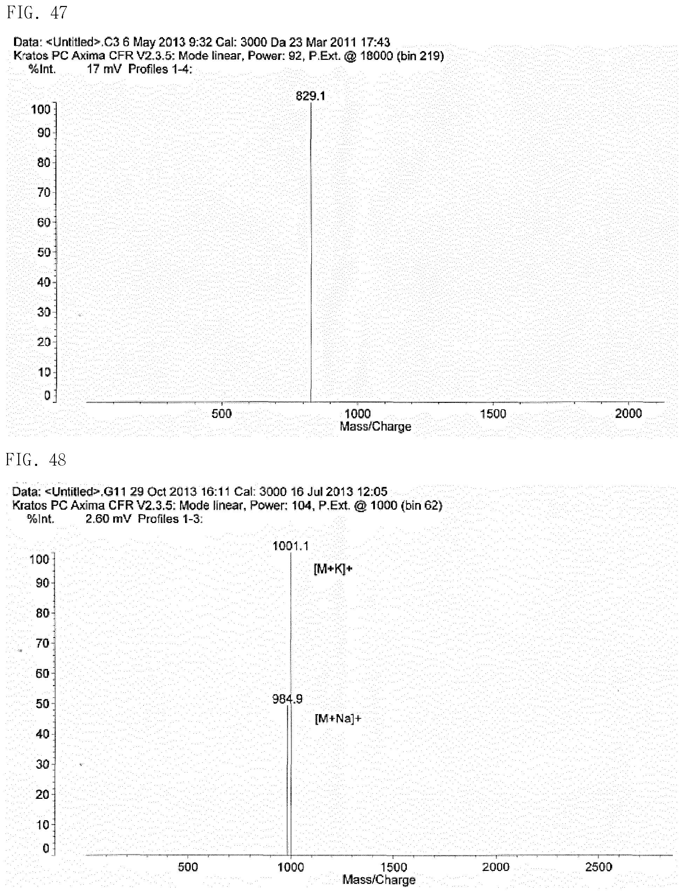

The purity and molecular weight of the HKFY-11 are shown in FIGs. 11 and 47, respectively.

### Example 1.12. Preparation of HKFY-12

In order to prepare His(trt)-Lys(caprylic)-d-Phe-Tyr (N6-decanoyl-N2-(Nt-trityl-L-histidyl)-L-lysyl-D-phenylalanyl-L-tyrosine, HKFY-12), Fmoc-Tyr(tBu) and DMF (dimethylformamide) were loaded onto the trityl resin to prepare Fmoc-Tyr(tBu)-trityl resin. DMF containing 20% piperidine and Fmoc-d-Phe-OH of Formula 8 and HOBt (hydroxyl-benzo triazole) were added to Fmoc-Tyr(tBu)-trityl resin to prepare Fmoc-d-Phe-Tyr(tBu)-trityl resin.

Fmoc-Lys(caprylic acid)-OH and HOBt (hydroxyl-benzo triazole) were added to the Fmoc-d-Phe-Tyr(tBu)-trityl resin to prepare Fmoc-Lys(caprylic acid)-d-Phe-Tyr(tBu)-trityl resin. DMF containing 20% piperidine was added to Fmoc-Lys(caprylic acid)-d-Phe-Tyr(tBu)-trityl resin, and Boc-His(trt)-OH and HOBt (hydroxyl-benzo triazole) were added to prepare Boc-His(trt)-Lys(caprylic acid)-d-Phe-Tyr(tBu)-trityl resin.

The protecting group was cleaved from the Boc-His(trt)-Lys(caprylic acid)-d-Phe-Tyr(tBu)-trityl resin and then purified to prepare His(trt)-Lys(caprylic)-d-Phe-Tyr.

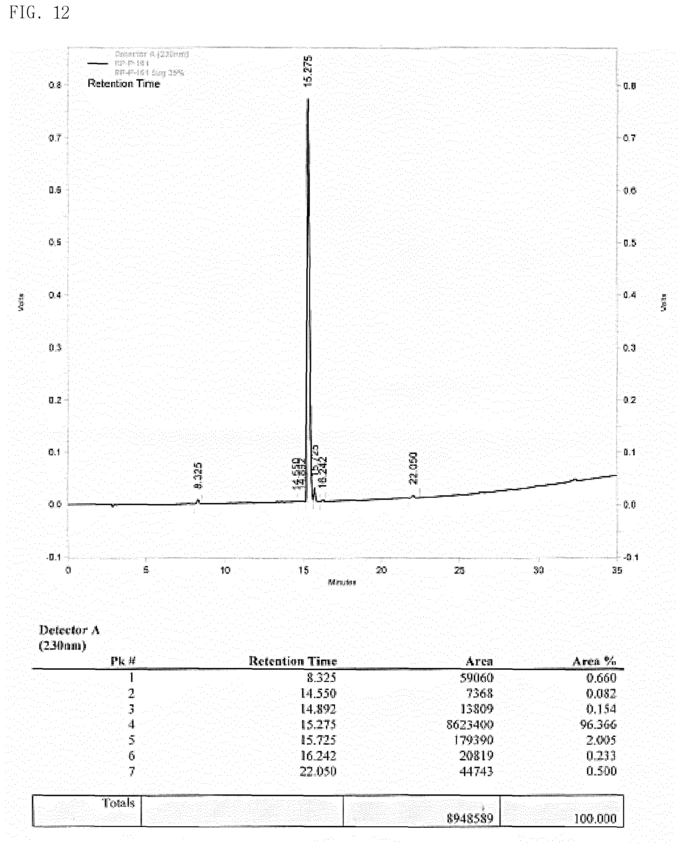

The purity and molecular weight of the HKFY-12 are shown in FIGs. 12 and 48, respectively.

### Example 1.13. Preparation of HKFY-13

His(trt)-Lys(caprylic)-d-Phe(4-Cl)-Tyr (((R)-2-((S)-2-((S)-2-amino-3-(1-trityl-1H-imidazol-4-yl)propanamido)-6-(non-1-en-2-ylamino)hexanamido)-3-(4-chlorophenyl)propanoyl)-L-tyrosine, HKFY-13) was prepared in the same manner except that Fmoc-d-Phe(4-Cl)-OH was used instead of Fmoc-d-Phe-OH obtained during the preparation process of Example 1.6.

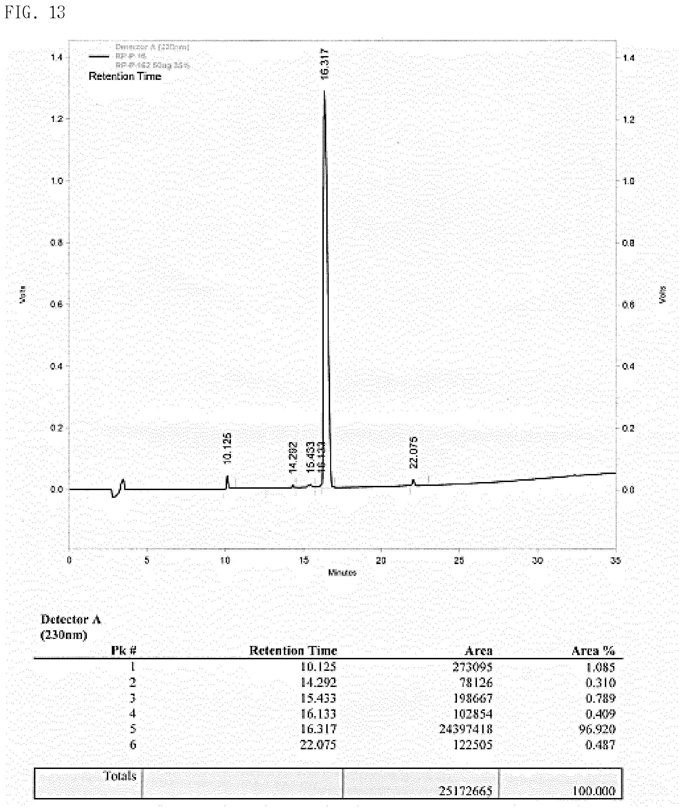

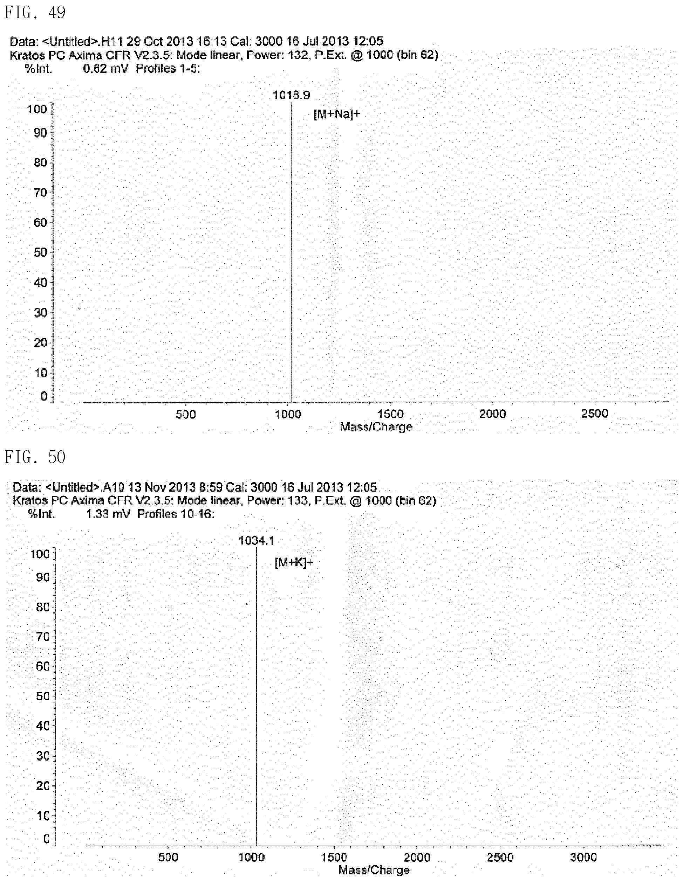

The purity and molecular weight of the HKFY-13 are shown in FIGs. 13 and 49, respectively.

### Example 1.14. Preparation of HKFY-14

Fmoc-d-Tyr(tBu)-trityl resin was prepared in the same manner except that Fmoc-d-Tyr(tBu)-OH was used instead of Fmoc-Tyr(tBu)-OH obtained during the preparation process of Example 1.13. During the subsequent process, Boc-His(trt)-OH was changed to Boc-d-His(trt)-OH to prepare d-His(trt)-Lys(caprylic)-d-Phe(4-Cl)-d-Tyr (((R)-2-((S)-2-((R)-2-amino-3-(1-trityl-1H-imidazol-4-yl)propanamido)-6-octanamidohexanamido)-3-(4-chlorophenyl)propanoyl)-D-tyrosine).

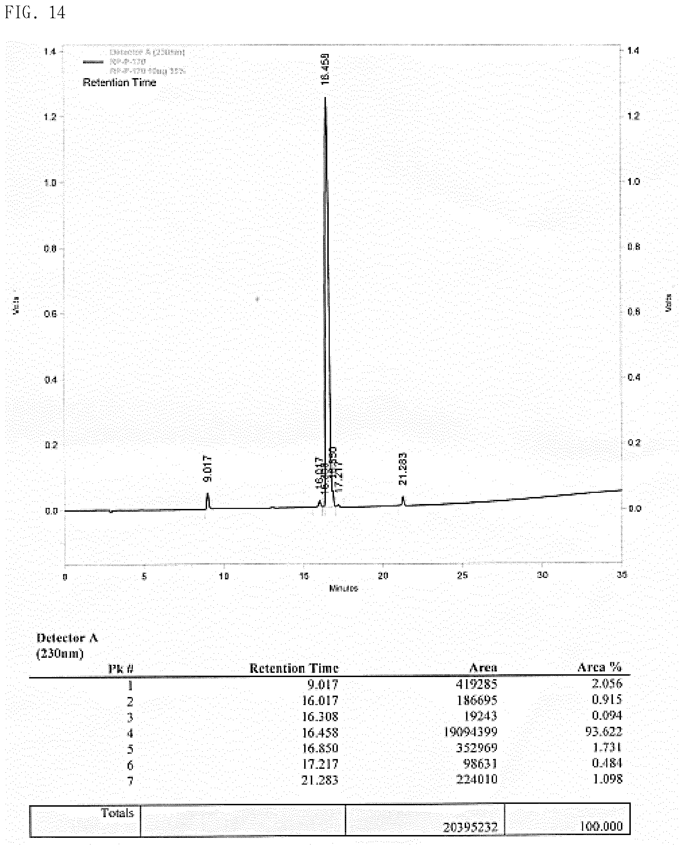

The purity and molecular weight of the HKFY-14 are shown in FIGs. 14 and 50, respectively.

### Example 1.15. Preparation of HKFY-15

His(trt)-Lys(caprylic)-d-Phe(4-Cl)-d-Tyr (((R)-2-(T(S)-2-((S)-2-amino-3-(1-trityl-1H-imidazol-4-yl)propanamido)-6-octanamidohexanamido)-3-(4-chlorophenyl)propanoyl)-D-tyrosine) was prepared in the same manner except that Boc-His(trt)-OH was used instead of Boc-d-His(trt)-OH obtained during the preparation process of Example 1.14.

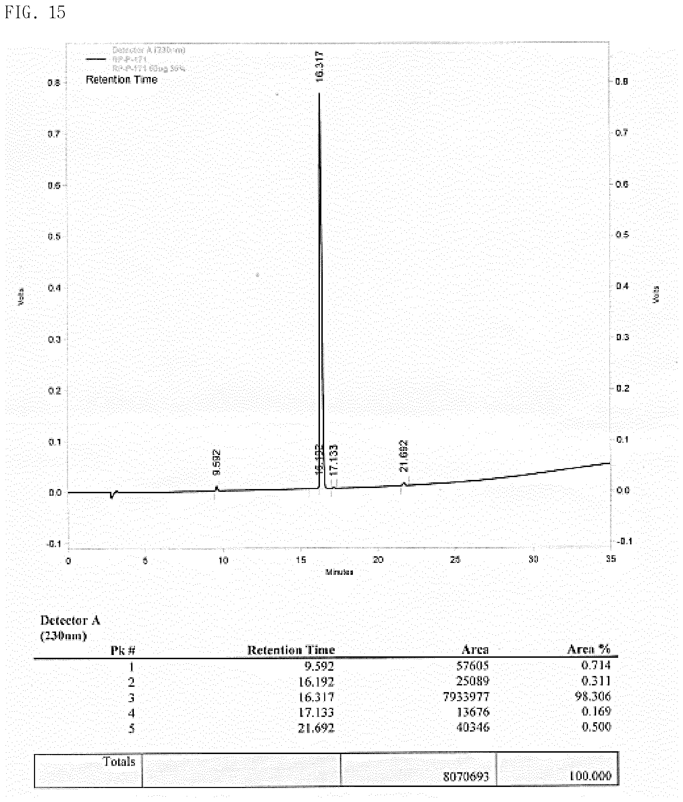

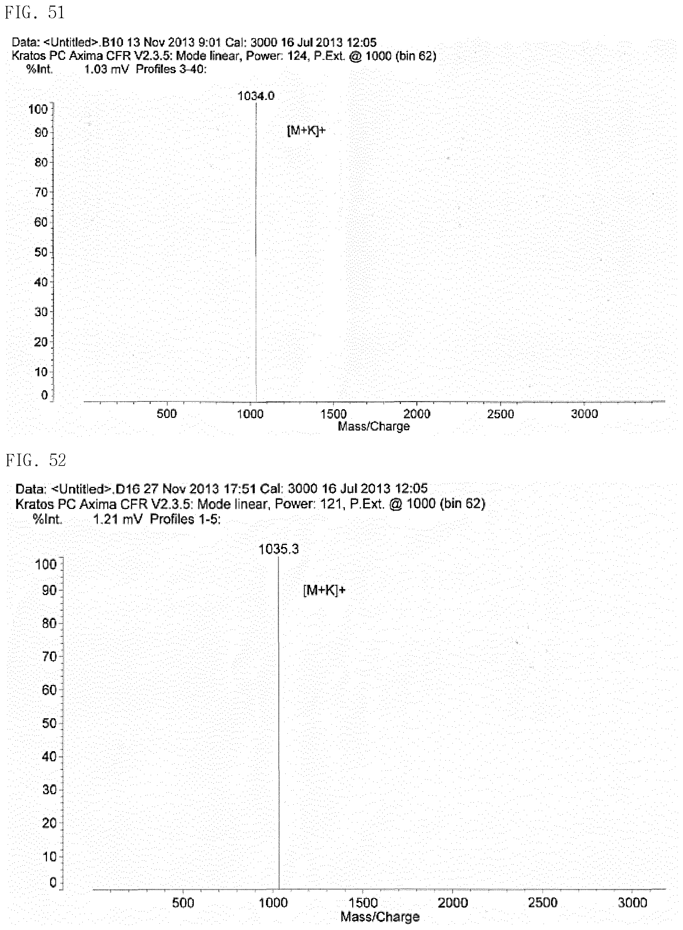

The purity and molecular weight of the HKFY-15 are shown in FIGs. 15 and 51, respectively.

### Example 1.16. Preparation of HKFY-16

His(trt)-d-Lys(caprylic)-d-Phe(4-Cl)-Tyr (((R)-2-(T(R)-2-(S)-2-amino-3-(1-trityl-1H-imidazol-4-yl)propanamido)-6-octanamidohexanamido)-3-(4-chlorophenyl)propanoyl)-L-tyrosine) was prepared in the same manner except that Fmoc-d-Lys(caprylic)-OH was used by replacing Fmoc-Lys(dde)-OH with Fmoc-d-Lys(dde)-OH in the preparation process using Fmoc-Lys(caprylic)-OH instead of Fmoc-Lys(dde)-OH obtained during the preparation process of Example 1.13.

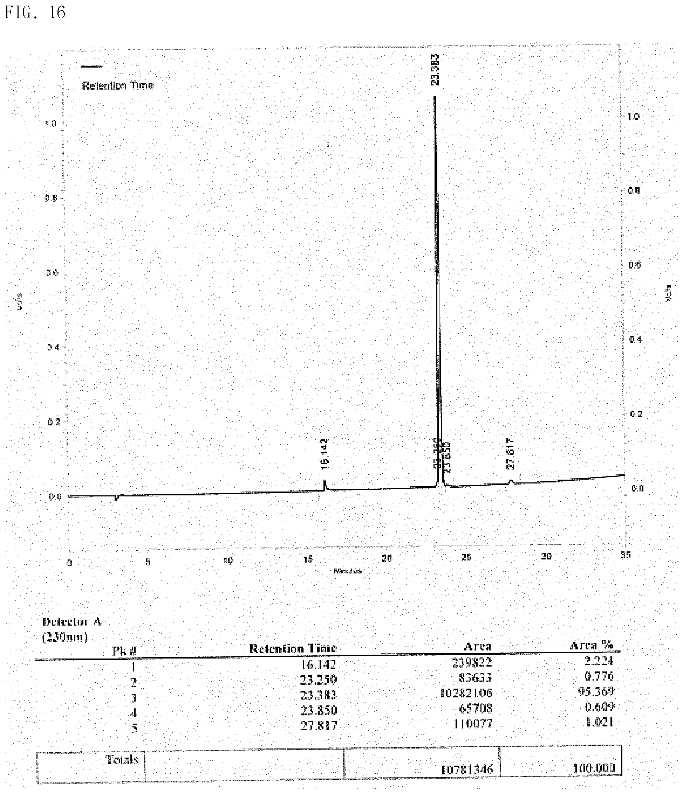

The purity and molecular weight of the HKFY-16 are shown in FIGs. 16 and 52, respectively.

### Example 1.17. Preparation of HKFY-17

His(3,3-diphenylethyl)-Lys(caprylic acid)-Phe-Tyr (N2-(Nt-(2,2-diphenylethyl)-L-histidyl)-N6-octanoyl-L-lysyl-L-phenylalanyl-L-tyrosine) was prepared in the same manner except that Boc-His(3,3-diphenylethyl)-OH was used instead of Boc-His(trt)-OH obtained during the preparation process of Example 1.1.

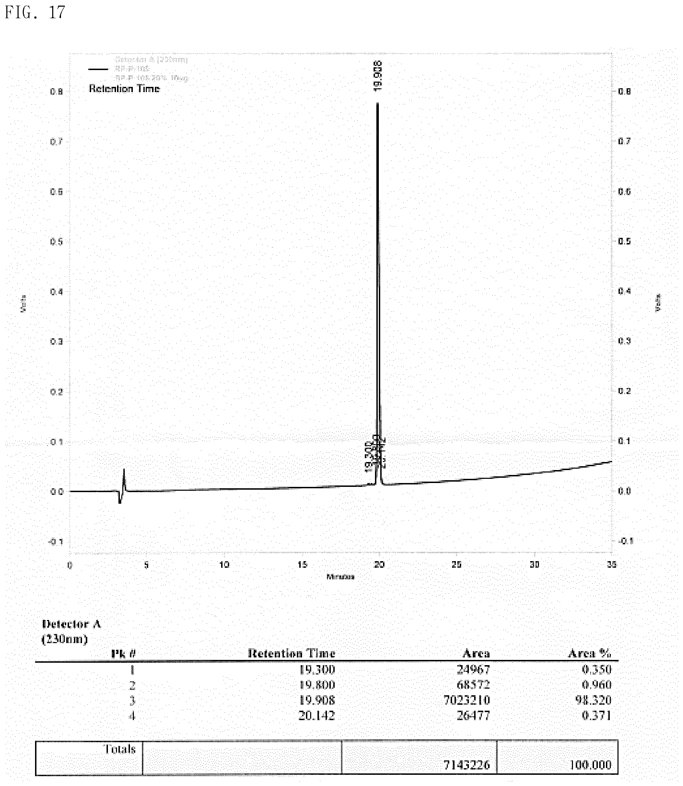

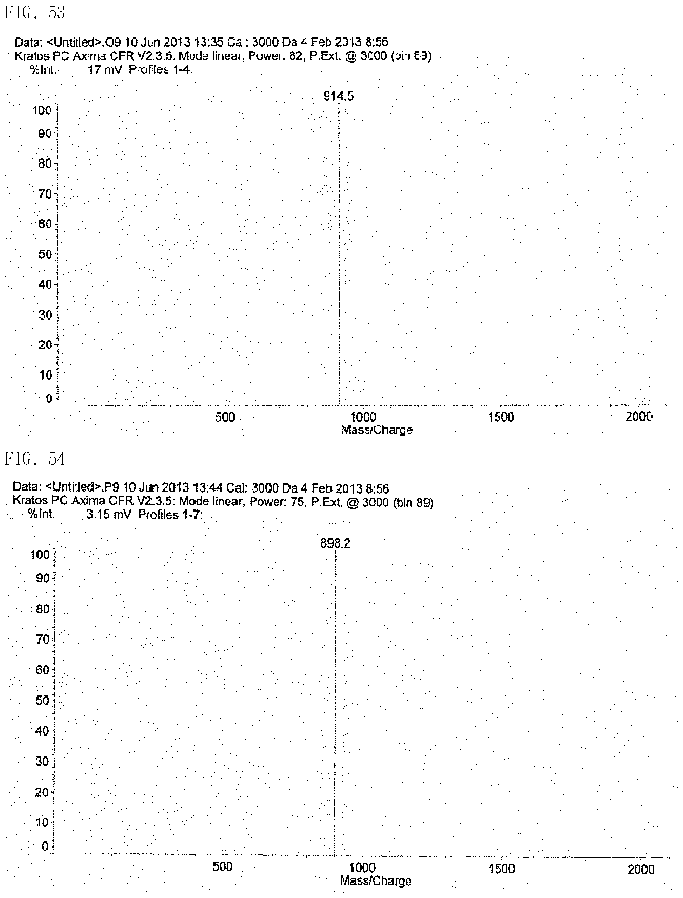

The purity and molecular weight of the HKFY-17 are shown in FIGs. 17 and 53, respectively.

### Example 1.18. Preparation of HKFY-18

His(Fm)-Lys(caprylic acid)-Phe-Tyr (N2-(Nt-(9H-fluoren-9-yl)-L-histidyl)-N6-octanoyl-L-lysyl-L-phenylalanyl-L-tyrosine) was prepared in the same manner except that Boc-His(Fm)-OH was used instead of Boc-His(trt)-OH obtained during the preparation process of Example 1.1.

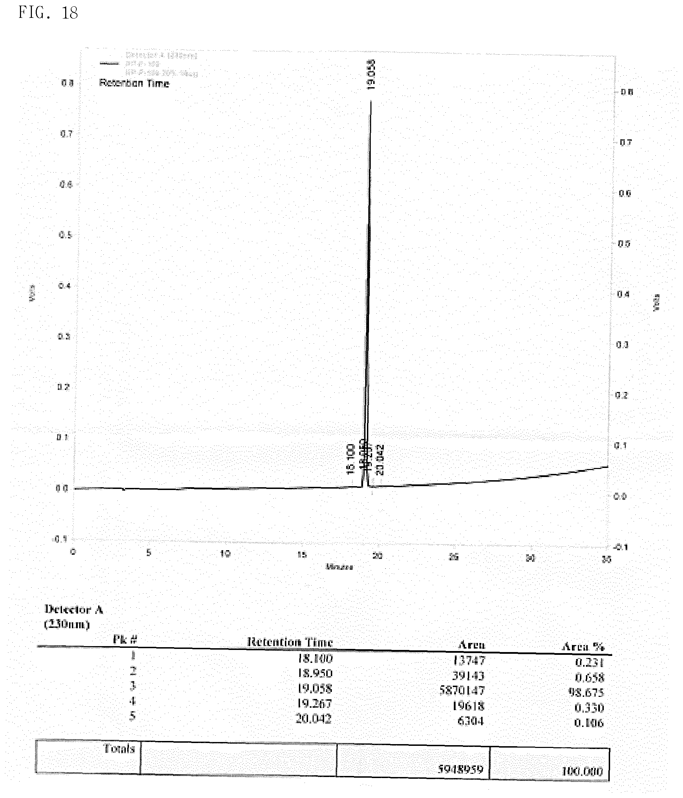

The purity and molecular weight of the HKFY-18 are shown in FIGs. 18 and 54, respectively.

### Example 1.19. Preparation of HKFY-19

His(phenylethyl)-Lys(caprylic acid)-Phe-Tyr (N6-octanoyl-N2-(Nt-phenethyl-L-histidyl)-L-lysyl-L-phenylalanyl-L-tyrosine) was prepared in the same manner except that Boc-His(phenylethyl)-OH was used instead of Boc-His(trt)-OH obtained during the preparation process of Example 1.1.

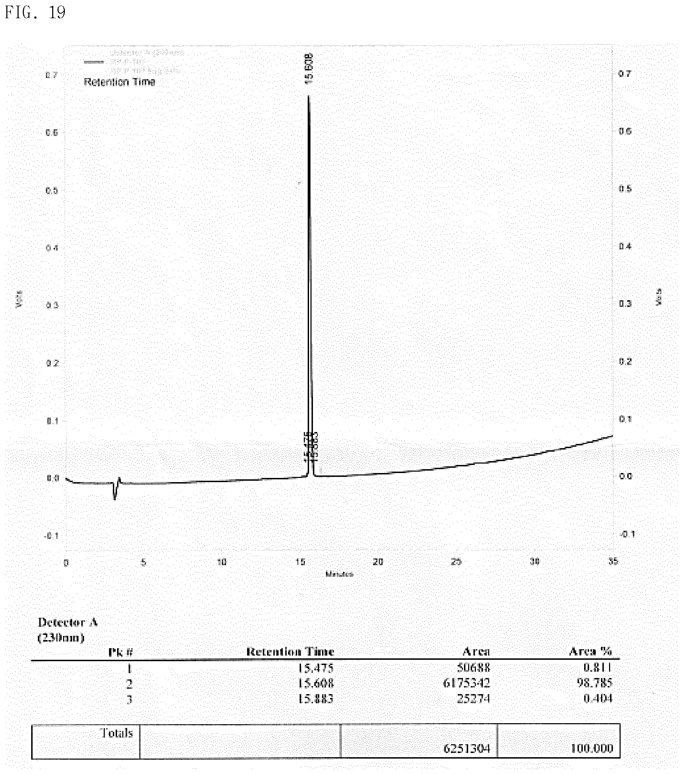

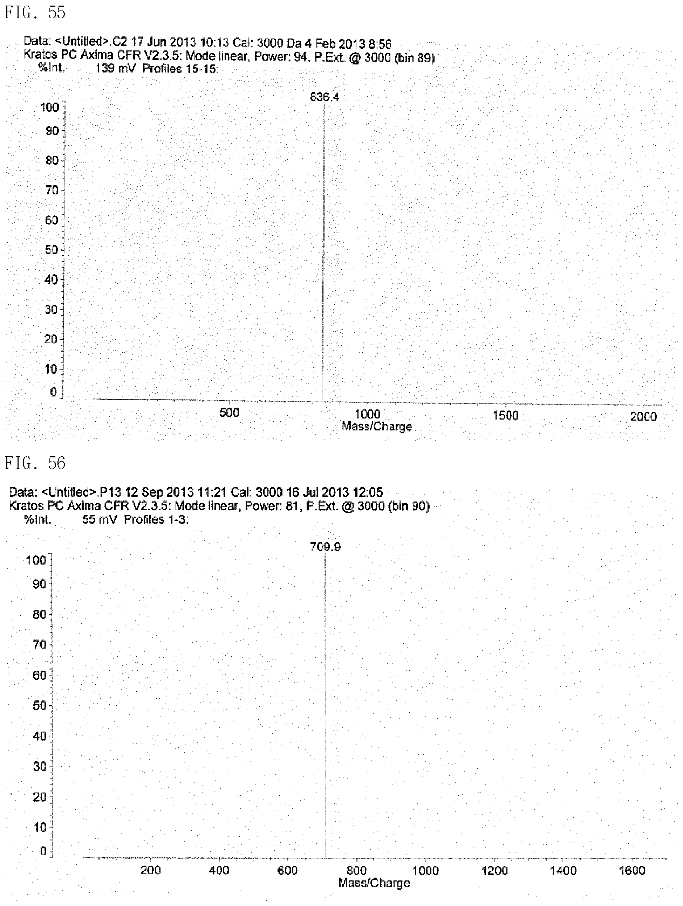

The purity and molecular weight of the HKFY-19 are shown in FIGs. 19 and 55, respectively.

### Example 1.20. Preparation of HKFY-20

His(methoxylbenzhydryl)-Lys(caprylic acid)-Phe-Tyr (N2-(Nt-( (4-methoxyphenyl)(phenyl)methyl)-L-histidyl)-N6-octanoyl-L-lysyl-L-phenylalanyl-L-tyrosine) was prepared in the same manner except that Boc-His(methoxylbenzhydryl)-OH was used instead of Boc-His(trt)-OH obtained during the preparation process of Example 1.1.

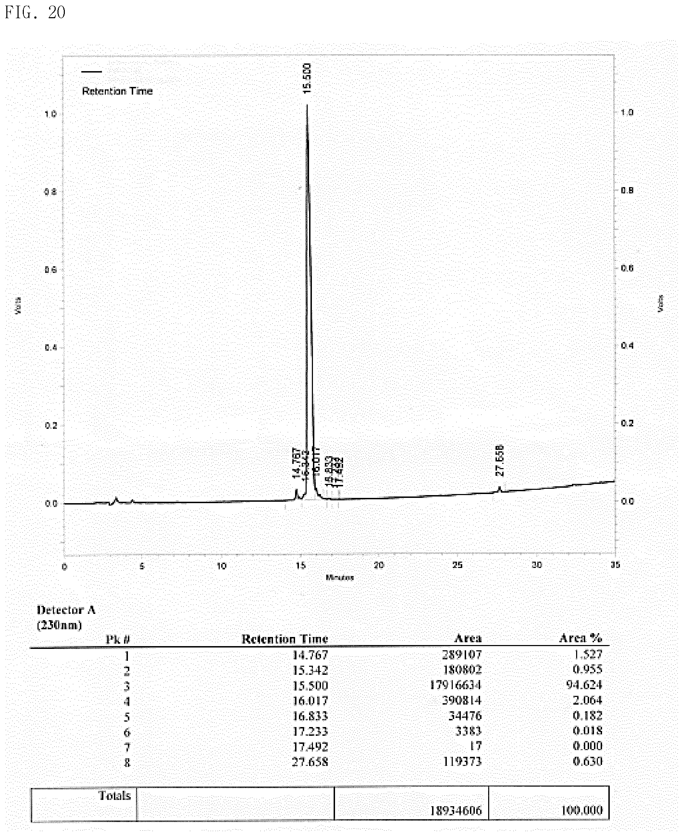

The purity and molecular weight of the HKFY-20 are shown in FIGs. 20 and 56, respectively.

### Example 1.21. Preparation of HKFY-21

His(4-chlorobenzhydryl)-Lys(caprylic acid)-Phe-Tyr (N2-(Nt-((R)-(4-chlorophenyl)(phenyl)methyl)-L-histidyl)-N6-octanoyl-L-lysyl-L-phenylalanyl-L-tyrosine) was prepared in the same manner except that Boc-His(4-chlorobenzhydryl)-OH was used instead of Boc-His(trt)-OH obtained during the preparation process of Example 1.1.

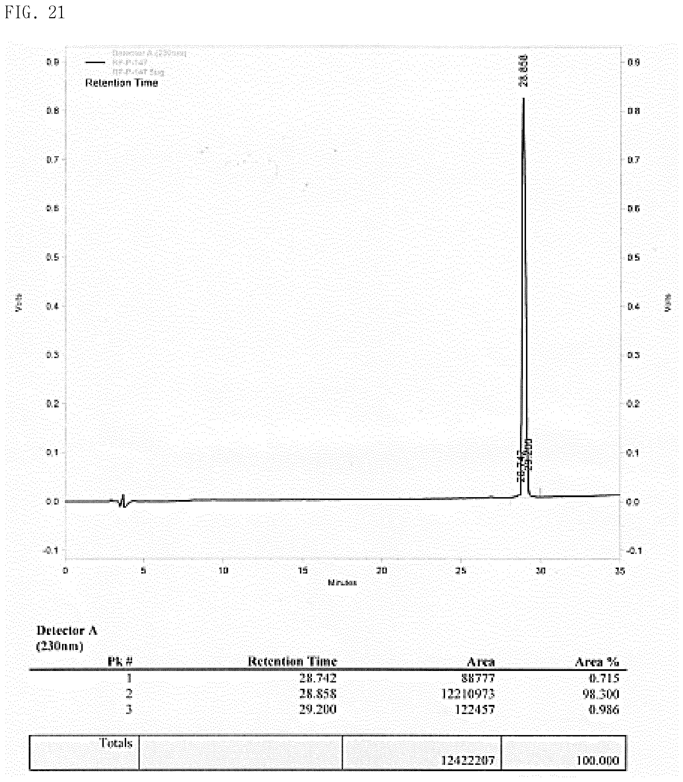

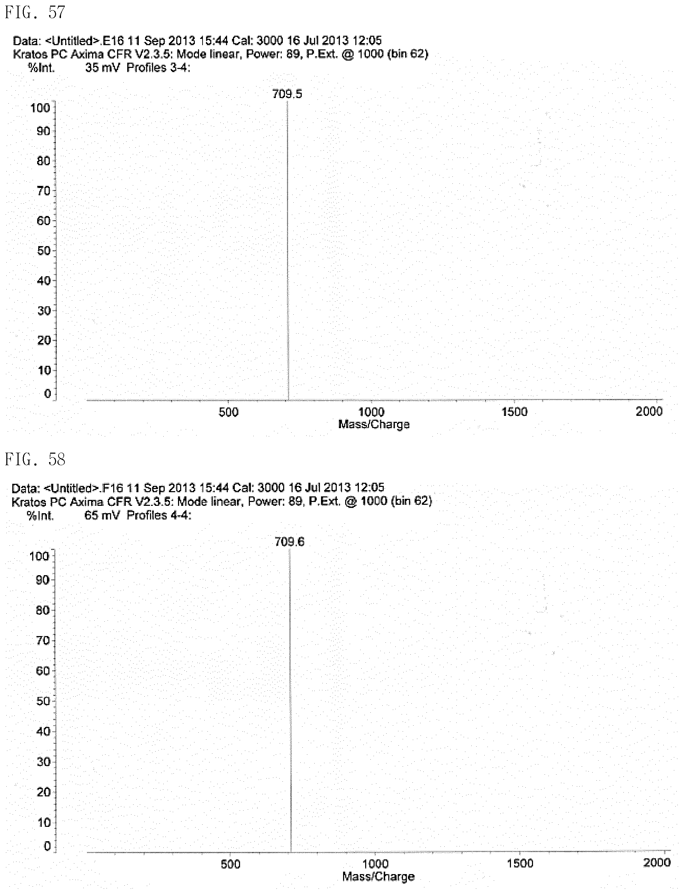

The purity and molecular weight of the HKFY-21 are shown in FIGs. 21 and 57, respectively.

### Example 1.22. Preparation of HKFY-22

His(methylbenzhydryl)-Lys(caprylic acid)-Phe-Tyr (N6-octanoyl-N2-(Nt-((R)-phenyl (p-tolyl)methyl)-L-histidyl)-L-lysyl-L-phenylalanyl-L-tyrosine) was prepared in the same manner except that Boc-His(methylbenzhydryl)-OH was used instead of Boc-His(trt)-OH obtained during the preparation process of Example 1.1.

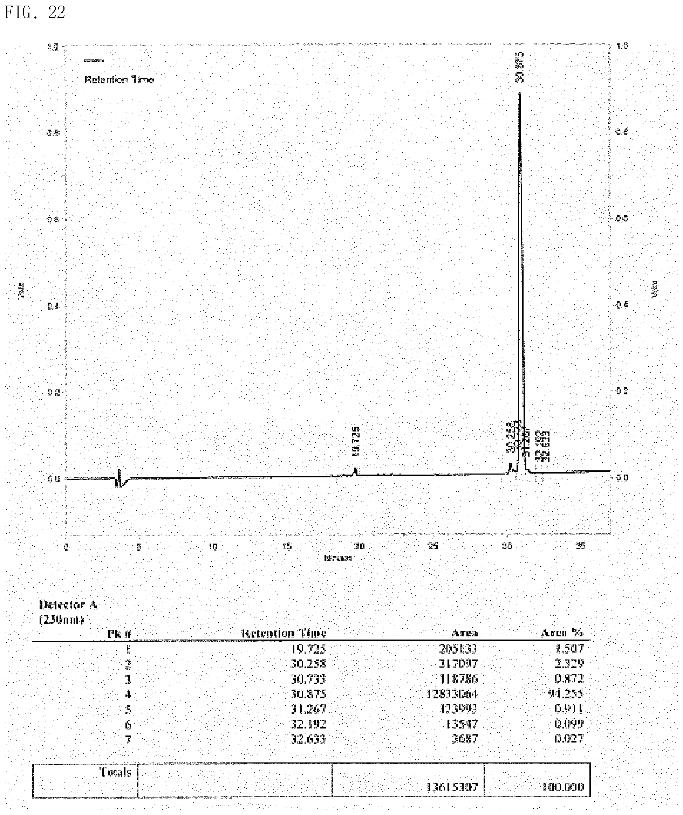

The purity and molecular weight of the HKFY-22 are shown in FIGs. 22 and 58, respectively.

### Example 1.23. Preparation of HKFY-23

His(adamantan-1-yl)-Lys(caprylic acid)-Phe-Tyr (N2-(Nt-(adamantan-1-yl)-L-histidyl)-N6-octanoyl-L-lysyl-L-phenylalanyl-L-tyrosine) was prepared in the same manner except that Boc-His(adamantan-1-yl)-OH was used instead of Boc-His(trt)-OH obtained during the preparation process of Example 1.1.

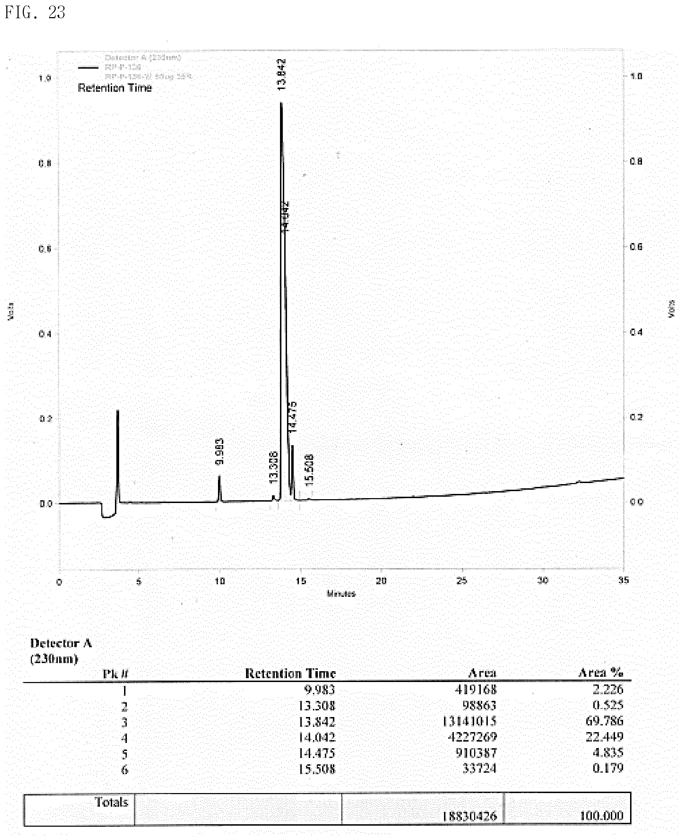

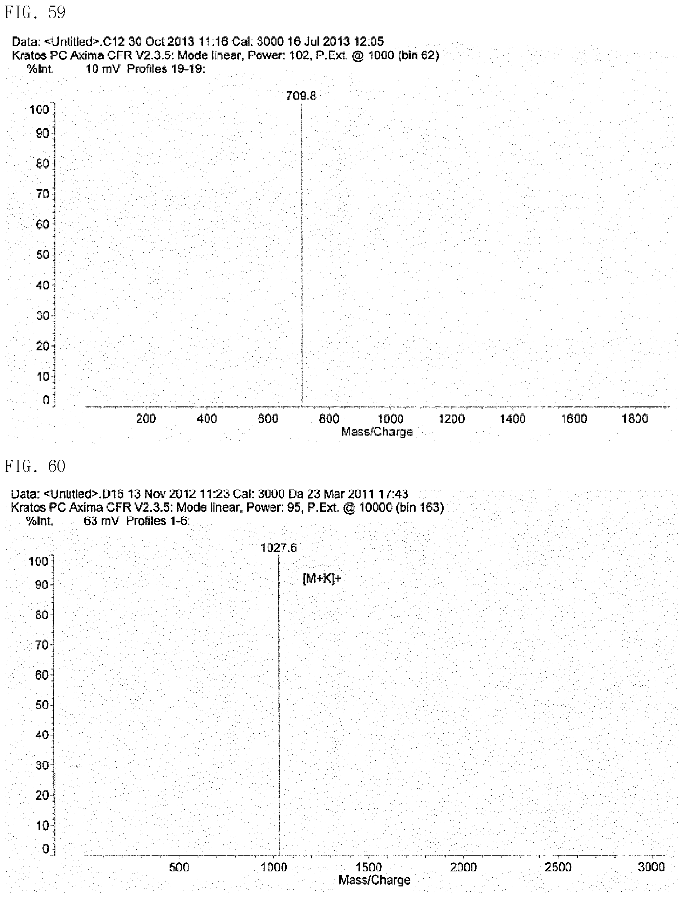

The purity and molecular weight of the HKFY-23 are shown in FIGs. 23 and 59, respectively.

### Example 1.24. Preparation of HKFY-24

His(trt)-Lys(capric)-Phe-Tyr (N6-decanoyl-N2-(tNLt-trityl-L-histidyl)-L-lysyl-L-phenylalanyl-L-tyrosine, HKFY-24) was prepared in the same manner except that capric acid instead of caprylic acid was added to Fmoc-Lys-OH obtained during the preparation process of Example 1.1.

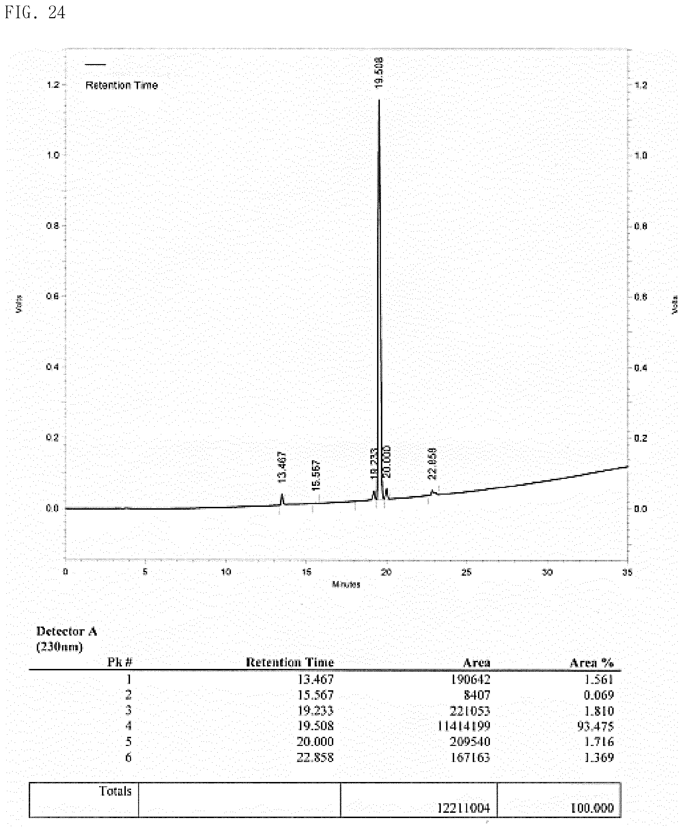

The purity and molecular weight of the HKFY-24 are shown in FIGs. 24 and 60, respectively.

### Example 1.25. Preparation of HKFY-25

His(trt)-Lys(lauric)-Phe-Tyr (N6-dodecanoyl-N2-(Nt-trityl-L-histidyl)-L-lysyl-L-phenylalanyl-L-tyrosine, HKFY-25) was prepared in the same manner except that lauric acid instead of caprylic acid was added to Fmoc-Lys-OH obtained during the preparation process of Example 1.1.

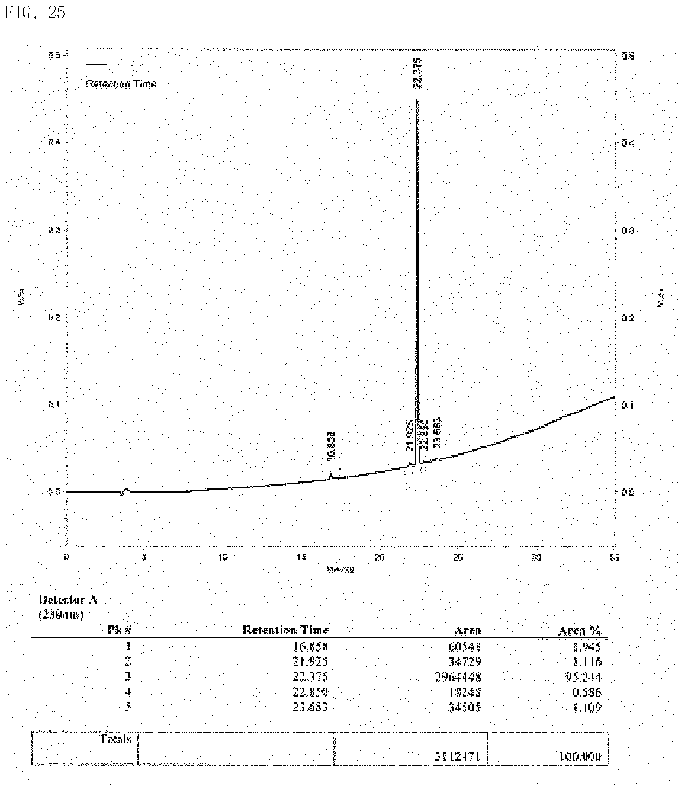

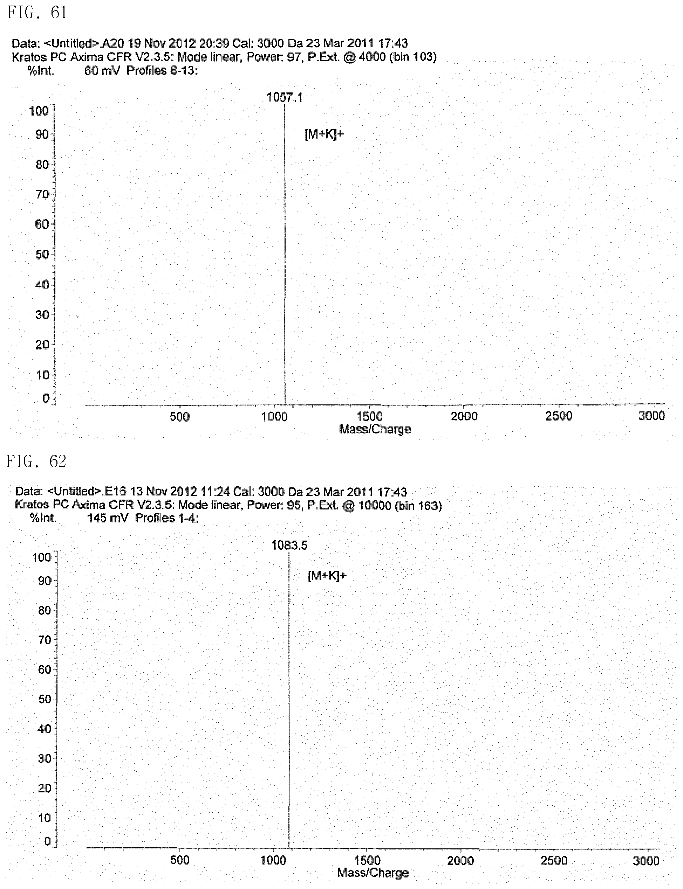

The purity and molecular weight of the HKFY-25 are shown in FIGs. 25 and 61, respectively.

### Example 1.26. Preparation of HKFY-26

His(trt)-Lys(myristic)-Phe-Tyr (N6-tetradecanoyl- N2-(Nt-trityl-L-histidyl)-L-lysyl-L-phenylalanyl-L-tyrosine, HKFY-26) was prepared in the same manner except that myristic acid instead of caprylic acid was added to Fmoc-Lys-OH obtained during the preparation process of Example 1.1.

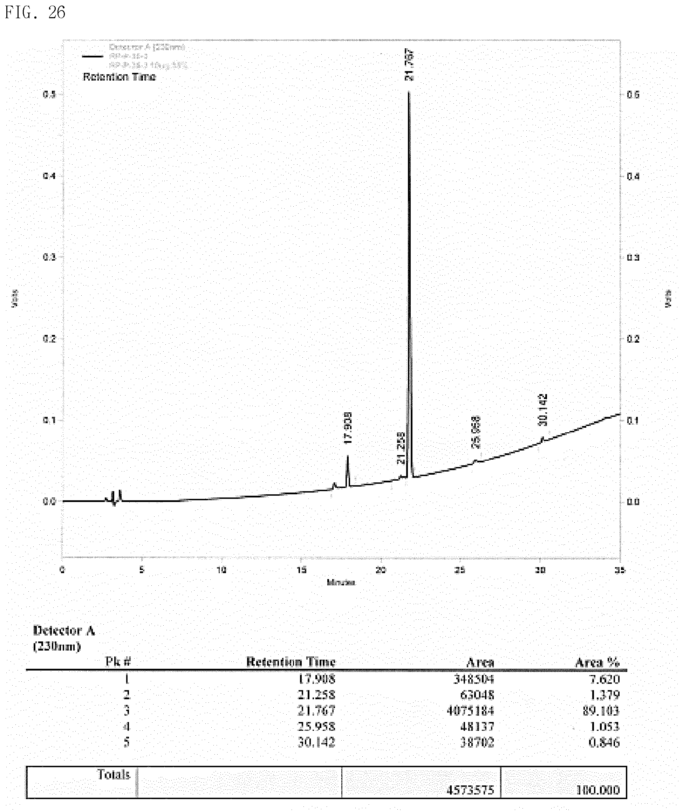

The purity and molecular weight of the HKFY-26 are shown in FIGs. 26 and 62, respectively.

### Example 1.27. Preparation of HKFY-27

His(trt)-Lys(palmitic)-Phe-Tyr (N6-palmitoyl-N2-(tNLt-triiyl-L-histidyl)-L-lysyl-L-phenylalanyl-L-tyrosine, HKFY-27) was prepared in the same manner except that palmitic acid instead of caprylic acid was added to Fmoc-Lys-OH obtained during the preparation process of Example 1.1.

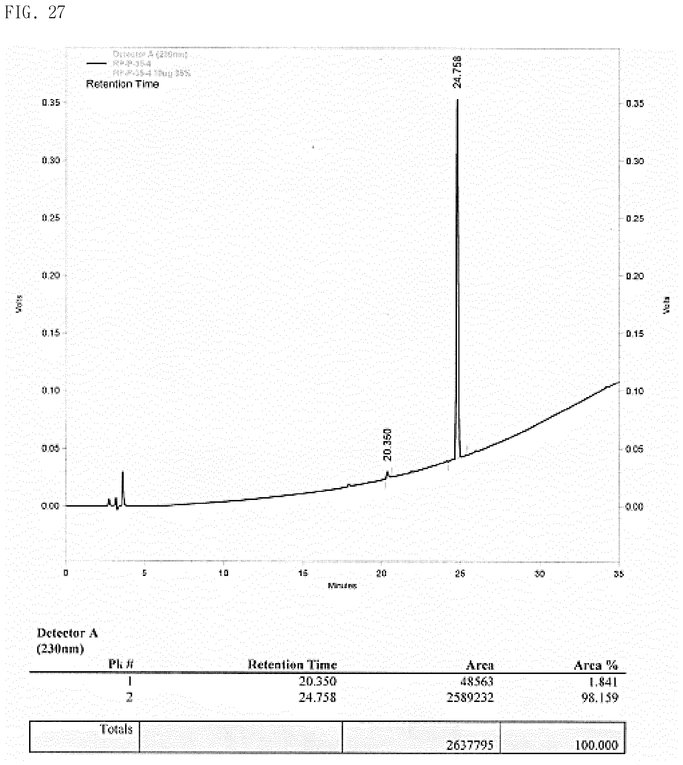

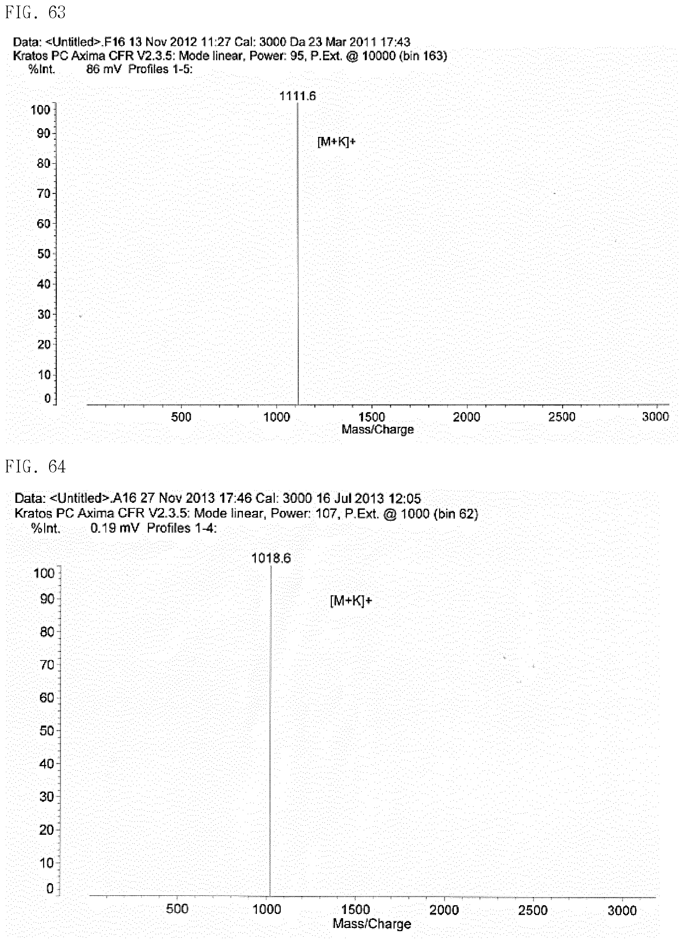

The purity and molecular weight of the HKFY-27 are shown in FIGs. 27 and 63, respectively.

### Example 1.28. Preparation of HKFY-28

His(trt)-Lys(caprylic)-d-Phe(4-F)-Tyr (((R)-2-((S)-2-((S)-2-amino-3-(1-trityl-1H-imidazol-4-yl)propanamido)-6-octanamidohexanamido)-3-(4-fluorophenyl)propanoyl)-L-tyrosine, HKFY-28) was prepared in the same manner except that Fmoc-d-Phe(4-F)-OH was used instead of Fmoc-d-Phe-OH obtained during the preparation process of Example 1.6.

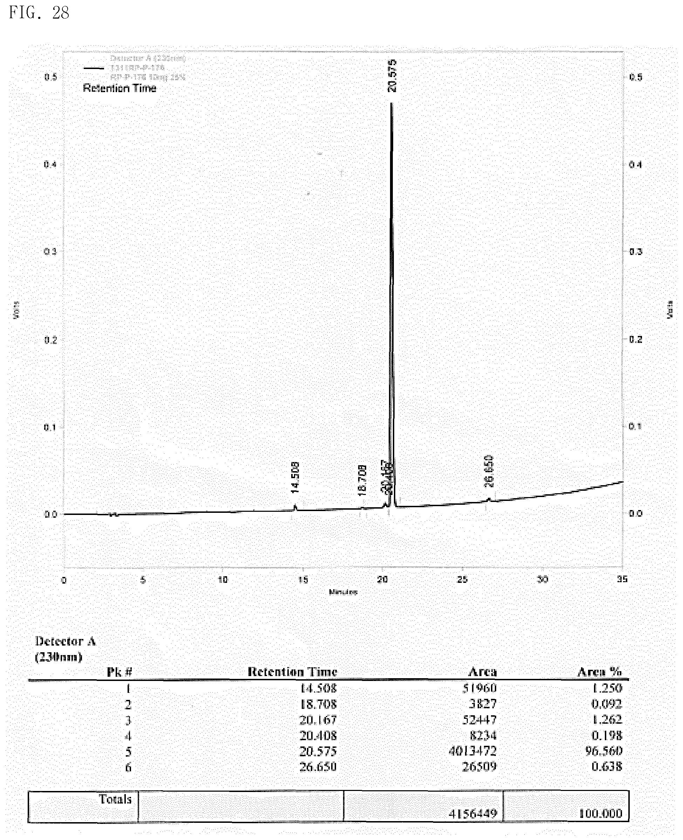

The purity and molecular weight of the HKFY-28 are shown in FIGs. 28 and 64, respectively.

### Example 1.29. Preparation of HKFY-29

His(trt)-Lys(caprylic)-d-Phe(4-Br)-Tyr (((R)-2-((S)-2-((S)-2-amino-3-(1-trityl-1H-imidazol-4-yl)propanamido)-6-octanamidohexanamido)-3-(4-bromophenyl)propanoyl)-L-tyrosine, HKFY-29) was prepared in the same manner except that Fmoc-d-Phe(4-Br)-OH was used instead of Fmoc-d-Phe-OH obtained during the preparation process of Example 1.6.

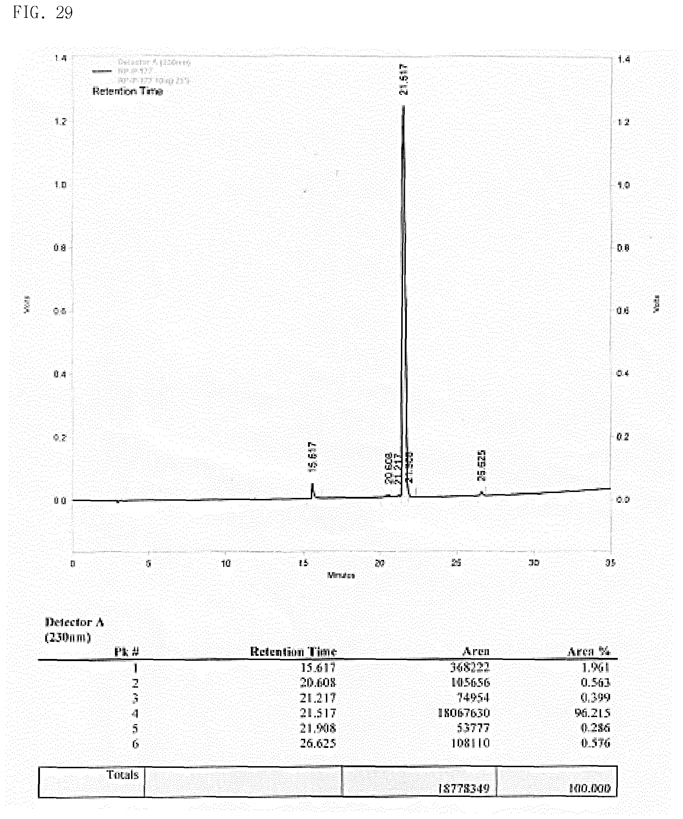

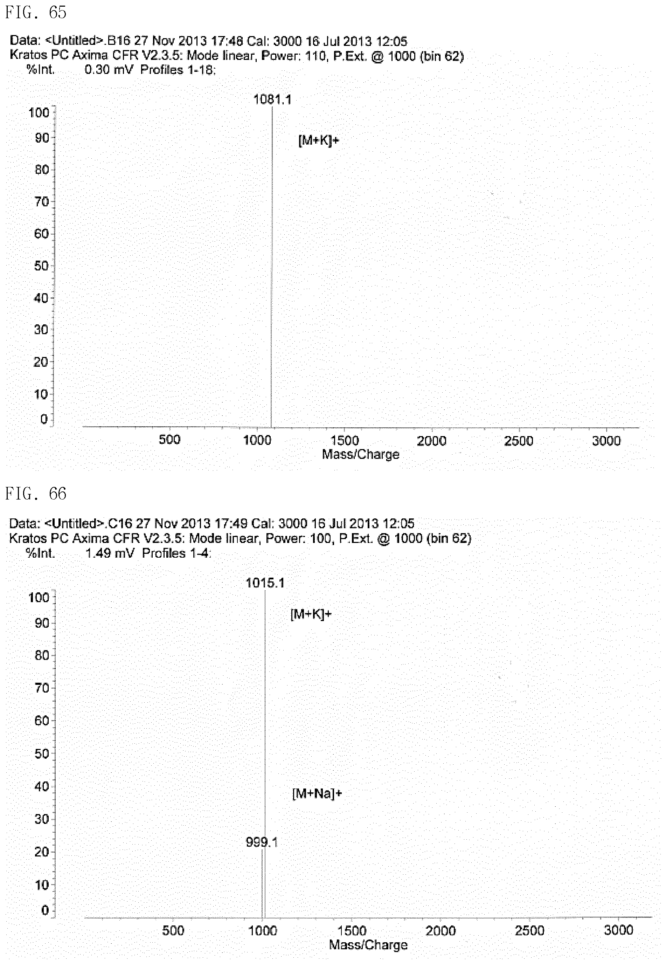

The purity and molecular weight of the HKFY-29 are shown in FIGs. 29 and 65, respectively.

### Example 1.30. Preparation of HKFY-30

His(trt)-Lys(caprylic)-d-Phe(4-methyl)-Tyr (((R)-2-((S)-2-((S)-2-amino-3-(1-trityl-1H-imidazol-4-yl)propanamido)-6-octanamidohexanamil)propanoyl)-L-tyrosine, HKFY-30) was prepared in the same manner except that Fmoc-d-Phe(4-methyl)-OH was used instead of Fmoc-d-Phe-OH obtained during the preparation process of Example 1.6.

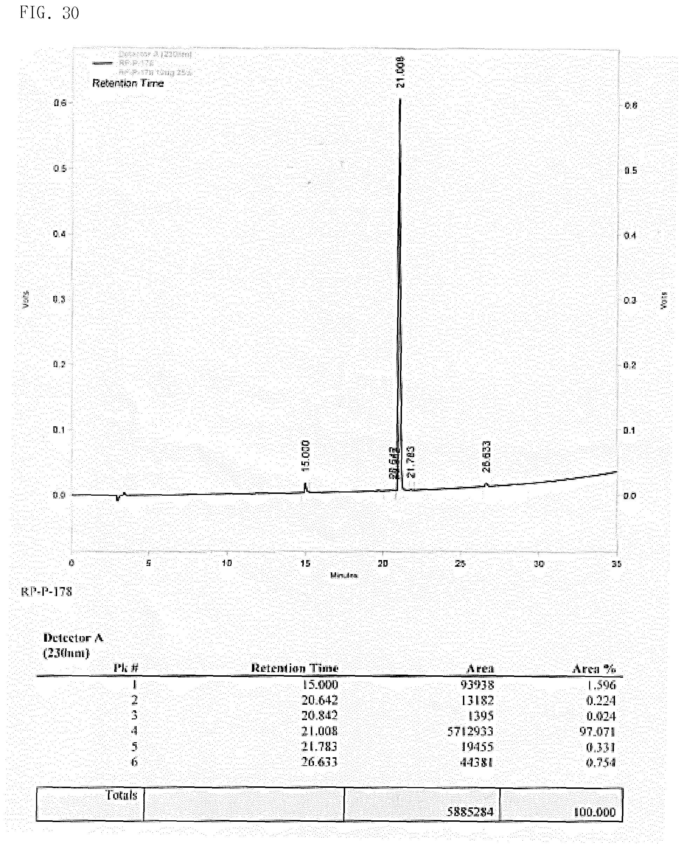

The purity and molecular weight of the HKFY-30 are shown in FIGs. 30 and 66, respectively.

### Example 1.31. Preparation of HKFY-31

d-His(trt)-Lys(caprylic)-d-Phe(4-methyl)-Tyr ((R)-2-((S)-2-((R)-2-amino-3-(1-trityl-1H-imidazol-4-yl)propanamido)-6-octanamidohexanamido)-3-(p-tolyl)propanoyl)-L-tyrosine) was prepared in the same manner except that Boc-d-His(trt)-OH was used instead of Boc-His(trt)-OH obtained during the preparation process of Example 1.30.

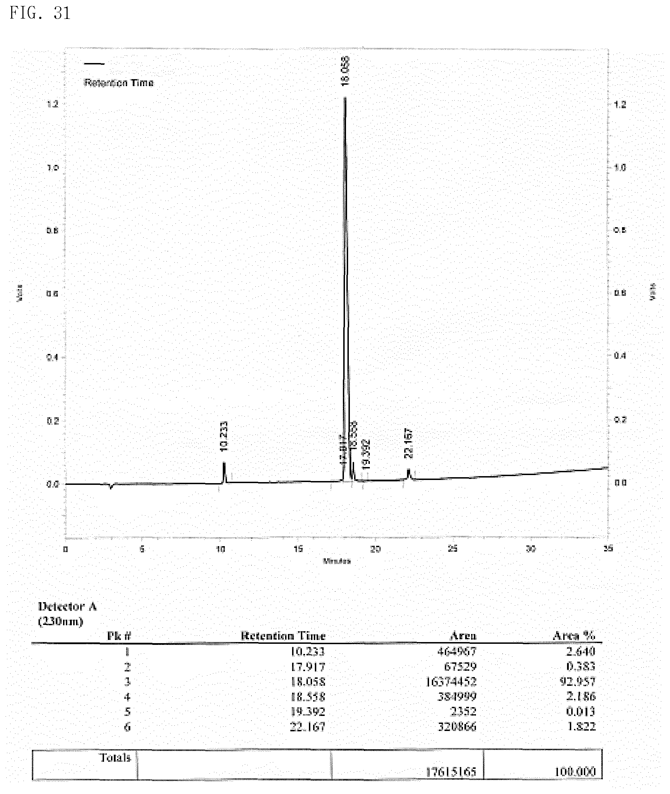

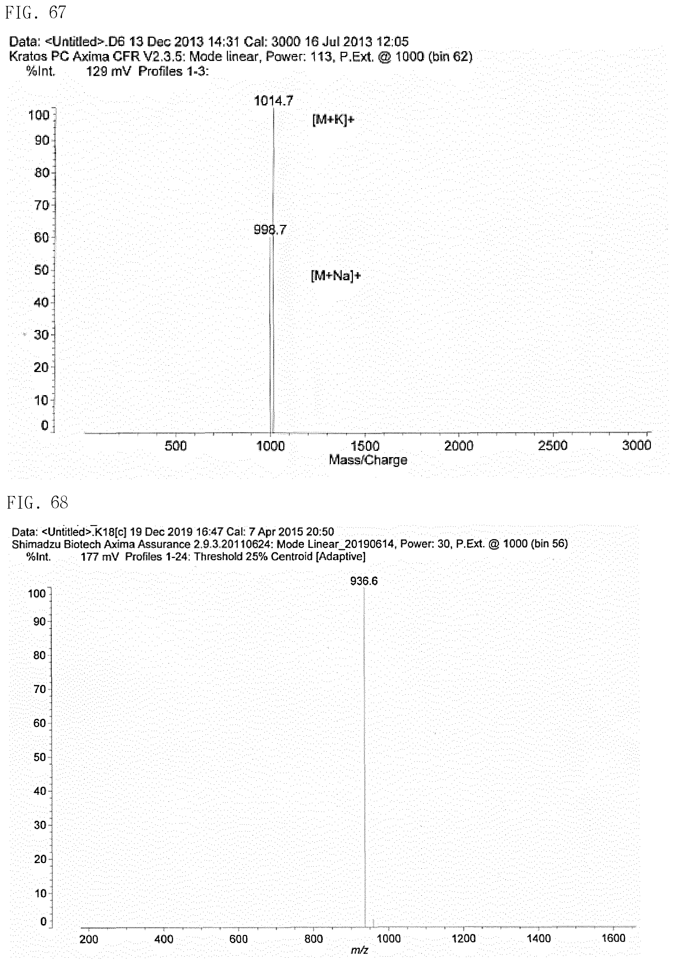

The purity and molecular weight of the HKFY-31 are shown in FIGs. 31 and 67, respectively.

### Example 1.32. Preparation of HKFY-32

His(1,3-difluorobenzhydryl)-Lys(caprylic)-d-Phe(methyl)-Tyr (((2R)-2-((2S)-2-((2S)-2-amino-3-(1-((2,4-difluorophenyl)(phenyl)methyl)-1H-imidazol-4-yl)propanamido)-6-octanamidohexanamido)-3-(p-tolyl)propanoyl)-L-tyrosine) was prepared in the same manner except that Boc-His(1,3-difluorobenzhydryl)-OH was used instead of Boc-His(benzhydryl)-OH among the constituent amino acids of Example 1.34. below.

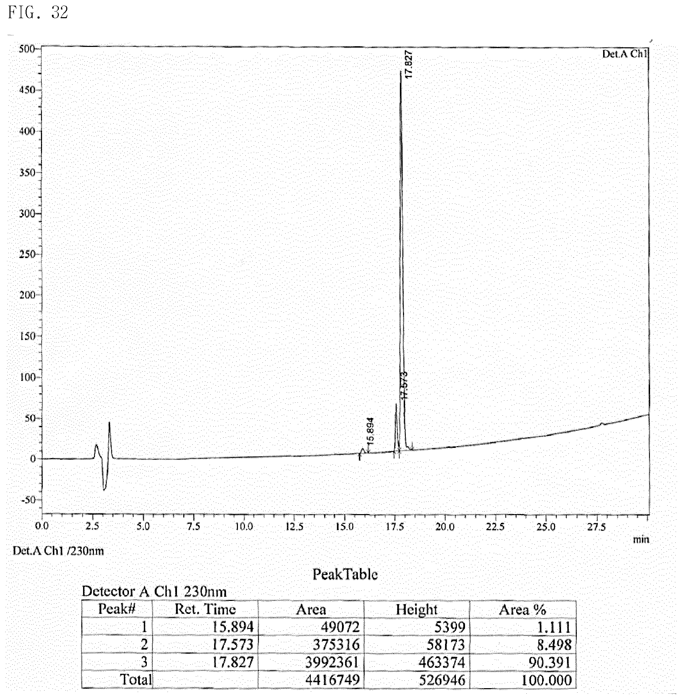

The purity and molecular weight of the HKFY-32 are shown in FIGs. 32 and 68, respectively.

### Example 1.33. Preparation of HKFY-33

His(benzhydryl)-Lys(caprylic)-Phe-Tyr (N2-(Nt-benzhydryl-L-histidyl)-N6-octanoyl-L-lysyl-L-phenylalanyl-L-tyrosine) was prepared in the same manner except that Boc-His(benzhydryl)-OH was used instead of Boc-His(trt)-OH obtained during the preparation process of Example 1.1.

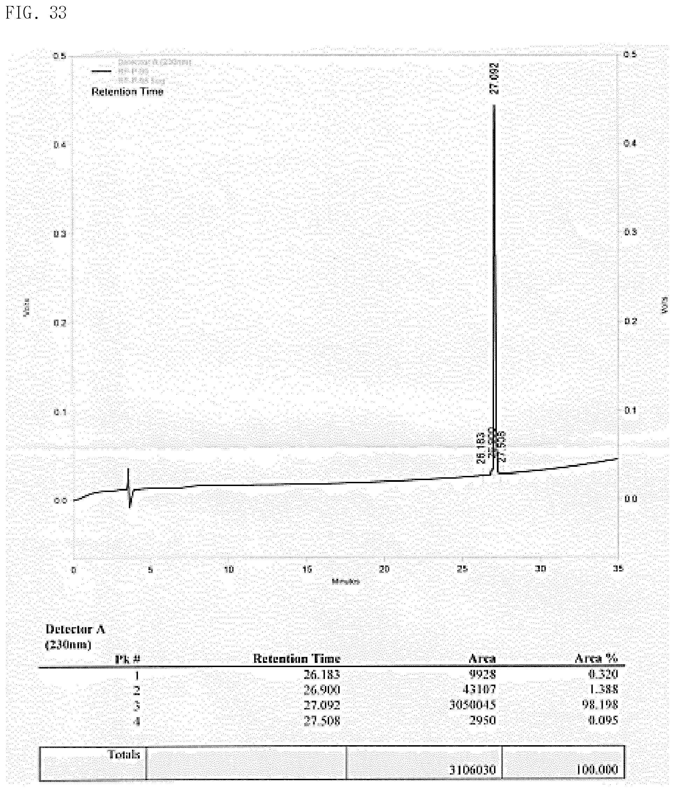

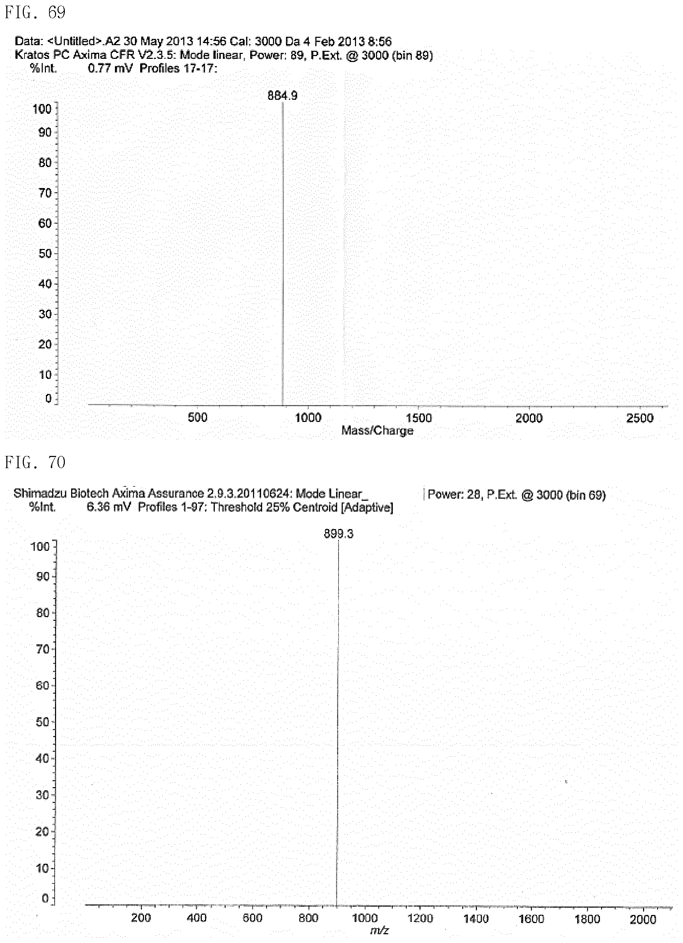

The purity and molecular weight of the HKFY-33 are shown in FIGs. 33 and 69, respectively.

### Example 1.34. Preparation of HKFY-34

In order to prepare His(benzhydryl)-Lys(caprylic)-d-Phe(4-methyl)-Tyr (((R)-2-((S)-2-((S)-2-amino-3-(1-benzhydryl-1H-imidazol-4-yl)propanamido)-6-octanamidohexanamido)-3-(p-tolyl)propanoyl)-L-tyrosine, HKFY-34), Fmoc-Tyr(tBu) and DMF (dimethylformamide) were loaded onto the trityl resin to prepare Fmoc-Tyr(tBu)-trityl resin. DMF containing 20% piperidine and Fmoc-d-Phe(4-methyl)-OH and HOBt (hydroxyl-benzo triazole) were added to Fmoc-Tyr(tBu)-trityl resin to prepare Fmoc-d-Phe(4-methyl)-Tyr(tBu)-trityl resin.

DMF containing 2% NH₂NH₂·H₂O was added to Fmoc-Lys(dde)-OH, and dde was removed to prepare Fmoc-Lys-OH. DMF containing caprylic acid, HOBt and DIC was added to the Fmoc-Lys-OH to prepare Fmoc-Lys(caprylic acid)-OH, and Boc-His(trt)-OH was used to prepare Boc-His(benzhydryl)-OH.

DMF containing 20% piperidine was added to the Fmoc-d-Phe(4-methyl)-Tyr(tBu)-trityl resin, and HOBt (hydroxyl-benzo triazole) was added to the Fmoc-Lys(caprylic acid)-OH to prepare Fmoc-Lys(caprylic acid)-d-Phe(4-methyl)-Tyr(tBu)-trityl resin. DMF containing 20% piperidine was added to Fmoc-Lys(caprylic acid)-d-Phe(4-methyl)-Tyr(tBu)-trityl resin, and Boc-His(benzhydryl)-OH and HOBt (hydroxyl-benzo triazole) were added to prepare a peptide of Boc-His(benzhydryl)-Lys(caprylic acid)-d-Phe(4-methyl)-Tyr(tBu)-trityl resin.

The protecting group was cleaved from the Boc-His(benzhydryl)-Lys(caprylic acid)-d-Phe(4-methyl)-Tyr(tBu)-trityl resin and then purified to prepare His(benzhydryl)-Lys(caprylic acid)-d-Phe(4-methyl)-Tyr-OH.

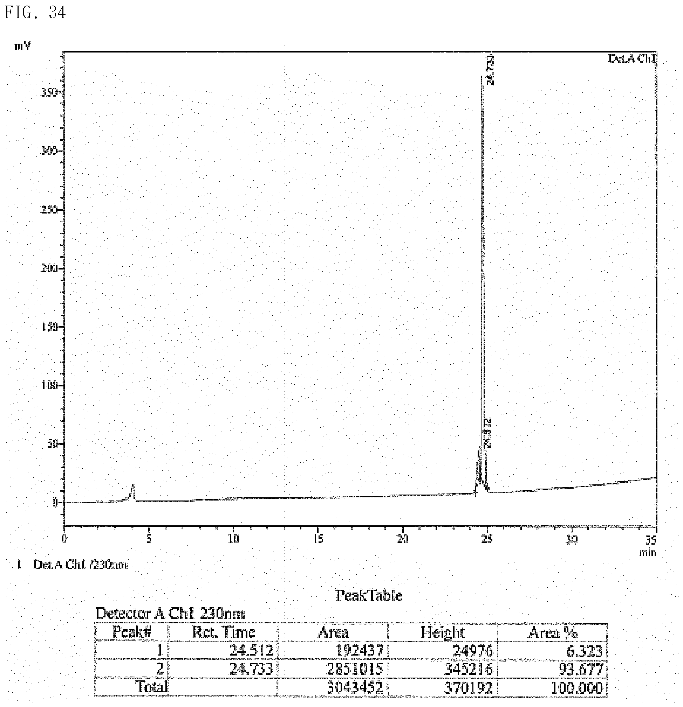

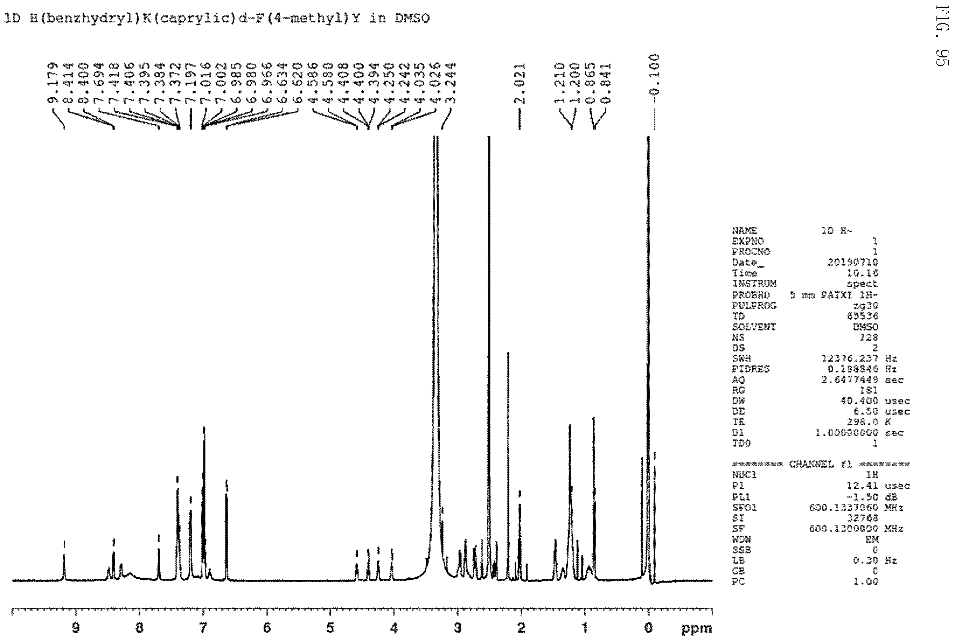

The purity, molecular weight and ¹H NMR of the HKFY-34 are shown in FIGs. 34, 70 and 95, respectively.

### Example 1.35. Preparation of HKFY-35

His(benzhydryl)-Lys(caprylic acid)-d-Phe(4-methyl)-Tyr ((SO₃H)-OH (S)-2-((R)-2-((S)-2-((S)-2-amino-3-(1-benzhydryl-1H-imidazol-4-yl)propanamido)-6-octanamidohexanamido)-3-(p-tolyl)propanamido)-3-(4-(sulfooxy)phenyl)propanoic acid) was prepared in the same manner except that Fmoc-Tyr(SO₃H)-OH was used instead of Fmoc-Tyr(tBu)-OH obtained during the preparation process of Example 1.34.

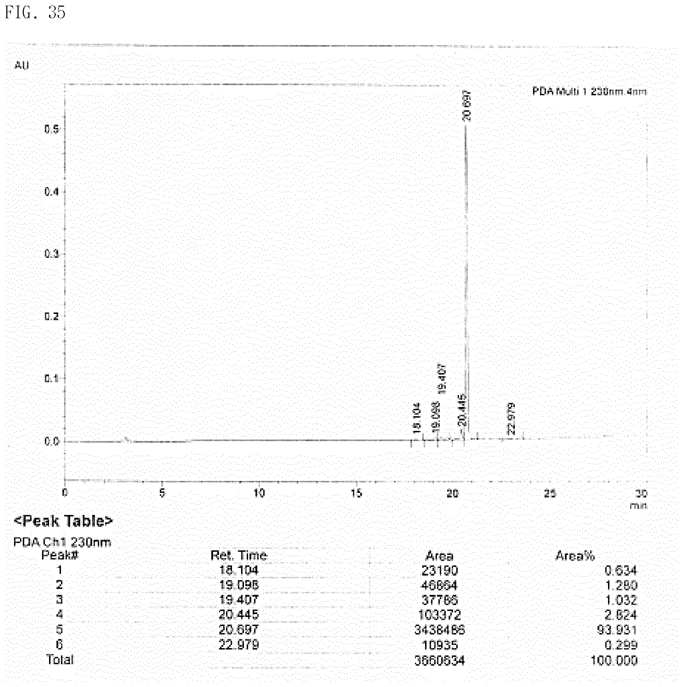

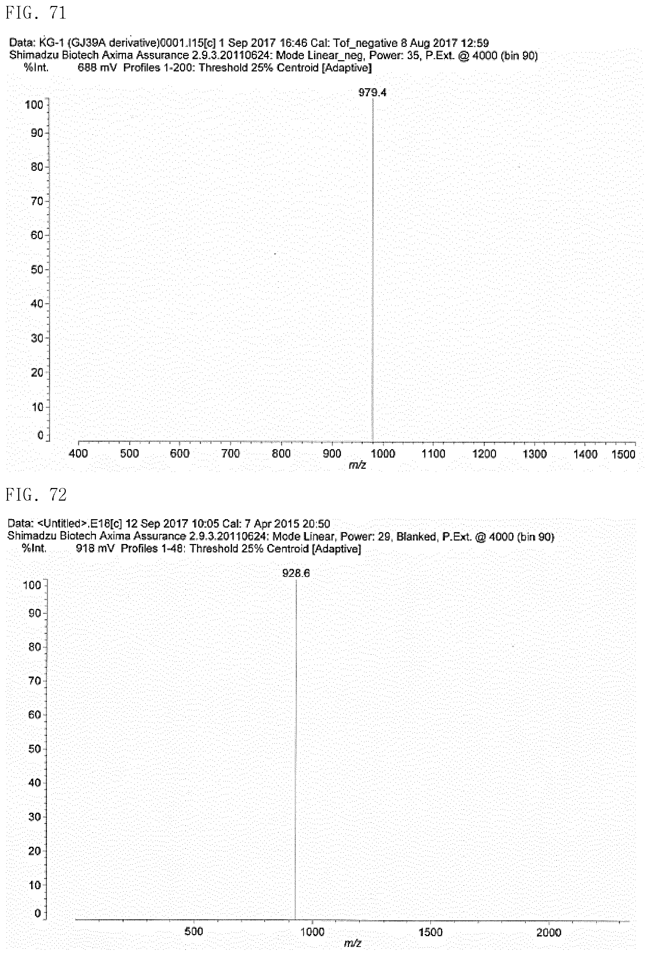

The purity and molecular weight of the HKFY-35 are shown in FIGs. 35 and 71, respectively.

### Example 1.36. Preparation of HKFY-36

His(benzhydryl)-Lys(caprylic acid)-d-Phe(4-methyl)-Tyr ((2.6-di-methyl)-OH (S)-2-((R)-2-((S)-2-((S)-2-amino-3-(1-benzhydryl-1H-imidazol-4-yl)propanamido)-6-octanamidohexanamido)-3-(p-tolyl)propanamido)-3-(4-hydroxy-2,6-dimethylphenyl)propanoic acid) was prepared in the same manner except that Fmoc-Tyr(2.6-di-methyl)-OH was used instead of Fmoc-Tyr(tBu)-OH obtained during the preparation process of Example 1.34.

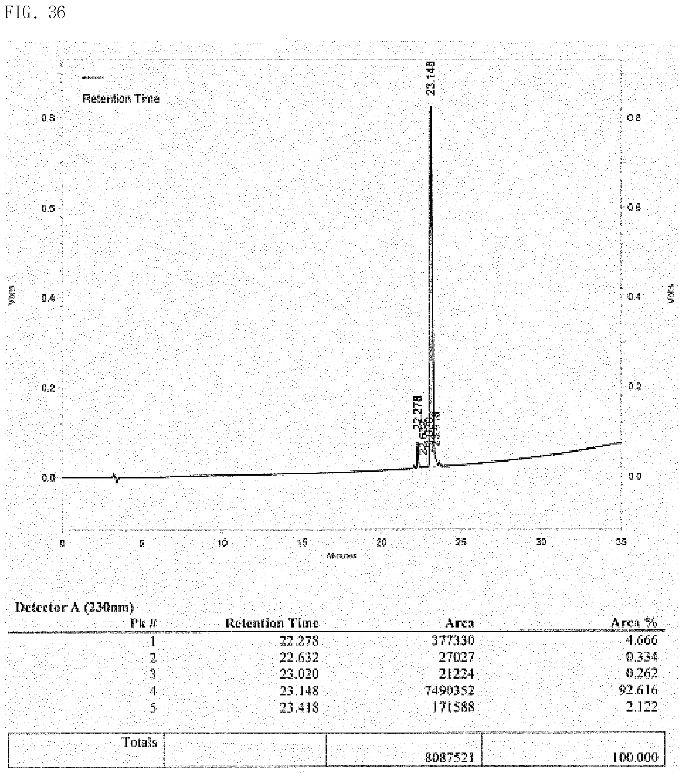

The purity and molecular weight of the HKFY-36 are shown in FIGs. 36 and 72, respectively.

### Example 1.37. Preparation of HKFY-37

DMF containing 20% piperidine was added to Fmoc-His(trt)-Lys(caprylic)-Phe-Tyr-OH-trityl resin obtained during the preparation process of Example 1.1., and HOBt (hydroxyl-benzo triazole) was added to Fmoc-Ser(tBu)-OH to prepare Fmoc-Ser(tBu)-His(trt)-Lys(caprylic)-Phe-Tyr-OH-trityl resin. DMF containing 20% piperidine was added to the resin, and HOBt (hydroxyl-benzo triazole) was added to Fmoc-Gly-OH to prepare Fmoc-Gly-Ser(tBu)-His(trt)-Lys(caprylic)-Phe-Tyr-OH-trityl resin.

The protecting group was cleaved from the Fmoc-Gly-Ser(tBu)-His(trt)-Lys(caprylic)-Phe-Tyr-OH-trityl resin and then purified to prepare Gly-Ser-His(trt)-Lys(caprylic acid)-Phe-Tyr (N2-(Na-glycyl-L-seryl-Nt-trityl-L-histidyl)-N6-octanoyl-L-lysyl-L-phenylalanyl-L-tyrosine).

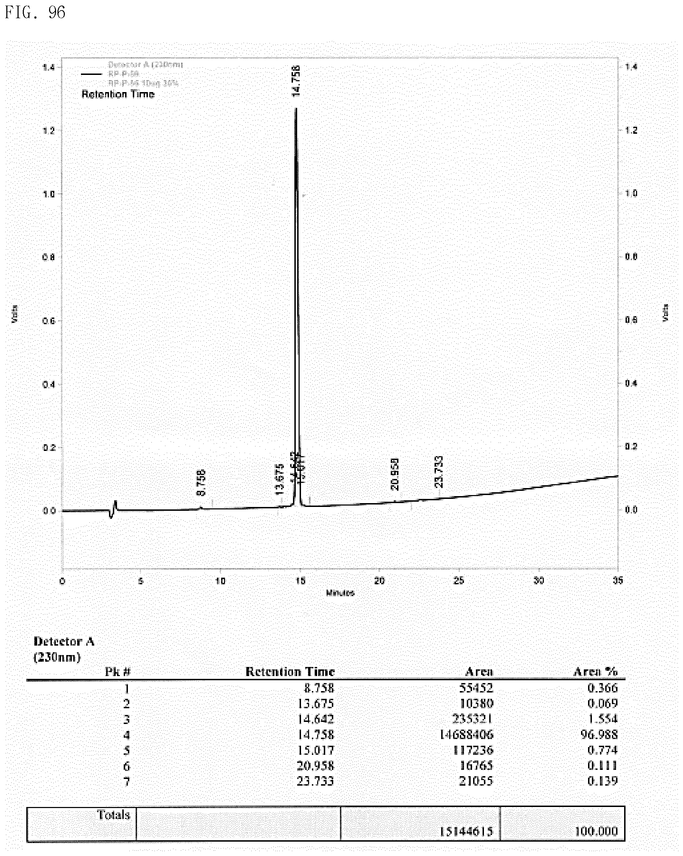

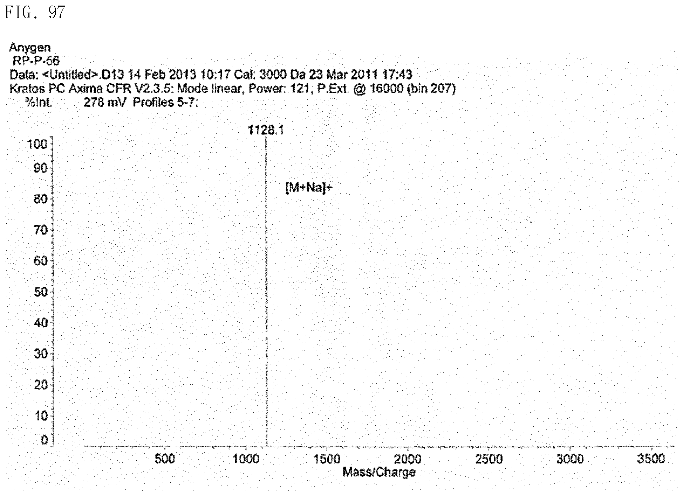

The purity and molecular weight of the HKFY-37 are shown in FIGs. 96 and 97, respectively.

### Example 1.38. Preparation of HKFY-38

In order to prepare HKFY-38 ((3S,6S,9S,12S,15S)-15-((S)-2-amino-3-(1-trityl-1H-imidazol-4-yl)propanamido)-12-benzyl-3-(((6S,9S,12S,15S)-1,18-diamino-6-(((S)-1-amino-1-oxo-3-phenylpropan-2-yl)carbamoyl)-12-(3-amino-3-oxopropyl)-1-imino-9-methyl-8,11,14,18-tetraoxo-2,7,10,13-tetraazaoctadecan-15-yl)carbamoyl)-9-(4-hydroxybenzyl)-6-(hydroxymethyl)-5,8,11,14,21-pentaoxo-4,7,10,13,20-pentaazaoctacosanoic acid), Fmoc-Phe-OH and DMF (dimethylformamide) were loaded onto the trityl resin to prepare Fmoc-Phe-trityl resin. DMF containing 20% piperidine and Fmoc-Arg(pbf)-OH and HOBt (hydroxyl-benzo triazole) were added to Fmoc-Phe-trityl resin to prepare Fmoc-Arg(pbf)-Phe-trityl resin. DMF containing 20% piperidine and Fmoc-Ala-OH and HOBt (hydroxyl-benzo triazole) were added to Fmoc-Arg(pbf)-Phe-trityl resin to prepare Fmoc-Ala-Arg(pbf)-Phe-trityl resin. Fmoc-Gln(trt)-OH, Fmoc-Gln(trt)-OH, Fmoc-Asp(tBu)-OH, Fmoc-Ser(tBu)-OH, Fmoc-Tyr(tbu)-OH, Fmoc-Phe-OH were sequentially added in the same manner as above to prepare Fmoc-Phe-Tyr(tBu)-Ser(tBu)-Asp(tBu)-Gln(trt)-Gln(trt)-Ala-Arg(pbf)-Phe-trityl resin.

DMF containing 20% piperidine and Fmoc-Lys(caprylic)-OH obtained from Example 1.1. and HOBt (hydroxyl-benzo triazole) were added to the resin to prepare Fmoc-Lys(caprylic)-Phe-Tyr(tBu)-Ser(tBu)-Asp(tBu)-Gln(trt)-Gln(trt)-Ala-Arg(pbf)-Phe-trityl resin.

DMF containing 20% piperidine and Fmoc-His(trt)-OH obtained from Example 1.1. and HOBt (hydroxyl-benzo triazole) were added to the resin to prepare Fmoc-His(trt)-Lys(caprylic)-Phe-Tyr(tBu)-Ser(tBu)-Asp(tBu)-Gln(trt)-Gln(trt)-Ala-Arg(pbf)-Phe-trityl resin.

The protecting group was cleaved from the Fmoc-His(trt)-Lys(caprylic)-Phe-Tyr(tBu)-Ser(tBu)-Asp(tBu)-Gln(trt)-Gln(trt)-Ala-Arg(pbf)-Phe-trityl resin and then purified to prepare His(trt)-Lys(caprylic)-Phe-Tyr-Ser-Asp-Gln-Gln-Ala-Arg-Phe.

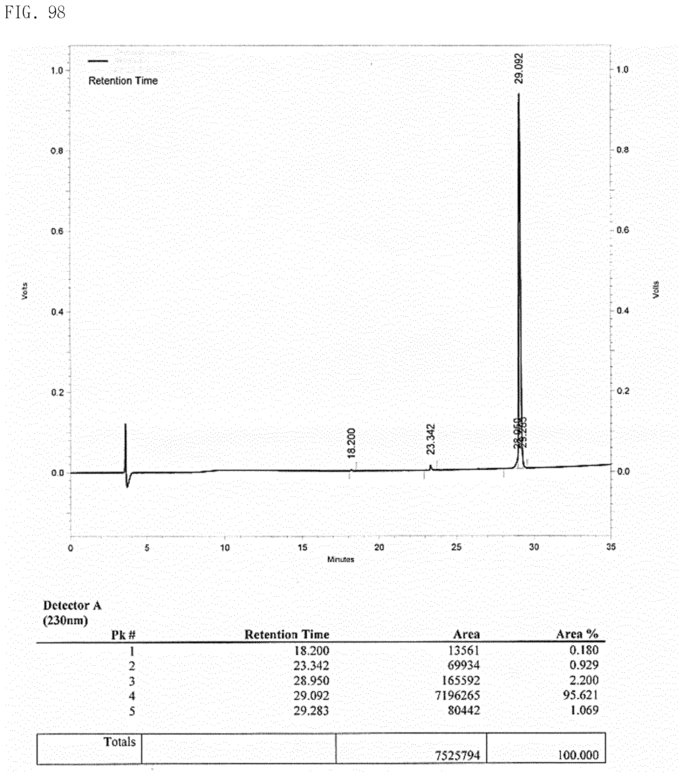

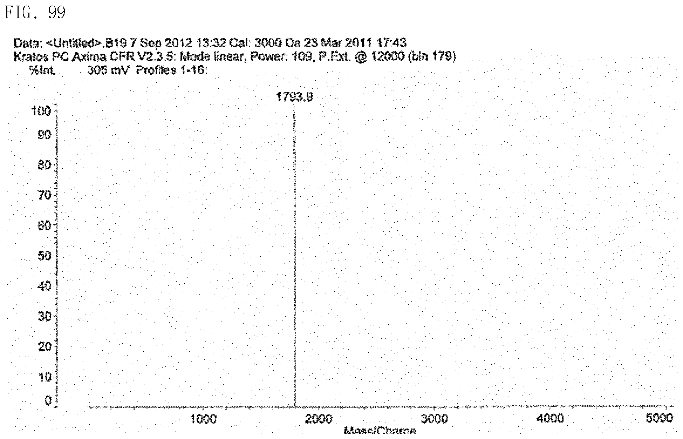

The purity and molecular weight of the HKFY-38 are shown in FIGs. 98 and 99, respectively.

### Example 1.39. Preparation of HKFY-39

In order to prepare HKFY-39 ((3S,6S,9S,12S,15S,18S)-3-((2-(((S)-1-(((S)-1-(((S)-1-amino-1-oxo-3-phenylpropan-2-yl)amino)-5-guanidino-1-oxopentan-2-yl)amino)-1-oxopropan-2-yl)amino)-2-oxoethyl)carbamoyl)-18-((S)-2-amino-3-(1-trityl-1H-imidazol-4-yl)propanamido)-6-(3-amino-3-oxopropyl)-15-benzyl-12-(4-hydroxybenzyl)-9-(hydroxymethyl)-5,8,11,14,17,24-hexaoxo-4,7,10,13,16,23-hexaazahentriacontanoic acid), DMF containing 20% piperidine and Fmoc-Gly-OH and HOBt (hydroxyl-benzo triazole) were added to Fmoc-Ala-Arg(pbf)-Phe-trityl resin obtained from Example 1.38. to prepare Fmoc-Gly-Ala-Arg(pbf)-Phe-trityl resin. DMF containing 20% piperidine and Fmoc-Asn(trt)-OH and HOBt (hydroxyl-benzo triazole) were added to Fmoc-Gly-Ala-Arg(pbf)-Phe-trityl resin to prepare Fmoc-Asn(trt)-Gly-Ala-Arg(pbf)-Phe-trityl resin. Fmoc-Gln(trt)-OH, Fmoc-Ser(tBu)-OH, Fmoc-Tyr(tbu)-OH, Fmoc-Phe-OH were sequentially added in the same manner as above to prepare Fmoc-Phe-Tyr(tBu)-Ser(tBu)-Gln(trt)-Asn(trt)-Gly-Ala-Arg(pbf)-Phe-trityl resin. Fmoc-Lys(caprylic)-OH obtained from Example 1.1. and HOBt (hydroxyl-benzo triazole) were added to the resin to prepare Fmoc-Lys(caprylic)-Phe-Tyr(tBu)-Ser(tBu)-Gln(trt)-Asn(trt)-Gly-Ala-Arg(pbf)-Phe-trityl resin.

DMF containing 20% piperidine and Fmoc-His(trt)-OH obtained from Example 1.1. and HOBt (hydroxyl-benzo triazole) were added to the resin to prepare Fmoc-His(trt)-Lys(caprylic)-Phe-Tyr(tBu)-Ser(tBu)-Gln(trt)-Asn(trt)-Gly-Ala-Arg(pbf)-Phe-trityl resin.

The protecting group was cleaved from the Fmoc-His(trt)-Lys(caprylic)-Phe-Tyr(tBu)-Ser(tBu)-Gln(trt)-Asn(trt)-Gly-Ala-Arg(pbf)-Phe-trityl resin and then purified to prepare His(trt)-Lys(caprylic)-Phe-Tyr-Ser-Gln-Asn-Gly-Ala-Arg-Phe.

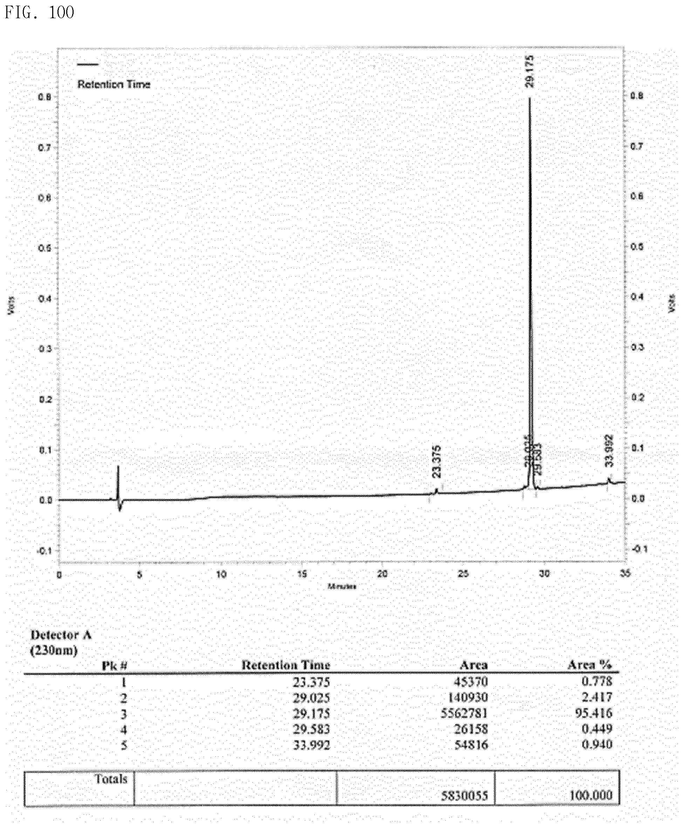

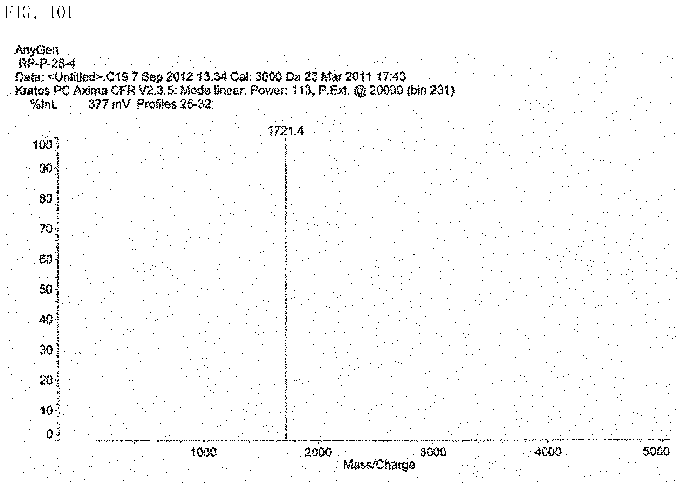

The purity and molecular weight of the HKFY-39 are shown in FIGs. 100 and 101, respectively.

### Example 1.40. Preparation of HKFY-40

DMF containing 20% piperidine and Fmoc-Gln(trt)-OH and HOBt (hydroxyl-benzo triazole) were added to the Fmoc-Ala-Arg(pbf)-Phe-trityl resin obtained from Example 1.39. to prepare Fmoc-Gln(trt)-Ala-Arg(pbf)-Phe-trityl resin.

Fmoc-Gln(trt)-OH, Fmoc-His(trt)-OH, Fmoc-Ser(tBu)-OH, Fmoc-Tyr(tBu)-OH, Fmoc-Phe()-OH were sequentially added in the same manner to prepare Fmoc-Phe-Tyr(tBu)-Ser(tBu)-His(trt)-Gln(trt)-Gln(trt)-Ala-Arg(pbf)-Phe-trityl resin.

Fmoc-Lys(caprylic)-OH obtained from Example 1.1. and Fmoc-His(trt)-OH were sequentially added to the resin in the same manner to prepare Fmoc-His(trt)-Lys(caprylic)-Phe-Tyr(tBu)-Ser(tBu)-His(trt)-Gln(trt)-Gln(trt)-Ala-Arg(pbf)-Phe-trityl resin.

The protecting group was cleaved from the Fmoc-His(trt)-Lys(caprylic)-Phe-Tyr(tBu)-Ser(tBu)-His(trt)-Gln(trt)-Gln(trt)-Ala-Arg(pbf)-Phe-trityl resin and then purified to prepare His(trt)-Lys(caprylic)-Phe-Tyr-Ser-His-Gln-Gln-Ala-Arg-Phe ((S)-2-((2S,5S,8S,11S,14S)-2-((1H-imidazol-4-yl)methyl)-14-((S)-2-amino-3-(1-trityl-1H-imidazol-4-yl)propanamido)-11-benzyl-8-(4-hydroxybenzyl)-5-(hydroxymethyl)-4,7,10,13,20-pentaoxo-3,6,9,12,19-pentaazaheptacosanamido)-N1-((S)-5-amino-1-(((S)-1-(((S)-1-(((S)-1-amino-1-oxo-3-phenylpropan-2-yl)amino)-5-guanidino-1-oxopentan-2-yl)amino)-1-oxopropan-2-yl)amino)-1,5-dioxopentan-2-yl)pentanediamide).

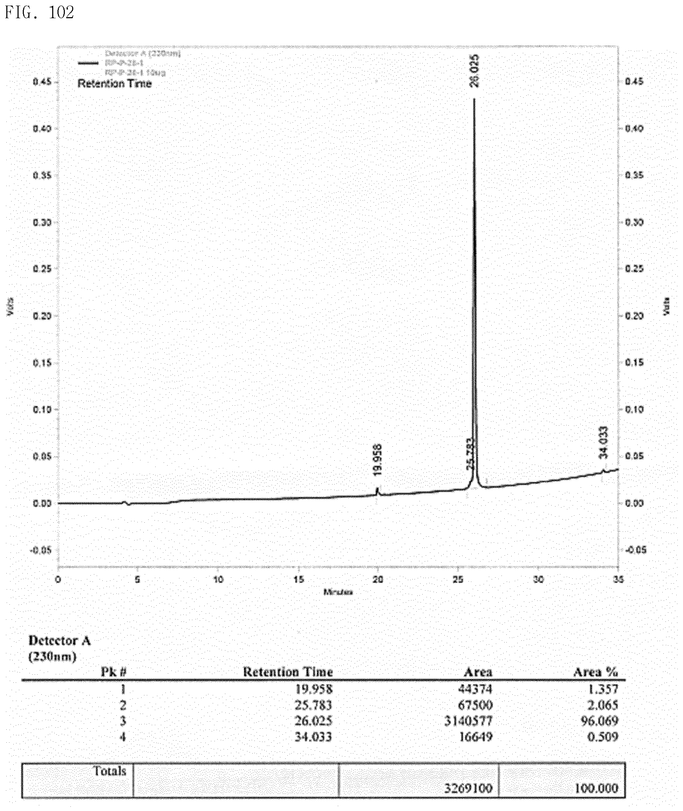

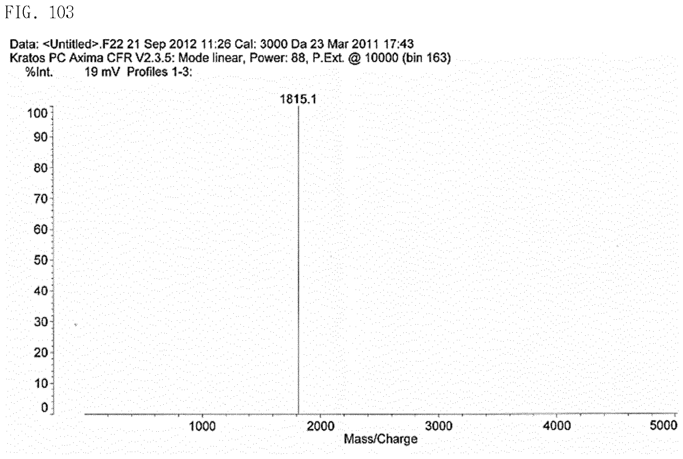

The purity and molecular weight of the HKFY-40 are shown in FIGs. 102 and 103, respectively.

### Example 1.41. Preparation of HKFY-41

DMF containing 20% piperidine and Fmoc-Gly-OH and HOBt (hydroxyl-benzo triazole) were added to the Fmoc-Ala-Arg(pbf)-Phe-trityl resin obtained from Example 1.39. to prepare Fmoc-Gly-Ala-Arg(pbf)-Phe-trityl resin. Fmoc-Asn(trt)-OH, Fmoc-Gln(trt)-OH, Fmoc-Tyr(tBu)-OH, Fmoc-Phe-OH, Fmoc-Lys(caprylic)-OH obtained from Example 1.1., Fmoc-His(trt)-OH were sequentially added to the resin in the same manner to prepare Fmoc-His(trt)-Lys(caprylic)-Phe-Tyr(tBu)-Gln(trt)-Asn(trt)-Gly-Ala-Arg(pbf)-Phe-trityl resin. The protecting group of the resin was cleaved to prepare His(trt)-Lys(caprylic)-Phe-Tyr-Gln-Asn-Gly-Ala-Arg-Phe ((3S,6S,9S,12S,15S,18S)-3-((2-(((S)-1-(((S)-1-(((S)-1-amino-1-oxo-3-phenylpropan-2-yl)amino)-5-guanidino-1-oxopentan-2-yl)amino)-1-oxopropan-2-yl)amino)-2-oxoethyl)carbamoyl)-18-((S)-2-amino-3-(1-trityl-1H-imidazol-4-yl)propanamido)-6-(3-amino-3-oxopropyl)-15-benzyl-12-(4-hydroxybenzyl)-9-(hydroxymethyl)-5,8,11,14,17,24-hexaoxo-4,7,10,13,16,23-hexaazahentriacontanoic acid).

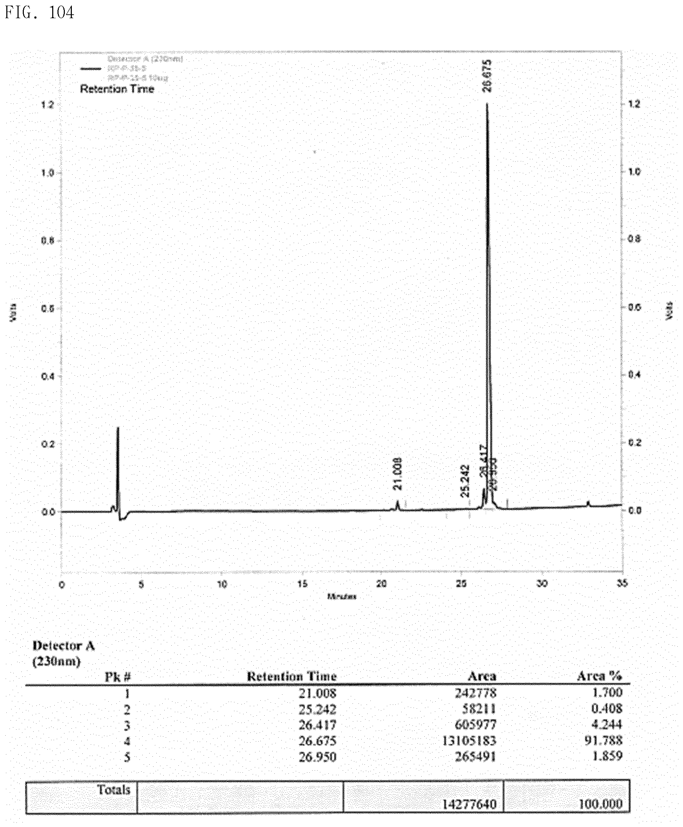

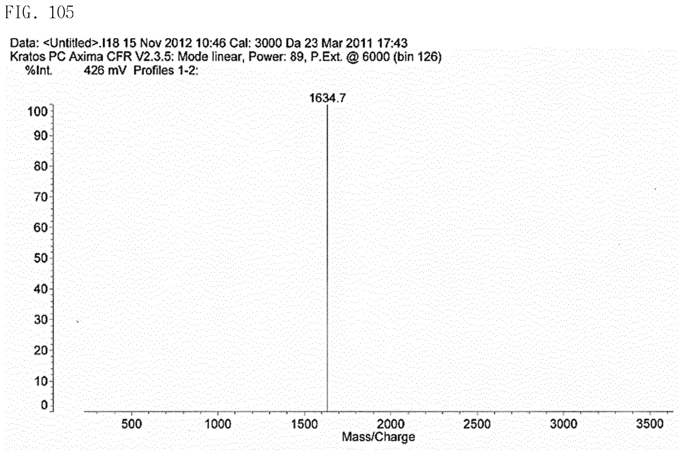

The purity and molecular weight of the HKFY-41 are shown in FIGs. 104 and 105, respectively.

### Example 1.42. Preparation of HKFY-42

DMF containing 20% piperidine and Fmoc-Gln(trt) and HOBt (hydroxyl-benzo triazole) were added to the Fmoc-Ala-Arg(pbf)-Phe-trityl resin obtained from Example 1.39. to prepare Fmoc-Gly-Ala-Arg(pbf)-Phe-trityl resin. Fmoc-Gln(trt)-OH, Fmoc-His(trt)-OH, Fmoc-Tyr(tBu)-OH, Fmoc-Phe-OH, Fmoc-Lys(caprylic)-OH obtained from Example 1.1., Fmoc-His(trt)-OH were sequentially added to the resin in the same manner to prepare Fmoc-His(trt)-Lys(caprylic)-Phe-Tyr(tBu)-His(trt)-Gln(trt)-Gln(trt)-Ala-Arg(pbf)-Phe-trityl resin. The protecting group of the resin was cleaved to prepare His(trt)-Lys(caprylic)-Phe-Tyr-His-Gln-Gln-Ala-Arg-Phe ((S)-2-((2S,5S,8S,11S)-2-((1H-imidazol-4-yl)methyl)-11-((S)-2-amino-3-(1-trityl-1H-imidazol-4-yl)propanamido)-8-benzyl-5-(4-hydroxybenzyl)-4,7,10,17-tetraoxo-3,6,9,16-tetraazatetracosanamido)-N1-((S)-5-amino-1-(((S)-1-(((S)-1-(((S)-1-amino-1-oxo-3-phenylpropan-2-yl)amino)-5-guanidino-1-oxopentan-2-yl)amino)-1-oxopropan-2-yl)amino)-1,5-dioxopentan-2-yl)pentanediamide).

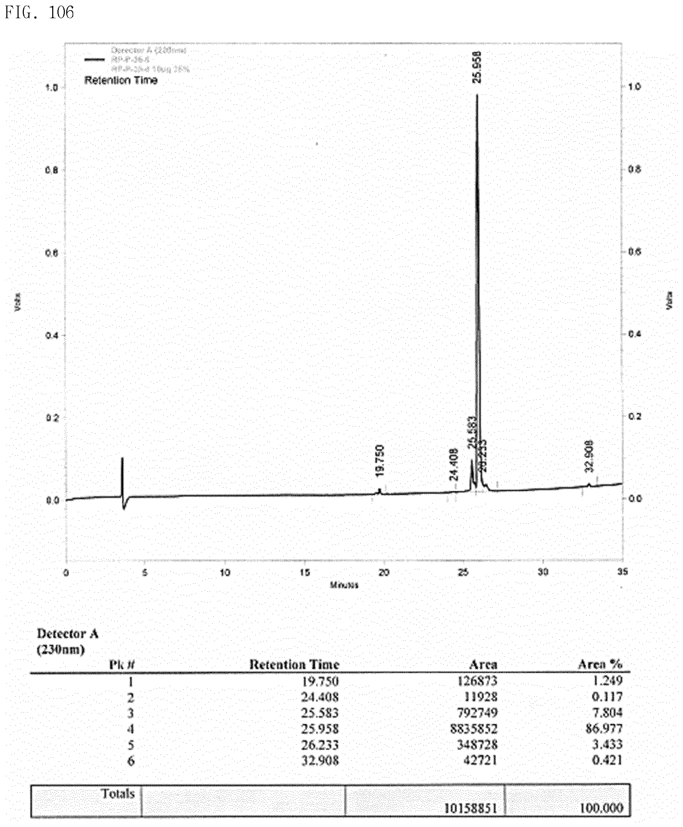

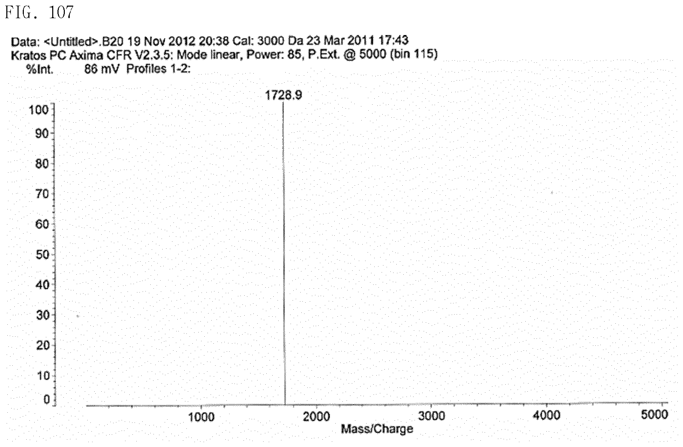

The purity and molecular weight of the HKFY-42 are shown in FIGs. 106 and 107, respectively.

### Example 1.43. Preparation of HKFY-43

Fmoc-His(trt)-Lys(caprylic)-Phe-Tyr(tBu)-Gln(trt)-Asn(trt)-Gln(trt)-Ala-Arg(pbf)-Phe-trityl resin was prepared in the same manner except that Fmoc-Gln(trt)-OH was used instead of Fmoc-Gly-OH among the constituent amino acids of Example 1.41. The protecting group of the resin was cleaved to prepare His(trt)-Lys(caprylic)-Phe-Tyr-Gln-Asn-Gln-Ala-Arg-Phe ((S)-N1-((S)-1-(((S)-1-(((S)-1-amino-1-oxo-3-phenylpropan-2-yl)amino)-5-guanidino-1-oxopentan-2-yl)amino)-1-oxopropan-2-yl)-2-((2S,5S,8S,11S,14S)-2-(2-amino-2-oxoethyl)-14-((S)-2-amino-3-(1-trityl-1H-imidazol-4-yl)propanamido)-5-(3-amino-3-oxopropyl)-11-benzyl-8-(4-hydroxybenzyl)-4,7,10,13,20-pentaoxo-3,6,9,12,19-pentaazaheptacosanamido)pentanediamide).

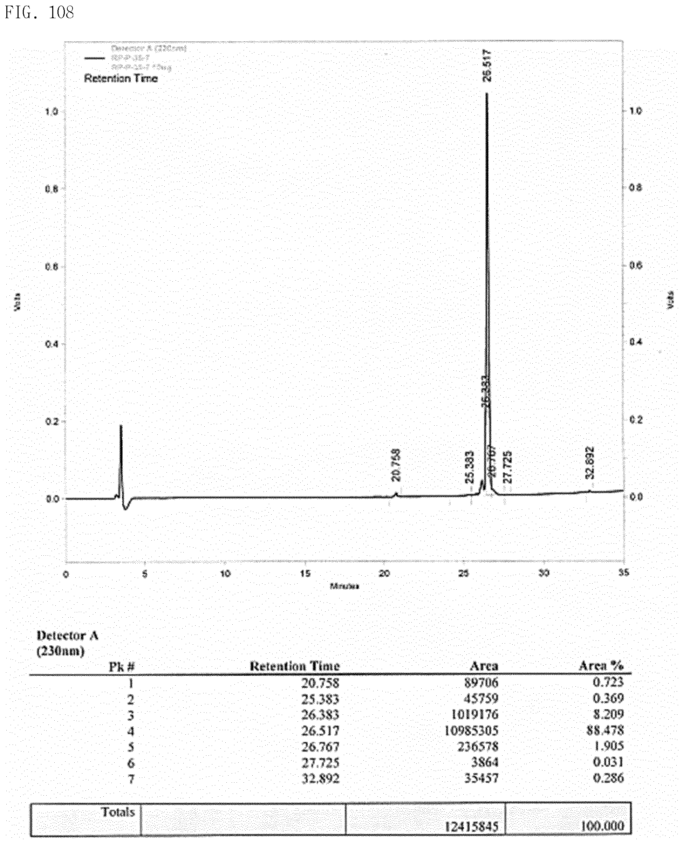

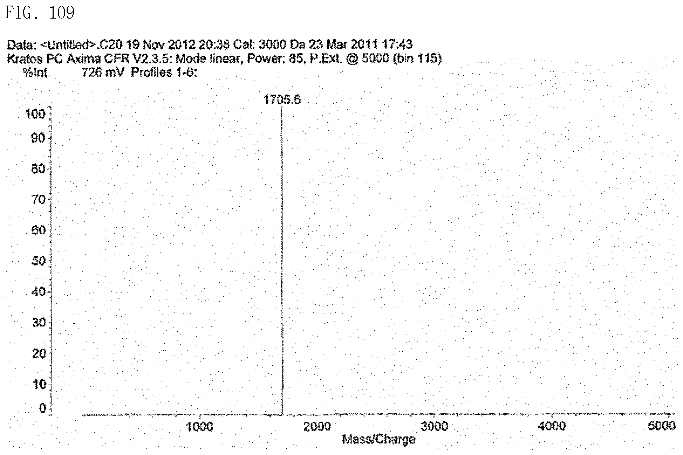

The purity and molecular weight of the HKFY-43 are shown in FIGs. 108 and 109, respectively.

### Example 1.44. Preparation of HKFY-44

In order to prepare His(trt)-Lys(caprylic)-Phe-Tyr-Trp, Fmoc-Trp(Boc)-OH and DMF were loaded onto the trityl resin to prepare Fmoc-Trp(Boc)-trityl resin. DMF containing 20% piperidine and Fmoc-Tyr(tBu)-OH and HOBt (hydroxyl-benzo triazole) were added to the Fmoc-Trp(Boc)-trityl resin to prepare Fmoc-Tyr(tBu)-Trp(Boc)-trityl resin. Fmoc-Phe-OH, Fmoc-Lys(caprylic)-OH obtained from Example 1.1., Fmoc-His(trt)-OH were sequentially added to the resin in the same manner to prepare Fmoc-His(trt)-Lys(caprylic)-Phe-Tyr(tBu)-Trp(Boc)-trityl resin. The protecting group of the resin was cleaved to prepare His(trt)-Lys(caprylic)-Phe-Tyr-Trp (N6-octanoyl-N2-(Nt-trityl-L-histidyl)-L-lysyl-L-phenylalanyl-L-tyrosyl-L-tryptophan).

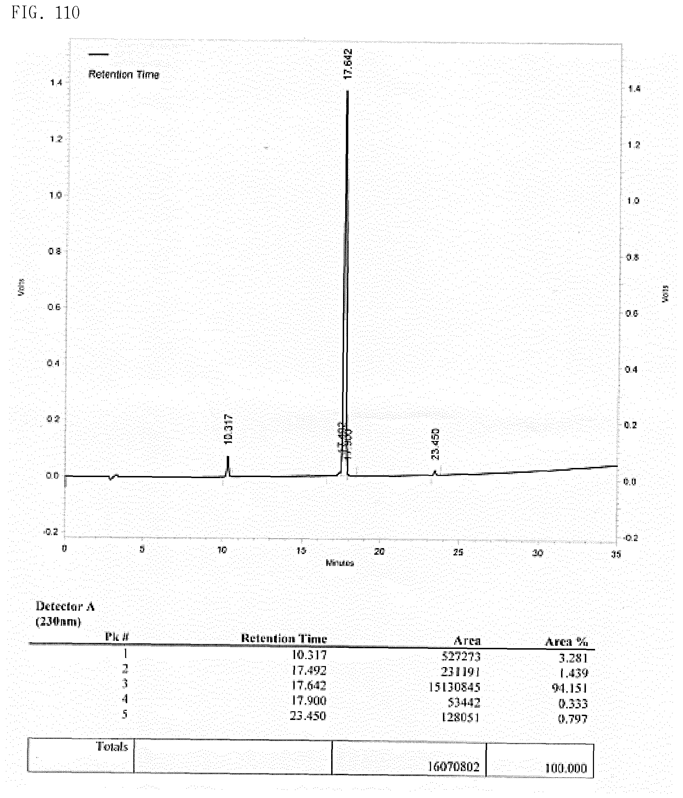

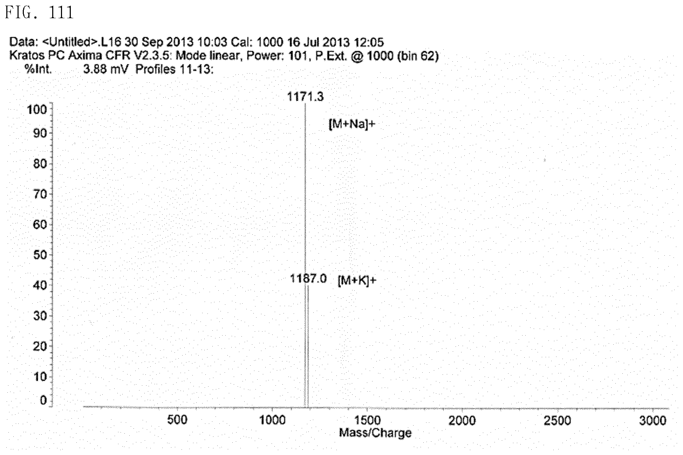

The purity and molecular weight of the HKFY-44 are shown in FIGs. 110 and 111, respectively.

### Example 1.45. Preparation of HKFY-45

D-biotin-His(trt)-K(caprylic)-Ala(biphenyl)-Tyr(Boc)-trityl resin was prepared in the same manner except that Fmoc-Ala(biphenyl)-OH was used instead of Fmoc-Phe-OH during the preparation process of Example 1.2. The protecting group of the resin was cleaved to prepare biotin-His(trt)-K(caprylic)-Ala(biphenyl)-Tyr (((S)-3-([1,1'-biphenyl]-4-yl)-2-((S)-6-octanamido-2-((S)-2-(5-((3as,4S,6aR)-2-oxohexahydro-1H-thieno[3,4-d]imidazol-4-yl)pentanamido)-3-(1-trityl-1H-imidazol-4-yl)propanamido)hexanamido)propanoyl)-L-tyrosine).

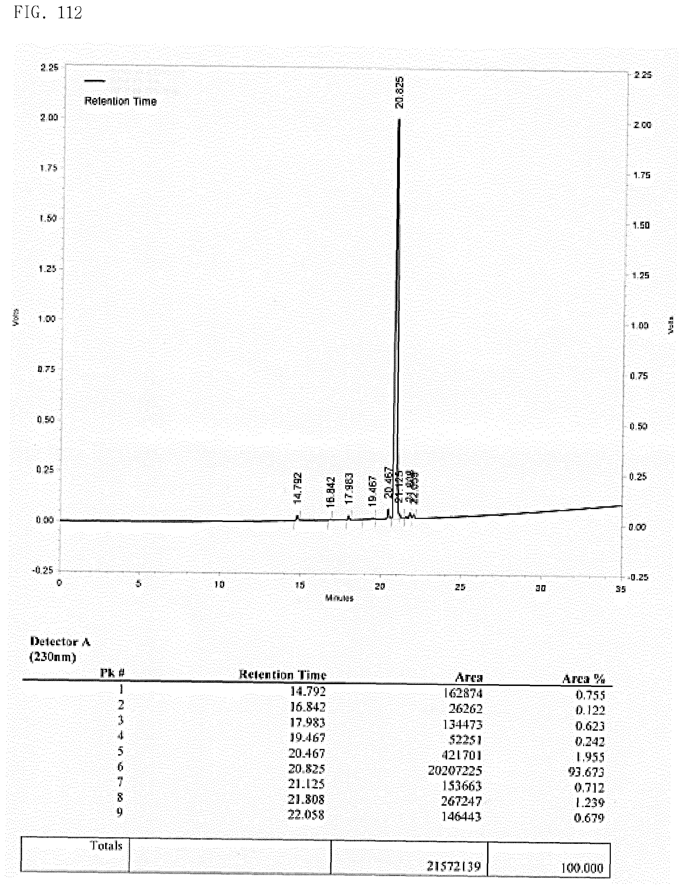

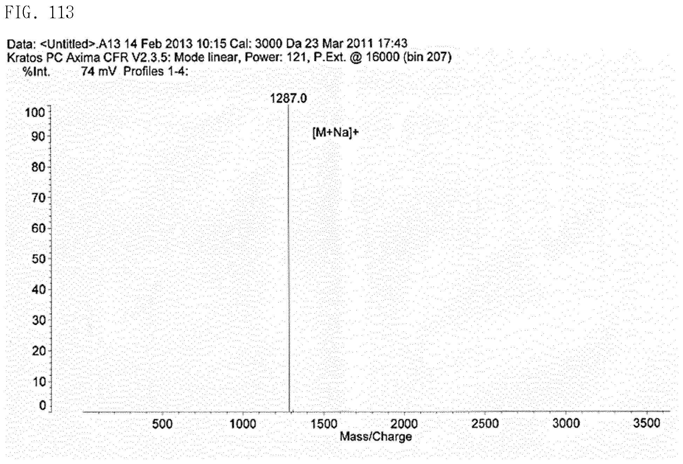

The purity and molecular weight of the HKFY-45 are shown in FIGs. 112 and 113, respectively.

### Example 1.46. Preparation of HKFY-46

Fmoc-Phe-OH and DMF were loaded onto the Fmoc-Trp(Boc)-trityl resin obtained from Example 1.44. to prepare Fmoc-Trp(Boc)-trityl resin. Fmoc-Lys(caprylic)-OH obtained from Example 1.1., Fmoc-His(trt)-OH were sequentially added to the resin in the same manner to prepare Fmoc-His(trt)-Lys(caprylic)-Phe-Trp(Boc)-trityl resin. The protecting group of the resin was cleaved to prepare His(trt)-Lys(caprylic)-Phe-Trp(N6-octanoyl-N2-(Nt-trityl-L-histidyl)-L-lysyl-L-phenylalanyl-L-tryptophan).

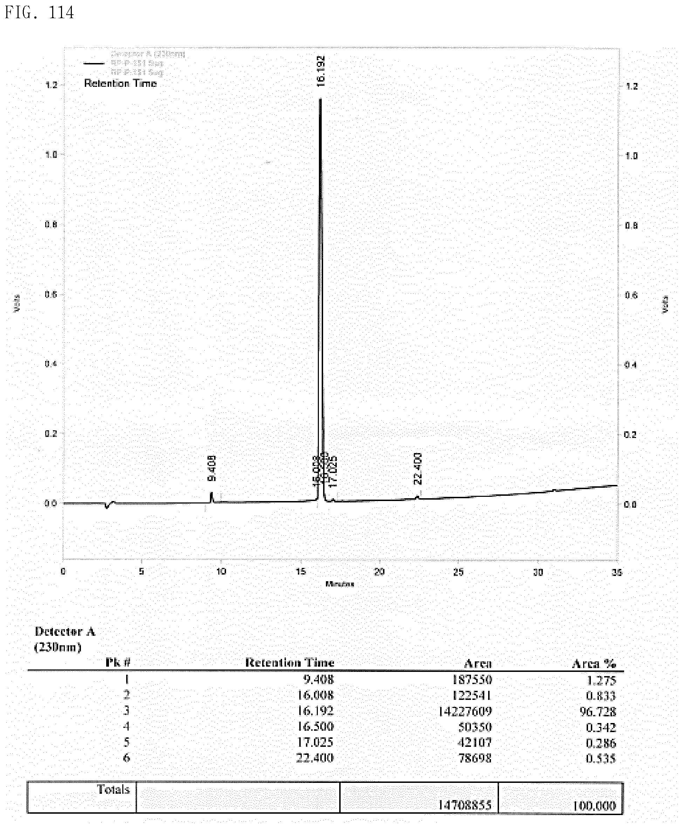

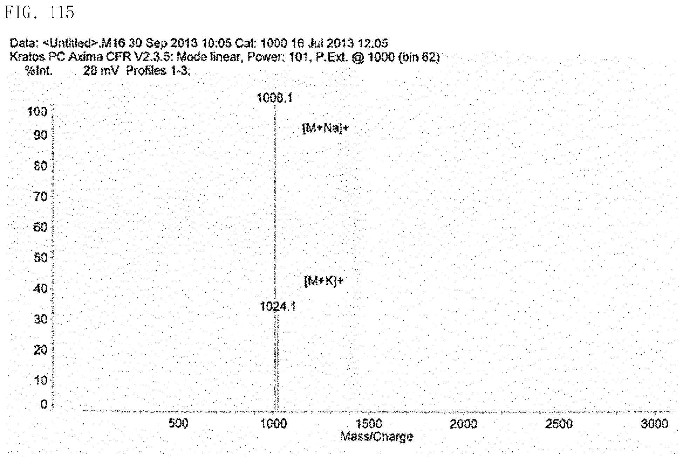

The purity and molecular weight of the HKFY-46 are shown in FIGs. 114 and 115, respectively.

### Example 1.47. Preparation of HKFY-47

In order to prepare N6-octanoyl-N2-(Nt-trityl-L-histidyl)-L-lysyl-L-tyrosyl-L-phenylalanine, Fmoc-Phe-OH and DMF were loaded onto the trityl resin to prepare Fmoc-Phe-trityl resin. Fmoc-Tyr(Boc)-OH, Fmoc-Lys(caprylic)-OH obtained from Example 1.1., Fmoc-His(trt)-OH were sequentially added to the resin in the same manner to prepare Fmoc-His(trt)-Lys(caprylic)-Tyr(Boc)-Phe-trityl resin. The protecting group of the resin was cleaved to prepare His(trt)-Lys(caprylic)-Tyr-Phe (N6-octanoyl-N2-(tNLt-triyl-L-histidyl)-L-lysyl-L-tyrosyl-L-phenylalanine).

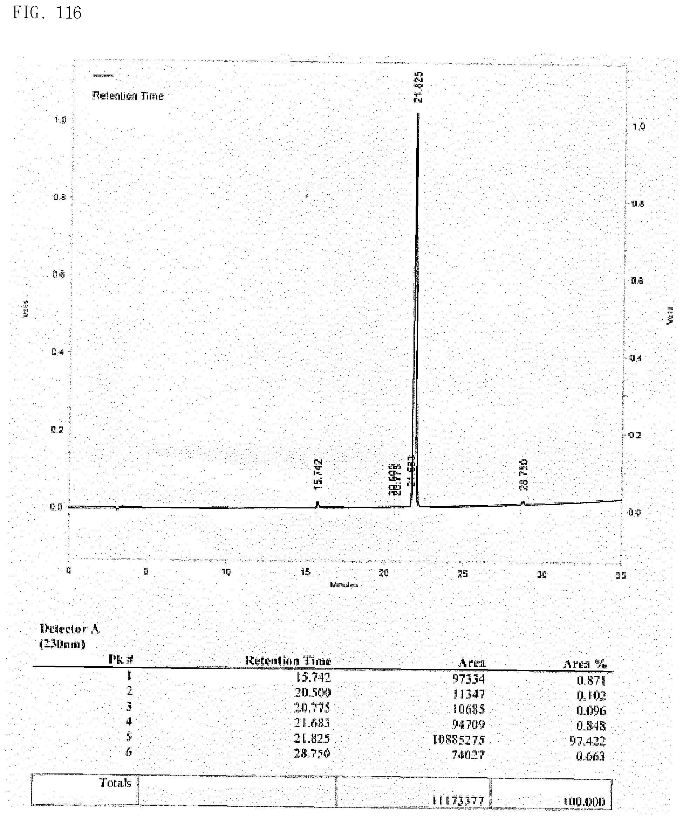

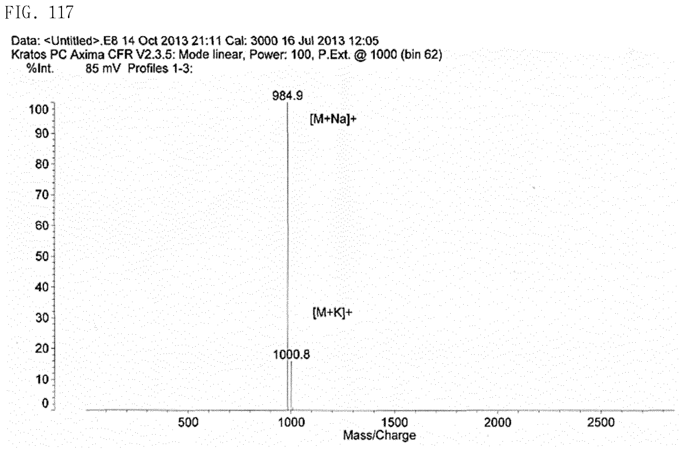

The purity and molecular weight of the HKFY-47 are shown in FIGs. 116 and 117, respectively.

### Example 1.48. Preparation of HKFY-48

In order to prepare HKFY-48 (N6-octanoyl-N2-(Nt-trityl-L-histidyl)-L-lysyl-L-tyrosyl-L-tyrosine), Fmoc-Tyr(Boc)-OH and DMF were loaded onto the trityl resin to prepare Fmoc-Tyr(Boc)-trityl resin. Fmoc-Tyr(Boc)-OH, Fmoc-Lys(caprylic)-OH obtained from Example 1.1., Fmoc-His(trt)-OH were sequentially added to the resin in the same manner to prepare Fmoc-His(trt)-Lys(caprylic)-Tyr(Boc)-Tyr(Boc)-trityl resin. The protecting group of the resin was cleaved to prepare His(trt)-Lys(caprylic)-Tyr-Tyr(N6-octanoyl-N2-(Nt-trityl-L-histidyl)-L-lysyl-L-tyrosyl-L-tyrosine).

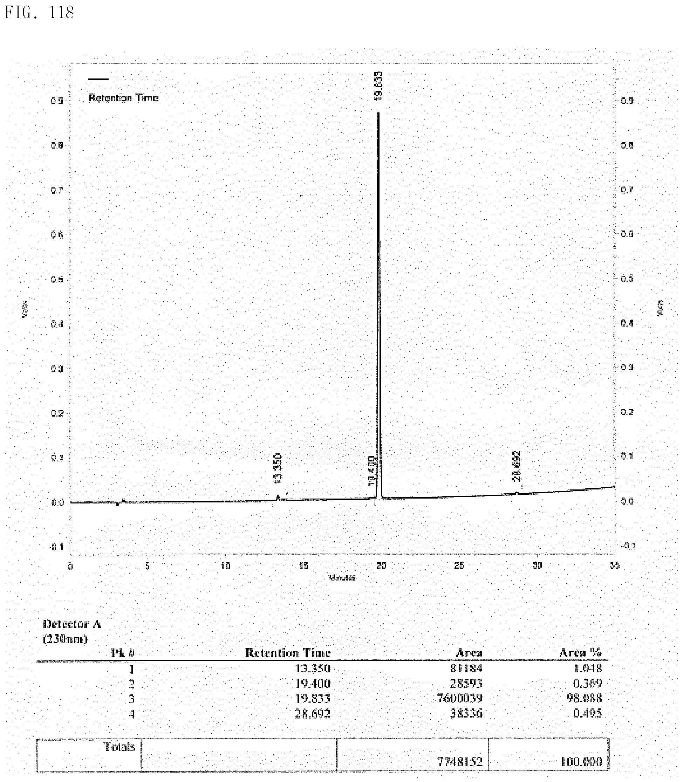

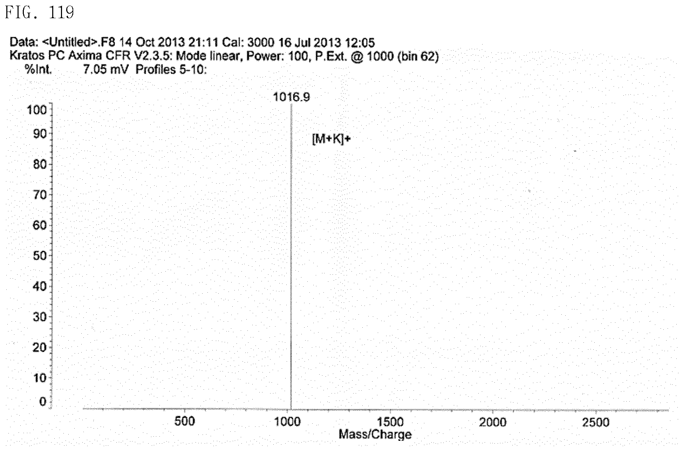

The purity and molecular weight of the HKFY-48 are shown in FIGs. 118 and 119, respectively.

### Example 1.49. Preparation of HKFY-49

In order to prepare HKFY-49 (N6-octanoyl-N2-(Nt-trityl-L-histidyl)-L-lysyl-L-phenylalanine), Fmoc-Lys(caprylic)-OH obtained from Example 1.1., and Fmoc-His(trt)-OH were sequentially added to the Fmoc-Phe-trityl resin obtained from Example 1.47. in the same manner to prepare Fmoc-His(trt)-Lys(caprylic)-Phe-trityl resin. The protecting group of the resin was cleaved to prepare His(trt)-Lys(caprylic)-Phe.

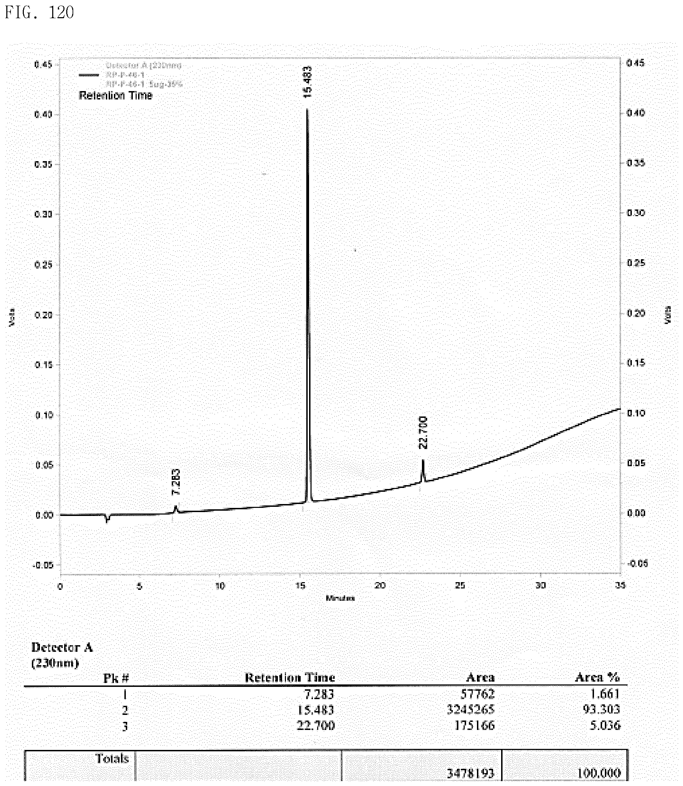

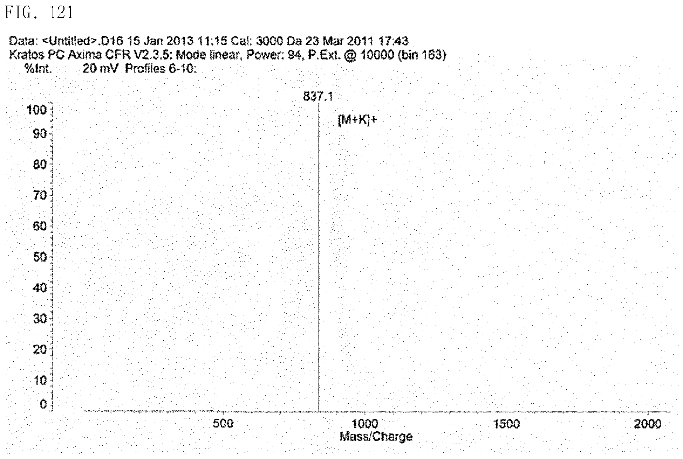

The purity and molecular weight of the HKFY-49 are shown in FIGs. 120 and 121, respectively.

### Example 1.50. Preparation of HKFY-50

In order to prepare HKFY-50 (N6-octanoyl-N2-(Nt-trityl-L-histidyl)-L-lysyl-L-phenylalanyl-L-serine), Fmoc-Ser(tBu)-OH and DMF were loaded onto the trityl resin to prepare Fmoc-Ser(tBu)-trityl resin. Fmoc-Phe-OH, Fmoc-Lys(caprylic)-OH obtained from Example 1.1., and Fmoc-His(trt)-OH were sequentially added to the resin in the same manner to prepare Fmoc-His(trt)-Lys(caprylic)-Phe-Ser-trityl resin. The protecting group of the resin was cleaved to prepare His(trt)-Lys(caprylic)-Phe-Ser.

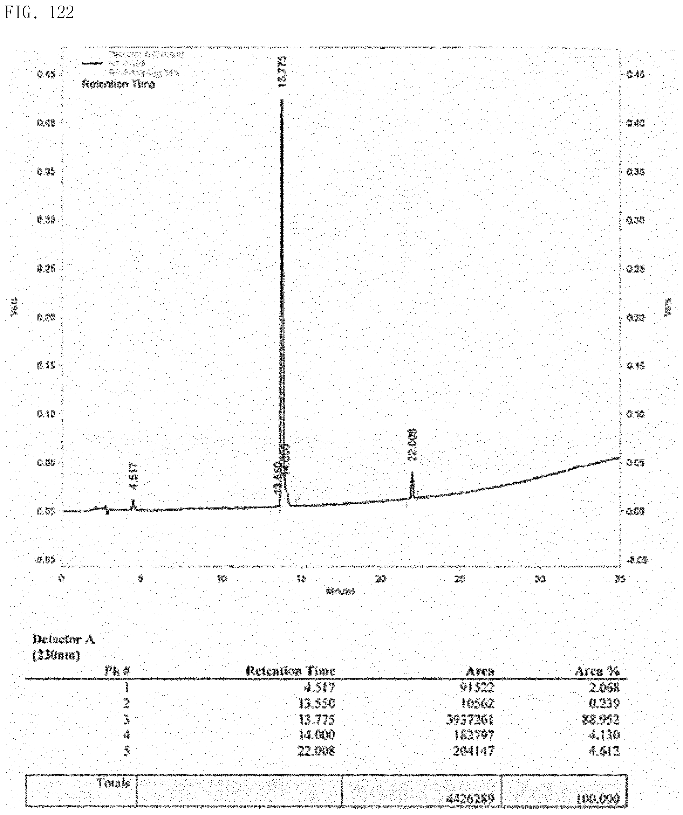

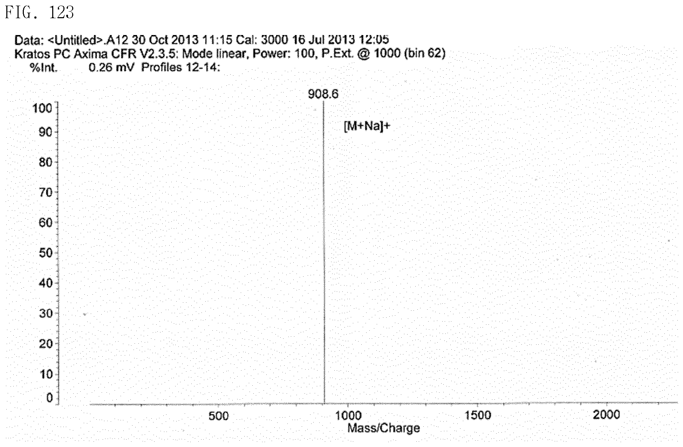

The purity and molecular weight of the HKFY-50 are shown in FIGs. 122 and 123, respectively.

### Example 1.51. Preparation of HKFY-51

His(trt)-Lys(caprylic)-Leu-Tyr (N6-octanoyl-N2-(Nt-trityl-L-histidyl)-L-lysyl-L-leucyl-L-tyrosine) was prepared in the same manner except that Fmoc-Leu-OH was used instead of Fmoc-Tyr(Boc)-OH among the constituent amino acids of Example 1.48.

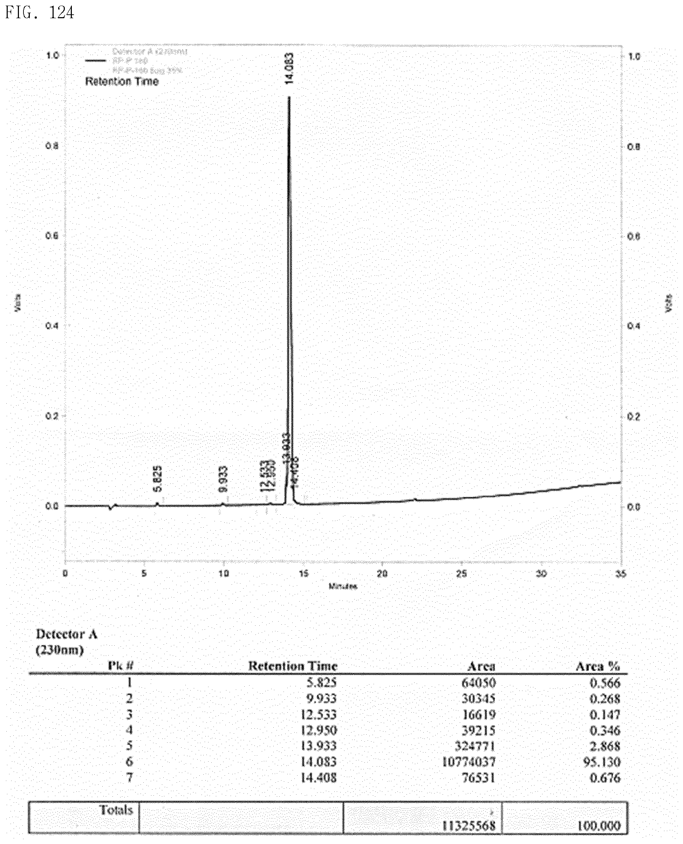

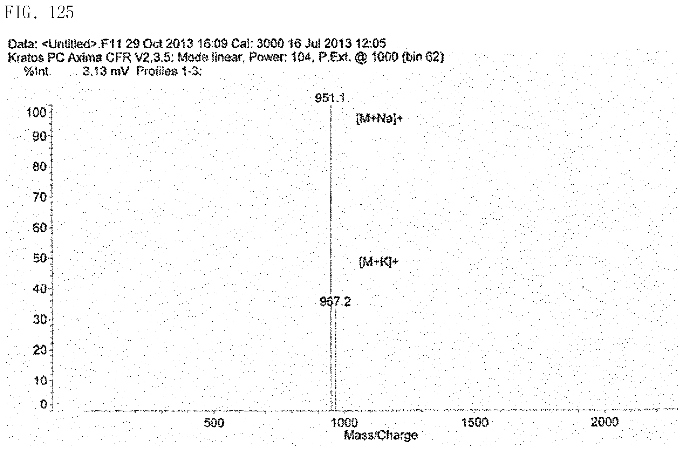

The purity and molecular weight of the HKFY-51 are shown in FIGs. 124 and 125, respectively.

### Example 1.52. Preparation of HKFY-52

Fmoc-Asp(tBu)-OH, Fmoc-Tyr(Boc)-OH, Fmoc-Phe-OH, and Fmoc-His(trt)-OH were sequentially added to the Fmoc-Gln(trt)-Gln(trt)-Ala-Arg(pbf)-Phe-trityl resin obtained from Example 1.38. in the same manner to prepare Fmoc-His(trt)-Phe-Tyr(Boc)-Asp(tBu)-Gln(trt)-Gln(trt)-Ala-Arg(pbf)-Phe-trityl resin. The protecting group of the resin was cleaved to prepare His(trt)-Phe-Tyr-Asp-Gln-Gln-Ala-Arg-Phe ((6S,9S,12S,15S,18S)-1-amino-6-(((S)-1-amino-1-oxo-3-phenylpropan-2-yl)carbamoyl)-18-((S)-2-((S)-2-((S)-2-amino-3-(1-trityl-1H-imidazol-4-yl)propanamido)-3-phenylpropanamido)-3-(4-hydroxyphenyl)propanamido)-12,15-bis(3-amino-3-oxopropyl)-1-imino-9-methyl-8,11,14,17-tetraoxo-2,7,10,13,16-pentaazaicosan-20-oic acid).

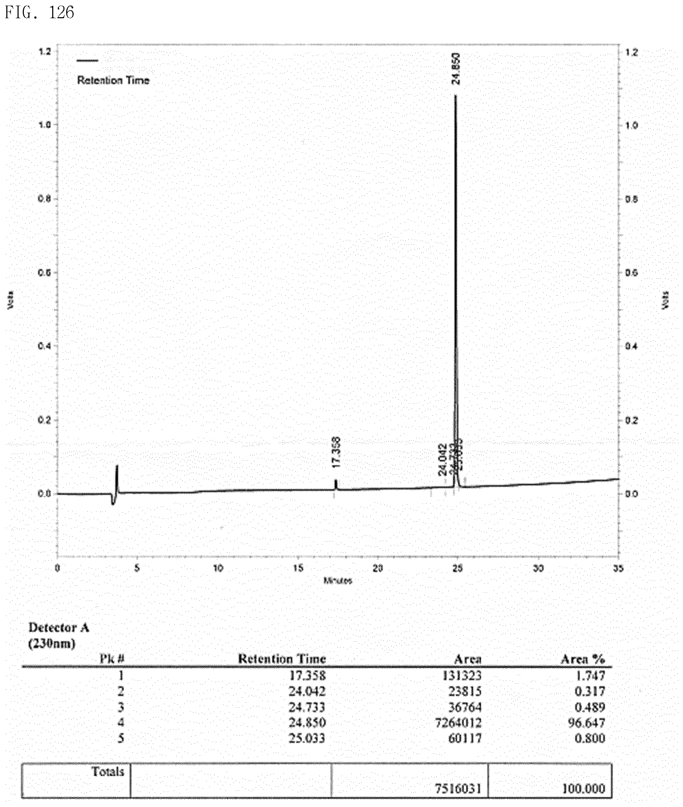

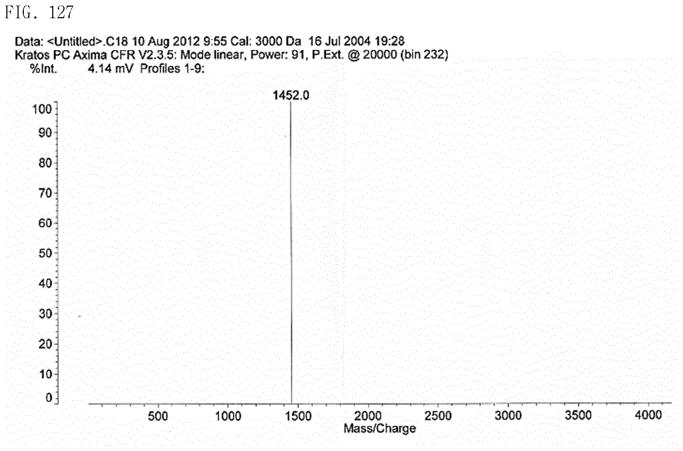

The purity and molecular weight of the HKFY-52 are shown in FIGs. 126 and 127, respectively.

### Example 1.53. Preparation of HKFY-53

Fmoc-Lys(caprylic)-OH obtained from Example 1.1., Fmoc-Ser(tBu)-OH, Fmoc-Gly-OH, and Fmoc-His(trt)-OH were sequentially added to the Fmoc-Phe-Tyr(Boc)-trityl resin obtained from Example 1.47. in the same manner to prepare Fmoc-His(trt)-Gly-Ser(tBu)-Lys(caprylic)-Phe-Tyr(Boc)-trityl resin. The protecting group of the resin was cleaved to prepare His(trt)-Gly-Ser-Lys(caprylic)-Phe-Tyr (N6-octanoyl-N2-(Nt-trityl-L-histidylglycyl-L-seryl)-L-lysyl-L-phenylalanyl-L-tyrosine).

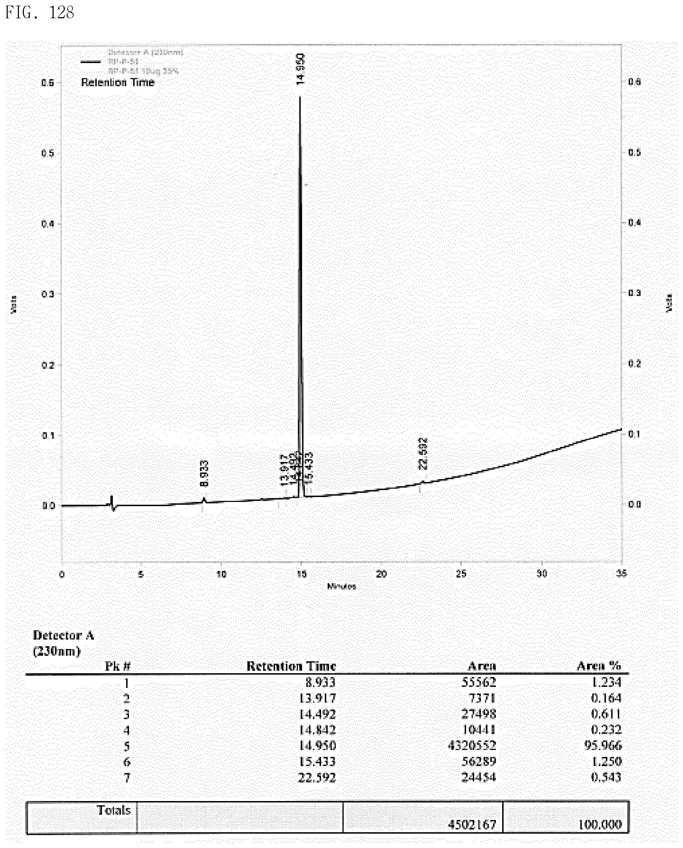

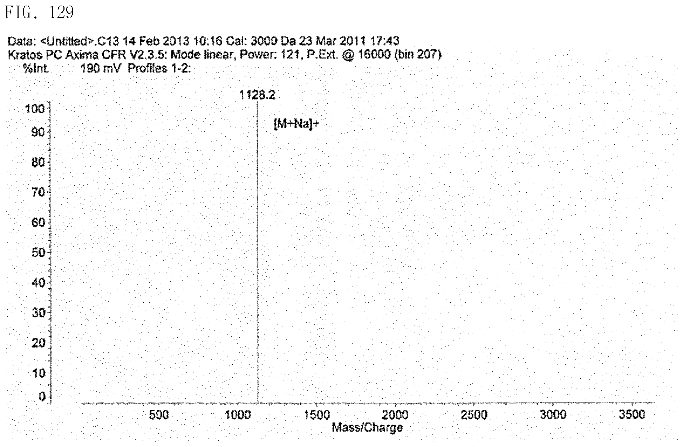

The purity and molecular weight of the HKFY-53 are shown in FIGs. 128 and 129, respectively.

### Example 1.54. Preparation of HKFY-54

Fmoc-Lys(caprylic)-OH obtained from Example 1.1. and DMF and HOBt were added to the Fmoc-Phe-Tyr(Boc)-trityl resin obtained from Example 1.47. to prepare Fmoc-Lys(caprylic)-Phe-Tyr(Boc)-trityl resin. The protecting group of the resin was cleaved to prepare Lys(caprylic)-Phe-Tyr (N6-octanoyl-L-lysyl-L-phenylalanyl-L-tyrosine).

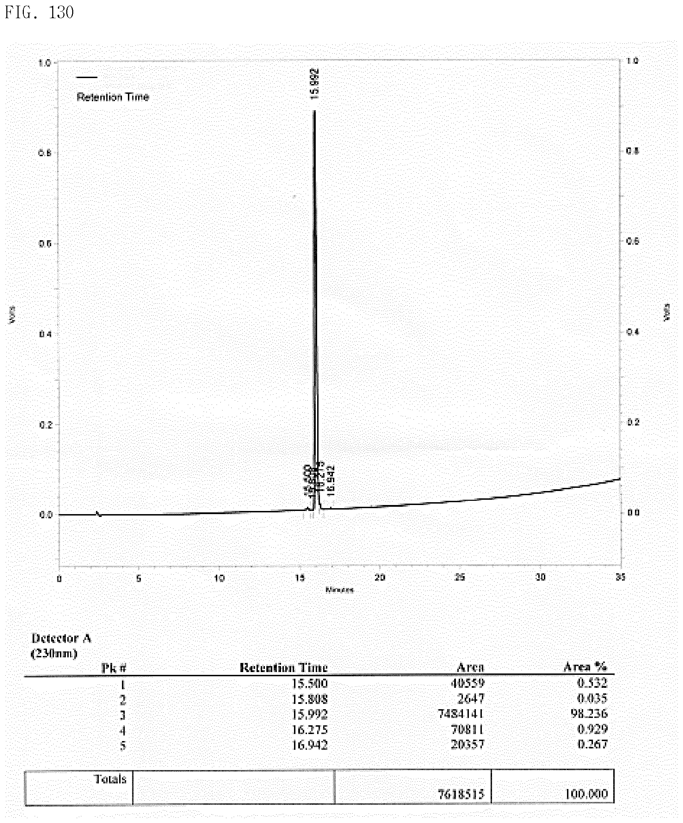

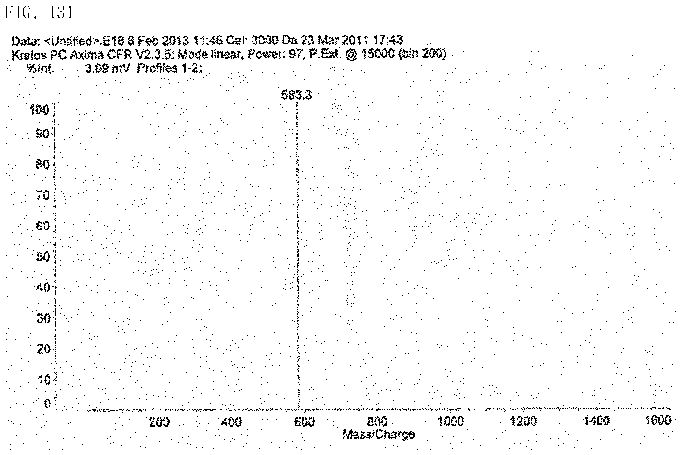

The purity and molecular weight of the HKFY-54 are shown in FIGs. 130 and 131, respectively.

### Example 1.55. Preparation of HKFY-55

Fmoc-His(trt)-OH, and Fmoc-Lys(caprylic)-OH obtained from Example 1.1. were sequentially added to the Fmoc-Phe-Tyr(Boc)-trityl resin obtained from Example 1.47. in the same manner to prepare Fmoc-Lys(caprylic)-His(trt)-Phe-Tyr(Boc)-trityl resin. The protecting group of the resin was cleaved to prepare Lys(caprylic)-His(trt)-Phe-Tyr (Na-(N6-octanoyl-L-lysyl)-Nt-trityl-L-histidyl-L-phenylalanyl-L-tyrosine).

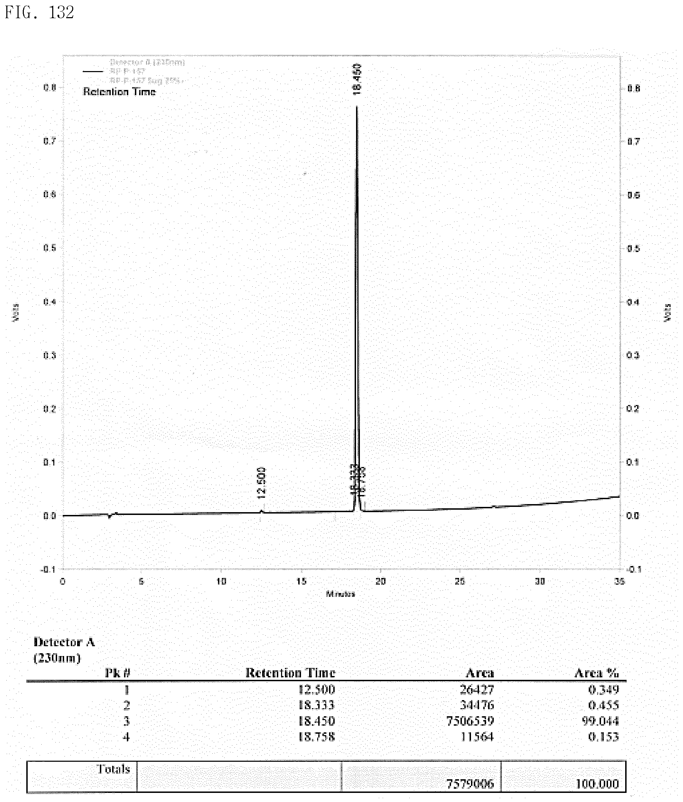

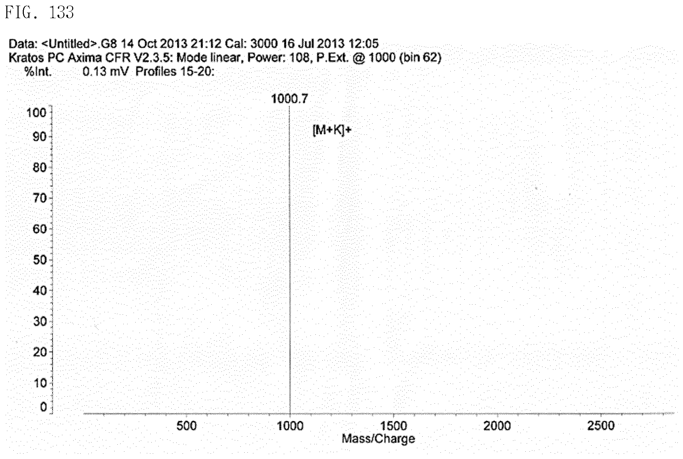

The purity and molecular weight of the HKFY-55 are shown in FIGs. 132 and 133, respectively.

### Example 1.56. Preparation of HKFY-56

Fmoc-Asp(tBu)-Gln(trt)-Gln(trt)-Ala-Arg(pbf)-Phe-trityl resin was prepared in the same manner except that Fmoc-Asp(tBu)-OH was used instead of Fmoc-Ser(tBu)-OH during the preparation process of Example 1.38. DMF containing 2% NH₂NH2·H₂O was added to Fmoc-Lys(dde)-OH, and dde was removed to prepare Fmoc-Lys-OH. DMF containing caproic acid and HOBt and DIC was added to the Fmoc-Lys-OH to prepare Fmoc-Lys(caproic acid)-OH.

Fmoc-Lys(caproic)-OH and Fmoc-His(trt)-OH were sequentially added in the same manner to prepare Fmoc-His(trt)-Lys(caproic)-Phe-Tyr(Boc)-Asp(tBu)-Gln(trt)-Gln(trt)-Ala-Arg(pbf)-Phe-trityl resin. The protecting group of the resin was cleaved to prepare His(trt)-Lys(caproic)-Phe-Tyr-Asp-Gln-Gln-Ala-Arg-Phe ((6S,9S,12S,15S,18S)-1-amino-6-(((S)-1-amino-1-oxo-3-phenylpropan-2-yl)carbamoyl)-18-((S)-2-((S)-2-((S)-2-((S)-2-amino-3-(1-trityl-1H-imidazol-4-yl)propanamido)-6-(non-1-en-2-ylamino)hexanamido)-3-phenylpropanamido)-3-(4-hydroxyphenyl)propanamido)-12,15-bis(3-amino-3-oxopropyl)-1-imino-9-methyl-8,11,14,17-tetraoxo-2,7,10,13,16-pentaazaicosan-20-oic acid).

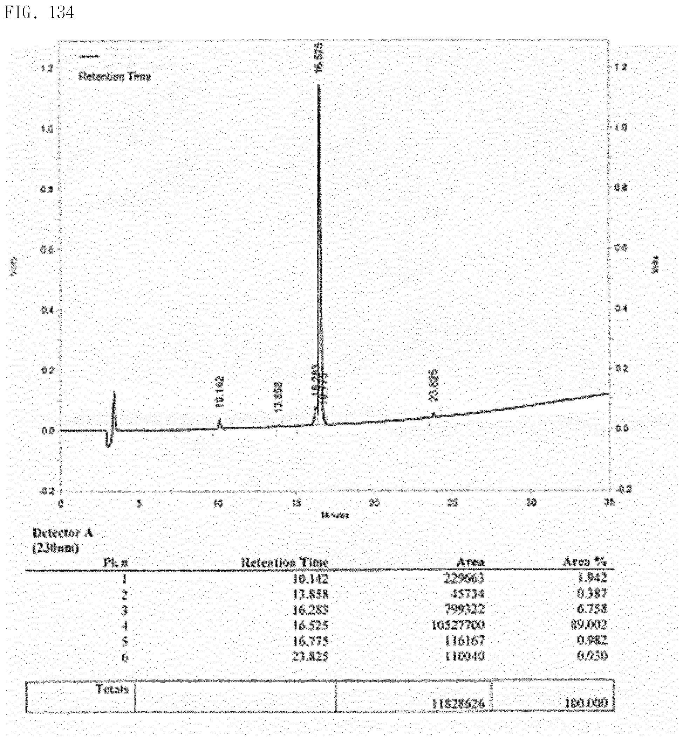

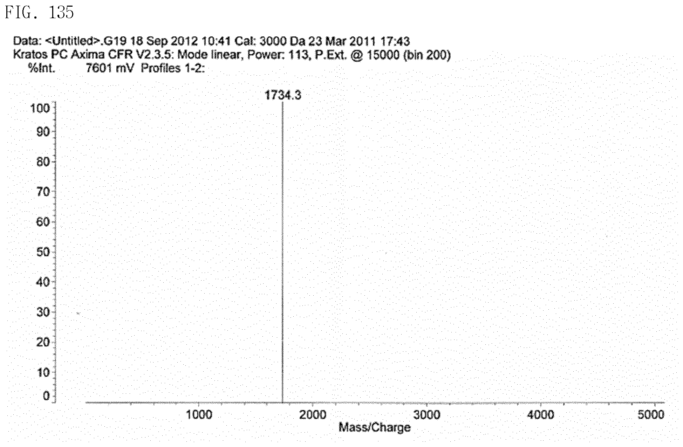

The purity and molecular weight of the HKFY-56 are shown in FIGs. 134 and 135, respectively.

### Example 1.57. Preparation of HKFY-57

His(2-phenyl)-Lys(caprylic)-d-Phe(methyl)-Tyr (((R)-2-((S)-2-((S)-2-amino-3-(2-phenyl-1H-imidazol-4-yl)propanamido)-6-octanamidohexanamido)-3-(p-tolyl)propanoyl)-L-tyrosine) was prepared in the same manner except that Boc-His(2-phenyl)-OH was used instead of Boc-His(benzhydryl)-OH among the constituent amino acids of Example 1.34.

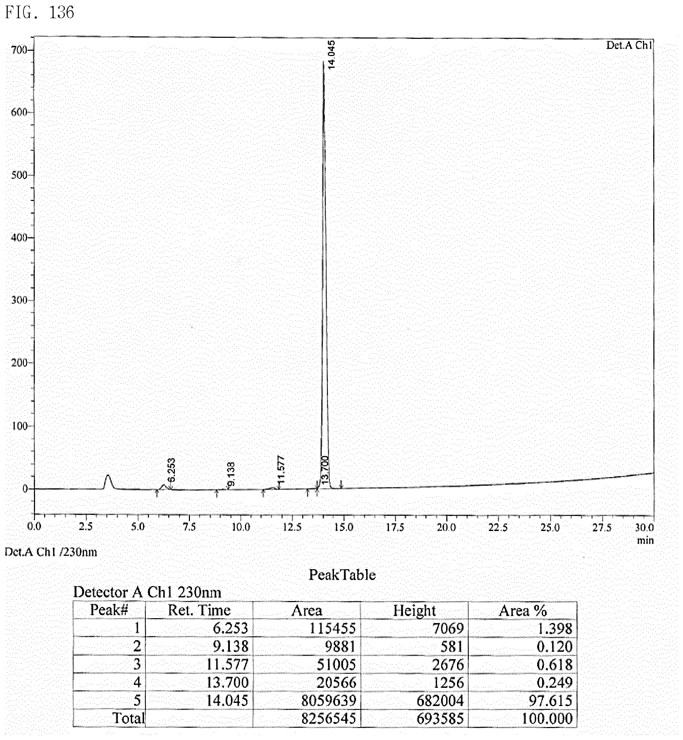

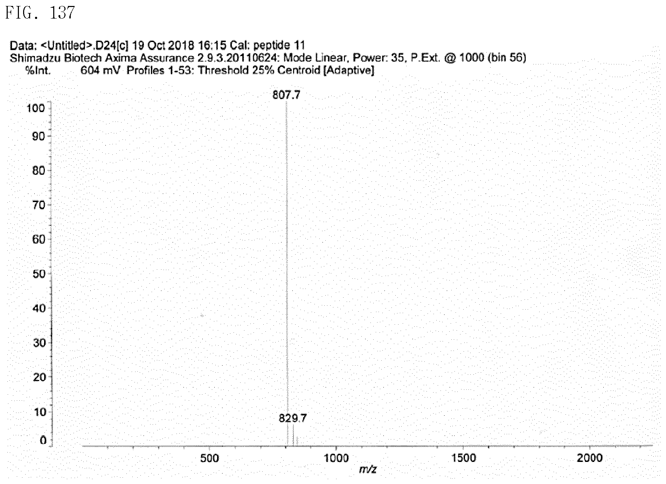

The purity and molecular weight of the HKFY-57 are shown in FIGs. 136 and 137, respectively.

### Example 1.58. Preparation of HKFY-58

His(1-phenyl)-Lys(caprylic)-d-Phe(methyl)-Tyr (((R)-2-((S)-2-((S)-2-amino-3-(1-phenyl-1H-imidazol-4-yl)propanamido)-6-octanamidohexanamido)-3-(p-tolyl)propanoyl)-L-tyrosine) was prepared in the same manner except that Boc-His(1-phenyl)-OH was used instead of Boc-His(benzhydryl)-OH among the constituent amino acids of Example 1.34.

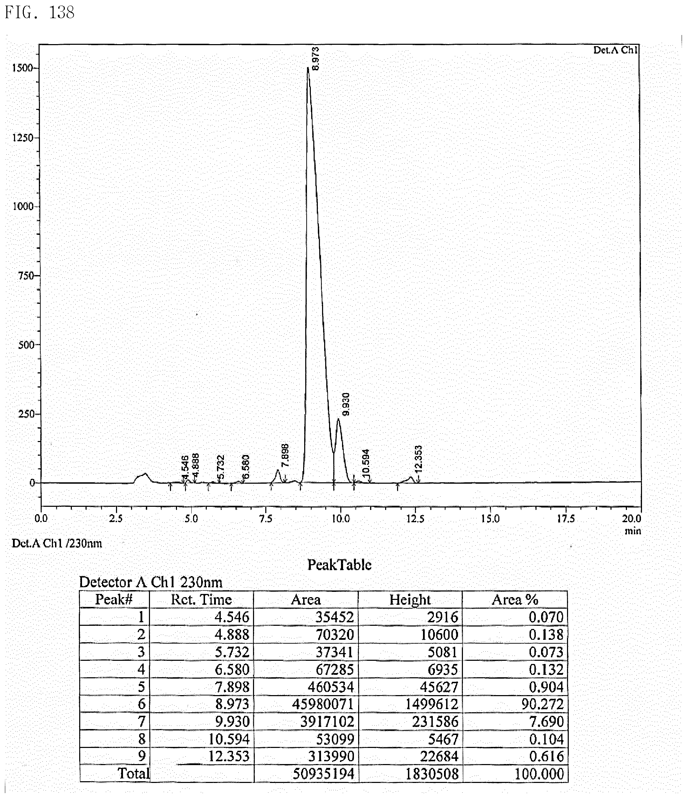

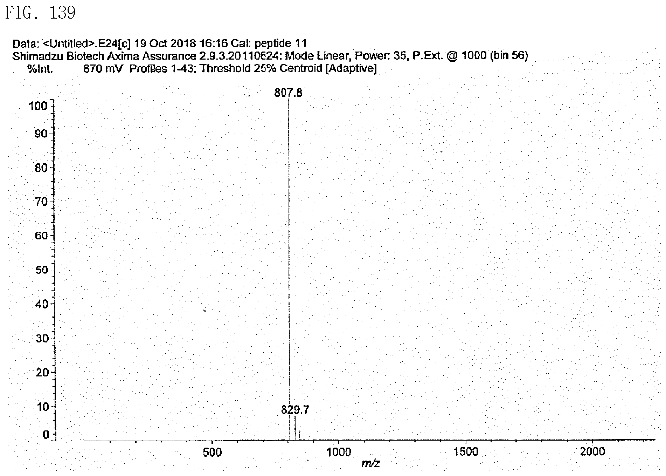

The purity and molecular weight of the HKFY-58 are shown in FIGs. 138 and 139, respectively.

### Example 1.59. Preparation of HKFY-59

His(1,2-diphenyl)-Lys(caprylic)-d-Phe(methyl)-Tyr (((R)-2-((S)-2-((S)-2-amino-3-(12-diphenyl-1H-imidazol-4-yl)propanamido)-6-octanamidohexanamido)-3-(p-tolyl)propanoyl)-L-tyrosine) was prepared in the same manner except that Boc-His(1,2-diphenyl)-OH was used instead of Boc-His(benzhydryl)-OH among the constituent amino acids of Example 1.34.

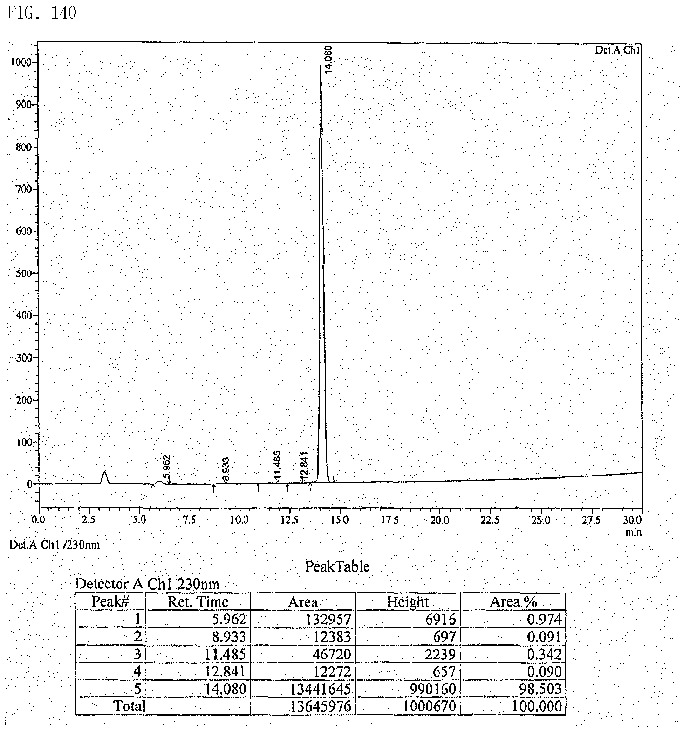

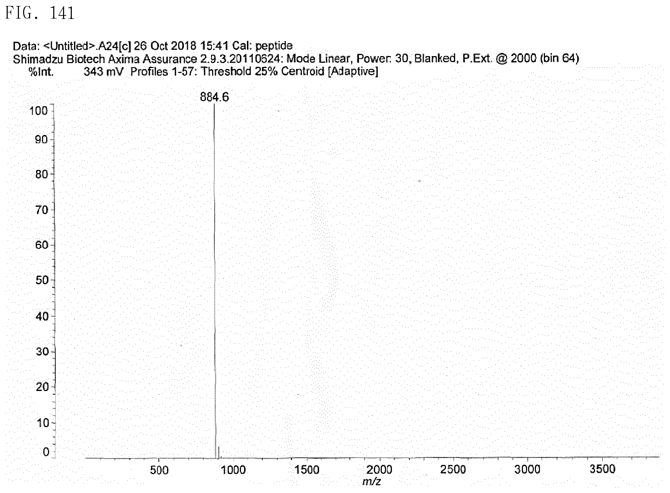

The purity and molecular weight of the HKFY-59 are shown in FIGs. 140 and 141, respectively.

### Example 1.60. Preparation of HKFY-60

His(2-(4-tert-butyl)phenyl)-Lys(caprylic)-d-Phe(methyl)-Tyr (((R)-2-((S)-2-((S)-2-amino-3-(2-(4-(tert-butyl)phenyl-1H-imidazol-4-yl)propanmido)-6-octanamidohexanamido)-3-(p-tolyl)propanoyl)-L-tyrosine) was prepared in the same manner except that Boc-His(2-(4-tert-butyl)phenyl)-OH was used instead of Boc-His(benzhydryl)-OH among the constituent amino acids of Example 1.34.

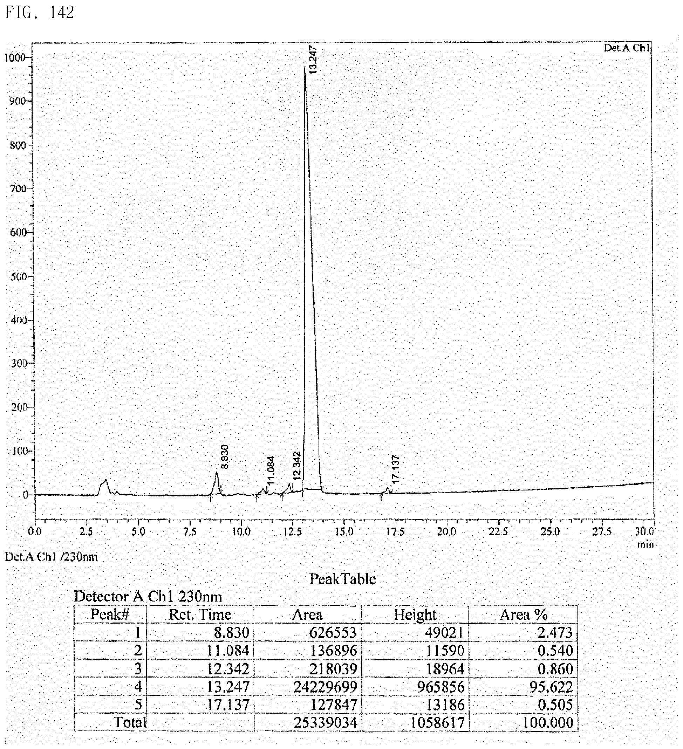

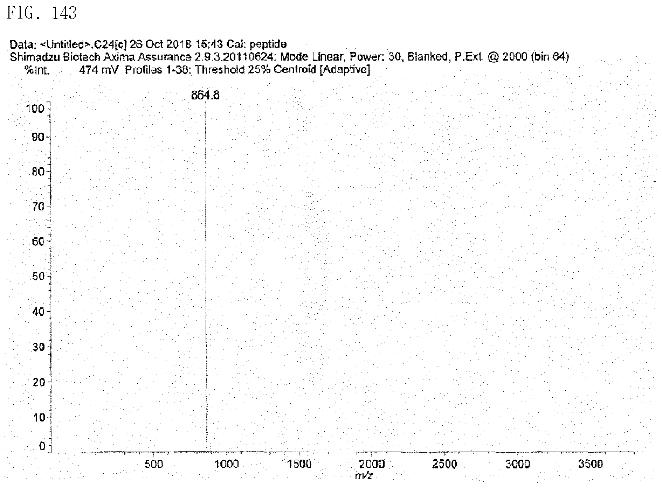

The purity and molecular weight of the HKFY-60 are shown in FIGs. 142 and 143, respectively.

### Example 1.61. Preparation of HKFY-61

His(1-(4-tert-butyl)phenyl)-Lys(caprylic)-d-Phe(methyl)-Tyr (((R)-2-((S)-2-((S)-2-amino-3-(1-(4-(tert-butynphenyn-1H-imidazol-4-yl)propanamido)-6-octanamidohexanamido)-3-(p-tolyl)propanoyl)-L-tyrosine) was prepared in the same manner except that Boc-His(1-(4-tert-butyl)phenyl)-OH was used instead of Boc-His(benzhydryl)-OH among the constituent amino acids of Example 1.34.

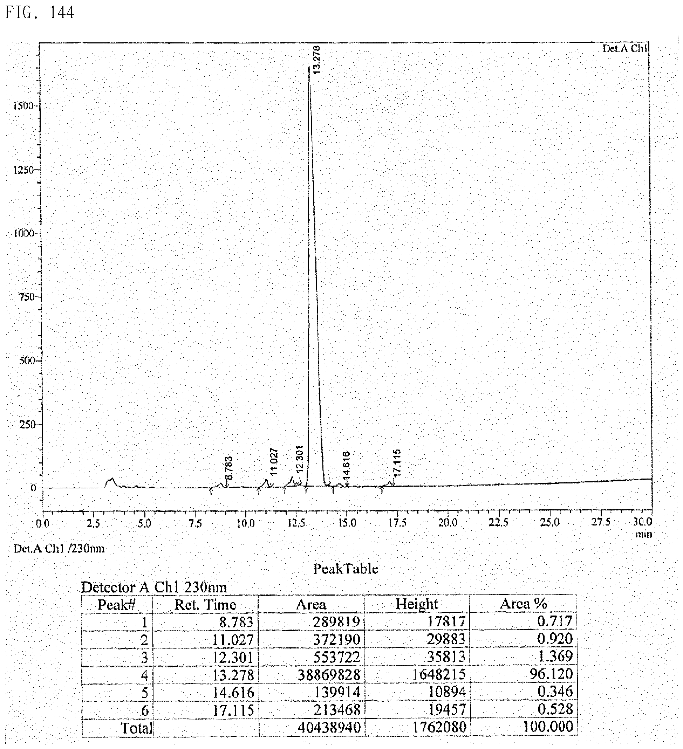

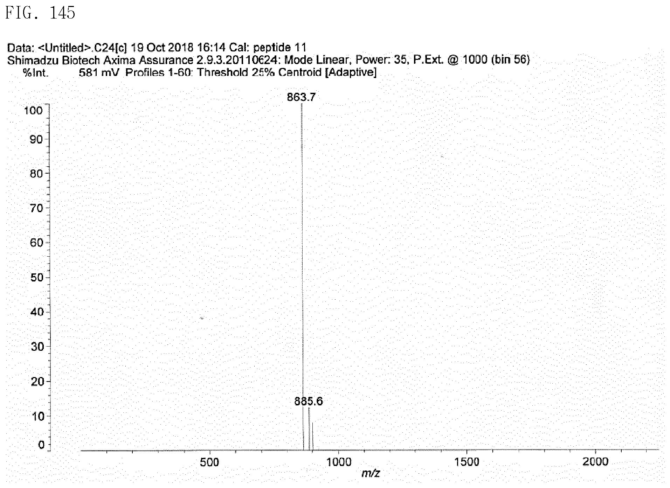

The purity and molecular weight of the HKFY-61 are shown in FIGs. 144 and 145, respectively.

### Example 1.62. Preparation of HKFY-62

d-His(benzhydryl)-Lys(caprylic)-d-Phe(methyl)-Tyr (((R)-2-((S)-2-((R)-2-amino-3-(1-benzhydryl-1H -imidazol-4-yl)propanamido)-6-octanamidohexanamido)-3-(p-tolyl)propanoyl)-L-tyrosine) was prepared in the same manner except that Boc-d-His(benzhydryl)-OH was used instead of Boc-His(benzhydryl)-OH among the constituent amino acids of Example 1.34.

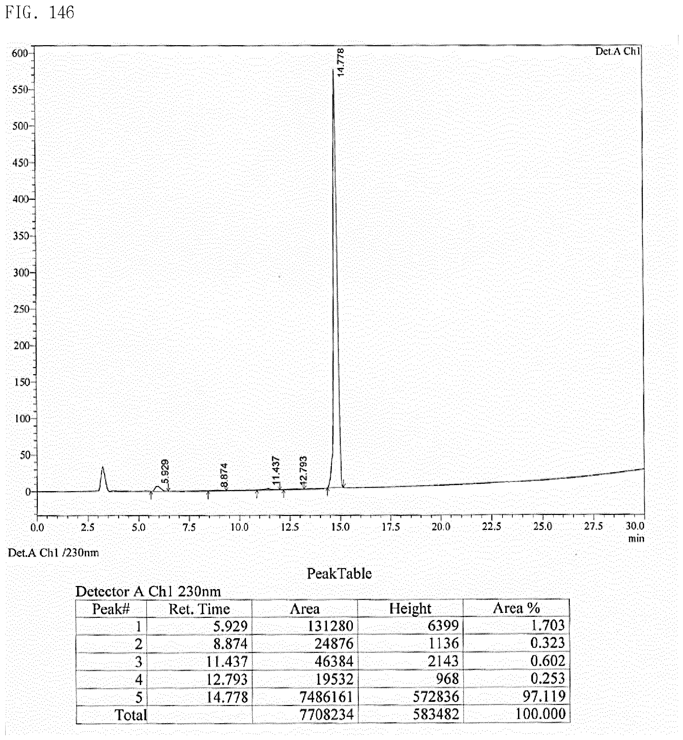

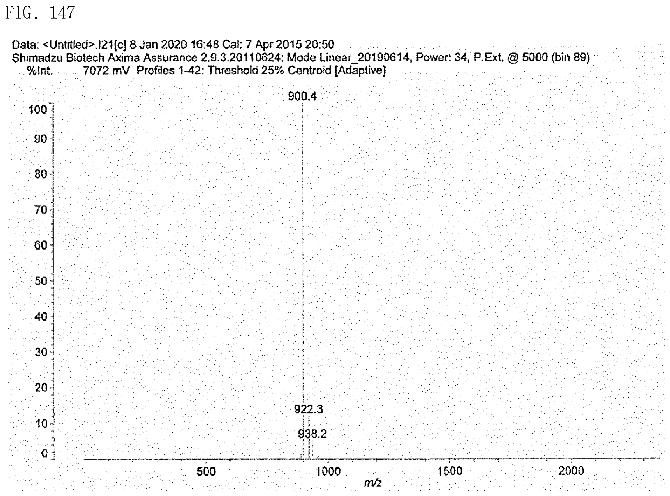

The purity and molecular weight of the HKFY-62 are shown in FIGs. 146 and 147, respectively.

### Example 1.63. Preparation of HKFY-63

His(thiophene)-Lys(caprylic)-d-Phe(methyl)-Tyr (((R)-2-(Y(S)-2-((S)-2-amino-3-(2-(thiophen-2-yl)-1H-imidazol-4-yl)propanamido)-6-octanamidohexanamido)-3-(p-tolyl)propanoyl)-L-tyrosine) was prepared in the same manner except that Boc-His(thiophene)-OH was used instead of Boc-His(benzhydryl)-OH among the constituent amino acids of Example 1.34.

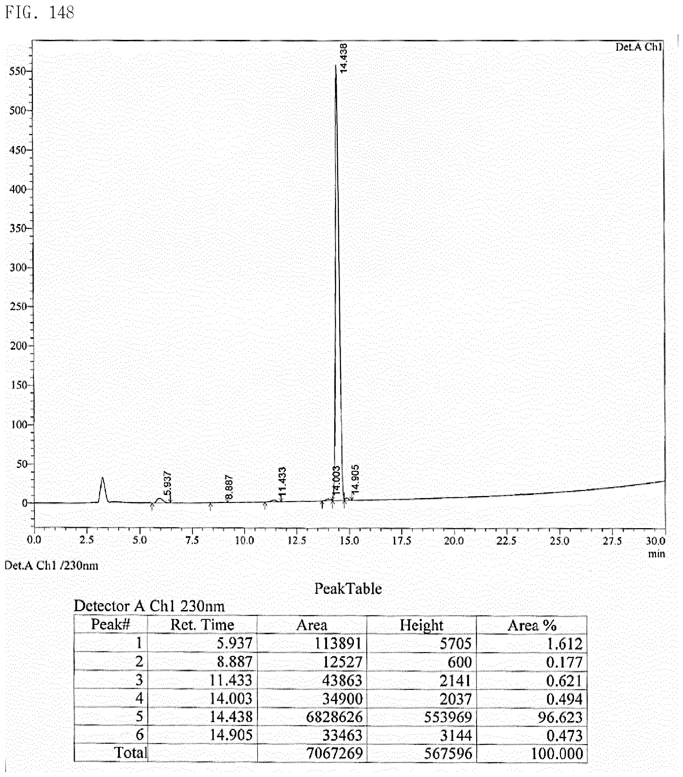

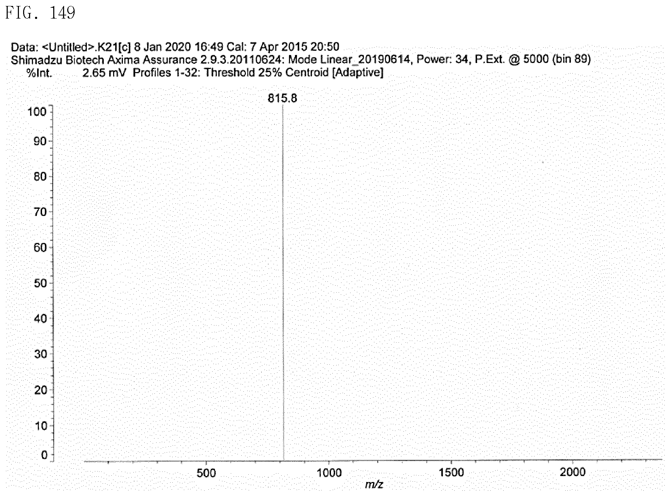

The purity and molecular weight of the HKFY-63 are shown in FIGs. 148 and 149, respectively.

### Example 1.64. Preparation of HKFY-64

His(4-methoxybenzhydryl)-Lys(caprylic)-d-Phe(methyl)-Tyr (((2R)-2-((2S)-2-((2S)-2-amino-3-(1-((4-methoxyphenyl)(phenyl)methyl)-1H-imidazol-4-yl)propanamido)-6-octanamidohexanamido)-3-(p-tolyl)propanoyl)-L-tyrosine) was prepared in the same manner except that Boc-His(4-methoxybenzhydryl)-OH was used instead of Boc-His(benzhydryl)-OH among the constituent amino acids of Example 1.34.

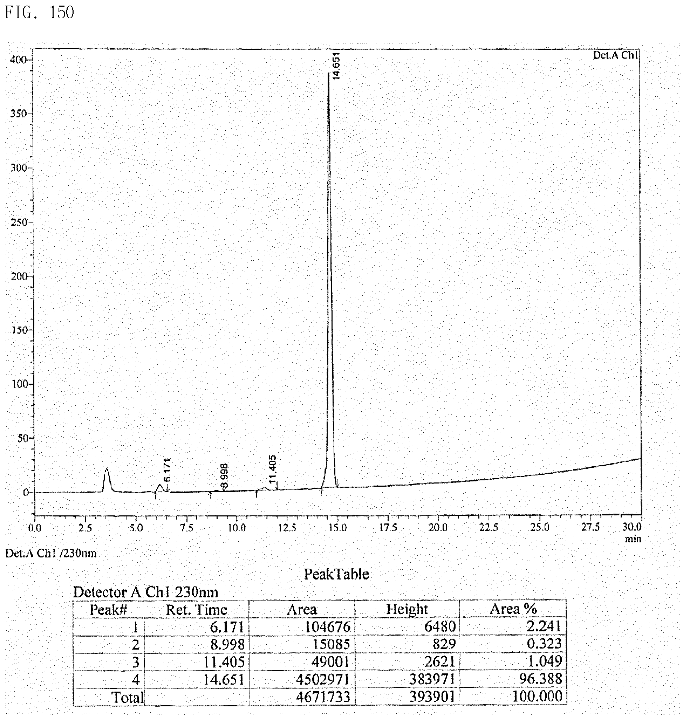

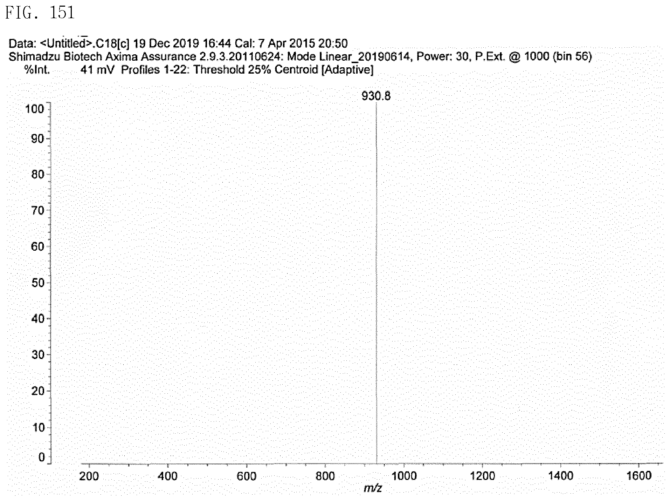

The purity and molecular weight of the HKFY-64 are shown in FIGs. 150 and 151, respectively.

### Example 1.65. Preparation of HKFY-65

His(4-hydroxybenzhydryl)-Lys(caprylic)-d-Phe(methyl)-Tyr (((2R)-2-((2S)-2-((2S)-2-amino-3 -(1-((4-hydroxyphenyl)(phenyl)methyl)-1H-imidazol-4-yl)propanamido)-6-octanamidohexanamido)-3-(p-tolyl)propanoyl)-L-tyrosine) was prepared in the same manner except that Boc-His(4-hydroxybenzhydryl)-OH was used instead of Boc-His(benzhydryl)-OH among the constituent amino acids of Example 1.34.

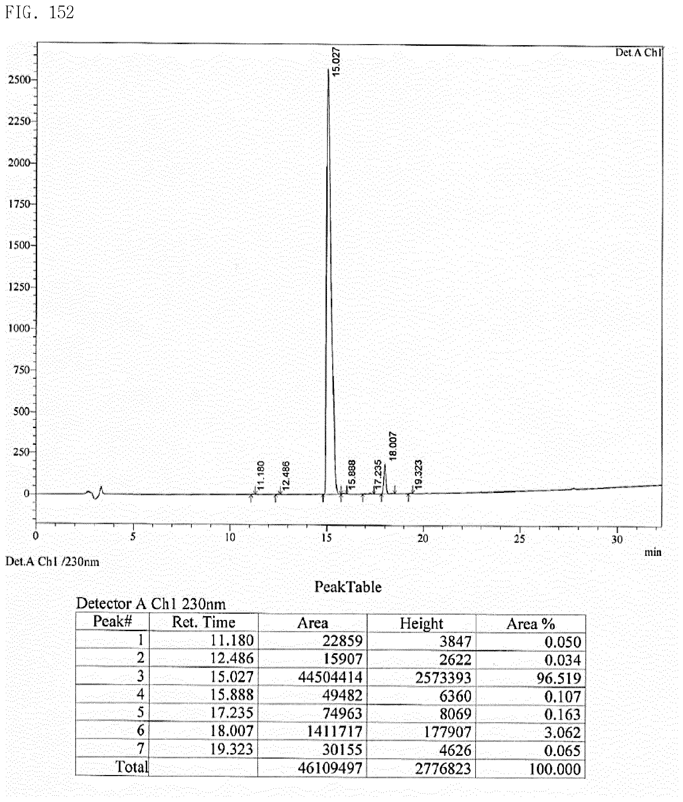

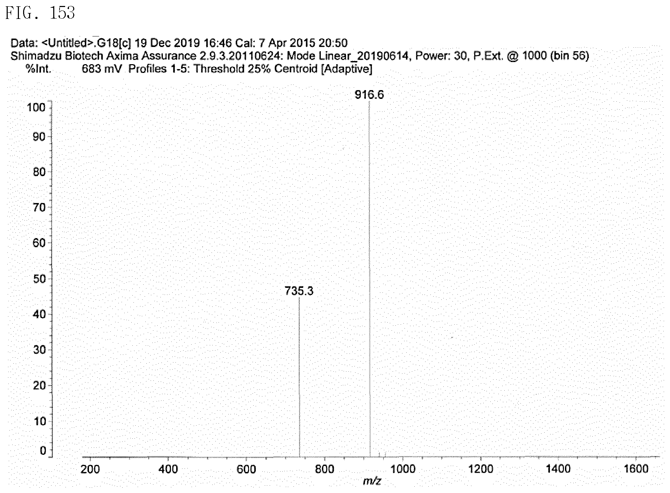

The purity and molecular weight of the HKFY-65 are shown in FIGs. 152 and 153, respectively.

### Example 1.66. Preparation of HKFY-66

His(benzhydryl)-Lys(caprylic)-d-Phe(4-methyl)-Tyr-NH₂, (N-((S)-5-((S)-2-amino-3-(1-benzhydryl-1H-imidazol-4-yl)propanamido)-6-(((R)-1-(((S)-1-amino-3-(4-hydroxyphenyl-1-oxopropan-2-yl)amino)-1-oxo-3-(p-tolyl)propan-2-yl)amino)-6-oxohexyl)octanamide) was prepared in the same manner except that Fmoc-Tyr-wang resin was used instead of the trityl resin among the constituent amino acids of Example 1.34.

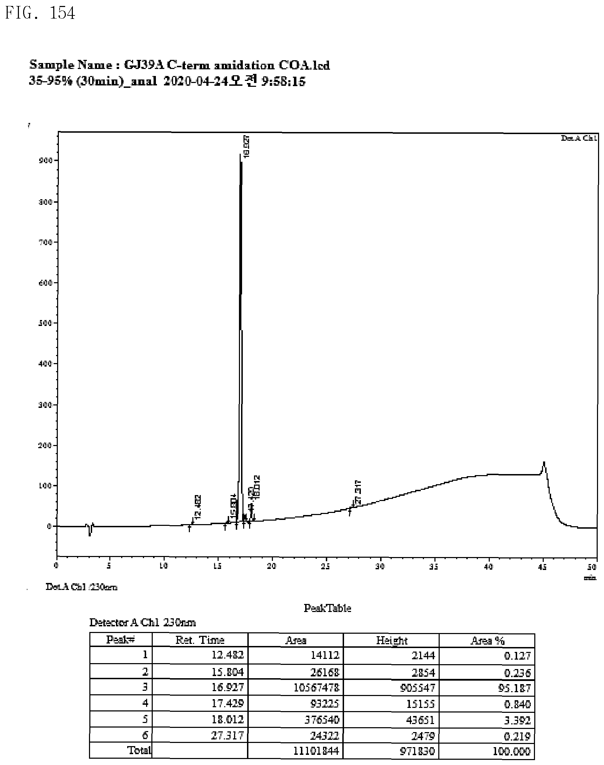

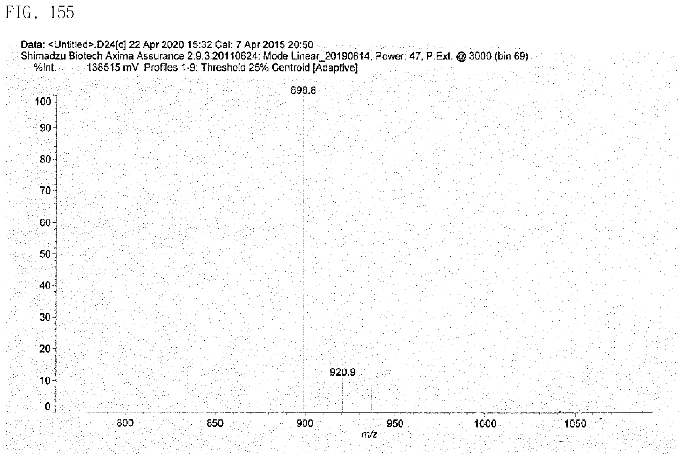

The purity and molecular weight of the HKFY-66 are shown in FIGs. 154 and 155, respectively.

### Example 1.67. Preparation of HKFY-67

His(benzhydryl)-Lys(caprylic)-d-Phe(4-methyl)-Tyr(3-chloro) ((S)-2-((R)-2-((S)-2-((S)-2-amino-3-(1-benzhydryl-1H-imidazol-4-yl)propanamido)-6-octanamidohexanamido)-3-(p-tolyl)propanamido)-3-(3-chloro-4-hydroxyphenyl)propanoic acid) was prepared in the same manner except that Fmoc-Tyr(3-chloro) was used instead of Fmoc-Tyr(tBu) among the constituent amino acids of Example 1.34.

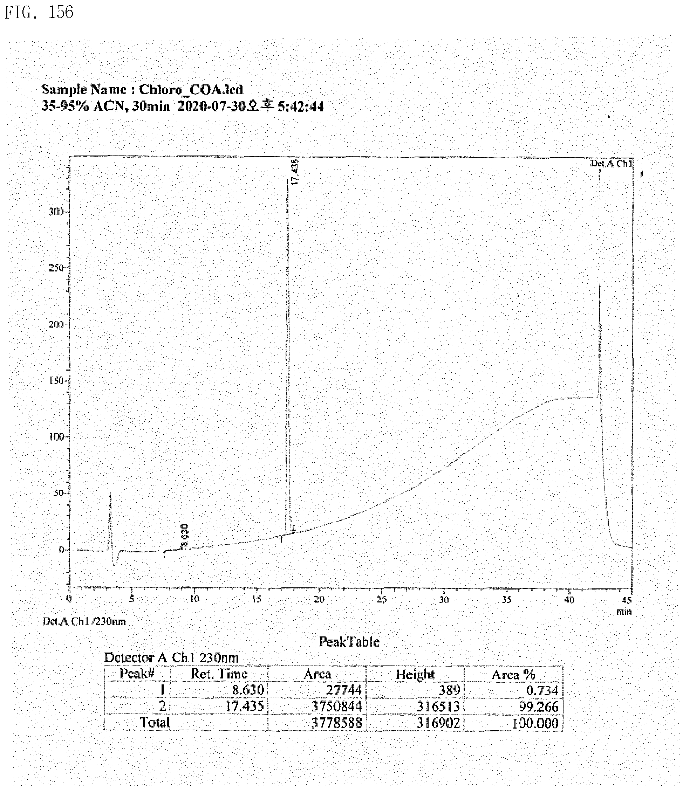

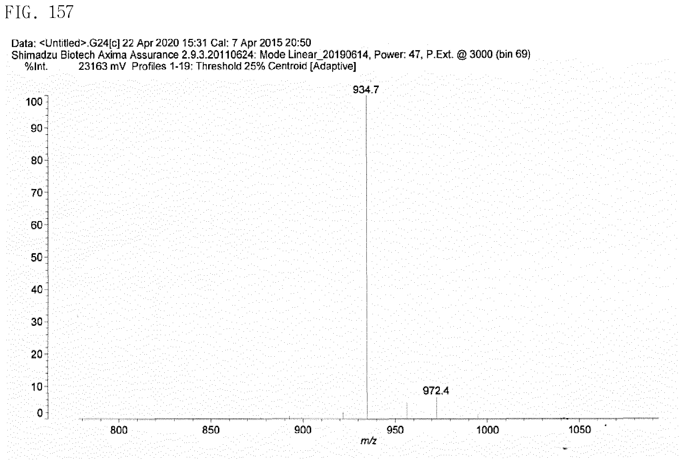

The purity and molecular weight of the HKFY-67 are shown in FIGs. 156 and 157, respectively.

### Example 1.68. Preparation of HKFY-68

His(benzhydryl)-Lys(caprylic)-d-Phe(4-methyl)-Tyr(3-nitro) ((S)-2-((R)-2-((S)-2-((S)-2-amino-3-(1-benzhydryl-1H-imidazol-4-yl)propanamido)-6-octanamidohexanamido)-3-(p-tolyl)propanamido)-3-(4-hydroxy-3-nitrophenyl)propanoic acid) was prepared in the same manner except that Fmoc-Tyr(3-nitro) was used instead of Fmoc-Tyr(tBu) among the constituent amino acids of Example 1.34.

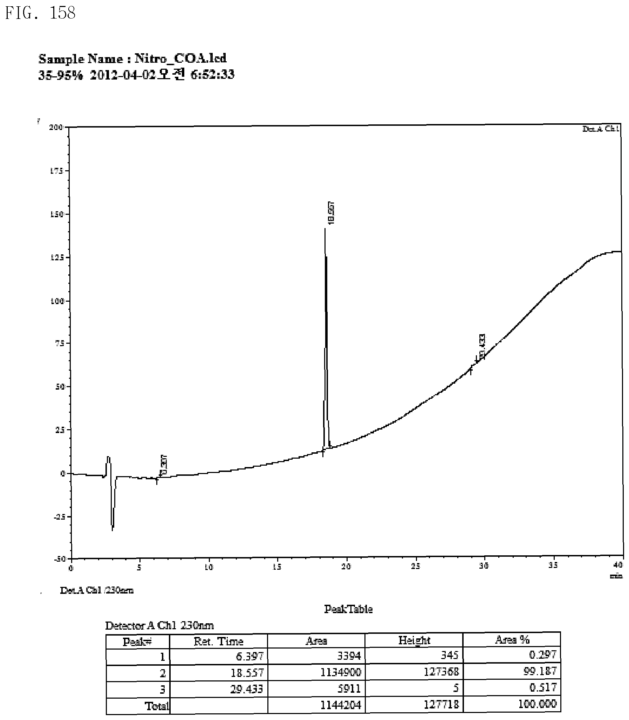

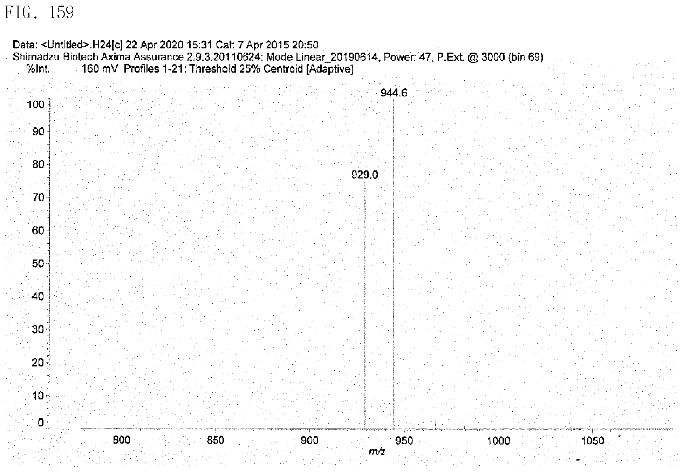

The purity and molecular weight of the HKFY-68 are shown in FIGs. 158 and 159, respectively.

### Example 1.69. Preparation of HKFY-69

His(benzhydryl)-Lys(caprylic)-d-Phe(4-methyl)-Tyr(3,5-nitro) ((S)-2-((R)-2-((S)-2-((S)-2-amino-3-(1-benzhydryl-1H-imidazol-4-yl)propanamido)-6-octanamidohexanamido)-3-(p-tolyl)propanamido)-3-(4-hydroxy-3,5-dinitrophenyl)propanoic acid) was prepared in the same manner except that Fmoc-Tyr(3,5-nitro) was used instead of Fmoc-Tyr(tBu) among the constituent amino acids of Example 1.34.

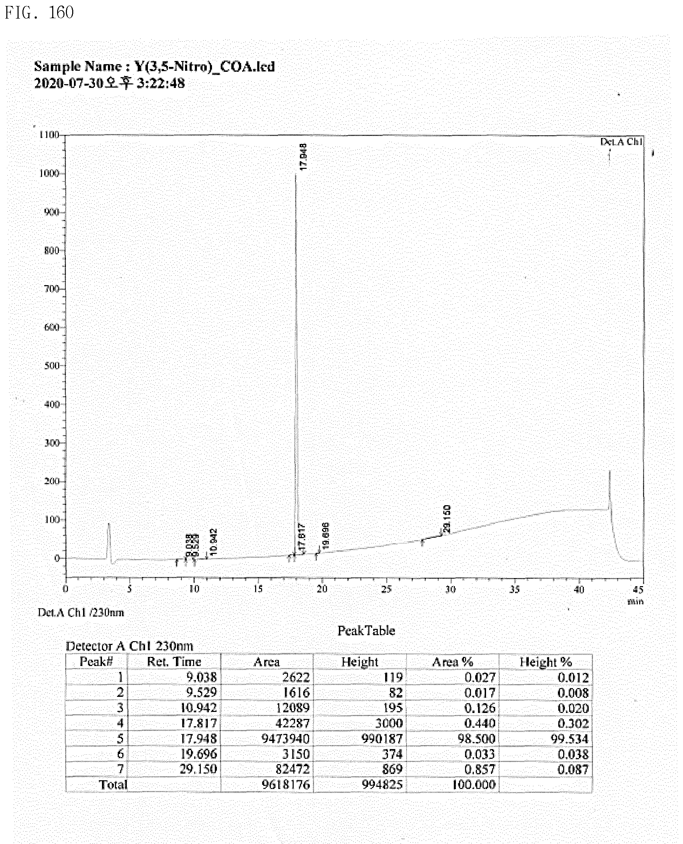

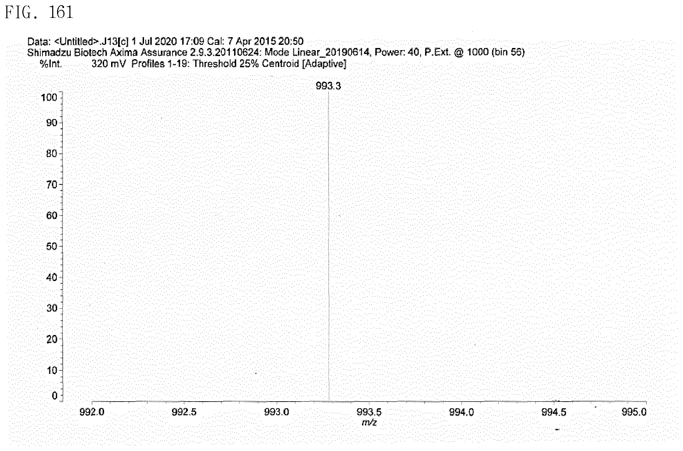

The purity and molecular weight of the HKFY-69 are shown in FIGs. 160 and 161, respectively.

### Example 1.70. Preparation of HKFY-70

His(benzhydryl)-Lys(caprylic)-d-Phe(4-methyl)-Tyr(3-amino) ((S)-2-((R)-2-((S)-2-((S)-2-amino-3-(1-benzhydryl-1H-imidazol-4-yl)propanamido)-6-octanamidohexanamido)-3-(p-tolyl)propanamido)-3-(3-amino-4-hydroxyphenyl)propanoic acid) was prepared in the same manner except that Fmoc-Tyr(3-amino) was used instead of Fmoc-Tyr(tBu) among the constituent amino acids of Example 1.34.

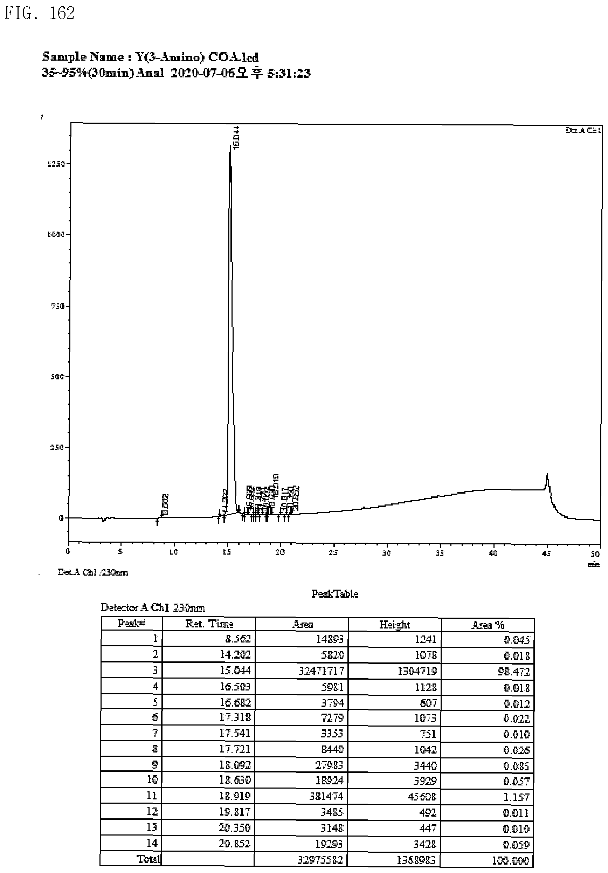

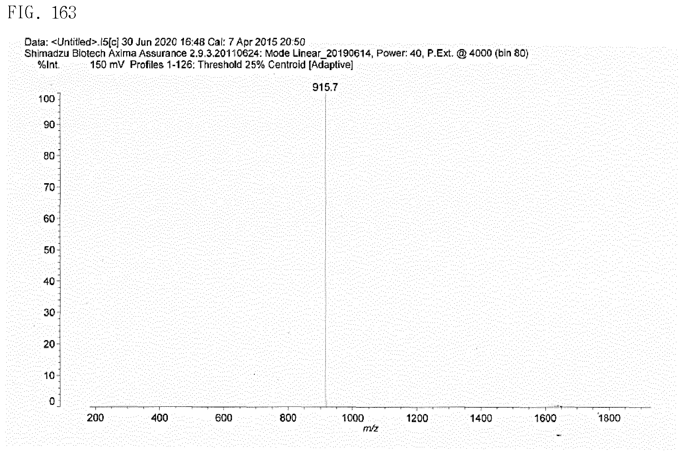

The purity and molecular weight of the HKFY-70 are shown in FIGs. 162 and 163, respectively.

### Example 1.71. Preparation of HKFY-71

His(benzhydryl)-Orn(caprylic)-d-Phe(4-methyl)-Tyr (((R)-2-((S)-2-((S)-2-amino-3-(1-benzhydryl-1H-imidazol-4-yl)propanamido)-5-octanamidopentanamido)-3-(p-tolyl)propanoyl)-L-tyrosine) was prepared in the same manner except that Fmoc-Orn(dde) was used instead of Fmoc-Lys(dde) among the constituent amino acids of Example 1.34.

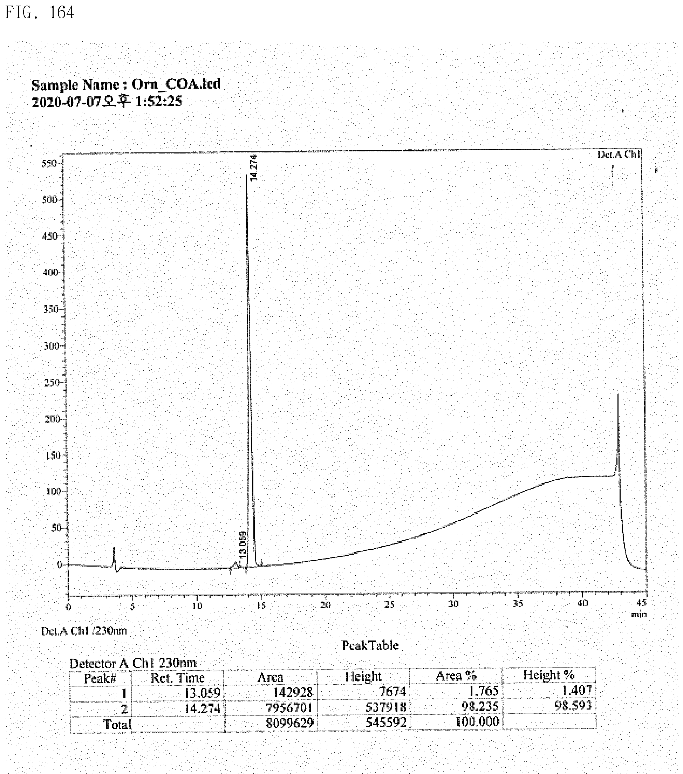

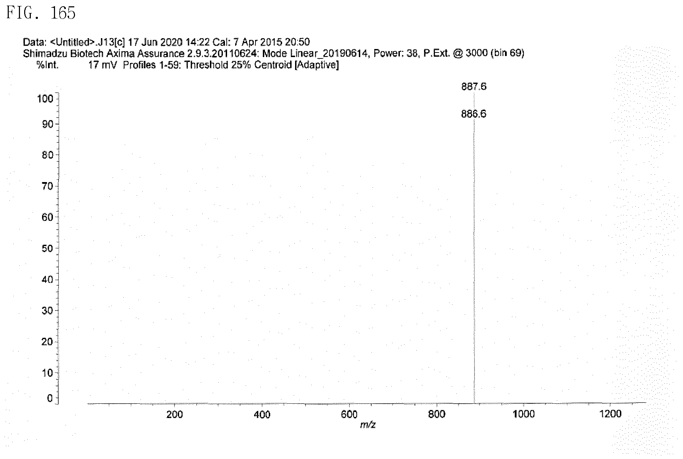

The purity and molecular weight of the HKFY-71 are shown in FIGs. 164 and 165, respectively.

### Example 1.72. Preparation of HKFY-72

Biotin-His(benzhydryl)-Lys(caprylic)-d-Phe(4-methyl)-Tyr (((S)-2-((S)-2-((S)-3-(1-benzhydryl-1H-imidazol-4-yl)-2-(5-((3aS,4S,6aR)-2-oxohexahydro-1H-thieno[3,4-dlimidazol-4-yl)pentanamido)propanamido)-6-octanamidohexanamido)-3-(p-tolyl)propanoyl)-L-tyrosine) was prepared in the same manner as in the synthesis process of D-biotin of Example 1.2. using His(benzhydryl)-Lys(caprylic acid)-d-Phe(4-methyl)-Tyr-OH of Example 1.34.

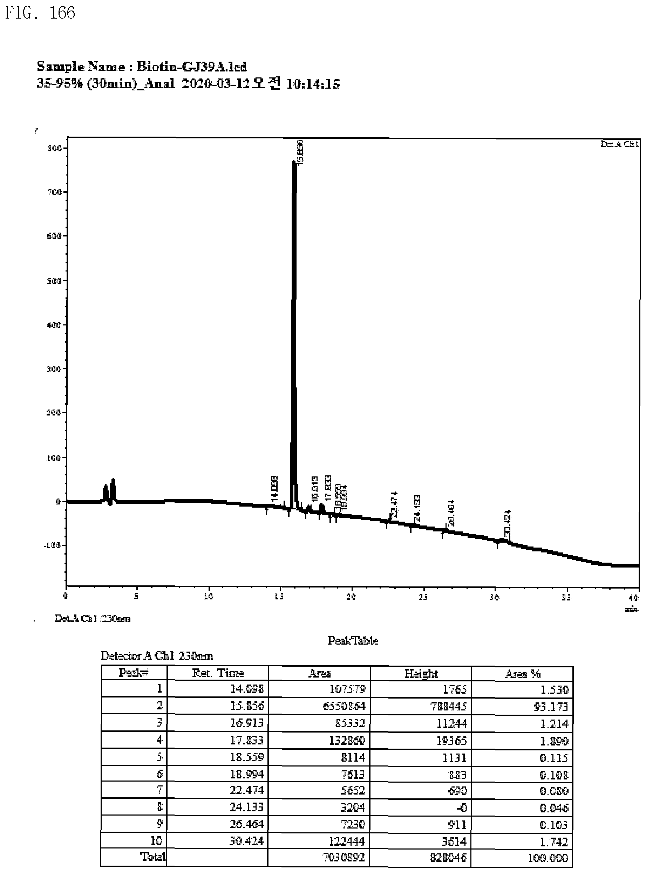

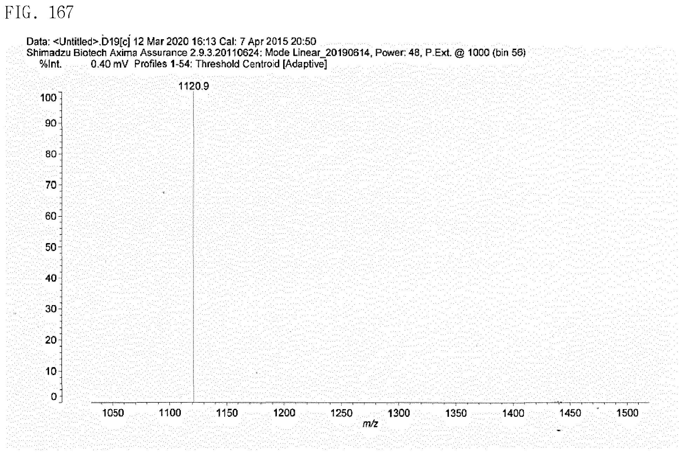

The purity and molecular weight of the HKFY-72 are shown in FIGs. 166 and 167, respectively.

### Example 1.73. Preparation of HKFY-73

H(trt)Lys(caprylic)-d-3Pal-Tyr (((R)-2-((S)-2-((S)-2-amino-3-(1-trityl-1H-imidazol-4-yl)propanamido)-6-octanamidohexanamido)-3-(pyridin-3-yl)propanoyl)-L-tyrosine) was prepared in the same manner except that Fmoc-d-3Pal was used instead of Fmoc-d-Phe-OH among the constituent amino acids of Example 1.12.

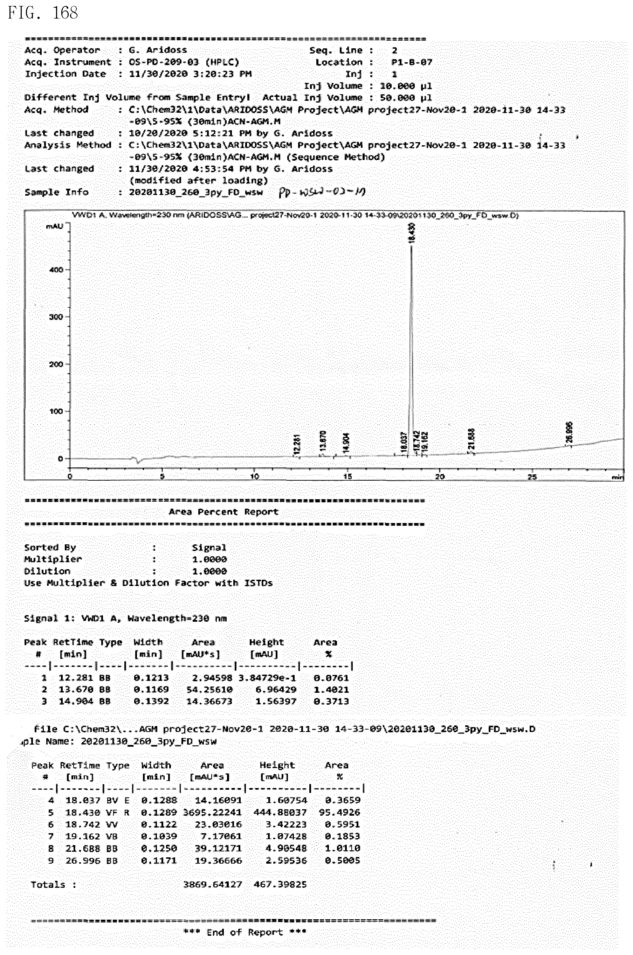

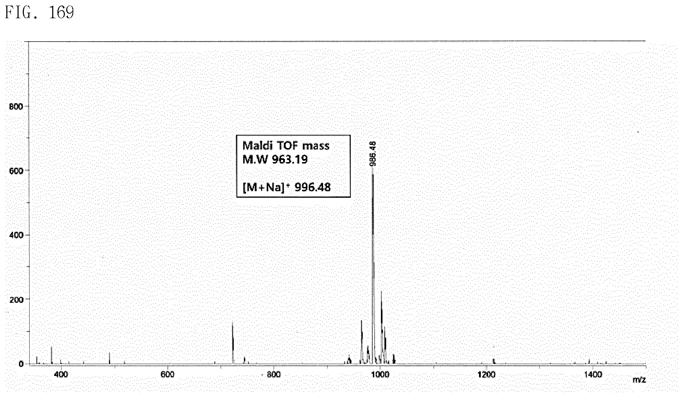

The purity and molecular weight of the HKFY-73 are shown in FIGs. 168 and 169, respectively.

### Example 1.74. Preparation of HKFY-74

H(trt)Lys(caprylic-Tyr (((R)-2-((S)-2-((S)-2-amino-3-(1-trityl-1H-imidazol-4-yl)propanamido)-6-octanamidohexanamido)-3-(naphthalen-2-yl)propanoyl)-L-tyrosine) was prepared in the same manner except that Fmoc-d-2Nal was used instead of Fmoc-d-3Pal among the constituent amino acids of Example 1.73.

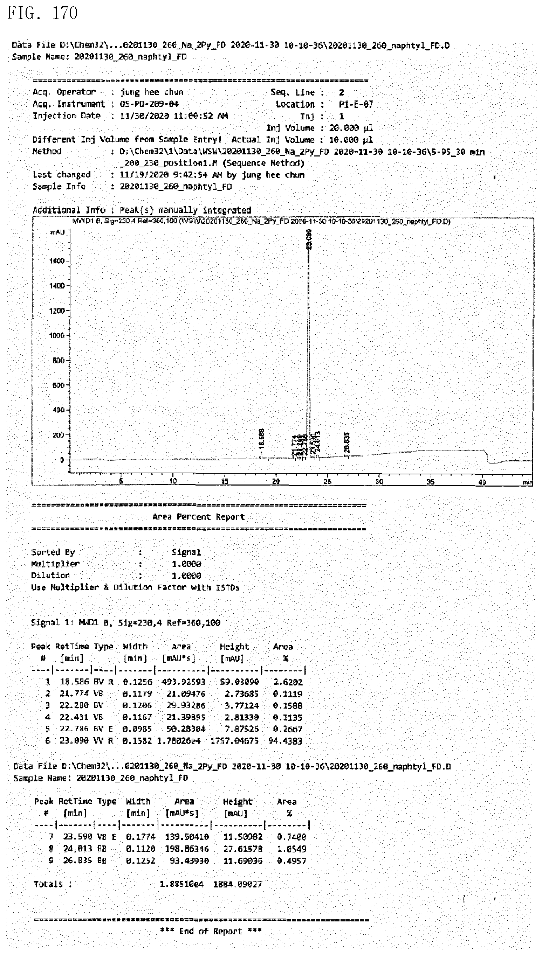

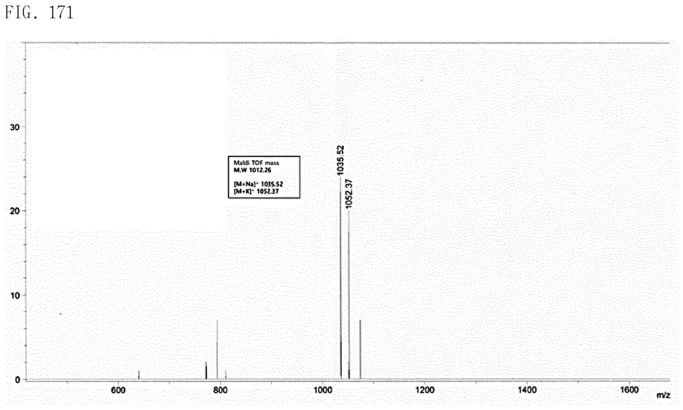

The purity and molecular weight of the HKFY-74 are shown in FIGs. 170 and 171, respectively.

### Example 1.75. Preparation of HKFY-75

His(tosyl)-Lys(caprylic)-d-Phe(4-methyl)-Tyr (((R)-2-((S)-2-((S)-2-amino-3-(1-tosyl-1H-imidazol-4-yl)propanamido)-6-octanamidohexanamido)-3-(p-tolyl)propanoyl)-L-tyrosine) was prepared in the same manner except that Boc-His(tosyl)-OH was used instead of Boc-His(benzhydryl)-OH among the constituent amino acids of Example 1.34.

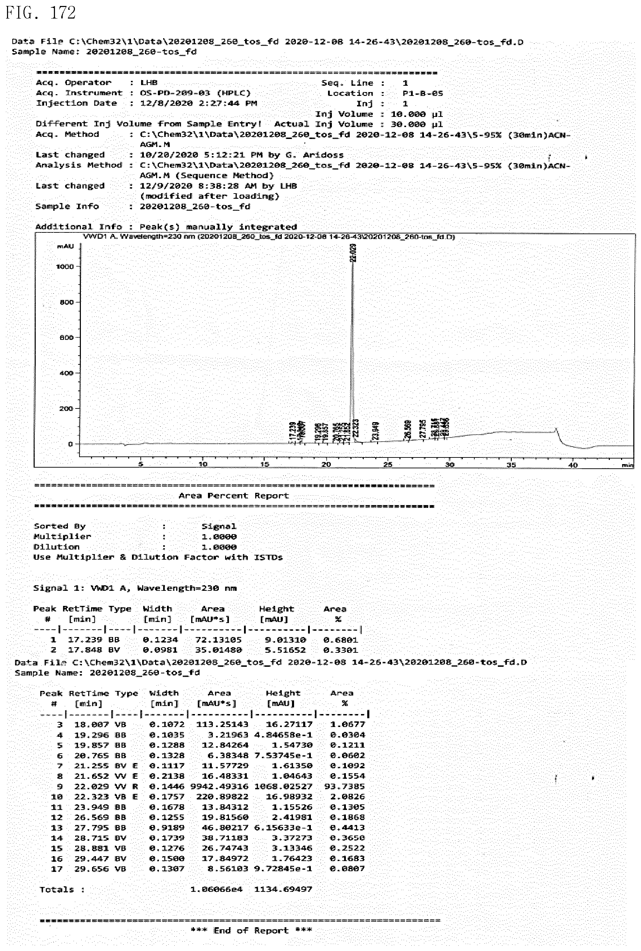

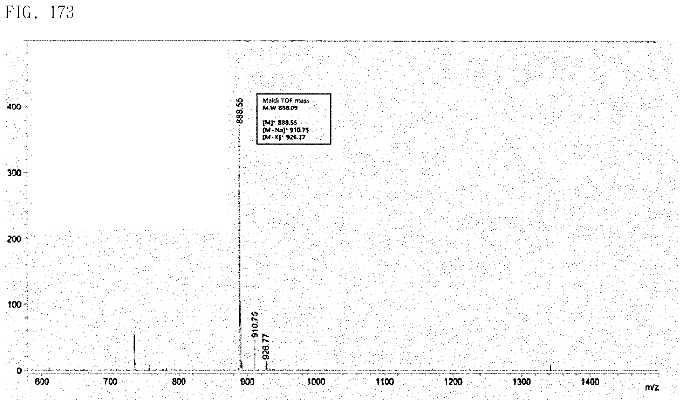

The purity and molecular weight of the HKFY-75 are shown in FIGs. 172 and 173, respectively.

### Example 1.76. Preparation of HKFY-76

His(trityl)-Lys(caprylic)-d-Phe(4-CF3)-Tyr (((R)-2-((S)-2-((S)-2-amino-3-(1-trityl-1H-imidazol-4-yl)propanamido)-6-octanamidohexanamido)-3-(4-(trifluoromethyl)phenyl)propanoyl)-L-tyrosine) was prepared in the same manner except that Fmoc-d-Phe(4-CF3) was used instead of Fmoc-d-3Pal among the constituent amino acids of Example 1.73.

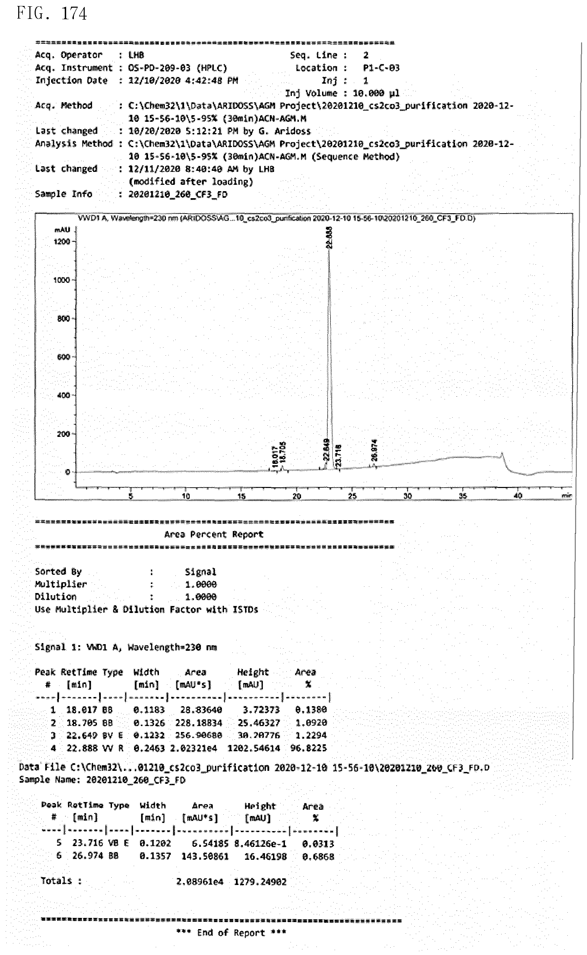

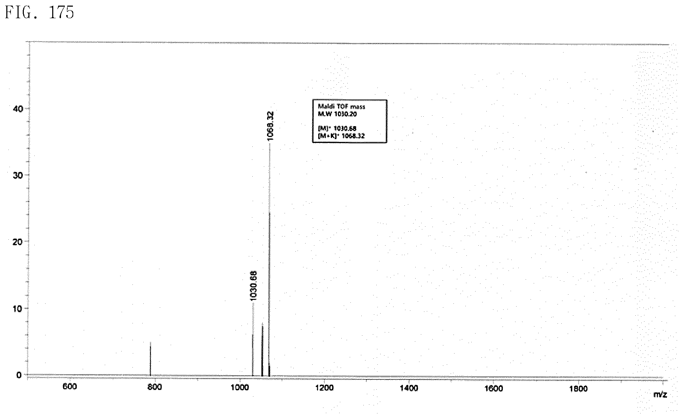

The purity and molecular weight of the HKFY-76 are shown in FIGs. 174 and 175, respectively.

### Example 1.77. Preparation of HKFY-77

His(tbm)-Lys(caprylic)-d-Phe(4-CF3)-Tyr (((R)-2-((S)-2-((S)-3-(1-((2'-(1H-tetrazol-5-yl)-[1,1'-biphenyl-4-yl)methyl)-1H-imidazol-4-yl)-2-aminopropanaido)-6-octanamidohexanamido)-3-(p-tolyl)propanoyl)-L-tyrosine) was prepared in the same manner except that Boc-His(tbm)-OH was used instead of Boc-His(benzhydryl)-OH among the constituent amino acids of Example 1.34.

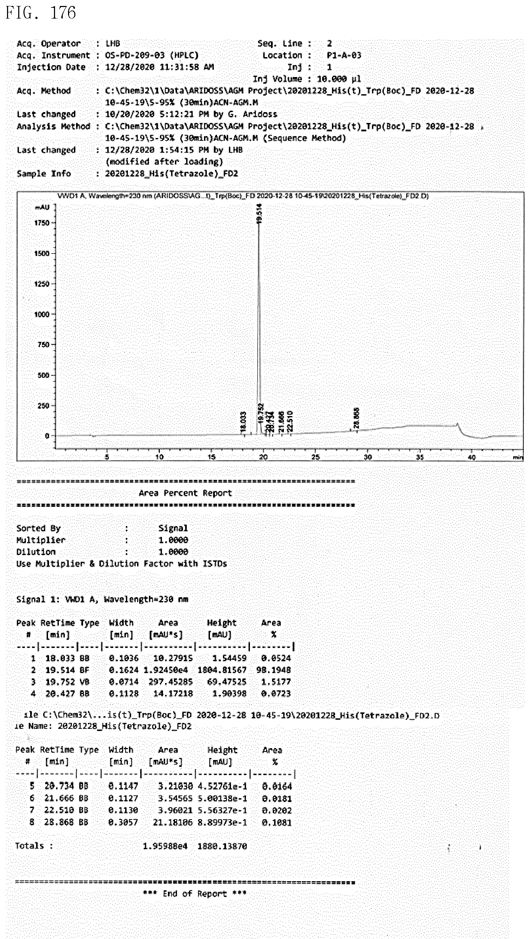

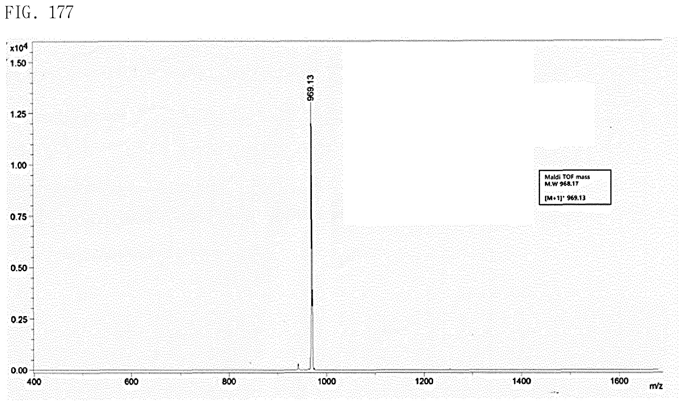

The purity and molecular weight of the HKFY-77 are shown in FIGs. 176 and 177, respectively.

### Example 1.78. Preparation of HKFY-78

Boc-Trp-Lys(caprylic)-d-Phe(4-methyl)-Tyr (((R)-2-((S)-2-((S)-2-((tert-butoxycarbonyl)amino)-3-(1H-indol-3-yl)propanamido)-6-octanamidohexanamido)-3-(p-tolyl)propanoyl)-L-tyrosine) was prepared in the same manner except that Boc-Trp-OH was used instead of Boc-His(benzhydryl)-OH among the constituent amino acids of Example 1.34.

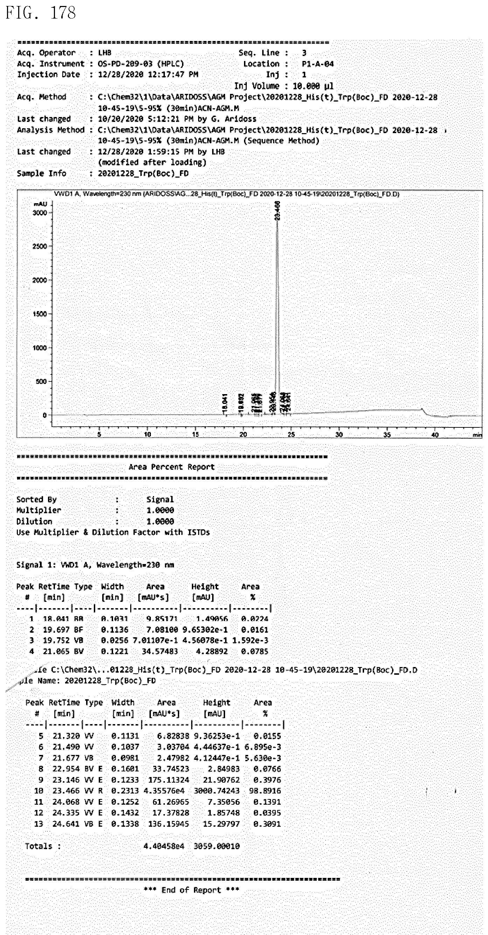

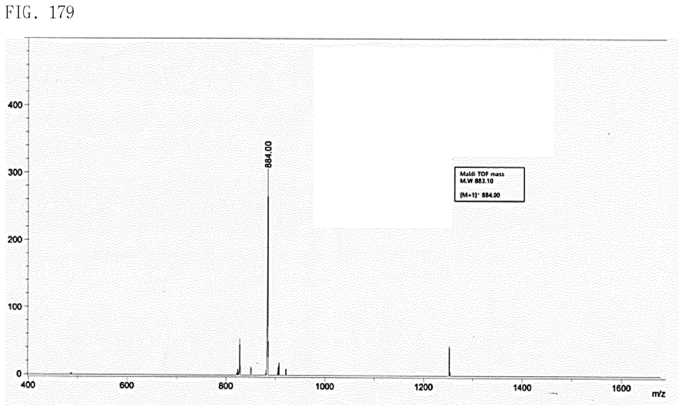

The purity and molecular weight of the HKFY-78 are shown in FIGs. 178 and 179, respectively.

### Example 1.79. Preparation of HKFY-79

Fmoc-Lys(dde) and DMF (dimethylformamide) were loaded onto the trityl resin to prepare Fmoc-Lys(dde)-trityl resin. Fmoc-Lys(caprylic acid)-trityl resin was prepared in the same manner as in the synthesis process of Example 1.1. Thereafter, Boc-His(benzhdyryl)-OH was used to prepare His(benzhydryl)-Lys(caprylic) (N2-(Nt-benzhydryl-L-histidyl)-N6-octanoyl-L-lysine).

The HKFY peptide derivatives prepared by the above preparation method are shown in Table 1 below.

**[Table 1]**

| No. | Amino acid sequence | IUPAC |
|---|---|---|
| HKFY-1 | His(trt)-Lys(caprylic)-Phe-Tyr | N6-octanoyl-N2-(Nt-trityl-L-histidyl)-L-lysyl-L-phenylalanyl-L-tyrosine |
| HKFY-2 | Biotin-His(trt)-Lys(caprylic)-Phe- Tyr | N6-octanoyl-N2-(Na-(5-((3aS,4S,6aR)-2-oxohexahydro-1H-thieno[3,4-d]imidazol-4-yl)pentanoyl)-Nt-trityl-L-histidyl)-L-lysyl-L-phenylalanyl-L-tyrosine |
| HKFY-3 | His(benzyl)-Lys(caprylic)-Phe-Tyr | N2-(Nt-benzyl-L-histidyl)-N6-octanoyl-L-lysyl-L-phenylalanyl-L-tyrosine |
| HKFY-4 | d-His(trt)-Lys(caprylic)-Phe-Tyr | N6-octanoyl-N2-(Nt-trityl-D-histidyl)-L-lysyl-L-phenylalanyl-L-tyrosine |
| HKFY-5 | His(4-methyltrityl)-Lys(caprylic)-Phe-Tyr | N2-(Nt-(diphenyl(p-tolyl)methyl)-L-histidyl)-N6-octanoyl-L-lysyl-L-phenylalanyl-L-tyrosine |
| HKFY-6 | His(DAMP-3)-Lys(caprylic)-Phe-Tyr | N2-((S)-2-amino-3-(1,3-diphenethyl-2,3-dihydro-1H-imidazol-4-yl)propanoyl)-N6-octanoyl-L-lysyl-L-phenylalanyl-L-tyrosine |
| HKFY-7 | His(DAMP-5)-Lys(caprylic)-Phe-Tyr | N2-((S)-2-amino-3-(3-(2-cyclohexylethyl)-2,3-dihydro-1H-imidazol-4-yl)propanoyl)-N6-octanoyl-L-lysyl-L-phenylalanyl-L-tyrosine |
| HKFY-8 | His(DAMP-2)-Lys(caprylic)-Phe-Tyr | N2-((S)-2-amino-3-(1,3-bis(2-cyclohexylethyl)-2,3-dihydro-1H-imidazol-4-yl)propanoyl)-N6-octanoyl-L-lysyl-L-phenylalanyl-L-tyrosme |
| HKFY-9 | His(2-phenylethyl)-Lys(caprylic)-Phe-Tyr | N6-octanoyl-N2-(Nt-phenethyl-L-histidyl)-L-lysyl-L-phenylalanyl-L-tyrosine |
| HKFY-10 | His(2-naphthalen-1-methyl)-Lys(caprylic)-Phe-Tyr | N2-(Nt-(naphthalen-1-ylmethyl)-L-histidyl)-N6-octanoyl-L-lysyl-L-phenylalanyl-L-tyrosine |
| HKFY-11 | His(2-cyclohexylethyl)-Lys(caprylic)-Phe-Tyr | N2-(Nt-(2-cyclohexylethyl)-L-histidyl)-N6-octanoyl-L-lysyl-L-phenylalanyl-L-tyrosine |
| HKFY-12 | His(trt)-Lys(caprylic)-d-Phe-Tyr | N6-(non-1-en-2yl)-N2-(Nt-trityl-L-histidyl)-L-lysyl-D-phenylalanyl-L-tyrosine |
| HKFY-13 | His(trt)-Lys(caprylic)-d-Phe(4-Cl)-Tyr | ((R)-2-((S)-2-((S)-2-amino-3-(1-trityl-1H-imidazol-4-yl)propanamido)-6-(non-1-en-2-ylamino)hexanamido)-3-(4-chlorophenyl)propanoyl)-L-tyrosine |
| HKFY-14 | d-His(trt)-Lys(caprylic)-d-Phe(4-Cl)-d-Tyr | ((R)-2-((S)-2-((R)-2-amino-3-(1-trityl-1H-imidazol-4-yl)propanamido)-6-octanamidohexanamido)-3-(4-chlorophenyl)propanoyl)-D-tyrosme |
| HKFY-15 | His(trt)-Lys(caprylic)-d-Phe(4-Cl)-d-Tyr | ((R)-2-((S)-2-((S)-2-amino-3-(1-trityl-1H-imidazol-4-yl)propanamido)-6-octanamidohexanamido)-3-(4-chlorophenyl)propanoyl)-D-tyrosme |
| HKFY-16 | His(trt)-d-Lys(caprylic)-d-Phe(4-Cl)-Tyr | ((R)-2-((R)-2-((S)-2-amino-3-(1-trityl-1H-imidazol-4-yl)propanamido)-6-octanamidohexanamido)-3-(4-chlorophenyl)propanoyl)-L-tyrosine |
| HKFY-17 | His(3,3-diphenylethyl)-Lys(caprylic)-Phe-Tyr | N2-(Nt-(2,2-diphenylethyl)-L-histidyl)-N6-octanoyl-L-lysyl-L-phenylalanyl-L-tyrosine |
| HKFY-18 | His(Fm)-Lys(caprylic)-Phe-Tyr | N2-(Nt-(9H-fluoren-9-yl)-L-histidyl)-N6-octanoyl-L-lysyl-L-phenylalanyl-L-tyrosine |
| HKFY-19 | His(phenethyl)-Lys(caprylic)-Phe-Tyr | N6-octanoyl-N2-(Nt-phenethyl-L-histidyl)-L-lysyl-L-phenylalanyl-L-tyrosine |
| HKFY-20 | His(4-methoxylbenzhydryl)-Lys(caprylic)-Phe-Tyr | N2-(Nt-((4-methoxyphenyl)(phenyl)methyl)-L-histidyl)-N6-octanoyl-L-lysyl-L-phenylalanyl-L-tyrosine |
| HKFY-21 | His(4-chlorobenzhydryl)-Lys(caprylic)-Phe-Tyr | N2-(Nt-((R)-(4-chlorophenyl)(phenyl)methyl)-L-histidyl)-N6-octanoyl-L-lysyl-L-phenylalanyl-L-tyrosine |
| HKFY-22 | His(4-methylbenzhydryl)-Lys(caprylic)-Phe-Tyr | N6-octanoyl-N2-(Nt-((R)-phenyl(p-tolyl)methyl)-L-histidyl)-L-lysyl-L-phenylalanyl-L-tyrosine |
| HKFY-23 | His(adamantan-1-yl)-Lys(caprylic)-Phe-Tyr | N2-(Nt-(adamantan-1-yl)-L-histidyl)-N6-octanoyl-L-lysyl-L-phenylalanyl-L-tyrosine |
| HKFY-24 | His(trt)-Lys(capric)-Phe-Tyr | N6-decanoyl-N2-(Nt-trityl-L-histidyl)-L-lysyl-L-phenylalanyl-L-tyrosine |
| HKFY-25 | His(trt)-L ys(lauric)-Phe-Tyr | N6-dodecanoyl-N2-(Nt-trityl-L-histidyl)-L-lysyl-L-phenylalanyl-L-tyrosine |
| HKFY-26 | His(trt)-L ys(myristic)-Phe-Tyr | N6-tetradecanoyl-N2-(Nt-trityl-L-histidyl)-L-lysyl-L-phenylalanyl-L-tyrosine |
| HKFY-27 | His(trt)-L ys(palmitic)-Phe-Tyr | N6-palmitoyl-N2-(Nt-trityl-L-histidyl)-L-lysyl-L-phenylalanyl-L-tyrosine |
| HKFY-28 | His(trt)-L ys(caprylic)-d-Phe(4-F)-Tyr | ((R)-2-((S)-2-((S)-2-amino-3-(1-trityl-1H-imidazol-4-yl)propanamido)-6-octanamidohexanamido)-3-(4-fluorophenyl)propanoyl)-L-tyrosine |
| HKFY-29 | His(trt)-Lys(caprylic)-d-Phe(4-Br)-Tyr | ((R)-2-((S)-2-((S)-2-amino-3-(1-trityl-1H-imidazol-4-yl)propanamido)-6-octanamidohexanamido)-3-(4-bromophenyl)propanoyl)-L-tyrosine |
| HKFY-30 | His(trt)-Lys(caprylic)-d-Phe(4-methyl)-Tyr | ((R)-2-((S)-2-((S)-2-amino-3-(1-trityl-1H-imidazol-4-yl)propanamido)-6-octanamidohexanamido)-3-(p-tolyl)propanoyl)-L-tyrosine |
| HKFY-31 | d-His(trt)-Lys(caprylic)-d-Phe(4-methl)-Tyr | ((R)-2-((S)-2-((R)-2-amino-3-(1-trityl-1H-imidazol-4-yl)propanamido)-6-octanamidohexanamido)-3-(p-tolyl)propanoyl)-L-tyrosine |
| HKFY-32 | His(1,3-difluorobenzhydryl)-Lys(caprylic)-d-Phe(4-methyl)-Tyr | ((2R)-2-((2S)-2-((2S)-2-amino-3-(1-((2,4-difluorophenyl)(phenyl)methyl)-1H-imidazol-4-yl)propanamido)-6-octanamidohexanamido)-3-(p-tolyl)propanoyl)-L-tyrosine |
| HKFY-33 | His(benzhydryl)-Lys(caprylic)-Phe-Tyr | N2-(Nt-benzhydryl-L-histidyl)-N6-octanoyl-L-lysyl-L-phenylalanyl-L-tyrosine |
| HKFY-34 | His(benzhydryl)-Lys(caprylic)-d-Phe(4-methyl)-Tyr | ((R)-2-((S)-2-((S)-2-amino-3-(1-benzhydryl-1H-imidazol-4-yl)propanamido)-6-octanamidohexanamido)-3-(p-tolyl)propanoyl)-L-tyrosine |
| HKFY-35 | His(benzhydryl)-Lys(caprylic)-d-Phe(4-methyl)-Tyr(SO₃H) | (S)-2-((R)-2-((S)-2-((S)-2-amino-3-(1-benzhydryl-1H-imidazol-4-yl)propanamido)-6-octanamidohexanamido)-3-(p-tolyl)propanamido)-3-(4-(sulfooxy)phenyl)propanoic acid |
| HKFY-36 | His(benzhydryl)-Lys(caprylic)-d-Phe(4-methyl)- Tyr(2,6-dimethyl) | (S)-2-((R)-2-((S)-2-((S)-2-amino-3-(1-benzhydryl-1H-imidazol-4-yl)propanamido)-6-octanamidohexanamido)-3-(p-tolyl)propanamido)-3-(4-hydroxy-2,6-dimethylphenyl)propanoic acid |
| HKFY-37 | Gly-Ser-His(trt)-Lys(caprylic)-Phe-Tyr | N2-(Na-glycyl-L-seiyl-Nt-trityl-L-histidyl)-N6-octanoyl-L-lysyl-L-phenylalanyl-L-tyrosine |
| HKFY-38 | His(trt)-Lys(caprylic)-Phe-Tyr-Ser-Asp-Gln-Gln-Ala-Arg-Phe | (3S,6S,9S,12S,15S)-15-((S)-2-amino-3-(1-trityl-1H-imidazol-4-yl)propanamido)-12-benzyl-3-(((6S,9S,12S,15S)-1,18-diamino-6-(((S)-1-amino-1-oxo-3-phenylpropan-2-yl)carbamoyl)-12-(3 -amino-3 -oxopropyl)-1-imino-9-methyl-8,11,14,18-tetraoxo-2,7,10,13-tetraazaoctadecan-15-yl)carbamoyl)-9-(4-hydroxybenzyl)-6-(hydroxymethyl)-5,8,11,14,21-pentaoxo-4,7,10,13,20-pentaazaoctacosanoic acid |
| HKFY-39 | His(trt)-Lys(caprylic)-Phe-Tyr-Ser-Gln-Asn-Gly-Ala-Arg-Phe | (3S,6S,9S,12S,15S,18S)-3-((2-(((S)-1-(((S)-1-(((S)-1-amino-1-oxo-3-phenylpropan-2-yl)amino)-5-guanidino-1-oxopentan-2-yl)amino)-1-oxopropan-2-yl)amino)-2-oxoethyl)carbamoyl)-18-((S)-2-amino-3-(1-trityl-1H-imidazol-4-yl)propanamido)-6-(3-amino-3-oxopropyl)-15-benzyl-12-(4-hydroxybenzyl)-9-(hydroxymethyl)-5,8,11,14,17,24-hexaoxo-4,7,10,13,16,23-hexaazahentriacontanoic acid |
| HKFY-40 | His(trt)-Lys(caprylic)-Phe-Tyr-Ser-His-Gln-Gln-Ala-Arg-Phe | (S)-2-((2S,5S,8S,11S,14S)-2-((1H-imidazol-4-yl)methyl)-14-((S)-2-amino-3-(1-trityl-1H-imidazol-4-yl)propanamido)-11-benzyl-8-(4-hydroxybenzyl)-5-(hydroxymethyl)-4,7,10,13,20-pentaoxo-3,6,9,12,19-pentaazaheptacosanamido)-N1-((S)-5-amino-1-(((S)-1-(((S)-1-(((S)-1-amino-1-oxo-3 -phenylpropan-2-yl)amino)-5-guanidino-1-oxopentan-2-yl)amino)-1-oxopropan-2-yl)amino)-1,5-dioxopentan-2-yl)pentanediamide |
| HKFY-41 | His(trt)-Lys(caprylic)-Phe-Tyr-Gln-Asn-Gly-Ala-Arg-Phe | (3S,6S,9S,12S,15S,18S)-3-((2-(((S)-1-(((S)-1-(((S)-1-amino-1-oxo-3-phenylpropan-2yl)amino)-5-guanidino-1-oxopentan-2-yl)amino)-1-oxopropan-2-yl)amino)-2-oxoethyl)carbamoyl)-18-((S)-2-amino-3-(1-trityl-1H-imidazol-4-yl)propanamido)-6-(3-amino-3 -oxopropyl)-15-benzyl-12-(4-hydroxybenzyl)-9-(hydroxymethyl)-5,8,11,14,17,24-hexaoxo-4,7,10,13,16,23-hexaazahentriacontanoic acid |
| HKFY-42 | His(trt)-Lys(caprylic)-Phe-Tyr-His-Gln-Gln-Ala-Arg-Phe | (S)-2-((2S,5S,8S,11S)-2-((1H-imidazol-4-yl)methyl)-11-((S)-2-amino-3-(1-trityl-1H-imidazol-4-yl)propanamido)-8-benzyl-5-(4-hydroxybenzyl)-4,7,10,17-tetraoxo-3,6,9,16-tetraazatetracosanamido)-N1-((S)-5-amino-1-(((S)-1-(((S)-1-(((S)-1-amino-1-oxo-3-phenylpropan-2-yl)amino)-5-guanidino-1-oxopentan-2-yl)amino)-1-oxopropan-2-yl)amino)-1,5-dioxopentan-2-yl)pentanediamide |
| HKFY-43 | His(trt)-Lys(caprylic)-Phe-Tyr-Gln-Asn-Gln-Ala-Arg-Phe | (S)-N1-((S)-1-(((S)-1-(((S)-1-amino-1-oxo-3-phenylpropan-2-yl)amino)-5-guanidino-1-oxopentan-2-yl)amino)-1-oxopropan-2-yl)-2-((2S,5S,8S,11S,14S)-2-(2-amino-2-oxoethyl)-14-((S)-2-amino-3-(1-trityl-1H-imidazol-4-yl)propanamido)-5-(3-amino-3-oxopropyl)-11-benzyl-8-(4-hydroxybenzyl)-4,7,10,13,20-pentaoxo-3,6,9,12,19-pentaazaheptacosanamido)pentanediamide |
| HKFY-44 | His(trt)-Lys(caprylic)-Phe-Tyr-Trp | N6-octanoyl-N2-(Nt-trityl-L-histidyl)-L-lysyl-L-phenylalanyl-L-tyrosyl-L-tryptophan |
| HKFY-45 | Biotin-His(trt)-Lys(caprylic)-Ala(biphenyl)-Tyr | ((S)-3-([1,1'-biphenyl]-4-yl)-2-((S)-6-octanamido-2-((S)-2-(5-((3aS,4S,6aR)-2-oxohexahydro-1H-thieno[3,4-d]imidazol-4-yl)pentanamido)-3-(1-trityl-1H-imidazol-4-yl)propanamido)hexanamido)propanoyl)-L-tyrosine |
| HKFY-46 | His(trt)-Lys(caprylic)-Phe-Trp | N6-octanoyl-N2-(Nt-trityl-L-histidyl)-L-lysyl-L-phenylalanyl-L-tryptophan |
| HKFY-47 | His(trt)-Lys(caprylic)-Tyr-Phe | N6-octanoyl-N2-(Nt-trityl-L-histidyl)-L-lysyl-L-tyrosyl-L-phenylalanine |
| HKFY-48 | His(trt)-Lys(caprylic)-Tyr-Tyr | N6-octanoyl-N2-(Nt-trityl-L-histidyl)-L-lysyl-L-tyrosyl-L-tyrosine |
| HKFY-49 | His(trt)-Lys(capiylic)-Phe | N6-octanoyl-N2-(Nt-trityl-L-histidyl)-L-lysyl-L-phenylalanine |
| HKFY-50 | His(trt)-Lys(caprylic)-Phe-Ser | N6-octanoyl-N2-(Nt-trityl-L-histidyl)-L-lysyl-L-phenylalanyl-L-serine |
| HKFY-51 | His(trt)-Lys(caprylic)-Leu-Tyr | N6-octanoyl-N2-(Nt-trityl-L-histidyl)-L-lysyl-L-leucyl-L-tyrosine |
| HKFY-52 | His(trt)-Phe-Tyr-Asp-Gln-Gln-Ala-Arg-Phe | (6S,9S,12S,15S,18S)-1-amino-6-(((S)-1-amino-1-oxo-3-phenylpropan-2-yl)carbamoyl)-18-((S)-2-((S)-2-((S)-2-amino-3-(1-trityl-1H-imidazol-4-yl)propanamido)-3-phenylpropanamido)-3-(4-hydroxyphenyl)propanamido)-12,15-bis(3-amino-3-oxopropyl)-1-imino-9-methyl-8,11,14,17-tetraoxo-2,7,10,13,16-pentaazaicosan-20-oic acid |
| HKFY-53 | His(trt)-Gly-Ser-Lys(caprylic)-Phe-Tyr | N6-octanoyl-N2-(Nt-trityl-L-histidylglycyl-L-seryl)-L-lysyl-L-phenylalanyl-L-tyrosine |
| HKFY-54 | Lys(caprylic)-Phe-Tyr | N6-octanoyl-L-lysyl-L-phenylalanyl-L-iyrosine |
| HKFY-55 | Lys(caprylic)-His(trt)-Phe-Tyr | Na-(N6-octanoyl-L-lysyl)-Nt-trityl-L-histidyl-L-phenylalanyl-L-tyrosine |
| HKFY-56 | His(trt)-Lys(caproic)-Phe-Tyr-Asp-Gln-Gln-Ala-Arg-Phe | (6S,9S,12S,15S,18S)-1-amino-6-(((S)-1-amino-1-oxo-3-phenylpropan-2-yl)carbamoyl)-18-((S)-2-((S)-2-((S)-2-((S)-2-amino-3-(1-trityl-1H-imidazol-4-yl)propanamido)-6-(non-1-en-2-ylamino)hexanamido)-3 - phenylpropanamido)-3-(4-hydroxyphenyl)propanamido)-12,15-bis(3-amino-3-oxopropyl)-1-imino-9-methyl-8,11,14,17-tetraoxo-2,7,10,13,16-pentaazaicosan-20-oic acid |
| HKFY-57 | His(2-phenyl)-Lys(caprylic)-d-Phe(4-methyl)-Tyr | ((R)-2-((S)-2-((S)-2-amino-3-(2-phenyl-1H-imidazol-4-yl)propanamido)-6-octanamidohexanamido)-3-(p-tolyl)propanoyl)-L-tyrosine |
| HKFY-58 | His(1-phenyl)-Lys(caprylic)-d-Phe(4-methyl)-Tyr | ((R)-2-((S)-2-((S)-2-amino-3-(1-phenyl-1H-imidazol-4-yl)propanamido)-6-octanamidohexanamido)-3-(p-tolyl)propanoyl)-L-tyrosine |
| HKFY-59 | His(1,2-diphenyl)-Lys(caprylic)-d-Phe(4-methyl)-Tyr | ((R)-2-((S)-2-((S)-2-amino-3-(1,2-diphenyl-1H-imidazol-4-yl)propanamido)-6-octanamidohexanamido)-3-(p-tolyl)propanoyl)-L-tyrosine |
| HKFY-60 | His(2-(4-tert-butyl)phenyl)-Lys(caprylic)-d-Phe(4-methyl)-Tyr | ((R)-2-((S)-2-((S)-2-amino-3-(2-(4-(tert-butyl)phenyl)-1H-imidazol-4-yl)propanamido)-6-octanamidohexanamido)-3-(p-tolyl)propanoyl)-L-tyrosine |
| HKFY-61 | His(1-(4-tert-butyl)phenyl)-Lys(caprylic)-d-Phe(4-methyl)-Tyr | ((R)-2-((S)-2-((S)-2-amino-3-(1-(4-(tert-butyl)phenyl)-1H-imidazol-4-yl)propanamido)-6-octanamidohexanamido)-3-(p-tolyl)propanoyl)-L-tyrosine |
| HKFY-62 | d-His(benzhydryl)-Lys(caprylic)-d-Phe(4-methyl)-Tyr | ((R)-2-((S)-2-((R)-2-amino-3-(1-benzhydryl-1H-imidazol-4-yl)propanamido)-6-octanamidohexanamido)-3-(p-tolyl)propanoyl)-L-tyrosine |
| HKFY-63 | His(thiophene)-Lys(caprylic)-d-Phe(4-methyl)-Tyr | ((R)-2-((S)-2-((S)-2-amino-3-(2-(thiophen-2-yl)-1H-imidazol-4-yl)propanamido)-6-octanamidohexanamido)-3-(p-tolyl)propanoyl)-L-tyrosine |
| HKFY-64 | His(4-methoxybenzhydryl)-Lys(caprylic)-d-Phe(4-methyl)-Tyr | ((2R)-2-((2S)-2-((2S)-2-amino-3-(1-((4-methoxyphenyl)(phenyl)methyl)-1H-imidazol-4-yl)propanamido)-6-octanamidohexanamido)-3-(p-tolyl)propanoyl)-L-tyrosine |
| HKFY-65 | His(4-hydroxybenzhydryl)-Lys(caprylic)-d-Phe(4-methyl)-Tyr | ((2R)-2-((2S)-2-((2S)-2-amino-3-(1-((4-hydroxyphenyl)(phenyl)methyl)-1H-imidazol-4-yl)propanamido)-6-octanamidohexanamido)-3-(p-tolyl)propanoyl)-L-tyrosine |
| HKFY-66 | His(benzhydryl)-Lys(caprylic)-d-Phe(4-methyl)-Tyr-NH2 | N-((S)-5-((S)-2-amino-3-(1-benzhydryl-1H-imidazol-4-yl)propanamido)-6-(((R)-1-(((S)-1-amino-3-(4-hydroxyphenyl)-1-oxopropan-2-yl)amino)-1-oxo-3-(p-tolyl)propan-2-yl)amino)-6-oxohexyl)octanamide |
| HKFY-67 | His(benzhydryl)-Lys(caprylic)-d-Phe(4-methyl)-Tyr(3-chloro) | (S)-2-((R)-2-((S)-2-((S)-2-ammo-3-(1-benzhydryl-1H-imidazol-4-yl)propanamido)-6-octanamidohexanamido)-3-(p-tolyl)propanamido)-3-(3 -chloro-4-hydroxyphenyl)propanoic acid |
| HKFY-68 | His(benzhydryl)-Lys(caprylic)-d-Phe(4-methyl)-Tyr(3-nitro) | (S)-2-((R)-2-((S)-2-((S)-2-amino-3-(1-benzhydryl-1H-imidazol-4-yl)propanamido)-6-octanamidohexanamido)-3-(p-tolyl)propanamido)-3-(4-hydroxy-3-nitrophenyl)propanoic acid |
| HKFY-69 | His(benzhydryl)-Lys(caprylic)-d-Phe(4-methyl)-Tyr(3,5-nitro) | (S)-2-((R)-2-((S)-2-((S)-2-amino-3-(1-benzhydryl-1H-imidazol-4-yl)propanamido)-6-octanamidohexanamido)-3-(p-tolyl)propanamido)-3-(4-hydroxv-3,5-dinitrophenyl)propanoic acid |
| HKFY-70 | His(benzhydryl)-Lys(caprylic)-d-Phe(4-methyl)-Tyr(3-amino) | (S)-2-((R)-2-((S)-2-((S)-2-amino-3-(1-benzhydryl-1H-imidazol-4-yl)propanamido)-6-octanamidohexanamido)-3-(p-tolyl)propanamido)-3 - (3-amino-4-hydroxyphenyl)propanoic acid |
| HKFY-71 | His(benzhydryl)-Orn(caprylic)-d-Phe(4-methyl)-Tyr | ((R)-2-((S)-2-((S)-2-amino-3-(1-benzhydryl-1H-imidazol-4-yl)propanamido)-5-octanamidopentanamido)-3-(p-tolyl)propanoyl)-L-tyrosine |
| HKFY-72 | Biotin-His(benzhydryl)-Lys(caprylic)-d-Phe(4-methyl)-Tyr | ((S)-2-((S)-2-((S)-3-(1-benzhydryl-1H-imidazol-4-yl)-2-(5-((3aS,4S,6aR)-2-oxohexahydro-1H-thieno[3,4-d]imidazol-4-yl)pentanamido)propanamido)-6-octanamidohexanamido)-3-(p-tolyl)propanoyl)-L-tyrosine |
| HKFY-73 | His(trt)-Lys(caprylic)-d-3Pal-Tyr | ((R)-2-((S)-2-((S)-2-amino-3-(1-trityl-1H-imidazol-4-yl)propanamido)-6-octanamidohexanamido)-3-(pyndin-3-yl)propanoyl)-L-tyrosme |
| HKFY-74 | His(trt)-Lys(caprylic)-d-2Nal-Tyr | ((R)-2-((S)-2-((S)-2-amino-3-(1-trityl-1H-imidazol-4-yl)propanamido)-6-octanamidohexanamido)-3-(naphthalen-2-yl)propanoyl)-L-tyrosine |
| HKFY-75 | His(tosyl)-Lys(caprylic)-d-Phe(4-methl)-Tyr | ((R)-2-((S)-2-((S)-2-amino-3-(1-tosyl-1H-imidazol-4-yl)propanamido)-6-octanamidohexanamido)-3-(p-tolyl)propanoyl)-L-tyrosme |
| HKFY-76 | His(trt)-Lys(caprylic)-d-Phe(4-CF3)-Tyr | ((R)-2-((S)-2-((S)-2-amino-3-(1-trityl-1H-imidazol-4-yl)propanamido)-6-octanamidohexanamido)-3-(4-(trifluoromethyl)phenyl)propanoyl)-L-tyrosine |
| HKFY-77 | His(tbm)-Lys(caprylic)-d-Phe(4-CF3)-Tyr | ((R)-2-((S)-2-((S)-3-(1-((2'-(1H-tetrazol-5-yl)-[1,1'-biphenyl]-4-yl)methyl)-1H-imidazol-4-yl)-2-aminopropanamido)-6-octanamidohexanamido)-3-(p-tolyl)propanoyl)-L-tyrosme |
| HKFY-78 | Boc-Trp-Lys(caprylic)-d-Phe(4-methyl)-Tyr | ((R)-2-((S)-2-((S)-2-((tert-butoxycarbonyl)amino)-3-(1H-indol-3-yl)propanamido)-6-octanamidohexanamido)-3-(p-tolyl)propanoyl)-L-tyrosine |
| HKFY-79 | His(benzhydryl)Lys(capr ylic) | N2-(Nt-benzhydryl-L-histidyl)-N6-octanoyl-L-lysine |

### Experimental Example 1. Analysis of HKFY derivatives

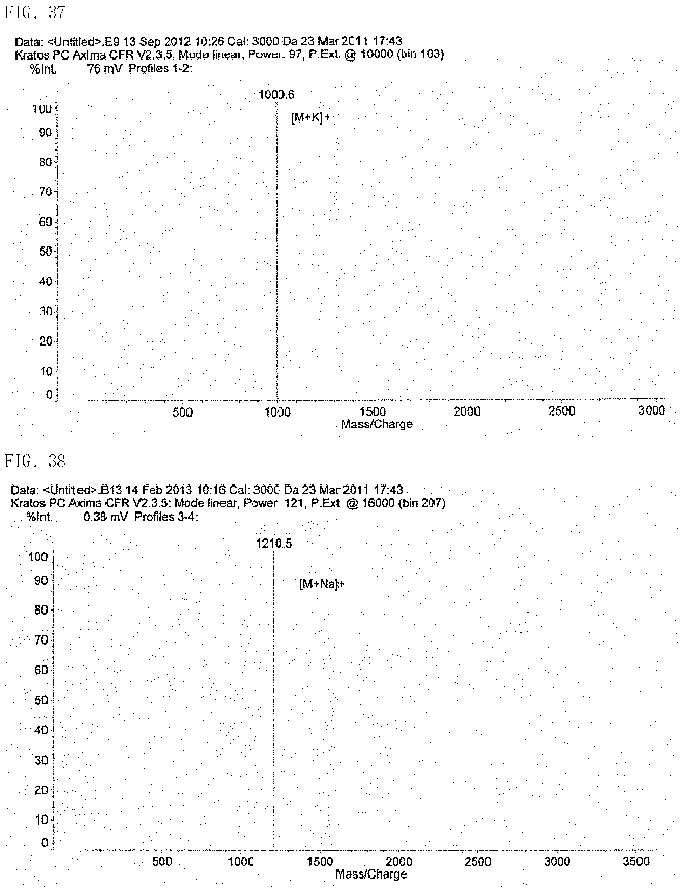

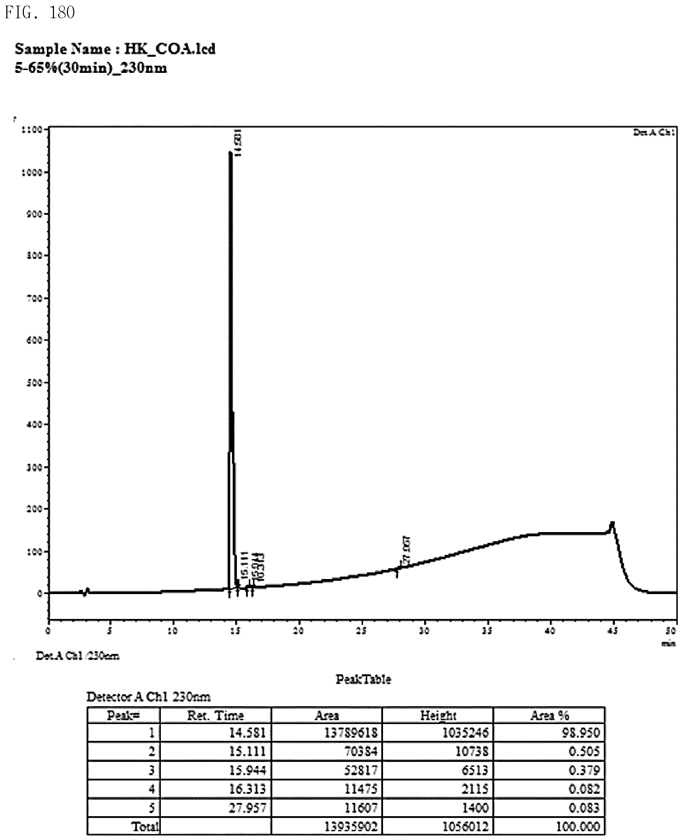

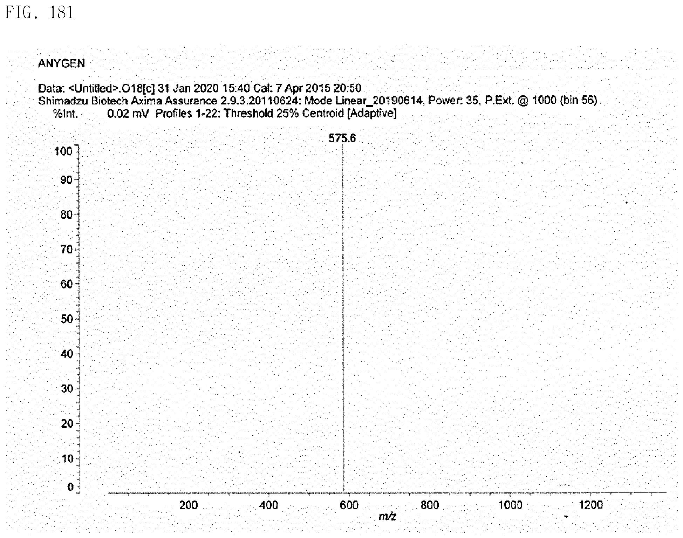

The HKFY derivatives prepared in Examples 1.1 to 1.79 were analyzed for purity and molecular weight using HPLC and Ion-Mass. The results are shown in Table 2 below, and the HPLC analysis results of the HKFY derivatives are shown in FIGs. 1 to 36, 96, 98, 100, 102, 104, 106, 108, 110, 112, 114, 116, 118, 120, 122, 124, 126, 128, 130, 132, 134, 136, 138, 140, 142, 144, 146, 148, 150, 152, 154, 156, 158, 160, 162, 164, 166, 168, 170, 172, 174, 176, 178 and 180, and the Ion-Mass analysis results of the HKFY derivatives are shown in FIGs. 37 to 72, 97, 99, 101, 103, 105, 107, 109, 111, 113, 115, 117, 119, 121, 123, 125, 127, 129, 131, 133, 135, 137, 139, 141, 143, 145, 147, 149, 151, 153, 155, 157, 159, 161, 163, 165, 167, 169, 171, 173, 175, 177, 179 and 181.

**[Table 2]**

| No. | Purity (%) | Molecular weight |
|---|---|---|
| HKFY-1 | 96.1 | 1000.6 |
| HKFY-2 | 94.8 | 1210.5 |
| HKFY-3 | 93.5 | 810.1 |
| HKFY-4 | 92.0 | 1000.5 |
| HKFY-5 | 86.4 | 976.2 |
| HKFY-6 | 95.2 | 956.6 |
| HKFY-7 | 99.2 | 844.9 |
| HKFY-8 | 93.4 | 969.1 |
| HKFY-9 | 98.7 | 823.3 |
| HKFY-10 | 98.5 | 873.1 |
| HKFY-11 | 97.5 | 829.1 |
| HKFY-12 | 96.3 | 1001.1 |
| HKFY-13 | 96.9 | 1018.9 |
| HKFY-14 | 96.6 | 1034.1 |
| HKFY-15 | 98.3 | 1034.0 |
| HKFY-16 | 95.3 | 1035.3 |
| HKFY-17 | 98.3 | 914.5 |
| HKFY-18 | 98.6 | 898.2 |
| HKFY-19 | 98.7 | 836.4 |
| HKFY-20 | 94.6 | 709.9 |
| HKFY-21 | 98.3 | 709.5 |
| HKFY-22 | 94.2 | 709.6 |
| HKFY-23 | 69.7 | 709.8 |
| HKFY-24 | 93.4 | 989.6 |
| HKFY-25 | 95.2 | 1019.1 |
| HKFY-26 | 89.1 | 1045.5 |
| HKFY-27 | 98.1 | 1073.6 |
| HKFY-28 | 96.5 | 980.6 |
| HKFY-29 | 96.2 | 1043.1 |
| HKFY-30 | 97.0 | 977.1 |
| HKFY-31 | 92.9 | 976.7 |
| HKFY-32 | 90.4 | 935.48 |
| HKFY-33 | 98.1 | 884.9 |
| HKFY-34 | 93.6 | 899.3 |
| HKFY-35 | 93.9 | 979.4 |
| HKFY-36 | 92.6 | 928.6 |
| HKFY-37 | 96.9 | 1128.1 |
| HKFY-38 | 95.6 | 1793.9 |
| HKFY-39 | 95.4 | 1721.4 |
| HKFY-40 | 96.1 | 1815.1 |
| HKFY-41 | 91.8 | 1634.7 |
| HKFY-42 | 87.0 | 1728.9 |
| HKFY-43 | 88.5 | 1705.6 |
| HKFY-44 | 94.2 | 1171.3 |
| HKFY-45 | 93.7 | 1287.0 |
| HKFY-46 | 96.7 | 986.1 |
| HKFY-47 | 97.4 | 962.8 |
| HKFY-48 | 98.0 | 978.9 |
| HKFY-49 | 93.3 | 799.1 |
| HKFY-50 | 88.9 | 886.6 |
| HKFY-51 | 95.1 | 928.2 |
| HKFY-52 | 96.6 | 1452.0 |
| HKFY-53 | 95.9 | 1105.2 |
| HKFY-54 | 98.2 | 583.3 |
| HKFY-55 | 99.0 | 962.7 |
| HKFY-56 | 95.6 | 1793.9 |
| HKFY-57 | 97.6 | 809.12 |
| HKFY-58 | 90.3 | 809.12 |
| HKFY-59 | 98.5 | 885.22 |
| HKFY-60 | 95.6 | 865.23 |
| HKFY-61 | 96.1 | 865.23 |
| HKFY-62 | 97.1 | 899.43 |
| HKFY-63 | 96.6 | 813.42 |
| HKFY-64 | 96.4 | 929.51 |
| HKFY-65 | 96.5 | 915.49 |
| HKFY-66 | 95.2 | 898 |
| HKFY-67 | 99.2 | 932.9 |
| HKFY-68 | 99.2 | 944 |
| HKFY-69 | 99.5 | 990 |
| HKFY-70 | 98.5 | 914.51 |
| HKFY-71 | 98.6 | 885.48 |
| HKFY-72 | 93.2 | 1126.21 |
| HKFY-73 | 95.5 | 963.19 |
| HKFY-74 | 94.4 | 1012.36 |
| HKFY-75 | 93.7 | 888.09 |
| HKFY-76 | 96.8 | 1030.2 |
| HKFY-77 | 98.2 | 968.17 |
| HKFY-78 | 98.9 | 883.1 |
| HKFY-79 | 95.2 | 575.35 |

### Experimental Example 2. Confirmation of binding capacity of HKFY derivatives to GPR39 receptor

In order to investigate the binding capacity of the HKFY derivatives prepared in Examples 1.1. to 1.79 above to GPR39, a luciferase assay system was used. The luciferase assay system is a method for confirming the degree of activity of a receptor in a cell through the binding of a ligand to a receptor in a cell.

Specifically, the fibroblast cell line CV-1 (1×10⁵ cells/ml, Korea Cell Line Bank) was cultured in a 96 well cell culture plate for 24 hours, and then treated with GPR39 inserted into adenovirus and SRE (serum response element) luciferase at 50 MOI (multiplicity of infection) and 25 MOI virus for 3 hours, respectively, and then cultured for 24 hours. In order to give a fasting process, after 24 hours, the medium was replaced with a serum-free medium and cultured for 16 hours, and then treated with each HKFY and HKFY derivative for 6 hours and cultured, and then the expression level of luciferase, a reporter gene, by activated GPR39 was quantified using a luminometer (Table 3).

**[Table 3]**

| No. | EC₅₀ (µM) | ECmax (-fold induction over basal) |
|---|---|---|
| HKFY-1 | 0.15 ± 0.01 | 20.51 ± 0.57 |
| HKFY-2 | 0.14 ± 0.07 | 15 ± 1.87 |
| HKFY-3 | 4.13± 0.15 | 9.2 ± 2.58 |
| HKFY-4 | 0.13 ± 0.07 | 16.8 ± 1.87 |
| HKFY-5 | 1.84 ± 0.2 | 16 ± 1.7 |
| HKFY-6 | 68.4 ± 1.17 | 15 ±2.12 |
| HKFY-7 | 1.56 ± 0.27 | 16 ± 2.2 |
| HKFY-8 | 33.65 ± 1.3 | 17.3 ± 1.22 |
| HKFY-9 | 42.7 ± 1.2 | 21 ± 2.52 |
| HKFY-10 | 46.5 ± 1.1 | 14 ± 2.12 |
| HKFY-11 | 57.8 ± 1.5 | 22 ± 2.2 |
| HKFY-12 | 0.035 ± 0.002 | 20.7 ± 2.3 |
| HKFY-13 | 0.014 ± 0.002 | 25.7 ± 2.4 |
| HKFY-14 | 0.02 ± 0.003 | 20.9 ± 1.4 |
| HKFY-15 | 0.098 ± 0.005 | 24.7 ± 2.6 |
| HKFY-16 | 0.24 ± 0.02 | 27.9 ± 3.4 |
| HKFY-17 | 8.12 ± 0.07 | 20 ± 0.57 |
| HKFY-18 | 2.29 ± 0.15 | 10 ± 1.48 |
| HKFY-19 | 24.6 ± 2.6 | 18 ± 1.7 |
| HKFY-20 | 159 ± 2.3 | 20.26 ± 2.7 |
| HKFY-21 | 136 ± 1.27 | 18.3 ± 2.5 |
| HKFY-22 | 59.8 ± 2.1 | 21.3 ± 1.8 |
| HKFY-23 | 2.6 ± 0.17 | 15 ± 1.37 |
| HKFY-24 | 0.24 ± 0.07 | 7.03 ± 0.57 |
| HKFY-25 | 0.63 ± 0.07 | 7.72 ± 0.57 |
| HKFY-26 | 0.28 ± 0.02 | 19.82 ± 0.57 |
| HKFY-27 | 0.56 ± 0.06 | 20.4 ± 0.57 |
| HKFY-28 | 0.074 ± 0.004 | 22.95 ± 2.58 |
| HKFY-29 | 0.31 ± 0.003 | 26.8 ± 1.87 |
| HKFY-30 | 0.008 ± 0.001 | 23.88 ± 3.04 |
| HKFY-31 | 0.02 ± 0.003 | 20.9 ± 2.51 |
| HKFY-32 | 0.002 ± 0.001 | 21.5 ± 2.7 |
| HKFY-33 | 0.15 ± 0.07 | 20 ± 0.57 |
| HKFY-34 | 0.007 ± 0.001 | 22.39 ± 4.59 |
| HKFY-35 | 0.28 ± 0.07 | 18.9 ± 0.41 |
| HKFY-36 | 0.15 ± 0.05 | 9.9 ± 0.28 |
| HKFY-37 | 1.84 ± 0.2 | 16 ± 1.7 |
| HKFY-38 | 3.5 ± 0.02 | 16.5 ± 0.02 |
| HKFY-39 | 0.78 ± 0.02 | 12.1 ± 0.02 |
| HKFY-40 | 1.3 ± 0.02 | 13.4 ± 0.02 |
| HKFY-41 | 9.55 ± 0.02 | 12.2 ± 0.02 |
| HKFY-42 | 1.58 ± 0.02 | 11.8 ± 0.02 |
| HKFY-43 | 1.21 ± 0.02 | 12.1 ± 0.02 |
| HKFY-44 | 0.37 ± 0.07 | 20 ± 0.47 |
| HKFY-45 | 0.14 ± 0.05 | 21 ± 2.58 |
| HKFY-46 | 0.26 ± 0.15 | 20 ± 0.28 |
| HKFY-47 | 0.3 ± 0.02 | 13 ± 1.7 |
| HKFY-48 | 0.33 ± 0.02 | 14.4 ± 2.7 |
| HKFY-49 | 0.32 ± 0.02 | 18.3 ± 1.8 |
| HKFY-50 | 0.7 ± 0.05 | 16.2 ± 1.5 |
| HKFY-51 | 0.12 ± 0.02 | 14.4 ± 1.9 |
| HKFY-52 | 56.4 ± 0.02 | 15.5 ± 2.2 |
| HKFY-53 | 0.34 ± 0.07 | 8 ± 2.12 |
| HKFY-54 | 25.9 ± 0.27 | 7.8 ± 2.5 |
| HKFY-55 | 2.49 ± 0.12 | 16.8 ± 2.5 |
| HKFY-56 | 0.03 ± 0.002 | 14.9 ± 1.8 |
| HKFY-57 | 0.26 ± 0.01 | 8.5 ± 2.3 |
| HKFY-58 | 0.02 ± 0.002 | 9.12 ± 2.1 |
| HKFY-59 | 0.55 ± 0.02 | 6.76 ± 2.2 |
| HKFY-60 | 0.19 ± 0.01 | 28.2 ± 2.5 |
| HKFY-61 | 0.02 ± 0.003 | 9.88 ± 1.8 |
| HKFY-62 | 0.83 ± 0.03 | 28.1 ± 2.1 |
| HKFY-63 | 0.006 ± 0.001 | 13.5 ± 1.8 |
| HKFY-64 | 0.007 ± 0.001 | 16 ± 1.5 |
| HKFY-65 | 0.003 ± 0.002 | 18.3 ± 2.2 |
| HKFY-66 | 0.011 ± 0.001 | 20.2 ± 3.5 |
| HKFY-67 | 0.043 ± 0.003 | 10.2 ± 1.51 |
| HKFY-68 | 0.013 ± 0.001 | 10.8 ± 1.41 |
| HKFY-69 | 0.015 ± 0.002 | 12.2 ± 2.5 |
| HKFY-70 | 0.02 ± 0.001 | 10.1 ± 1.6 |
| HKFY-71 | 0.012 ± 0.003 | 16.4 ± 2.51 |
| HKFY-72 | 0.011 ± 0.002 | 10.4 ± 1.12 |
| HKFY-73 | 0.023 ± 0.001 | 11.6 ± 2.3 |
| HKFY-74 | 0.008 ± 0.001 | 18.8 ± 3.2 |
| HKFY-75 | 0.001 ± 0.002 | 15.9 ± 4.12 |
| HKFY-76 | 0.007 ± 0.001 | 14.2 ± 3.75 |
| HKFY-77 | 0.007 ± 0.002 | 11.6 ± 2.14 |
| HKFY-78 | 0.009 ± 0.001 | 11.4 ± 1.54 |
| HKFY-79 | 0.97 ± 0.03 | 11.2 ± 1.4 |

As a result, referring to Table 3, it was confirmed that all HKFY derivatives had high activity at nM to µM level for GPR39. In addition, among the analogs in which various fatty acids were conjugated to Lys, the analog conjugated to caprylic acid showed the most excellent activity. Derivatives exhibiting nM level of activity are considered to have highly selective and specific activity for GPR39.

On the other hand, HKFY-30, HKFY-34, HKFY-63, HKFY-64, and HKFY-65 derivatives exhibited a higher activity level than other HKFY derivatives. In particular, HKFY-30 and HKFY-34 composed of d-Phe(methyl) exhibited the most excellent activity and high ECmax value.

### Experimental Example 3. Confirmation of selectivity and specificity of HKFY derivatives for GPR39

In order to confirm the selectivity and specificity of the HKFY derivatives for GPR39, the degree of activity was confirmed by a luciferase assay system and a calcium influx assay system (FIG. 73 and Table 4).

Specifically, the luciferase assay system analyzed the degree of activity using HKFY-34 among the HKFY derivatives in Experimental Example 2 above (top in FIG. 73). In the calcium influx assay system, the fibroblast cell line CV-1 (1×10⁵ cells/ml) was transfected with a mixture of human GPR39 (pCDNA3.1_hGPR39, American Type Culture Collection), Gaq (pCDNA3.1_Gαq, American Type Culture Collection), Aequrin (pCDNA3.1_aequrin, American Type Culture Collection) and lipofectamine (American Type Culture Collection), and then the medium was replaced with a serum-free medium, and after 16 hours of fasting, treated with HKFY-34 for 6 hours and cultured, and then the degree of binding of calcium activated by activated GPR39 to aequrin was quantified using a luminometer (bottom in FIG. 73).

**[Table 4]**

| Component | Reporter gene assay | | Ca²⁺ influx assay | |
|---|---|---|---|---|
| | EC₅₀ (nM) | ECmax (-fold induction over basal) | EC₅₀ (nM) | ECmax (-fold induction over basal) |
| HKFY-34 | 6.7 ± 0.7 | 22.39 ±4.59 | 2.7 ± 0.5 | 2.4 ± 4.59 |
| HKFY | N.D | - | - | - |

As a result, referring to FIG. 73 and Table 4, it was confirmed that when treated with HKFY-34, the binding capacity and activity to the GPR39 receptor were excellent.

In addition, through the activity test of the HKFY derivatives for similar receptors to GPR39, ghrelin receptor family (ghrelin receptor (GHSR), motilin receptor (MTLR), neurotensin receptor (NTSR1 & NTSR2), neuromedin U receptor (NMU1 & NMU2), TRH (thyrotropin releasing hormone) receptor (TRHR)), the selectivity and specificity for the GPR39 receptor were confirmed.

Specifically, the degree of activity between the intrinsic ligand and HKFY-34 for each receptor was analyzed using a luciferase assay system (FIG. 74).

As a result, referring to FIG. 74, it was confirmed that the activity of HKFY-34 for GHSR, MTLR, NTSR1 & NTSR2, NMU1 & NMU2 and TRHR was very low.

These results suggest that the HKFY derivative can be used as an agonist that selectively and specifically binds to the GPR39 receptor.

### Experimental Example 4. Confirmation of insulin secretion ability according to HKFY derivative treatment

The GPR39 receptor is widely distributed in the pancreas, and it is known that glucose-dependent insulin secretion ability is impaired in GPR39 KO mice (Endocrinology 2009, 150:2577-2585).

In this regard, in order to confirm the effect of the HKFY derivative, a candidate GPR39 agonist, on insulin secretion ability, a glucose-dependent insulin secretion ability test was performed in INS-1 (Korea Cell Line Bank), a rat insulinoma cell (FIG. 75).

Specifically, when INS-1 cells were treated with 1 or 10 µM of HKFY-34 in 2.8 mM glucose similar to a physiological blood glucose level or 11.1 mM glucose, a high blood glucose level, the insulin secretion ability was confirmed using a rat insulin ELISA.

As a result, referring to FIG. 75, in the case of a low blood glucose level, the change in insulin secretion level according to HKFY-34 treatment was insignificant, but in the case of a high blood glucose level, insulin secretion was enhanced at all treatment concentrations of HKFY-34.

In addition, through GPR39 knock-down experiment using GPR39 siRNA, it was confirmed that HKFY-34 enhances insulin secretion through the GPR39 receptor. In particular, the fact that the insulin secretion ability was not increased in cells treated with GPR39 siRNA compared to scrambled siRNA in 11.1 mM glucose suggests that HKFY-34 enhances the glucose-dependent insulin secretion ability through the GPR39 receptor.

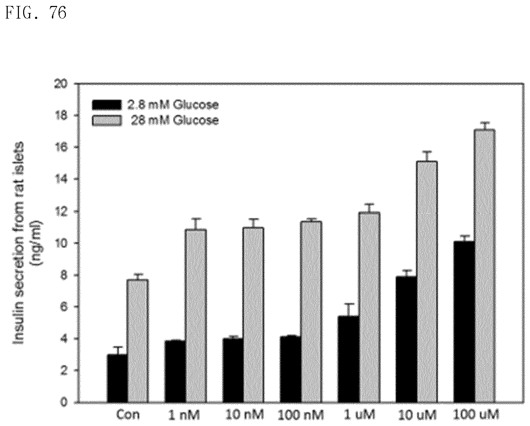

In addition, the glucose-dependent insulin secretion ability of the HKFY derivative for rat islet was confirmed (FIG. 76).

Specifically, the pancreas was extracted from a 8-week-old SD rat (Damul Science), and islets were isolated. The isolated islets were treated with HKFY-34 at different concentrations (1 nM, 10 nM, 100 nM, 1 µM, 10 µM and 100 µM) in 2.8 mM or 28 mM glucose, and then the amount of the secreted insulin was measured using a rat insulin ELISA.

As a result, referring to FIG. 76, it was confirmed that HKFY-34 had the insulin secretion ability in a concentration dependent (dose-dependent) manner.

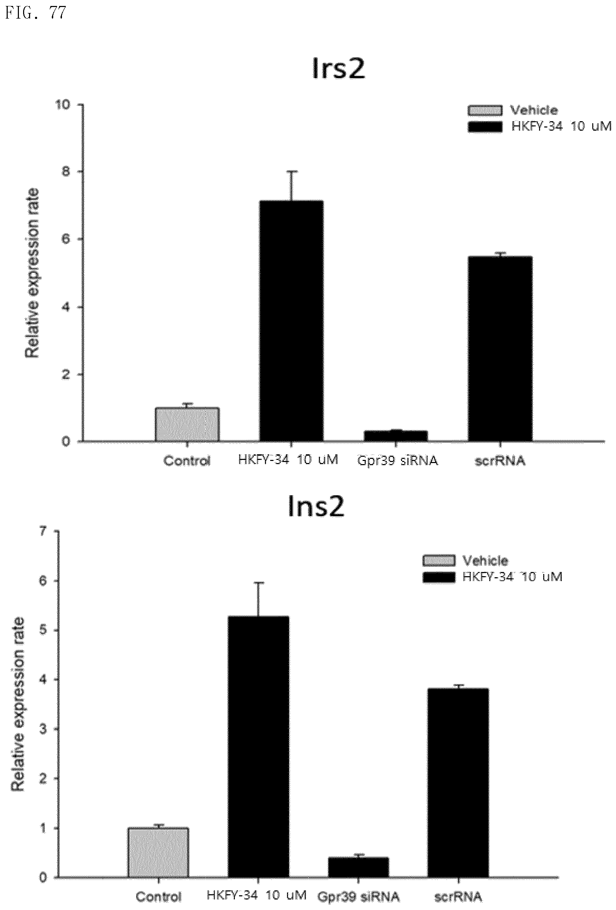

The mRNA expression levels of Irs2 and Ins2, which are important factors for proliferation and differentiation of pancreatic beta cells, were analyzed through RT-PCR (FIG. 77).

As a result, referring to FIG. 77, the mRNA expression levels of Irs2 and Ins2 were increased due to HKFY-34. On the other hand, the mRNA expression of Irs2 and Ins2 was hardly achieved in the GPR39 siRNA treated group, suggesting that the mRNA expression of both Irs2 and Ins2 was regulated by GPR39.

### Experimental Example 5. Confirmation of lipolytic ability by HKFY derivatives

Lipolysis is regulated by β-adrenergic receptors, which are mainly distributed in adipocytes, and obesity due to hyper-proliferation of adipocytes can be regulated through normal lipolysis.

In a study through the control of a high-fat or low-fat diet for GPR39 KO, it was confirmed that the increase in body weight and the accumulation of body fat were enhanced in GPR39 KO mice (Gastroenterology 2006, 131:1131-1141).

Accordingly, the expression level of the GPR39 receptor in adipocytes and adipose tissue was analyzed using western blot (FIG. 78). As a result, referring to FIG. 78, the GPR39 receptor was expressed in the adipocyte 3T3-L1 cells (Korea Cell Line Bank) and adipose tissue (white adipose tissue, WAT) of mice (C57BL6/J, Central Lab. Animal Inc.). In addition, the expression level of GPR39 was decreased in 3T3-L1 knocked down using GPR39 siRNA compared to the control group (con). The above results suggest that GPR39 is lacking in adipose tissue of GPR39 KO mice.

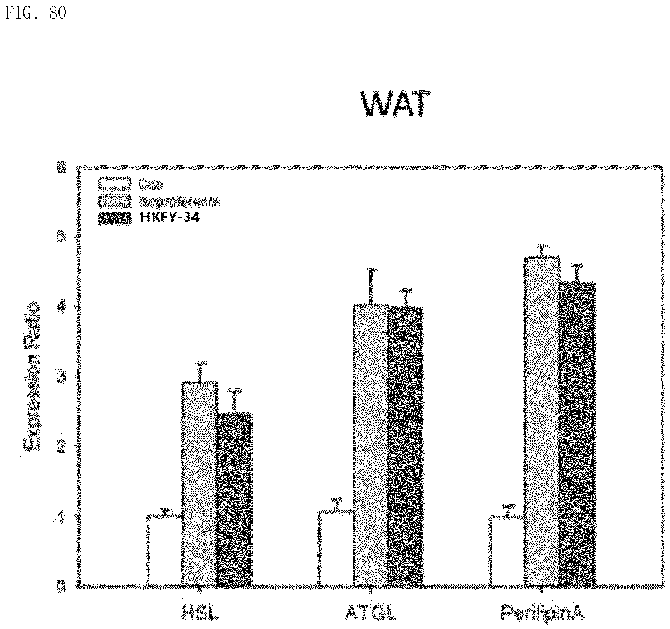

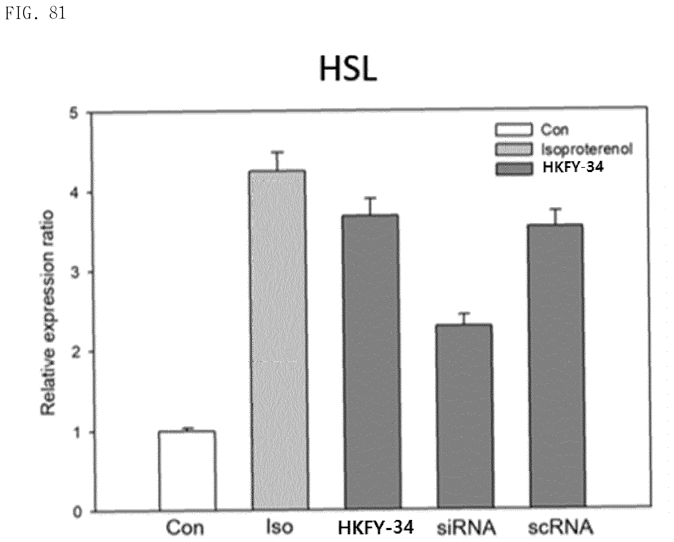

In addition, the mRNA expression levels of HSL (hormone-sensitive lipase), ATGL (adipose triglyceride lipase) and Perilipin A, which are important factors for lipolysis signaling according to HKFY-34 treatment, were analyzed through RT-PCR (FIG. 79). As a result, referring to FIG. 79, when 3T3-L1 cells and WAT cells were treated with HKFY-34, the expression of all factors was increased to a degree similar to that of isoproterenol, a positive control group (FIGs. 79 and 80). In addition, it was confirmed that the mRNA expression of all factors was enhanced due to GPR39 through GPR39 siRNA (FIGs. 81 to 83).

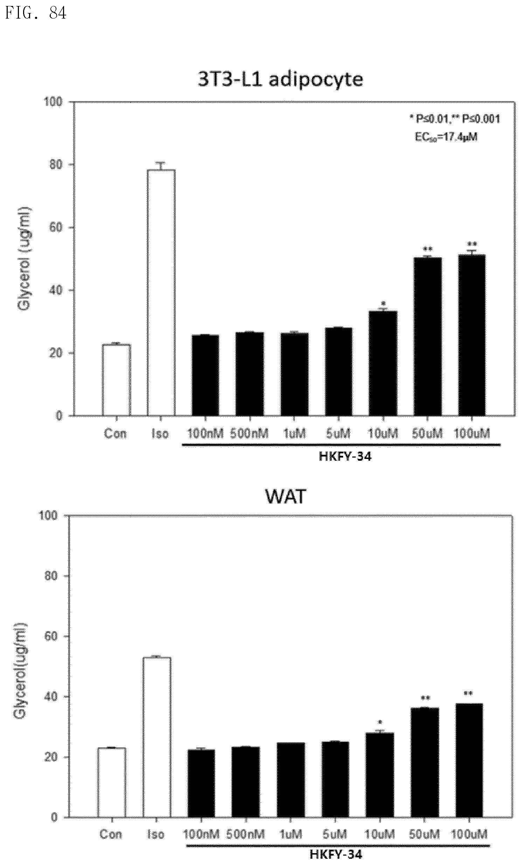

In addition, lipolytic ability according to the HKFY-34 treatment by concentration (100 nM, 500 nM, 1 µM, 10 µM, 50 µM and 100 µM) in 3T3-L1 cells and WAT cells was confirmed (FIG. 84). When the signal of lipolysis is transmitted, lipid droplet glycerol and free fatty acid in adipocytes are secreted as final products and come out of the cell. At this time, the degree of lipolysis can be confirmed by quantifying the secreted glycerol. Isoproterenol, a positive control group, is an agonist of β-adrenergic receptors and mainly promotes lipolytic ability in adipocytes.

As a result, referring to FIG. 84, the lipolytic ability in 3T3-L1 cells and adipose tissue was increased in a concentration dependent manner to HKFY-34. In particular, when the HKFY-34 concentration was 10 µM or higher, the lipolytic ability was significantly increased.

### Experimental Example 6. Analysis of action mechanism of lipolytic ability according to HKFY derivative treatment

In order to analyze the mechanism of lipolytic ability according to the HKFY derivative treatment in 3T3-L1 cells and WAT cells, the expression levels of pERK and pPKA were analyzed (FIGs. 85 and 86).

Specifically, protein was purified from 3T3L-1 cells (Korea Cell Line Bank) and WAT (tissue culture using mice purchased from Central Lab. Animal Inc.) treated with the HKFY derivatives, and then each antibody for pERK, ERK, pPKA, and PKA expressed in mice was used, and the expression level of each protein due to the derivatives was measured through western blot.

As a result, referring to FIGs. 85 and 86, when both 3T3-L1 cells (FIG. 85) and WAT cells (FIG. 86) were treated with HKFY-34, the expression of pERK and pPKA was increased compared to ERK and PKA. These results suggest that it has the same signaling and lipolysis regulatory mechanism as the β-adrenergic receptor, which regulates the major lipolysis of adipocytes.

### Experimental Example 7. Glucose tolerance test according to HKFY derivative treatment

The glucose tolerance level in a mouse model of diabetes mellitus disease according to the HKFY derivative treatment was analyzed (FIG. 87).

Specifically, male db/db mice (5 to 7-week-old, Central Lab. Animal Inc.), which is a mouse model of diabetes mellitus disease, were fasted for 16 hours, and then HKFY-34 was administered subcutaneously at a concentration of 0.5, 1, 5 and 10 mg/kg, respectively. After 30 minutes, glucose (1.5 g/kg) was intraperitoneally administered. After 0, 15, 30, 45, 60, 90, 120 and 180 minutes, blood was collected from the tail of the mice, and the blood glucose was measured with a blood glucose meter (acc-check, Roche, Germany) to analyze the glucose tolerance.

As a result, referring to FIG. 87, the mouse model of diabetes mellitus disease has significant glucose tolerance in a concentration dependent manner to the treated HKFY-34.

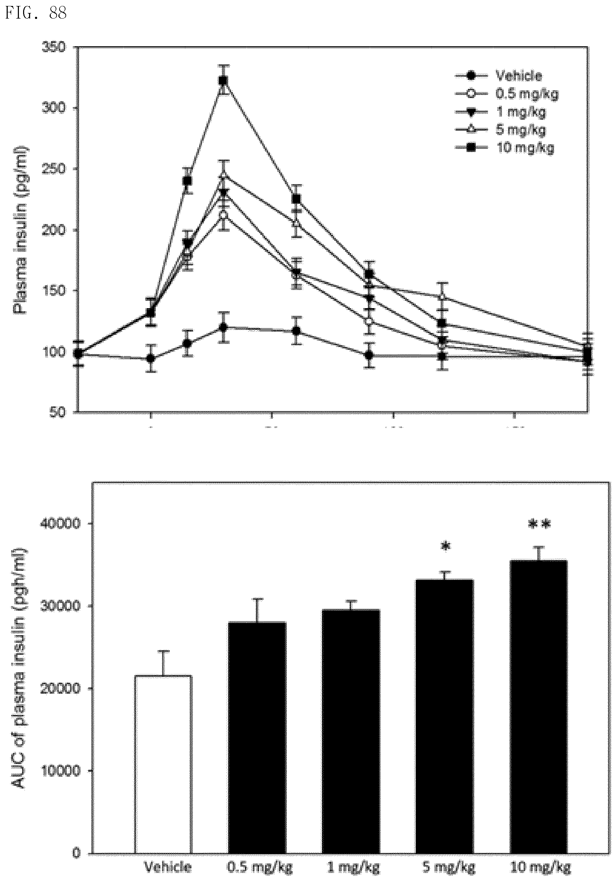

In addition, the blood insulin level was analyzed simultaneously with the glucose tolerance test for the mouse model of diabetes mellitus disease (FIG. 88).

Specifically, after 0, 15, 30, 60, 90, 120 and 180 minutes, blood was collected from the orbital veins of mice, and the insulin level in plasma was analyzed using mouse ELISA.

As a result, referring to FIG. 88, the insulin secretion was increased in a concentration dependent manner to the treated HKFY-34 in a mouse model of diabetes mellitus disease. In particular, when treated with HKFY-34 at a concentration of 5 and 10 mg/kg, the insulin secretion level was significantly increased.

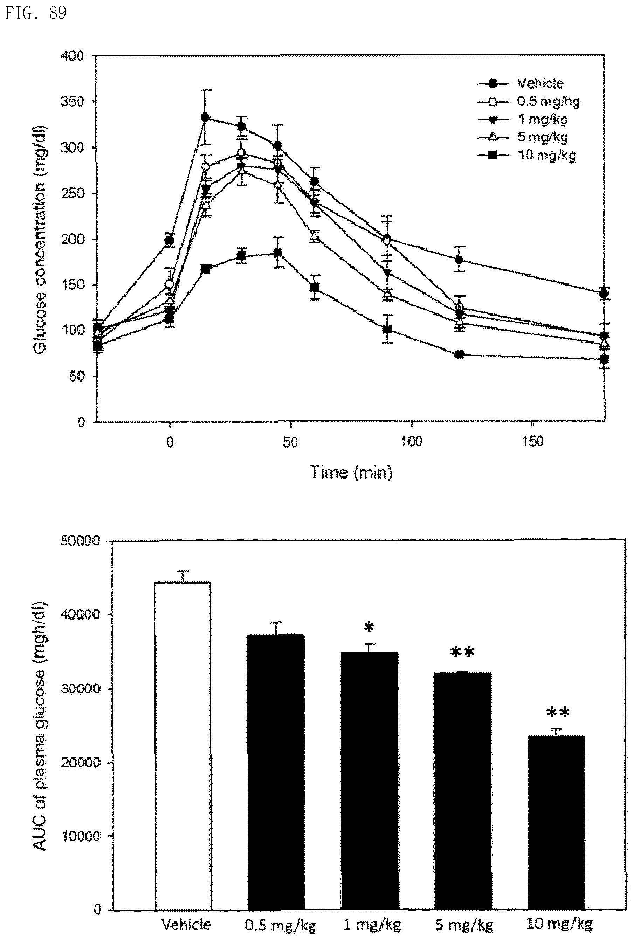

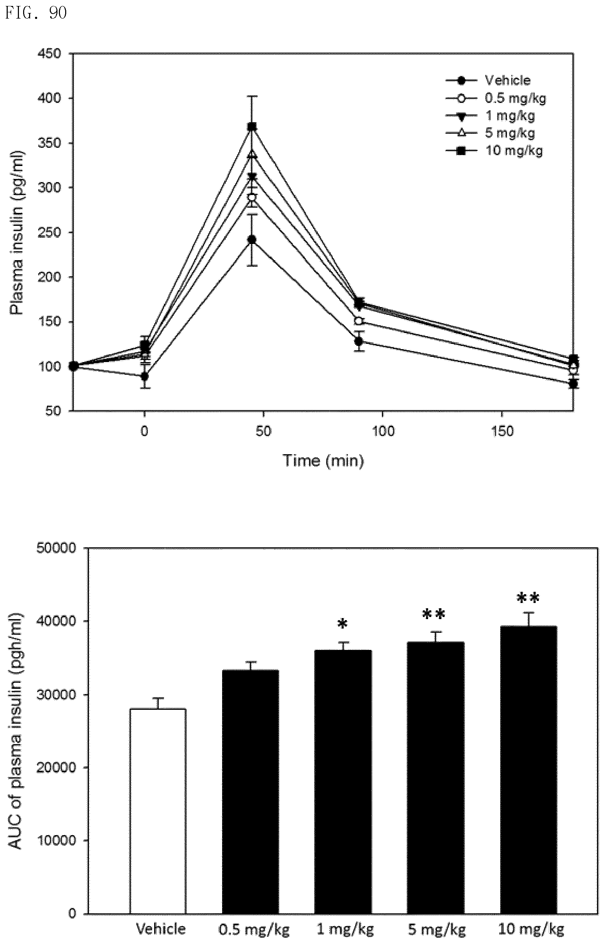

In addition, the glucose tolerance level and insulin level according to HKFY-34 treatment in a mouse model of obesity disease were analyzed (FIGs. 89 and 90).

Specifically, in the glucose tolerance level and insulin level analysis experiment, the experiment was performed by changing the mouse model of diabetes mellitus disease into a mouse model of obesity disease (DIO), in which obesity was induced by providing male C57BL6/J mice (6 to 7-week-old, Central Lab. Animal Inc.) with a high-fat feed (60% fat chow) for 5-6 weeks.

As a result, referring to FIGs. 89 and 90, the mouse model of obesity disease had glucose tolerance in a concentration dependent manner to the treated HKFY-34 (FIG. 89), and the insulin secretion level was increased (FIG. 90).

In addition, the glucose tolerance level in the GPR39 KO mouse model induced by a high-fat diet according to the HKFY derivative treatment was analyzed (FIG. 91).

Specifically, obesity was induced by providing male GPR39 KO mice (6 to 7-week-old, Central Lab. Animal Inc.) with a high-fat feed (60% fat chow) for 5-6 weeks. HKFY-34 was administered subcutaneously at a concentration of 0.5, 1, 5 and 10 mg/kg, respectively, to the 16, 17 or 18-week-old GPR39 KO mice, in which obesity was induced. After 30 minutes, glucose (1.5 g/kg) was intraperitoneally administered. After 0, 15, 30, 45, 60, 90, 120 and 180 minutes, blood was collected from the tail of the mice, and the blood glucose was measured with a blood glucose meter (acc-check, Roche, Germany) to analyze the glucose tolerance.

As a result, referring to FIG. 91, even if the 16, 17 and 18-week-old GPR39 KO mice were treated with HKFY-34, significant glucose tolerance was not observed. These results suggest that HKFY-34 acts specifically on GPR39.

### Experimental Example 8. Confirmation of anti-diabetic effect according to HKFY derivative treatment

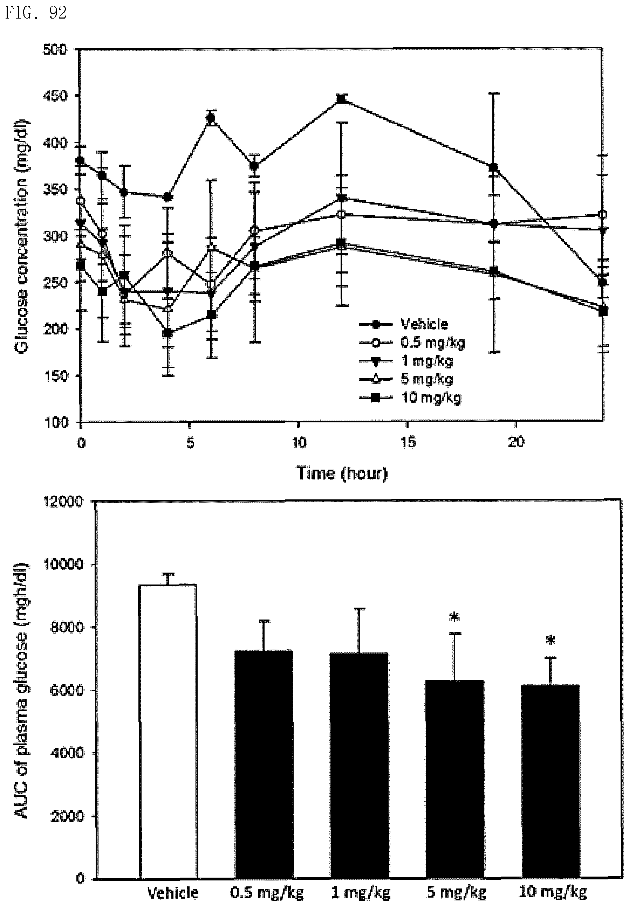

The drop in blood glucose level in a mouse model of diabetes mellitus disease according to the HKFY derivative treatment was analyzed (FIG. 92).

Specifically, HKFY-34 was subcutaneously administered at a concentration of 0.5, 1, 5 and 10 mg/kg, respectively, to male db/db mice (5 to 7-week-old), which is a mouse model of diabetes mellitus disease. After 0, 1, 2, 4, 6, 8, 12, 18 and 24 hours, blood was collected from the tail of the mice, and the blood glucose was measured with a blood glucose meter (acc-check, Roche, Germany) to confirm the anti-diabetic effect.

As a result, referring to FIG. 92, it was confirmed that the effect of drop in blood glucose level was exhibited in a concentration-dependent manner to HKFY-34 treatment in a mouse model of diabetes mellitus disease. In particular, when treated with HKFY-34 at a concentration of 5 and 10 mg/kg, the effect of drop in blood glucose was significantly increased.

## Claims

1. A compound represented by Formula 1 below or a pharmaceutically acceptable salt thereof: 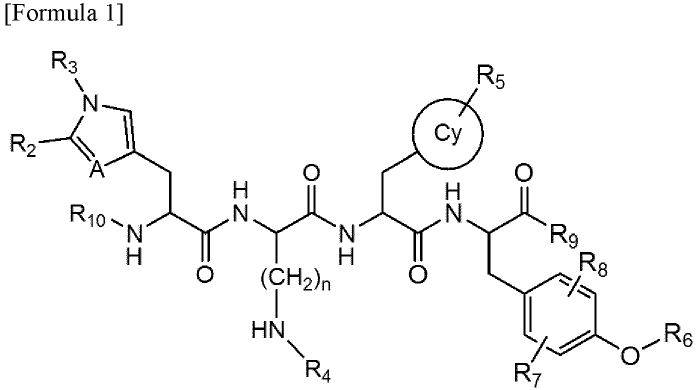 in Formula 1
A is -C(-Ro)-, -N= or -N(-R₁)-,
Cy is C₆₋₁₄ aryl or 5 to 6-membered heteroaryl,
R₁ and R₃ are each independently hydrogen, Ra, an amine protecting group, C₁₋₆ alkyl substituted with 1 to 3 Ra, C₃₋₁₀ cycloalkyl, or 5 to 6-membered heterocyclyl, wherein said heterocyclyl has or does not have 1 to 3 substituents selected from phenylethyl and cyclohexylethyl,
R₂ is hydrogen, Ra, or 5 to 6-membered heterocyclyl,
R₀ is hydrogen, or R₀ and R₂ are linked to each other to form a benzene ring together with the two carbon atoms to which they are attached,
Ra is each independently C₃₋₁₀ cycloalkyl or C₆₋₁₄ aryl, wherein said aryl has or does not have one or more substituents selected from the group consisting of halogen, -OH, C₆₋₁₄ aryl substituted with 5 to 6-membered heteroaryl, C₁₋₆ alkyl and C₁₋₆ alkoxy,
n is 1 to 10,
R₄ is hydrogen, C₁₋₁₀ alkyl or C₁-C₂₀ alkylcarbonyl,
R₅ is hydrogen, halogen, CF₃ or C₁₋₆ alkyl,
R₆ is hydrogen, C₁₋₁₀ alkyl or -S(=O)₂OH,
R₇ and R₈ are each independently hydrogen, halogen, nitro, amine or C₁₋₆ alkyl,
R₉ is -OH or -NH₂, and
R₁₀ is hydrogen, an amine protecting group or biotin,
wherein said heterocyclyl and heteroaryl each independently contain at least one heteroatom selected from the group consisting of N, S and O.

2. The compound or pharmaceutically acceptable salt thereof according to claim 1, wherein R₁ is hydrogen,

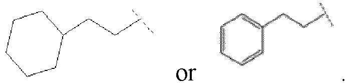

3. The compound or pharmaceutically acceptable salt thereof according to claim 1, wherein
R₂ is hydrogen, 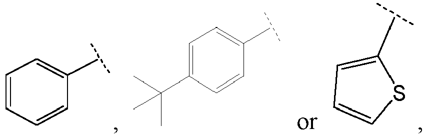 and
R₀ is hydrogen, or R₀ and R₂ are linked to each other to form a benzene ring together with the two carbon atoms to which they are attached.

4. The compound or pharmaceutically acceptable salt thereof according to claim 1, wherein
R₃ is one selected from the group consisting of

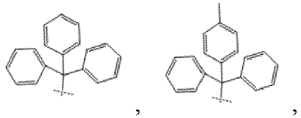

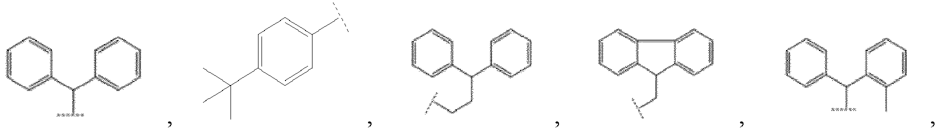

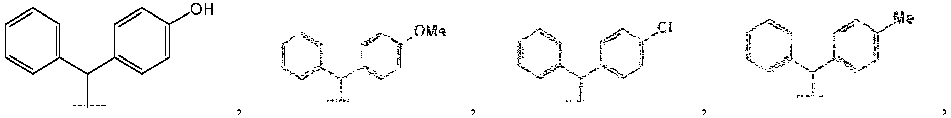

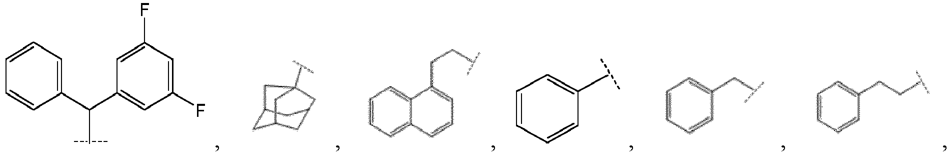

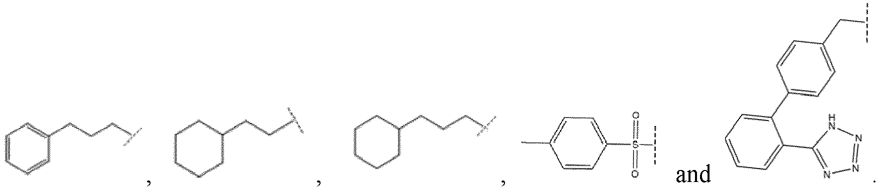

5. The compound or pharmaceutically acceptable salt thereof according to claim 1, wherein A is -C(-Ro)- or -N=.

6. The compound or pharmaceutically acceptable salt thereof according to claim 1, wherein R₃ is 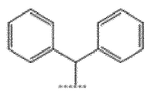 .

7. The compound or pharmaceutically acceptable salt thereof according to claim 1, whereinn is 3 or 4, and R₄ is heptylcarbonyl.

8. The compound or pharmaceutically acceptable salt thereof according to claim 1, wherein R₅ is methyl or CF₃.

9. The compound or pharmaceutically acceptable salt thereof according to claim 1, wherein R₆ is hydrogen or S(=O)₂OH.

10. The compound or pharmaceutically acceptable salt thereof according to claim 1, wherein R₇ and R₈ are each independently hydrogen or amine.

11. The compound or pharmaceutically acceptable salt thereof according to claim 1, wherein R₉ is -OH.

12. The compound or pharmaceutically acceptable salt thereof according to claim 1, wherein R₁₀ is hydrogen, p-toluenesulfonyl (Ts), t-butoxycarbonyl (Boc) or biotin.

13. The compound or pharmaceutically acceptable salt thereof according to claim 1, wherein said Cy is pyridyl, naphthyl or phenyl.

14. The compound or pharmaceutically acceptable salt thereof according to claim 1, wherein the compound is any one selected from the group consisting of:
1) His(trt)-Lys(caprylic)-Phe-Tyr,
2) Biotin-His(trt)-Lys(caprylic)-Phe-Tyr,
3) His(benzyl)-Lys(caprylic)-Phe-Tyr,
4) d-His(trt)-Lys(caprylic)-Phe-Tyr,
5) His(4-methyltrityl)-Lys(caprylic)-Phe-Tyr,
6) His(DAMP-3)-Lys(caprylic)-Phe-Tyr,
7) His(DAMP-5)-Lys(caprylic)-Phe-Tyr,
8) His(DAMP-2)-Lys(caprylic)-Phe-Tyr,
9) His(2-phenylethyl)-Lys(caprylic)-Phe-Tyr,
10) His(2-naphthalen-1-methyl)-Lys(caprylic)-Phe-Tyr,
11) His(2-cyclohexylethyl)-Lys(caprylic)-Phe-Tyr,
12) His(trt)-Lys(caprylic)-d-Phe-Tyr,
13) His(trt)-Lys(caprylic)-d-Phe(4-Cl)-Tyr,
14) d-His(trt)-Lys(caprylic)-d-Phe(4-Cl)-d-Tyr,
15) His(trt)-Lys(caprylic)-d-Phe(4-Cl)-d-Tyr,
16) His(trt)-d-Lys(caprylic)-d-Phe(4-Cl)-Tyr,
17) His(3,3-diphenylethyl)-Lys(caprylic)-Phe-Tyr,
18) His(Fm)-Lys(caprylic)-Phe-Tyr,
19) His(phenethyl)-Lys(caprylic)-Phe-Tyr,
20) His(4-methoxylbenzhydryl)-Lys(caprylic)-Phe-Tyr,
21) His(4-chlorobenzhydryl)-Lys(caprylic)-Phe-Tyr,
22) His(4-methylbenzhydryl)-Lys(caprylic)-Phe-Tyr,
23) His(adamantan-1-yl)-Lys(caprylic)-Phe-Tyr,
24) His(trt)-Lys(capric)-Phe-Tyr,
25) His(trt)-Lys(lauric)-Phe-Tyr,
26) His(trt)-Lys(myristic)-Phe-Tyr,
27) His(trt)-Lys(palmitic)-Phe-Tyr,
28) His(trt)-Lys(caprylic)-d-Phe(F)-Tyr,
29) His(trt)-Lys(caprylic)-d-Phe(Br)-Tyr,
30) His(trt)-Lys(caprylic)-d-Phe(4-methyl)-Tyr,
31) d-His(trt)-Lys(caprylic)-d-Phe(4-methyl)-Tyr,
32) His(1,3-difluorobenzhydryl)-Lys(caprylic)-d-Phe(4-methyl)-Tyr,
33) His(benzhydryl)-Lys(caprylic)-Phe-Tyr,
34) His(benzhydryl)-Lys(caprylic)-d-Phe(4-methyl)-Tyr,
35) His(benzhydryl)-Lys(caprylic)-d-Phe(4-methyl)-Tyr(SO₃H),
36) His(benzhydryl)-Lys(caprylic)-d-Phe(4-methyl)-Tyr(2,6-di-methyl),
57) His(2-phenyl)-Lys(caprylic)-d-Phe(4-methyl)-Tyr,
58) His(1-phenyl)-Lys(caprylic)-d-Phe(4-methyl)-Tyr,
59) His(1,2-diphenyl)-Lys(caprylic)-d-Phe(4-methyl)-Tyr,
60) His(2-(4-tert-butyl)phenyl)-Lys(caprylic)-d-Phe(4-methyl)-Tyr,
61) His(1-(4-tert-butyl)phenyl)-Lys(caprylic)-d-Phe(4-methyl)-Tyr,
62) d-His(benzhydryl)-Lys(caprylic)-d-Phe(4-methyl)-Tyr,
63) His(thiophene)-Lys(caprylic)-d-Phe(4-methyl)-Tyr,
64) His(4-methoxybenzhydryl)-Lys(caprylic)-d-Phe(4-methyl)-Tyr,
65) His(4-hydroxybenzhydryl)-Lys(caprylic)-d-Phe(4-methyl)-Tyr,
66) His(benzhydryl)-Lys(caprylic)-d-Phe(4-methyl)-Tyr-NH₂,
67) His(benzhydryl)-Lys(caprylic)-d-Phe(4-methyl)-Tyr(3-chloro),
68) His(benzhydryl)-Lys(caprylic)-d-Phe(4-methyl)-Tyr(3-nitro),
69) His(benzhydryl)-Lys(caprylic)-d-Phe(4-methyl)-Tyr(3,5-nitro),
70) His(benzhydryl)-Lys(caprylic)-d-Phe(4-methyl)-Tyr(3-amino),
71) His(benzhydryl)-Orn(caprylic)-d-Phe(4-methyl)-Tyr,
72) Biotin-His(benzhydryl)-Lys(caprylic)-d-Phe(4-methyl)-Tyr,
73) His(trt)-Lys(caprylic)-d-3Pal-Tyr,
74) His(trt)-Lys(caprylic)-d-2Nal-Tyr,
75) His(tosyl)-Lys(caprylic)-d-Phe(4-methyl)-Tyr,
76) His(trt)-Lys(caprylic)-d-Phe(4-CF3)-Tyr,
77) His(tbm)-Lys(caprylic)-d-Phe(4-CF3)-Tyr, and
78) Boc-Trp-Lys(caprylic)-d-Phe(4-methyl)-Tyr.

15. The compound or pharmaceutically acceptable salt thereof according to claim 1, wherein two amino acids or derivatives thereof are further bound to the N-terminus of the compound represented by Formula 1.

16. The compound or pharmaceutically acceptable salt thereof according to claim 15, wherein the compound is (X₁)-(X₂)-[His(trt)-Lys(caprylic)-Phe-Tyr],
wherein X₁ and X₂ are each independently any one amino acid selected from 20 amino acids or derivatives thereof.

17. The compound or pharmaceutically acceptable salt thereof according to claim 16, wherein the compound is 37) Gly-Ser-His(trt)-Lys(caprylic)-Phe-Tyr.

18. The compound or pharmaceutically acceptable salt thereof according to claim 1, wherein 1 to 10 amino acids or derivatives thereof are further bound to the C-terminus of the compound represented by Formula 1.

19. The compound or pharmaceutically acceptable salt thereof according to claim 18, wherein
the compound is [His(trt)-Lys(caprylic)-Phe-Tyr]-(X₃)ₐ-(X₄)_{b}-(X₅)_{c}-(X₆)_{d}-(X₇)ₑ-(X₈)_{f}-(X₉)_{g},
wherein X₃ to X₉ are each independently any one amino acid selected from 20 amino acids or derivatives thereof, and
a to g are 0 or 1, and at least one of them is 1.

20. The compound or pharmaceutically acceptable salt thereof according to claim 19, wherein the compound is any one selected from the group consisting of:
38) His(trt)-Lys(caprylic)-Phe-Tyr-Ser-Asp-Gln-Gln-Ala-Arg-Phe,
39) His(trt)-Lys(caprylic)-Phe-Tyr-Ser-Gln-Asn-Gly-Ala-Arg-Phe,
40) His(trt)-Lys(caprylic)-Phe-Tyr-Ser-His-Gln-Gln-Ala-Arg-Phe,
41) His(trt)-Lys(caprylic)-Phe-Tyr-Gln-Asn-Gly-Ala-Arg-Phe,
42) His(trt)-Lys(caprylic)-Phe-Tyr-His-Gln-Gln-Ala-Arg-Phe,
43) His(trt)-Lys(caprylic)-Phe-Tyr-Gln-Asn-Gln-Ala-Arg-Phe, and
44) His(trt)-Lys(caprylic)-Phe-Tyr-Trp.

21. A compound or a pharmaceutically acceptable salt thereof, wherein the compound consists of [His(trt)-Lys(caprylic)]-(X₁₀)ₕ-(X₁₁)ᵢ,
wherein X₁₀ to X₁₁ are each independently any one amino acid selected from 20 amino acids or derivatives thereof, and
h and i are 0 or 1, and at least one of them is 1.

22. The compound or pharmaceutically acceptable salt thereof according to claim 21, wherein the compound is any one selected from the group consisting of:
45) Biotin-His(trt)-Lys(caprylic)-Ala(biphenyl)-Tyr,
46) His(trt)-Lys(caprylic)-Phe-Trp,
47) His(trt)-Lys(caprylic)-Tyr-Phe,
48) His(trt)-Lys(caprylic)-Tyr-Tyr,
49) His(trt)-Lys(caprylic)-Phe,
50) His(trt)-Lys(caprylic)-Phe-Ser, and
51) His(trt)-Lys(caprylic)-Leu-Tyr.

23. A compound or a pharmaceutically acceptable salt thereof, wherein the compound is any one selected from the group consisting of:
52) His(trt)-Phe-Tyr-Asp-Gln-Gln-Ala-Arg-Phe,
53) His(trt)-Gly-Ser-Lys(caprylic)-Phe-Tyr,
54) Lys(caprylic)-Phe-Tyr,
55) Lys(caprylic)-His(trt)-Phe-Tyr
56) His(trt)-Lys(caproic)-Phe-Tyr-Asp-Gln-Gln-Ala-Arg-Phe, and
79) His(benzhydryl)Lys(caprylic).

24. A pharmaceutical composition for preventing or treating diabetes mellitus, comprising the compound or pharmaceutically acceptable salt thereof according to any one of claims 1 to 23 as an active ingredient.

25. A health functional food composition for preventing or improving diabetes mellitus, comprising the compound or nutritionally acceptable salt thereof according to any one of claims 1 to 23 as an active ingredient.

26. A pharmaceutical composition for preventing or treating obesity, comprising the compound or pharmaceutically acceptable salt thereof according to any one of claims 1 to 23 as an active ingredient.

27. A health functional food composition for preventing or improving obesity, comprising the compound or nutritionally acceptable salt thereof according to any one of claims 1 to 23 as an active ingredient.

28. A cosmetic composition for preventing or improving obesity, comprising the compound or pharmaceutically acceptable salt thereof according to any one of claims 1 to 23 as an active ingredient.

29. A method for preventing or treating diabetes mellitus, comprising administering the compound or pharmaceutically acceptable salt thereof according to any one of claims 1 to 23 to a mammal.

30. A method for preventing or treating obesity, comprising administering the compound or pharmaceutically acceptable salt thereof according to any one of claims 1 to 23 to a mammal.

31. Use of the compound or pharmaceutically acceptable salt thereof according to any one of claims 1 to 23, for the manufacture of a medicament for preventing or treating diabetes mellitus.

32. Use of the compound or pharmaceutically acceptable salt thereof according to any one of claims 1 to 23, for the manufacture of a medicament for preventing or treating obesity.
